# EUROPEAN PATENT APPLICATION

(11) **EP 3 950 962 A2**
(43) Date of publication of application: **09.02.2022**
(21) Application number: 20782477.2
(22) Date of filing: 03.04.2020
(51) Int. Cl.: C12Q 1/6886, C12Q 1/6806, C12Q 1/6813, C12Q 1/6816

(54) **METHOD FOR DIAGNOSING CANCER USING CFDNA**

(30) Priority: 05.04.2019 KR 20190039974; 29.11.2019 KR 20190157368
(71) Applicant: Genopsy, Inc., Seoul 07573 (KR)
(72) Inventor: CHO, Youngnam, Seoul 07573 (KR)
(74) Representative: Dehns
(86) International application number: PCT/KR2020/004602
(87) International publication number: WO 2020/204674

(57) **Abstract**

The diagnostic method of the present invention relates to a technique in which small-sized cfDNA is concentrated and isolated from a liquid sample such as urine, cerebrospinal fluid, plasma, blood, pleural fluid, or body fluid, and then a biomarker overexpressed in a specific cancer is detected with ultra-high sensitivity without PCR. The detection method according to an example of the present invention does not require a PCR amplification reaction and thus can greatly reduce time taken to diagnose cancer. In addition, since it can be directly analyzed in the field, it can be used as a point-of-care testing (POCT) that can simultaneously search a large number of genes within a short time.

## Description

### [Technical Field]

The present invention relates to a method for diagnosing cancer using cell-free DNA having a double helix structure, and more specifically, the present invention relates to a method for detecting a biomarker gene that is specifically expressed or overexpressed in cancer without amplification and a device for using the same.

### [Background Art]

Recently, the importance of early diagnosis of cancer diseases has been greatly emphasized worldwide. Therefore, research on methods for early diagnosis of cancer is increasing. However, to date, methods for diagnosis of cancer are performed by invasive methods such as collection of a tissue sample and endoscopic examination. In particular, the biopsy is performed by extracting a part of a suspected disease area and observing it under a microscope. Therefore, in order to use a needle, a punch, an endoscope, or a laparoscope to collect a tissue sample, the human body needs to be incised, and thus, the discomfort that the patient feels is not small, and scar remains and it takes a long time to recover.

As an alternative to invasive diagnosis and test methods, molecular diagnostic methods using liquid biopsy have attracted attention. Since the liquid biopsy uses a non-invasive method, the test results can be quickly confirmed. In addition, unlike a tissue sample that could analyze only a portion of the disease, the liquid biopsy can perform analysis on the disease from various angles. In particular, the liquid biopsy is expected to exert excellent utility in the diagnosis of cancer. In particular, it is expected that detailed observations of cancer occurrence and metastasis and the like will be possible by analyzing DNA derived from cancer cells present in the blood of each body part only by examining body fluid such as blood and urine.

The molecular diagnostic method is a representative technique of in vitro diagnostics, and is a diagnosis technique by detecting a change in DNA or RNA from a sample containing genetic information such as blood and urine through numerical value or imaging. Since this has the advantages of high accuracy and no need for biopsy, attempts have been made to apply it to a cancer diagnosis technique based on the advantages of the cost reduction along with rapid development of a genome analysis technique.

Meanwhile, cell-free DNA (hereinafter referred to as cfDNA) means DNA derived from cells present in plasma. The cfDNA usually has a double helix structure, as well as often has a coiled coil structure. The cfDNA may be derived from tumor cells. In addition, cfDNA derived from tumor cells may be found in body fluids such as blood, plasma, or urine obtained from cancer patients.

The cfDNA found in cancer patients is often derived from cell necrosis, cell death or normal cells and/or cancer cells of the urinary system. Such cfDNA is released into urine, blood, and the like through various processes. Therefore, with the development of techniques for isolating and detecting cfDNA in biological samples such as blood, plasma or urine, it is prodicted that the liquid biopsy may be a more effective and reliable tool for monitoring patients at risk for cancer. In particular, since urine, cerebrospinal fluid(CSF), plasma, pleural fluid, ascites, blood, or body fluid is an easily obtainable sample, it is possible to collect a large amount of samples by a simple and non-invasive method through repeated sampling.

However , the method for early diagnosis of cancer by analyzing cfDNA in a liquid sample such as blood or urine and detecting a mutation present in genes has many difficulties due to the current level of technology. Therefore, there is a need for not only the development of a method for easily detecting cfDNA, but also a technique for enhancing detection sensitivity and accurately diagnosing cancer early.

Meanwhile, Korean Patent No. 10-1751962 discloses that a polymerase chain reaction is performed using a primer to detect cfDNA, and cfDNA can be quantified using a probe that can complementarily bind to cfDNA. However, there are still a problem that separate polymerase and experimental equipments and materials are required for performing the polymerase chain reaction, and there is a problem that diagnosis in the field is not easy.

In addition, Korean Patent No. 10-1701618 discloses a nanostructure in which the properties of the surface can be changed by changing the electric field in order to effectively separate cfDNA. The nanostructure can bind or dissociate cfDNA through electrical changes, thereby easily separating cfDNA from a sample. However, there is still a limitation to use the polymerase chain reaction in order to confirm which cfDNA is present.

In order to amplify cfDNA by performing the polymerase chain reaction, not only several kinds of primer sets are required, but also the performance of complicated steps is required, which takes a lot of time. Therefore, research has been continuously conducted to overcome the limitations of having to perform PCR and to develop a method for analyzing cfDNA with high accuracy. In addition, because cfDNA is found at an extremely low level in a liquid sample, research has been continuously conducted to develop a method for obtaining and analyzing DNA with high accuracy and a less amount of sample and an efficient analytical method.

### [Disclosure]

### [Technical Problem]

Conventionally, in order to detect cfDNA, it is necessary to denature double-stranded cfDNA into single-stranded DNA so that a primer complementary to cfDNA can be bound. Therefore, in order to detect cfDNA, a process of applying heat is necessary, and a process of reacting with a polymerase or the like is necessary.

However, the present inventors have found that there are many DNA sections in which double-stranded DNA is unwound into a single strand during the transcription process because the DNA transcription process is actively occurring in cancer cells, and a probe is capable of binding to the cfDNA released from these cancer cells even without denaturation process, and the present invention was completed based on these findings.

Therefore, one aspect of the present invention provides a method for detecting a biomarker that is specifically expressed or overexpressed in cancer cells present in a liquid sample such as plasma or urine using a probe having a sequence complementary to cfDNA without PCR or amplification process of nucleic acid.

According to another aspect, provided herein is a method of detecting a mutation (for example, SNP) of a cancer cell biomarker without PCR or amplification process of nucleic acid.

### [Technical Solution]

In order to achieve the above object, one aspect of the present invention provides a method for diagnosing cancer by detecting a gene derived from cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the cfDNA is derived from cancer cells, and the probe having a sequence complementary to cfDNA complementarily binds to a gene known as a cancer biomarker, and the method is a method for diagnosing cancer or predicting the prognosis of cancer.

### [Advantageous Effects]

The method for diagnosing cancer of the present invention relates to a technique in which small-sized cfDNA is isolated from a liquid sample such as urine, cerebrospinal fluid, plasma, blood, pleural fluid, or body fluid, and then a biomarker specifically expressed or overexpressed in cancer is detected with ultra-high sensitivity without PCR. The detection method according to an example of the present invention does not require a PCR amplification reaction and thus can greatly reduce time taken to diagnose cancer. In addition, since it can be directly analyzed in the field, it can be used as a pointof-care testing (POCT) that can simultaneously search a large number of genes within a short time. In particular, according to the method of the present invention, since it is possible to distinguish cfDNA present in the blood of a normal person from cfDNA present in the blood of a cancer patient, various cancers can be effectively detected.

### [Description of Drawings]

Figure 1A shows a scanning electron microscope (SEM) image of the positively charged nanowires (PEI/Ppy NWs).
Figure 1B shows a scanning electron microscope image of the nanoparticles to which HRP/streptavidin is bound.

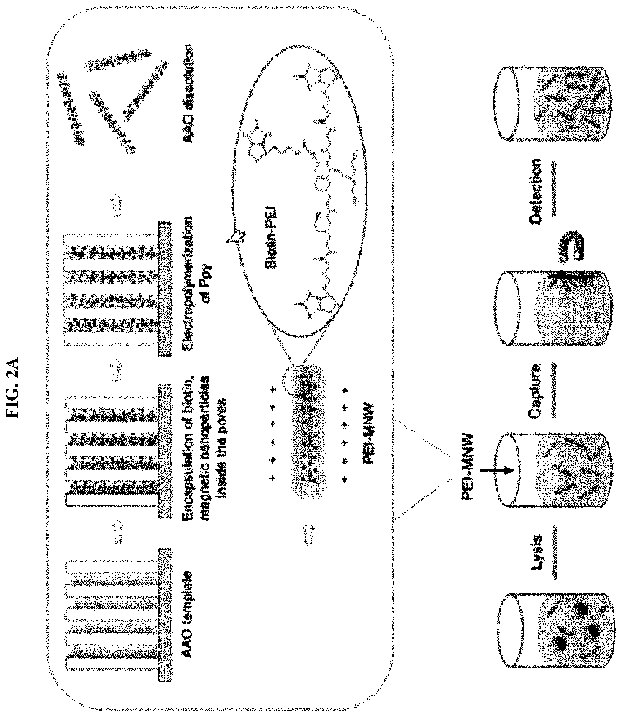

Figure 2A shows a conceptual schematic diagram of the preparation of the nanostructures (PEI/mPpy NWs) with polyethyleneimine (PEI), a cationic polymer, bound to the surface of the nanostructures, and a method for detecting and recovering cfDNA using the same.

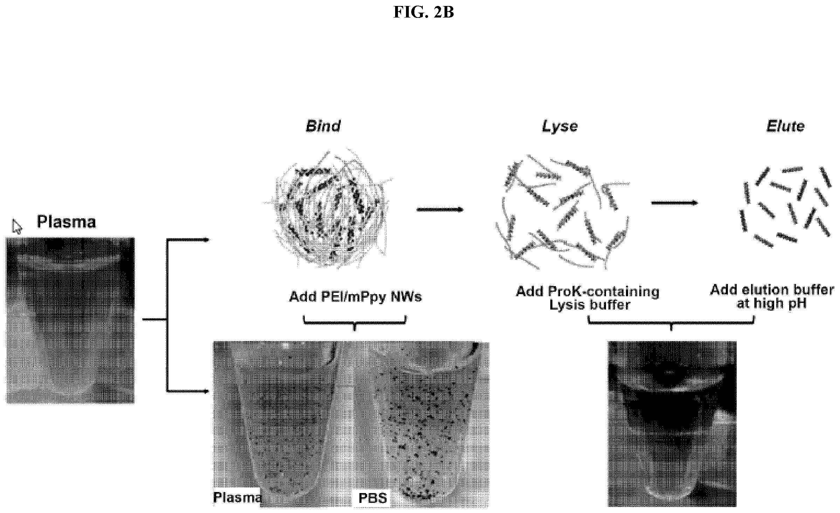

Figure 2B is a figure showing a photograph of a process of detecting and recovering cfDNA using the magnetic nanostructures (PEI/mPpy NWs) with polyethyleneimine (PEI), a cationic polymer, bound to the surface of the nanostructures for each process.
Figure 3 is a schematic view showing a process of recovering cfDNA using an Eppendorf tube.
Figure 4 and Figure 5 are figures showing a measured level of PD-L1 DNA expression and PD-L1 mRNA expression from cfDNA of a PD-L1 positive cancer cell line or a PD-L1 negative cancer cell line.
Figure 6 and Figure 7 are figures showing a measured level of EpCAM DNA expression and EpCAM mRNA expression from cfDNA of an EpCAM positive cancer cell line or an EpCAM negative cancer cell line.
Figure 8 and Figure 9 are figures showing a measured level of FOLR1 DNA expression and FOLR1 mRNA expression from cfDNA of an FOLR1 positive cancer cell line or an FOLR1 negative cancer cell line.
Figure 10 and Figure 11 are figures showing a measured level of EGFR DNA expression and EGFR mRNA expression from cfDNA of an EGFR positive cancer cell line or an EGFR negative cancer cell line.
Figure 12 and Figure 13 are figures showing a measured level of ERBB2 DNA expression and ERBB2 mRNA expression from cfDNA of an ERBB2 positive cancer cell line or an ERBB2 negative cancer cell line.
Figure 14 and Figure 15 are figures showing a measured level of OGT DNA expression from cfDNA of an OGT positive cancer cell line or an OGT negative cancer cell line.
Figures 16 to 18 are figures showing a measured level of CEA DNA expression from cfDNA of a CEA positive cancer cell line or a CEA negative cancer cell line.
Figure 19 and Figure 20 are figures showing a measured level of PSA DNA expression from cfDNA of a PSA positive cancer cell line or a PSA negative cancer cell line.
Figure 21 and Figure 22 are figures showing a measured level of CA19-9 DNA expression from cfDNA of a CA19-9 positive cancer cell line or a CA19-9 negative cancer cell line.
Figure 23 and Figure 24 are figures showing a measured level of CA125 DNA expression from cfDNA of a CA125 positive cancer cell line or a CA125 negative cancer cell line.
Figure 25 and Figure 26 are figures showing a measured level of AFP DNA expression from cfDNA of an AFP positive cancer cell line or an AFP negative cancer cell line.
Figures 27 to 29 are figures showing a measured level of DNA expression of PSA, PSMA, PAP, and PAC3 using the plasma obtained from prostate cancer patients.
Figures 30 to 32 are figures showing a measured level of DNA expression of PSA, PSMA, PAP, and PAC3 using the plasma obtained from normal persons.
Figure 33 and Figure 34 are figures showing a measured level of DNA expression of NSE, SCC, CEA, Cyfra21-1, and TPA using the plasma obtained from lung cancer patients.
Figure 35 is a figure showing a measured level of DNA expression of NSE, SCC, CEA, Cyfra21-1, and TPA using the plasma obtained from normal persons.
Figures 36 to 38 are figures showing a measured level of DNA expression of CEA, NSE, TG, and CALCA using the plasma obtained from thyroid cancer patients.
Figure 39 and Figure 40 are figures showing a measured level of DNA expression of CEA, NSE, TG, and CALCA using the plasma obtained from normal persons.
Figure 41 and Figure 42 are figures showing a measured level of DNA expression of OGT, FGFR3, TP53, NMP22, and Cyfra21-1 using the urine obtained from bladder cancer patients.
Figure 43 and Figure 44 are figures showing a measured level of DNA expression of OGT, FGFR3, TP53, NMP22, and Cyfra21-1 using the urine obtained from cystitis patients.
Figure 45 and Figure 46 are figures showing a measured level of DNA expression of OGT, FGFR3, TP53, NMP22, and Cyfra21-1 using the urine obtained from normal persons.
Figure 47 and Figure 48 are figures showing a measured level of DNA expression of CA27-29, CA15-3, and CEA using the plasma obtained from breast cancer patients.
Figure 49 and Figure 50 are figures showing a measured level of DNA expression of CA27-29, CA15-3, and CEA using the plasma obtained from normal persons.
Figure 51 and Figure 52 are figures showing a measured level of DNA expression of CEA and CA19-9 using the plasma obtained from colorectal cancer patients.
Figures 53 to 55 are figures showing a measured level of DNA expression of CEA and CA19-9 using the plasma obtained from normal persons.
Figure 56 is a figure showing a measured level of DNA expression of CA19-9, CA125, and CEA using the plasma obtained from biliary tract cancer patients.
Figure 57 and Figure 58 are figures showing a measured level of DNA expression of CA19-9, CA125, and CEA using the plasma obtained from normal persons.
Figure 59 is a figure showing a measured level of DNA expression of CEA, CA19-9, CGB, and Cyfra21-1 using the plasma obtained from gastric cancer patients.
Figure 60 and Figure 61 are figures showing a measured level of DNA expression of CEA, CA19-9, CGB, and Cyfra21-1 using the plasma obtained from normal persons.
Figures 62 to 65 are figures showing a measured level of DNA expression of CA19-9, CA125, and CEA using the plasma obtained from pancreatic cancer patients.
Figures 66 to 69 are figures showing a measured level of DNA expression of CA19-9, CA125, and CEA using the plasma obtained from normal persons.
Figure 70 is a figure showing a measured level of DNA expression of CPT1A using the plasma obtained from lung cancer patients.
Figure 71 is a figure showing a measured level of DNA expression of CPT1A using the plasma obtained from normal persons.
Figure 72 is a figure showing a measured level of DNA expression of CPT1A using the urine obtained from bladder cancer patients.
Figure 73 is a figure showing a measured level of DNA expression of CPT1A using the urine obtained from normal persons.
Figure 74 is a figure showing a measured level of DNA expression of PD-L1 from cfDNA of a PD-L1 positive cancer cell line or a PD-L1 negative cancer cell line that is not treated with IFN-γ, and a result of measuring whether PD-L1 is detected by the method of one embodiment of the present invention
Figure 75 is a figure showing a measured level of DNA expression of IFNG (IFN-γ) from cfDNA of a PD-L1 positive cancer cell line or a PD-L1 negative cancer cell line that is not treated with IFN-γ.
Figure 76 is a figure showing a measured level of DNA expression of IFNR1 (IFN-γ receptor) from cfDNA of a PD-L1 positive cancer cell line or a PD-L1 negative cancer cell line that is not treated with IFN-γ.
Figures 77 to 79 are figures showing a measured level of DNA expression of PD-L1, IFNG, and IFNR1 from cfDNA of a PD-L1 positive cancer cell line or a PD-L1 negative cancer cell line that is treated with IFN-γ.
Figure 80 is a figure showing a measured level of DNA expression of PD-L1 from cfDNA of a PD-L1 positive cancer cell line or a PD-L1 negative cancer cell line depending on the presence or absence of IFN-γ treatment.
Figure 81 is a graph showing a measured level of DNA expression of IFN-γ from cfDNA of a PD-L1 positive cancer cell line or a PD-L1 negative cancer cell line depending on the presence or absence of IFN-γ treatment.
Figure 82 is a figure showing a measured level of DNA expression of IFNR1 from cfDNA of a PD-L1 positive cancer cell line or a PD-L1 negative cancer cell line depending on the presence or absence of IFN-γ treatment.
Figure 83 is a figure showing a measured level of DNA expression of PD-L1 from cfDNA of a PD-L1 positive cancer cell line or a PD-L1 negative cancer cell line depending on the presence or absence of IFN-γ treatment.
Figure 84 is a schematic view showing the detection step of the present invention.
Figure 84A shows a schematic diagram of the method for analyzing the gene mutation within about 60 minutes through the reaction with a probe and HRP/streptavidin-nanoparticle (HRP/st-tagged NP) after obtaining cfDNA from the patient's body fluid, using the nanowires (PEI/Ppy NWs) with polyethyleneimine (PEI) bound to the surface of the nanowires.
Figure 84B is a schematic view showing a method for detecting unstable cfDNA using the nanowires, the probe, and the HRP/streptavidin nanoparticles.
Figure 84C is a figure showing a process of detecting the gene mutation through a spin column using the nanowires that do not comprise magnetic nanoparticles. In one embodiment of the present invention, a step of treating with a lysis buffer may be further included.
Figure 84D shows a time series flow diagram of the method for detecting unstable cfDNA in a sample such as blood, cerebrospinal fluid, or pleural fluid.
Figure 84E shows a time series flow diagram of the method for detecting unstable cfDNA in a sample such as urine.
Figure 84F is a schematic view showing the difference in denaturation conditions according to the state of cfDNA obtained from the blood.
Figure 84G is a schematic view showing the difference in denaturation conditions according to the state of cfDNA obtained from the urine, saliva, and sputum.
Figure 85 is a figure showing the isolation of cfDNA through a spin column using the nanowires that do not comprise magnetic nanoparticles. The upper photograph is a SEM image of the spin column before centrifugation, and the lower photograph is a SEM image of the spin column in which cfDNA is isolated after centrifugation.
Figure 86 is a figure showing a measured level of DNA expression of a cancer-related biomarker such as AKL Fusion and PIK3CA from the blood obtained from lung cancer patients using an injection needle.
Figure 87 is a figure showing a measured level of DNA expression of a cancer-related biomarker such as AKL Fusion and PIK3CA from the blood obtained from lung cancer patients using a lancet.
Figure 88 is a figure showing a measured level of DNA expression of a cancer-related biomarker such as AKL Fusion from the blood obtained from normal persons using an injection needle.
Figure 89 is a figure showing a measured level of DNA expression of a cancer-related biomarker such as AKL Fusion from the blood obtained from normal persons using a lancet.
Figure 90 is a figure of confirming a level of EML4-ALK expression from cfDNA of EML4-ALK variant 3a/b positive cell (H2228) and EML4-ALK negative cell (A549, H1993, PC9, RT4) cancer cell lines through RT-PCR.
Figure 91 is a figure of confirming a level of EML4-ALK expression from cfDNA of EML4-ALK variant 3a/b positive cell (H2228) and EML4-ALK negative cell (A549, H1993, PC9, RT4) cancer cell lines through Western blot.
Figure 92 is a graph showing a result of confirming a level of EML4-ALK expression from cfDNA of EML4-ALK variant 3a/b positive cell (H2228) and EML4-ALK negative cell (A549, H1993, PC9, RT4) cancer cell lines through RT-PCR and Western blot.
Figure 93 is a figure showing a measured level of DNA expression of EML4-ALK fusion var. 1 or EML4-ALK fusion var.3 from cfDNA of EML4-ALK variant 3a/b positive cell (H2228) and EML4-ALK negative cell (A549, H1993, PC9, RT4) cancer cell lines.
Figure 94 is a graph showing a measured level of DNA expression of EML4-ALK fusion var. 1 or EML4-ALK fusion var.3 from cfDNA of EML4-ALK variant 3a/b positive cell (H2228) and EML4-ALK negative cell (A549, H1993, PC9, RT4) cancer cell lines.
Figure 95 is a figure showing a measured level of DNA expression of a cancer-related biomarker such as EML4-ALK fusion var.3, KRAS, SYP, NCAM1, and NKX2-1 from the blood obtained from small cell lung cancer patients. As a result, EML4-ALK fusion was found to be the same in ctDNA in cancer tissue and blood, and as a result of ctDNA, EML4-ALK fusion was found to be var.3, not var.1, indicating that Crizotinib, an ALK TKI, was not well responsive.
Figure 96 is a figure showing a measured level of DNA expression of a cancer-related biomarker such as EML4-ALK fusion var.1 from the blood obtained from cancer patients. As a result, EML4-ALK fusion was found to be the same in ctDNA in cancer tissue and blood, and as a result of ctDNA, EML4-ALK fusion was found to be var.1, not var.3, indicating that Crizotinib, an ALK TKI, was well responsive and patient's response of partial response (PR) was obtained.
Figure 97 is a figure showing a measured level of DNA expression of a cancer-related biomarker such as EML4-ALK fusion var.3 from the blood obtained from cancer patients. As a result, EML4-ALK fusion was found to be the same in ctDNA in cancer tissue and blood, and as a result of ctDNA, EML4-ALK fusion was found to be var.3, not var.1, indicating that Crizotinib, an ALK TKI, will be not well responsive, and thus alectinib is prescribed from the beginning to await patient's response.
Figure 98 is a figure showing a measured level of DNA expression of a cancer-related biomarker such as EML4-ALK fusion var.3, BRAFV800E, and TP53 from the blood obtained from cancer patients. As a result, EML4-ALK fusion was found to be the same in ctDNA in cancer tissue and blood, and as a result of ctDNA, EML4-ALK fusion was found to be var.3, not var.1, indicating that Crizotinib, an ALK TKI, was not well responsive (PD).
Figure 99 is a figure showing a measured level of DNA expression of a cancer-related biomarker such as EML4-ALK fusion var.1 from the blood obtained from cancer patients. As a result, EML4-ALK fusion was found to be the same in ctDNA in cancer tissue and blood, and as a result of ctDNA, EML4-ALK fusion was found to be var.1, not var.3, indicating that Crizotinib, an ALK TKI, was well responsive and patient's response of partial response (PR) was obtained.
Figure 100 is a figure showing a measured level of DNA expression of a cancer-related biomarker such as EML4-ALK fusion var.1 from the blood obtained from cancer patients. As a result, EML4-ALK fusion was found to be the same in ctDNA in cancer tissue and blood, and as a result of ctDNA, EML4-ALK fusion was found to be var.1, not var.3, indicating that an ALK TKI was well responsive and patient's response of partial response (PR) was obtained.
Figure 101 is a figure of confirming a level of protein expression of OGT from cfDNA of each cancer cell line *in vitro* through Western blot.
Figure 102 is a figure of confirming a level of mRNA expression of OGT from cfDNA of each cancer cell line *in vitro* through RT-PCR.
Figure 103 is a graph showing a result of confirming a level of mRNA expression of OGT from cfDNA of each cancer cell line *in vitro* through RT-PCR.
Figure 104 is a figure showing a measured level of DNA expression of OGT from cfDNA of each cancer cell line *in vitro.*
Figure 105 is a graph showing a result of measuring a level of DNA expression of OGT from cfDNA of each cell line *in vitro.*
Figure 106 is a figure showing a result of confirming a level of expression of OGT from each cell line *in vitro* through Western blot, RT-PCR, and cfDNA detection.
Figure 107 is a photograph of cfDNA of OGT detected in the nanowires that do not comprise magnetic nanoparticles from each cell line *in vitro.*
Figure 108 is a graph of quantifying cfDNA obtained from the urine of normal persons, cystitis patients, and bladder cancer patients.
Figure 109 is a graph of analyzing a level of DNA expression of OGT from cfDNA obtained from the urine of normal persons, cystitis patients, and bladder cancer patients.
Figure 110 is a graph of analyzing a level of DNA expression of OGT from cfDNA obtained from the urine of normal persons, cystitis patients, and bladder cancer patients.
Figures 111 to 113 are figures of analyzing a level of DNA expression of OGT from cfDNA obtained from the urine of several cancer patients as a blind test.
Figures 114 to 116 are figures of analyzing a level of DNA expression of BRAF V600E and TERT C250T from cfDNA obtained from the tissue of thyroid cancer patients.
Figures 117 to 121 are figures of measuring a level of DNA expression of a cancer-related biomarker such as SYP, CgA, NCAM1, and NKX2-1 using the blood obtained from small cell lung cancer patients. In particular, in Korea Cancer Center Hospital, the patient of Figure 120 was confirmed to have small cell lung cancer (SCLC), and crizotinib was prescribed, but it was confirmed to have no effect.
Figure 122 is a figure of measuring a level of DNA expression of SYP, CgA, NCAM1, and NKX2-1 using the blood obtained from non-small cell lung cancer patients.
Figure 123 is a figure showing a level of DNA expression of CEA using the blood obtained from lung cancer patients before and after anti-cancer treatment, and the prognosis of patients.
Figures 124 to 128 are figures of measuring a level of DNA expression of a cancer-related biomarker such as NSE and CEA using the blood obtained from lung cancer patients.
Figures 129 to 133 are figures of measuring a level of DNA expression of a cancer-related biomarker such as NSE and CEA using the blood obtained from normal persons.
Figure 134 is a figure of measuring a level of DNA expression of a cancer-related biomarker such as PSA, PSMA, PAP, and PCA3 using the blood obtained from prostate cancer patients.
Figure 135 is a figure of measuring a level of DNA expression of a cancer-related biomarker such as PSA, PSMA, PAP, and PCA3 using the blood obtained from normal persons.
Figure 136 is a figure of measuring a level of DNA expression of TMPRSS2-ERG fusion using the blood obtained from prostate cancer patients and normal persons.
Figure 137 is a figure of measuring a level of DNA expression of CEA, NSE, TG (thyroglobulin), and CALCA using the blood obtained from thyroid cancer patients.
Figure 138 is a figure of measuring a level of DNA expression of CEA, NSE, TG (thyroglobulin), and CALCA using the blood obtained from normal persons.
Figure 139 is a figure of measuring a level of DNA expression of BRAF mutation (V600E), TERT Promotor mutation (C228T, C250T) using the blood obtained from thyroid cancer patients and normal persons.
Figure 140 is a figure of measuring a level of DNA expression of OGT, FGFR3, TP53, NMP22, and Cyfra21-1 using the urine obtained from bladder cancer patients, hematuria patients, and normal persons.
Figure 141 is a figure of measuring a level of DNA expression of CA27-29 and CEA using the blood obtained from breast cancer patients and normal persons.
Figure 142 is a figure of measuring a level of DNA expression of CEA and CA19-9 using the blood obtained from colorectal cancer patients and normal persons.
Figure 143 and Figure 144 are figures of measuring a level of DNA expression of CA19-9, CEA, and CA123 using the blood obtained from biliary tract cancer patients and normal persons.
Figure 145 is a figure of measuring a level of DNA expression of CEA, CA19-9, CGB, and Cyfra21-1 using the blood obtained from gastric cancer patients and normal persons.
Figure 146 is a figure of measuring a level of DNA expression of CA125 and CEA using the blood obtained from ovarian cancer patients and normal persons.
Figure 147 is a figure of measuring a level of DNA expression of CEA, CA19-9, and CA125 using the blood obtained from pancreatic cancer patients.
Figure 148 is a figure of measuring a level of DNA expression of CEA, CA19-9, and CA125 using the blood obtained from normal persons.
Figure 149 and Figure 150 are results of early diagnosis by measuring a level of DNA expression of a cancer-related biomarker using the blood obtained from normal persons (PC: positive control, PC is a tool for confirming whether an experiment of early cancer diagnosis was accurately performed and is irrelevant to a result of early cancer diagnosis).
Figure 151 and Figure 152 are figures of organizing biomarkers according to cancer types used in one example of the present invention.
Figure 153 shows a result of confirming through the absorbance the presence or absence of the binding of cfDNA present in the urine of HPV positive cervical cancer patients (HPV16 (+) and HPV18 (+)) and a HPV negative healthy control (HPV-) with a probe specific to HPV 18 or HPV 16.
Figure 154 shows a result of confirming the presence or absence of the binding of cfDNA with each probe after sequentially reacting cfDNA isolated from the urine of cervical cancer patients with probes specific to HPV 16, EGFR19 deletion, HPV 18, and EGFR 21 L858R.
Figure 155 is a table of analyzing gene mutations of lung cancer patients using cfDNA obtained from the plasma of 151 lung cancer patients.
Figure 156 shows a result of confirming gene mutations of lung cancer patients by obtaining cfDNA from the plasma of patients without an EGFR mutation (wild type), patients with EGFR exon19 deletion, and lung cancer patients with EGFR exon 21 L858R, mixing with a probe specific to EGFR exon19 Del, and then analyzing the absorbance (ΔOD, 500 nm to 650 nm) values of UV spectrum.
Figure 157 shows a result of obtaining cfDNA from the plasma of lung cancer patients with EGFR exon19 deletion, mixing with a probe specific to EGFR exon19 Del, and then analyzing the specificity and sensitivity of gene mutations.
Figure 158 shows a result of confirming gene mutations of patients by obtaining cfDNA from the plasma of patients without EGFR mutation (wild type), patients with EGFR exon19 deletion, and lung cancer patients with EGFR exon 21 L858R, adding a probe specific to EGFR exon21 L858R, and then analyzing the absorbance (ΔOD, 500 nm to 650 nm) values of UV spectrum
Figure 159 shows a result of obtaining cfDNA from the plasma of lung cancer patients with EGFR exon 21 L858R, adding a probe specific to EGFR exon 21 L858R, and then analyzing the specificity and sensitivity of gene mutations of patients.
Figure 160 shows the sequences of CP and DP of EGFR exon 19 deletion. In this study, CP_1 and DP were used to analyze the cfDNA gene mutation of lung cancer patients. In this case, CP is a probe that is designed to complementarily bind to a sequence that contains or is adjacent to a mutated portion, and DP means a probe that is designed to complementarily bind to a portion spaced apart a mutated sequence.
Figure 161 shows the sequences of CP and DP of EGFR exon 20 T790M. In this study, CP2 and DP were used to analyze the cfDNA gene mutation of lung cancer patients.
Figure 162 shows the sequences of CP and DP of EGFR exon 21 L858R. In this study, CP2 and DP were used to analyze the cfDNA gene mutation of lung cancer patients.
Figure 163 shows a result of confirming whether cfDNA was detected by the color change and UV absorbance when reacting cfDNA obtained from the plasma of lung cancer patients with EGFR exon 19 deletion and EGFR exon 20 T790M gene mutations and a probe specific to EGFR exon 19 deletion (De119), EGFR exon 20 T790M, and EGFR exon 21 L858R, and then adding HRP/streptavidin nanoparticles (including a large amount of HRP).
Figure 164 shows a result of confirming whether cfDNA was detected by the color change and UV absorbance when reacting cfDNA obtained from the plasma of lung cancer patients with EGFR exon 19 deletion and EGFR exon 20 T790M gene mutations that are the same as in Figure 163 and a probe specific to EGFR exon 19 deletion (De119), EGFR exon 20 T790M, and EGFR exon 21 L858R, and then adding an HRP/streptavidin complex (a complex of HRP and streptavidin bound 1:1). It was confirmed that noise was generated in the case of the HRP/streptavidin complex compared to the HRP/streptavidin nanoparticles.
Figure 165 shows a result of confirming and comparing the consistency of the genotype with cancer tissue through the analysis of the result of extracting cfDNA from the plasma of 5 lung cancer patients with EGFR exon19 deletion and exon20 T790M gene mutations, and then reacting with a probe specific to EGFR exon 19 Del, EGFR exon 20 T790M, EGFR exon 21 L858R and HRP/streptavidin nanoparticles (HRP/st-tagged NPs) and the result of reacting with a probe specific to EGFR exon19 Del, EGFR exon 20 T790M, EGFR exon 21 L858R and an HRP/streptavidin complex (HRP and streptavidin bound 1:1).
Figure 166 shows a result of confirming by the UV absorbance that the gene mutation was observed only in EGFR exon 20 T790M and EGFR exon 21 L861Q in the same manner as a cancer tissue, when mixing a probe specific to EGFR exon 19 deletion (19 Del), EGFR exon 20 T790M, EGFR exon 21 L858R, and EGFR exon L861Q and HRP/st-tagged NP all at once in order to detect the gene mutation of cfDNA obtained from the plasma of lung cancer patients with EGFR exon 20 T790M and EGFR exon 21 L861Q gene mutations.
Figure 167 shows a result of confirming that the ALK-EML4 fusion and ALK point mutation (I1171N/T) genotypes were detected in the same manner as a cancer tissue, when mixing a probe specific to ALK-EML4 fusion and ALK point mutation (T1151, L1152P, L1152R, C1156Y, 11171N/T) and HRP/st-tagged NP all at once in order to detect the gene mutation of cfDNA obtained from the plasma of lung cancer patients with ALK-EML4 fusion and ALK point mutation (I1171N/T) gene mutations.
Figure 168 shows a result of confirming that the BRAF V600E gene mutation was detected in the same manner as the genotype of patients, when mixing a probe specific to BRAF V600E and HRP/st-tagged NP all at once in order to detect the gene mutation of cfDNA obtained from plasma of thyroid cancer patients with BRAF V600E gene mutation.
Figure 169 shows a result of detecting unstable cfDNA according to treatment conditions after denaturing samples collected from the blood of normal persons under various temperature conditions.
Figure 170 shows a result of detecting unstable cfDNA according to treatment conditions after denaturing samples collected from the blood of patients under various temperature conditions.
Figure 171 shows a result of detecting unstable cfDNA according to treatment conditions after denaturing fDNA obtained from the mutation cell line under various temperature conditions.
Figure 172 shows a result of detecting unstable cfDNA according to treatment conditions after treating fDNA obtained from the mutation cell line with DNase at 37 °C for 30 minutes.
Figure 173 shows a result of detecting unstable cfDNA according to treatment conditions after treating fDNA obtained from the mutation cell line with DNase at 37 °C for 60 minutes.
Figure 174 shows a result of detecting unstable cfDNA according to treatment conditions after treating fDNA obtained from the mutation cell line with DNase at 37 °C for 120 minutes.
Figure 175 shows a result of treating with 1 µL or 2 µL of DNase at 24 °C for 120 minutes in order to confirm the difference between unstable cfDNA and stable cfDNA according to the activity of DNase.
Figure 176 shows a result of treating with 1 µL or 2 µL of DNase at 3 °C for 120 minutes in order to confirm the difference between unstable cfDNA and stable cfDNA according to the activity of DNase.

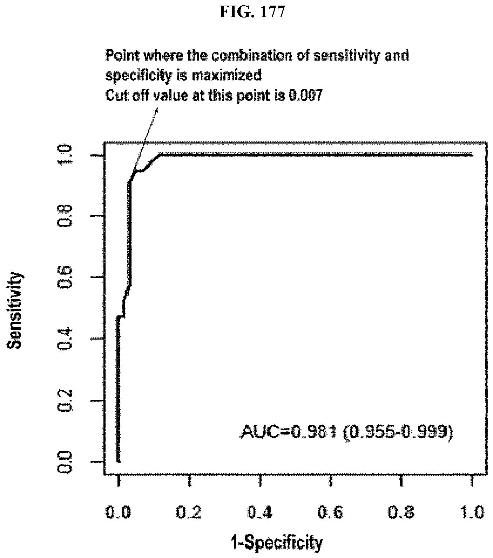

Figure 177 shows an embodiment of the cutoff value when detecting the EML4-ALK fusion gene using cfDNA in the plasma of lung cancer patients as an example of the present invention.

### [Best Mode]

### <Definition of Term>

As used herein, the term "cell-free DNA" is also referred to as cfDNA. In addition, cfDNA may be circulating tumor DNA (ctDNA), which is a cancer cell-derived DNA that may be found in a biological sample such as urine, cerebrospinal fluid, plasma, blood, or body fluid derived from cancer patients due to tumor cells. In addition, cfDNA may be present in a biological sample such as urine, cerebrospinal fluid, pleural fluid, ascites, plasma, blood, saliva, sputum, or body fluid. In this case, cfDNA may have a size of 80 bp to 10 kbp, 100 bp to 1 kbp, 120 bp to 500 bp. In addition, cfDNA may have a size of 150 bp to 200 bp, and may usually have a size of 165 bp to 170 bp. In addition, the cfDNA may include a small sized cfDNA of about 80 bp or less.

As used herein, the term "unstable cfDNA" means cfDNA that is thermodynamically unstable compared to "stable cfDNA." In other words, unstable cfDNA may be denatured in less severe conditions than conditions in which stable cfDNA is denatured. The reason why the unstable cfDNA is generated is that the unstable cfDNA has an unstable double helix structure. Specifically, cfDNA derived from a gene that is overexpressed in cancer cells may be an example of unstable cfDNA.

As used herein, the term "cfDNA having an unstable double helix structure" is characterized in that it has a lower Tm value than that of cfDNA having a stable double helix structure, or that it is denatured in a condition in which cfDNA having a stable double helix structure is not denatured. The Tm refers to a melting temperature and means a temperature at which 50% of double-stranded DNA is converted into single-stranded DNA. The Tm value is proportional to the length of DNA and may vary depending on the nucleotide sequence. However, since in genomic DNA a large number of nucleotides are bound by hydrogen bonds, it should be heated at 92 °C to 95 °C for 5 minutes or more, or at 98 °C for 2 minutes or more. In addition, at temperatures lower than 90 °C, genomic DNA is not easily denatured. In this case, assuming that cfDNA having a stable double helix structure has an average nucleotide of about170 bp, it may have a Tm value similar to that of genomic DNA.

However, the "cfDNA having an unstable double helix structure" has a lower Tm value than that of cfDNA having a stable double helix structure. Therefore, when the cfDNA having a stable double helix structure is denatured in any one condition selected from the group consisting of i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 60 minutes; v) a condition of heating at about 25 °C to about 40 °C for about 10 minutes to about 120 minutes; vi) a condition of treating with protease for about 10 seconds to about 30 minutes; vii) a condition of treating with DNase for about 10 seconds to about 30 minutes, and viii) a condition of treating with a chemical substance (for example, sodium hydroxide, DMSO, a surfactant, and the like), and then subjected to a binding reaction with a 15-mer to 30-mer probe having a sequence complementary to a portion of the sequence of cfDNA, the cfDNA having a stable double helix structure does not bind with the above probe. In this case, "ambient temperature" means room temperature and may be about 18 °C to about 25 °C. Further, in addition to the above conditions, a condition of heating at about 40 °C to about 65 °C for about 5 minutes to about 80 minutes may be further included.

However, when treating the cfDNA having an unstable double helix structure in any one condition of the above described conditions i) to viii), and then performing a binding reaction with a 15-mer to 30-mer probe, it was confirmed that the cfDNA bound with the above probe. In this case, the above probe may be a about 15-mer to about 30-mer or about 20-mer to about 25-mer probe and may be a about 21-mer, about 22-mer, about 23-mer, or about 24-mer probe.

In this case, the cfDNA having an unstable double helix structure may be a circulating tumer DNA(ctDNA).

As used herein, the term "probe" means DNA or RNA for detecting a target cfDNA. The probe may have a sequence designed to be capable of complementarily binding to unstable cfDNA. As used herein, the term "probe having a sequence complementary to cfDNA" means a probe having a nucleic acid sequence capable of complementarily binding to cfDNA of the desired double helix structure that is desired to be detected and is present in fluid sample like plasma.

In this case, the probe may be constructed in two ways. One may be a first probe (hereinafter referred to as CP) designed to be capable of binding to a damaged region of a gene, and the other may be a second probe (hereinafter referred to as DP) designed to be capable of binding to a periphery of a damaged region. The DP may be designed to complementarily bind to a sequence located about 10 bp to about 100 bp, about 20 bp to about 50 bp away from a targeted DNA sequence or a region where damage occurred.

As used herein, the term "complementarily binding" means that the probe can bind to the target cfDNA and form a duplex under appropriate hybridization conditions, and the probe has a sequence that is at least about 75%, at least about 80%, at least about 85%, at least about 90%, at least about 95%, or about 100% complementary to the target sequence of cfDNA.

"Hybridization conditions" can be determined empirically by those skilled in the art based on, for example, the length of the probe, the complementarity of the probe, and the salt concentration (i.e., ionic strength) in the hybridization buffer. In general, stringent hybridization conditions are those in which a polynucleotide can preferentially bind to its complementary sequence, and also can bind with a higher affinity relative to any other region on the target. Exemplary stringent conditions for hybridization to its complement of a polynucleotide sequence having 20 bases may be about 50% G+C content, 50 mM salt (Na+), and annealing temperature of 60 °C. In the case of a longer sequence, hybridization may be carried out at higher temperature. In general, stringent conditions are the conditions in which annealing is carried out at about 5 °C below the melting temperature of the polynucleotide. The "melting temperature" is a temperature at which 50% of the polynucleotide complementary to the target polynucleotide complementarily binds at a given ion strength, pH, and polynucleotide concentration.

In the present specification, it was confirmed that damaged cfDNA can be effectively detected even when the first probe and the second probe are used at the same time or the first probe or the second probe is used respectively. In addition, the probe may be in a form in which a material such as biotin is bound in order to bind with a marker. Alternatively, the probe may be directly bound to a marker or bound through a linker. In this case, the marker may be a nanoparticle, a fluorescent dye, a fluorescent protein, or an enzyme. In addition, the probe may be added simultaneously with the marker, and may be added sequentially.

In one embodiment of the present invention, a probe capable of complementarily binding to a target cfDNA may complementarily bind to a region comprising a sequence specific to the following cancer cells. For example, in the case of the sequence specific to ovarian cancer or breast cancer, it may be a SNP present in BRCA1 exon 7, BRCA1 exon 10, BRCA1 exon 11, BRCA1 exon 15. In addition, in the case of the sequence specific to gastric cancer, it may be a SNP present in TP53, and in the case of the sequence specific to colorectal cancer, it may be a SNP present in MSH2. In the case of the sequence specific to lung cancer, it may be a SNP present in EGFR. In addition, in the case of the sequence specific to liver cancer, it may be selected from a SNP present in FGFR3.

Biomarker genes derived from cancer cells and specific to cancer cells are known to those skilled in the art. For example, the following documents can be referred: Circulating Cell-Free DNA in Plasma/Serum of Lung Cancer Patients as a Potential Screening and Prognostic Tool, Pathak et al, Clinical Chemistry October 2006 vol. 52 no. 10 1833-1842; Cell-free Tumor DNA in Blood Plasma As a Marker for Circulating Tumor Cells in Prostate Cancer, Schwarzenbach et al, Clin Cancer Res Feb. 1, 2009 15; 1032; Cell-free DNA: measurement in various carcinomas and establishment of normal reference range, Wua et al, Clinica Chimica Acta, Volume 321, Issues 1-2, July 2002, Pages 77-87; Detection of Circulating Tumour DNA in the Blood (Plasma/Serum) of Cancer Patients, Anker et al, Cancer and Metastasis Reviews 1999, Volume 18, Issue 1, pp 65-73; Cell-free nucleic acids as biomarkers in cancer patients, Schwarzenbach et al, Nature Reviews Cancer 11, 426-437 (June 2011); Circulating Tumor-Specific DNA: A Marker for Monitoring Efficacy of Adjuvant Therapy in Cancer Patients, Fiegl et al, Cancer Res Feb. 15, 2005 65; 1141.

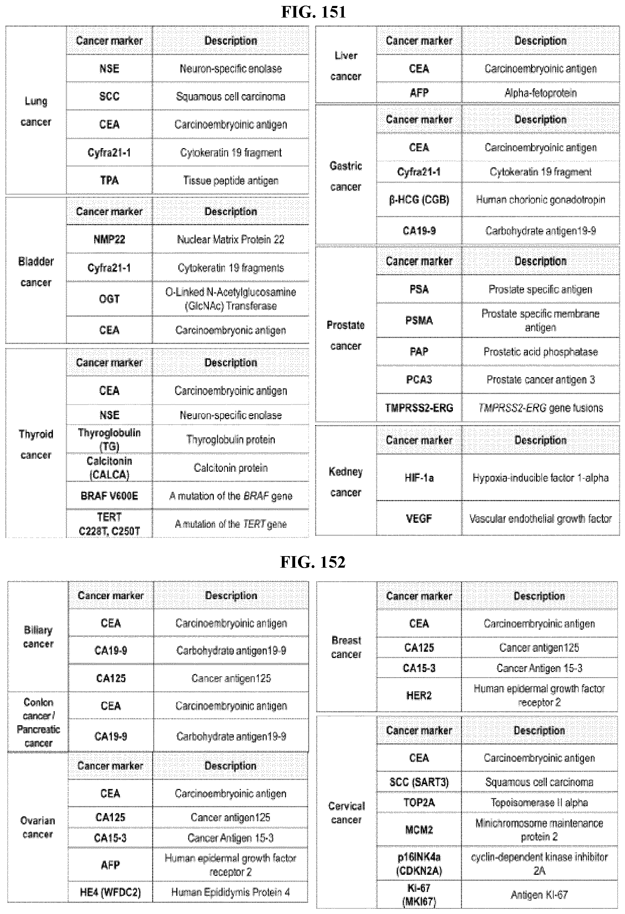

In one embodiment of the present invention, a probe capable of complementarily binding to a target cfDNA can complementarily bind to a region overexpressed in the following cancer cells. As such, the region overexpressed in cancer cells may be a biomarker of cancer cells. The biomarkers of cancer cells may be genes shown in Figure 151 and Figure 152, but are not limited thereto. In addition, throughout the present specification, various biomarker genes for specific tumor/cancer cells and exemplary probes that complementarily bind to the biomarker genes are described by the examples. In addition, the probe may further comprise biotin or an avidin-based protein. Specifically, the marker may further include any one selected from the group consisting of avidin, streptavidin, or a combination thereof. Preferably, the probe may be in the form to which biotin is bound.

As used herein, the term "separated biological sample" means a sample of urine, saliva, cerebrospinal fluid, pleural fluid, ascites, plasma, blood, sputum, or body fluid separated from the human body. The separated biological sample may be a liquid sample separated from the human body. In this case, plasma may be obtained from the blood.

As used herein, the term "positively charged material" is a positively charged material, and may be used in the form of a nanoparticle, a nanowire, a network, or a filter, but is not limited thereto. One embodiment of the "positively charged material" may be a nanowire or a membrane having a positively charged surface. The nanowire or membrane may be manufactured using a conductive polymer. The conductive polymer may be any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline. Although the length and diameter may be appropriately adjusted depending on the manufacturing method, for nanowires, the diameter can be selected from a range of about 50 nm to about 500 nm, about 100 nm to about 500 nm, about 100 nm to about 400 nm, about 150 nm to about 350 nm, about 200 nm to about 400 nm, or about 100 nm to about 300 nm, and the length can be selected from a range of several µm to about 100 µm, about 10 µm to about 100 µm, about 15 µm to about 50 µm, about 15 µm to about 40 µm, or about 15 µm to about 30 µm.

In one embodiment, it may be a nanowire having a diameter of about 200 nm and a length of about 18 µm. In addition, the nanowire may be manufactured in a form of including biotin.

The surface of the nanowire or membrane may be modified by a cationic polymer. The kind of the cationic polymer is not limited. One embodiment of the cationic polymer may be polyethyleneimine (PEI) or polylysine (PLL). In addition, it may be cationic branched polymer polyethyleneimine. The nanowires or membranes modified by such cationic polymer may have a positive charge on their surfaces. To capture cfDNA, the surface charge of the nanowire or membrane may be from about 20 mV to about 80 mV, from about 30 mV to about 60 mV, and from about 35 mV to about 50 mV. Further, the surface charge may be about 36 mV, about 37 mV, about 38 mV, about 39 mV, about 40 mV, about 41 mV, about 42 mV, about 43 mV, or about 44 mV.

In one embodiment, the positively charged nanowires can successfully capture cfDNA efficiently even at a low concentration. In particular, due to the characteristics of the nanowires such as the large surface area for binding with target molecules such as DNA and the enhanced mobility for facilitating interaction with DNA, cfDNA can be effectively captured.

As used herein, the term "marker" is a material for effectively detecting and/or quantifying cfDNA having a double helix structure derived from cancer cells, and specifically, may be a quantum dot, a material that degrades a specific substrate to cause a color reaction, a material that emits luminescence when irradiated with a certain wavelength, and the like. Specifically, the marker may be a fluorescent protein such as GFP (Green Fluorescent Protein), YFP (Yellow Fluorescent Protein), RFP (Red Fluorescent Protein), or CFP (Cyan Fluorescent Protein). In addition, the marker may be a chromogenic enzyme or bioluminescent enzyme, such as alkaline phosphatase (AP), Horsesadish peroxidase (HRP), or beta-galactosidase (BGAL).

The chromogenic enzyme mediates a chromogenic reaction or a luminescent reaction by reacting with a substrate. For HRP, ABTS, OPD, AmplexRed, DAB, AEC, TMB, Homovanillic acid, Luminol and the like can be used as such a substrate. For AP, BCIP (5-Bromo-4-Chloro-3-Indolyl Phosphate)/NBT (nitriblue tetrazolium), pNPP (p-Nitrophenyl Phosphate), Fast Red TR/Naphthol AS-MX and CDP-Star (Disodium 2-chloro -5-(4-methoxyspiro[1,2-dioxetane-3,2'-(5-chlorotricyclo[3.3.1.1^{3.7}]decan])-4-yl]-1-phenyl phosphate) and the like can be used. For BGAL, any substrate selected from the group consisting of X-gal (5-bromo-4-chloro-3-indolyl-β-d-galactopyranoside) or ONPG (ortho-Nitrophenyl-β-galactoside) can be used together. The bioluminescent enzyme may be a luciferase derived from firefly *(Photinus pyralis*), sea pansy *(Renilla sp.),* copepod *Metridiia longa,* bacteria of the genus *Vibrio*, or dinoflagellate.

The marker may further include a material capable of binding to the probe. Specifically, when biotin is bound to the probe, the marker may further include an avidine-based protein. Specifically, the marker may further include any one selected from the group consisting of avidin, streptavidin, or a combination thereof.

In addition, the marker may include biotin. In this case, the probe may further include an avidin-based protein. Specifically, the marker may further include any one selected from the group consisting of avidin, streptavidin, or a combination thereof.

In one embodiment of such a marker, it may be used in the form of nanoparticles in which streptavidin and HRP are bound to nanoparticles composed of a conductive polymer and hyaluronic acid. In this case, the conductive polymer is as described above, and preferably may be poly(pyrrole). In another embodiment, it may be used in the form of nanoparticles in which streptavidin and a fluorescent protein are bound to the nanoparticles composed of a conductive polymer and hyaluronic acid. The size of the HRP nanoparticles may be about 20 nm to about 150 nm, and may be about 30 nm to about 120 nm, about 40 nm to about 100 nm. In addition, The size of the HRP nanoparticles may be about 50 nm, about 60 nm, about 65 nm, about 70 nm, about 75 nm, or about 80 nm.

In addition, a substrate suitable for a marker may be used together to cause a color reaction of the marker. In this case, the substrate may be added simultaneously with the marker, but may be added before and after adding the marker. The markers and substrates can be used by known methods. In one embodiment, when HRP is used as a marker, ABTS (2,2'-Azinobis [3-ethylbenzothiazoline-6-sulfonic acid]-diammonium salt), OPD (o-Phenylenediamine dihydrochloride), AmplexRed, DAB (3,3'-diaminobenzidine tetrahydrochloride), AEC (3-Amino-9-ethylcarbazole), TMB (3,3',5,5'-Tetramethylbenzidine), Homovanillic acid, or Luminol may be used as a substrate. In addition, in one embodiment, when a fluorescent protein is used, a marker may be detected by the presence or absence of light having a wavelength emitted after irradiating light of a specific wavelength rather than a substrate.

According to one embodiment, the present diagnostic method can detect the target cfDNA with a high degree of precision and accuracy, and for example, it is possible to effectively detect even when the target cfDNA is contained in a very small amount in a biological sample. Therefore, it can be usefully used for the detection of cancer cell in early stage.

By detecting the presence or absence of cfDNA encoding a biomarker of a specific abnormal cells/tissue, for example a specific cancer, present in a biological sample, the diagnosis, prognosis, or metastatis status of the cancer in question can be confirmed, and resistance/tolerance to conventional treatment methods can be also predicted.

### <Prostate Cancer>

### Method for Diagnosing Prostate Cancer

One aspect of the present invention provides a method for diagnosing prostate cancer by detecting a gene derived from prostate cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a prostate cancer biomarker. In this case, a gene known as a prostate cancer biomarker may be a gene encoding a protein overexpressed in prostate cancer.

Specifically, the probe having a sequence complementary to cfDNA may complementarily bind to at least any one gene selected from the group consisting of *KLK3, FOLH1*, *PCA3, PDE4D7, SFMBT2, EFEMP1*, *RETN, ACADL, AGR2, COL1A1*, *FAM13C*, *GPX8, GRHL2, HNF1A*, *HOXB13*, *KLK2, MYBPC1*, *NROB1*, *PITX2, SFRP4, SLCO1B3*, *TMEFF2, TMPRSS2-ERG*, and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with avidin-based protein with a solution of PEI to which biotin is bound, cationic branched polyethyleneimine (cationic branched PEI) may be further bound to the nanowires through the interaction of biotin-avidin-based protein. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of prostate cancer cells. Specifically, cfDNA may include a nucleic acid sequence overexpressed in prostate cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.007 or more when the absorbance (optical density) is measured using a marker. More specifically, in the level or reference of the nucleic acid sequence overexpressed in cancer cells, an OD value that is determined from a maximum value of sensitivity and specificity by measuring an absorbance using a marker and then drawing a receiver operation characteristic curve may be used as a reference. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from prostate cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in prostate cancer cells may be any one selected from the group consisting of *KLK3* (NCBI Gene ID: 354), *FOLH1* (NCBI Gene ID: 2346), *ACPP* (NCBI Gene ID: 55), *PCA3* (NCBI Gene ID: 50652), *PDE4D7* (NCBI Gene ID: 5144), *SFMBT2* (NCBI Gene ID: 57713), *EFEMP1* (NCBI Gene ID: 2202), *CB1* Gene ID: 56729), *ACADL* (NCBI Gene ID: 33), *AGR2* (NCBI Gene ID: 10551), *COL1A1* (NCBI Gene ID: 1277), *FAM13C* (NCBI Gene ID: 220965), *GPX8* (NCBI Gene ID: 493869), *GRHL2* (NCBI Gene ID: 79977), *HNF1A* (NCBI Gene ID: 6927), *HOXB13* (NCBI Gene ID: 10481), *KLK2* (NCBI Gene ID: 3817), *MYBPC1* (NCBI Gene ID: 4604), *NROB1* (NCBI Gene ID: 190), *PITX2* (NCBI Gene ID: 5308), *SFRP4* (NCBI Gene ID: 6424), *SLCOIB3* (NCBI Gene ID: 28234), *TMEFF2* (NCBI Gene ID: 23671), *CPT1A* (NCBI Gene ID: 1374), *IFNG* (NCBI Gene ID: 3458), *CD274* (NCBI Gene ID: 29126), *FOLR1* (NCBI Gene ID: 2348), *EPCAM(NCBI* Gene ID: 4072), *OGT* (NCBI Gene ID: 8473), *TMPRSS2-ERG*, and a combination thereof.

In one embodiment, the gene specifically present in prostate cancer may be *KLK3, FOLH1*, *PCA3, PDE4D7, SFMBT2, EFEMP1*, *RETN, ACADL, AGR2, COL1A1*, *FAM13C, GPX8, GRHL2, HNF1A*, *HOXB13*, *KLK2, MYBPC1*, *NR0B1*, *PITX2, SFRP4, SLCO1B3*, *TMEFF2, TMPRSS2-ERG* genes, and additionally, one or more additional marker genes selected from the group consisting of *ACPP, CPT1A*, *IFNG, CD279, CD274, ERBB2, EGFR, FOLR1,* and *EPCAM* may be additionally detected to diagnose prostate cancer. The additional marker gene is not a marker gene specific for prostate cancer, but when used in combination with the gene specifically present in prostate cancer, the sensitivity and specificity of the method for diagnosing prostate cancer can be significantly enhanced.

As used herein, the term *"KLK3"* refers to a gene encoding kallikrein-3, gamma-seminoprotein or prostate specific antigen (PSA). The PSA is a proteolytic enzyme synthesized in epithelial cells of the prostate, and is rarely expressed in tissues other than the prostate, and thus is a useful tumor marker used for screening prostate cancer. In addition, the PSA is usefully used for screening prostate cancer as well as for determining recurrence after surgery.

As used herein, the term "*FOLH1*" refers to a gene encoding a prostate-specific membrane antigen (PSMA). It is known that the PSMA is highly expressed in the prostate, and the expression of the PSMA in prostate cancer cells is increased by about 8 times to about 12 times as compared to that of normal prostate cells. The PSMA is used as a tumor marker for the diagnosis of prostate cancer.

As used herein, the term "*ACPP*" refers to a gene encoding prostatic acid phosphatase (PAP). The PAP is an enzyme produced by the prostate. The PAP shows an increase in expression in men suffering from prostate cancer or prostate disease, and thus, is used as an indicator of prostate cancer or prostate disease.

As used herein, the term *"PCA3"* refers to a gene expressed in the form of non-coding RNA in human prostate tissue. The PCA3 is expressed only in human prostate tissue and is highly overexpressed in prostate cancer cells. The PCA3 is used as a tumor marker for prostate cancer.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "avidin" refers to a homotetramer protein produced in the fallopian tubes of birds, reptiles, and amphibians, and is distributed in the egg white, and binds to biotin with a high affinity. Although its function in nature has been not yet investigated, it is estimated to be used for inhibiting the growth of bacteria by binding to biotin, which is necessary for the growth of bacteria.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from prostate cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Prostate Cancer Diagnostic Kit

Other aspect of the present invention provides a diagnostic kit for diagnosing prostate cancer, comprising a probe to which biotin is bound complementarily binding to a gene specifically expressed in prostate cancer; a positively charged material; a marker to which an avidin-based protein is bound; and a manual.

In this case, the gene specifically expressed in prostate cancer may be any one or more selected from the group consisting of *KLK3, FOLH1*, *PCA3, PDE4D7, SFMBT2, EFEMP1*, *RETN, ACADL, AGR2, COL1A1*, *FAM13C, GPX8, GRHL2, HNF1A*, *HOXB13*, *KLK2, MYBPC1*, *NR0B1*, *PITX2, SFRP4, SLCO1B3*, *TMEFF2, TMPRSS2-ERG*, and a combination thereof.

In addition, the manual may describe that the kit is configured to be capable of diagnosing prostate cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *ACPP*, *CPT1A*, *IFNG, CD274, FOLR1*, *EPCAM, OGT,* and a combination thereof.

In addition, the probe, the positively charged material, and the marker are as described above.

### Prostate Cancer Diagnostic Device

Other aspect of the present invention provides a device for diagnosing prostate cancer by detecting a gene derived from prostate cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in prostate cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from prostate cancer in the sample depending on whether the marker is detected.

In this case, the gene specifically expressed in prostate cancer may be any one or more selected from the group consisting of *KLK3, FOLH1*, *PCA3, PDE4D7, SFMBT2, EFEMP1*, *RETN, ACADL, AGR2, COL1A1*, *FAM13C, GPX8, GRHL2, HNF1A*, *HOXB13*, *KLK2, MYBPC1*, *NR0B1*, *PITX2, SFRP4, SLC01B3*, *TMEFF2, TMPRSS2-ERG*, and a combination thereof.

In addition, it may further comprise a probe to which biotin is bound complementarily binding to at least any one gene selected from the group consisting of *ACPP*, *CPT1A*, *IFNG, CD274, FOLR1*, *EPCAM, OGT,* and a combination thereof.

### <Lung Cancer>

### Method for Diagnosing Lung cancer

One aspect of the present invention provides a method for diagnosing lung cancer by detecting a gene derived from lung cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a lung cancer biomarker. In this case, a gene known as a lung cancer biomarker may be a gene encoding a protein overexpressed in lung cancer.

Specifically, the probe having a sequence complementary to cfDNA may complementarily bind to at least any one gene selected from the group consisting of *ENO2*, *SART3, KRT19*, *PLAT, EGFR, ALK, ROS1*, *RET, ERBB2, PI3K*, *S100P*, *MMP11*, *CDCA7, S100A2*, *ETV4*, *TOP2A, UBE2C,* and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin- streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of lung cancer cells. Specifically, cfDNA may include a nucleic acid sequence overexpressed in lung cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker.

More specifically, in the level or reference of the nucleic acid sequence overexpressed in cancer cells, an OD value is about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from lung cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes.

The gene overexpressed in lung cancer cells may be any one selected from the group consisting of *ENO2(NCBI Gene ID: 2026), SART3(NCBI Gene ID: 9733), ACPP(NCBI Gene ID: 55), KRT19(NCBI Gene ID: 3880), PLAT(NCBI Gene ID: 5327), EGFR(NCBI Gene ID: 1956), KRAS(NCBI Gene ID: 3845), ALK(NCBI Gene ID: 238), ROS1(NCBI Gene ID: 6098), RET(NCBI Gene ID: 5979), ERBB2(NCBI Gene ID: 2064), PI3K(NCBI Gene ID: 5291), S100P(NCBI Gene ID: 6286), MMP11 (NCBI Gene ID: 4320), CDCA7(NCBI Gene ID: 83879), S100A2(NCBI Gene ID: 6273), ETV4(NCBI Gene ID: 2118), TOP2A(NCBI Gene ID: 7153), UBE2C(NCBI Gene ID: 11065), CPT1A(NCBI Gene ID: 1374), IFNG(NCBI Gene ID: 3458), CD274(NCBI Gene ID: 29126), FOLR1(NCBI Gene ID: 2348), EPCAM(NCBI Gene ID: 4072), OGT(NCBI Gene ID: 8473),* and a combination thereof.

In one embodiment, the gene specifically present in lung cancer may be *SART3, PLAT, ALK, ROS1, PI3K*, *S100P*, *CDCA7, S100A2*, *ETV4* genes, and additionally, *ENO2*, *ACPP, KRT19*, *EGFR, KRAS, RET, ERBB2, MMP11*, *TOP2A, UBE2C, CPT1A*, *IFNG, CD279, CD274, ERBB2, EGFR, FOLR1*, or *EPCAM* gene may be additionally detected to diagnose lung cancer.

As used herein, the term *"ENO2*" refers to a gene encoding an enzyme known as gammaenolase or enolase 2 or NSE (neuron specific enolase). The NSE is used as a tumor marker of small cell lung cancer, neuroblastoma, and medullary thyroid cancer.

As used herein, the term *"SART3"* refers to a gene encoding squamous cell carcinoma antigen (SCCA). The SCCA is used as a tumor marker because it exhibits positive in the blood of patients with many squamous cell cancers such as vulvar cancer, vaginal cancer, esophageal cancer, tongue cancer, and pharynx cancer as well as squamous cell cancer of the cervix.

As used herein, the term "*KRT19*" refers to a gene encoding a protein known as Cyfra 21-1, CK-19 (cytokeratin-19), or K19 (keratin-19). It is known that the Cyfra 21-1 is associated with cancers originated from epithelial cells such as lung cancer and head and neck cancer. In addition, it has been reported that the blood level of the Cyfra 21-1 in patients suffering from pneumonia or lung disease is increased as compared to normal persons, and the Cyfra 21-1 is used as a tumor indicator.

As used herein, the term *"PLAT'* refers to a gene encoding TPA (tissue plasminogen activator), which is a protein involved in blood clot disruption.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from lung cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Lung Cancer Diagnostic Kit

Other aspect of the present invention provides a diagnostic kit for diagnosing lung cancer, comprising a probe to which biotin is bound complementarily binding to a gene specifically expressed in lung cancer; a positively charged material; a marker to which an avidin-based protein is bound; and a manual.

In this case, the gene specifically expressed in lung cancer may be any one or more selected from the group consisting of *SART3, PLAT, ALK, ROS1, PI3K*, *S100P*, *CDCA7, S100A2, ETV4,* and a combination thereof.

In addition, the manual may describe that the kit is configured to be capable of diagnosing lung cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *ENO2*, *ACPP, KRT19*, *EGFR, KRAS, RET, ERBB2, MMP11*, *TOP2A, UBE2C, CPT1A*, *IFNG, CD279, CD274, ERBB2, EGFR, FOLR1, EPCAM,* and a combination thereof.

In addition, the probe, the positively charged material, and the marker are as described above.

### Lung Cancer Diagnostic Device

Other aspect of the present invention provides a device for diagnosing lung cancer by detecting a gene derived from lung cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in lung cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from lung cancer in the sample depending on whether the marker is detected.

In this case, the gene specifically expressed in lung cancer may be any one or more selected from the group consisting of *SART3, PLAT, ALK, ROS1, PI3K*, *S100P*, *CDCA7, S100A2*, *ETV4,* and a combination thereof.

In addition, it may further comprise a probe to which biotin is bound complementarily binding to at least any one gene selected from the group consisting of *ENO2*, *ACPP, KRT19*, *EGFR, KRAS, RET, ERBB2, MMP11*, *TOP2A, UBE2C, CPT1A*, *IFNG, CD279, CD274, ERBB2, EGFR, FOLR1, EPCAM,* and a combination thereof.

### <Thyroid Cancer>

### Method for Diagnosing Thyroid cancer

One aspect of the present invention provides a method for diagnosing thyroid cancer by detecting a gene derived from thyroid cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a thyroid cancer biomarker. In this case, a gene known as a thyroid cancer biomarker may be a gene encoding a protein overexpressed in thyroid cancer.

Specifically, the probe having a sequence complementary to cfDNA may complementarily bind to at least any one gene selected from the group consisting of *TG, CALCA, APOC1, HIG2*, and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin protein with a solution of PEI to which biotin is bound, cationic branched polyethyleneimine (cationic branched PEI) may be further bound to the nanowires through the interaction of biotin- streptavidin protein. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of thyroid cancer cells. Specifically, cfDNA may include a nucleic acid sequence overexpressed in thyroid cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring a absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from thyroid cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in thyroid cancer cells may be any one selected from the group consisting of *ACPP(NCBI* Gene ID: 55), *ENO2*(NCBI Gene ID: 2026), *TG*(NCBI Gene ID: 7038), *CALCA*(NCBI Gene ID: 796), *APOC1*(NCBI Gene ID: 341), *HIG2*(NCBI Gene ID: 29923), *TYRO3*(NCBI Gene ID: 7301), *CPT1A(NCBI* Gene ID: 1374), *IFNG(NCBI* Gene ID: 3458), *CD274(NCBI* Gene ID: 29126), *FOLR1*(NCBI Gene ID: 2348), *EPCAM(NCBI* Gene ID: 4072), and a combination thereof.

In one embodiment, the gene specifically present in thyroid cancer may be *TG, CALCA, APOC1, HIG2* genes, and additionally, *ENO2*, *ACPP, TYRO3*, *CPT1A*, *IFNG, CD279, CD274, ERBB2, EGFR, FOLR1*, or *EPCAM* gene may be additionally detected to diagnose thyroid cancer.

As used herein, the term *"TG"* refers to a gene encoding thyroglobulin (Tg). The thyroglobulin is produced only in the thyroid gland in the human body, and when thyroid cancer develops or metastasizes, the level of thyroglobulin in the blood is increased. The level of thyroglobulin in the blood is used as a thyroid cancer marker.

As used herein, the term *"CALCA"* refers to a gene encoding a calcitonin gene-related peptide.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from thyroid cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Thyroid Cancer Diagnostic Kit

Other aspect of the present invention provides a diagnostic kit for diagnosing thyroid cancer, comprising a probe to which biotin is bound complementarily binding to a gene specifically expressed in thyroid cancer; a positively charged material; a marker to which an avidin-based protein is bound; and a manual.

In this case, the gene specifically expressed in thyroid cancer may be any one or more selected from the group consisting *of TG, CALCA, APOC1, HIG2,* and a combination thereof.

In addition, the manual may describe that the kit is configured to be capable of diagnosing thyroid cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *ENO2*, *ACPP, TYRO3*, *CPT1A*, *IFNG*, *CD279, CD274, ERBB2, EGFR, FOLR1, EPCAM,* and a combination thereof.

In addition, the probe, the positively charged material, and the marker are as described above.

### Thyroid Cancer Diagnostic Device

Other aspect of the present invention provides a device for diagnosing thyroid cancer by detecting a gene derived from thyroid cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in thyroid cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from thyroid cancer in the sample depending on whether the marker is detected.

In this case, the gene specifically expressed in thyroid cancer may be any one or more selected from the group consisting *of TG, CALCA, APOC1, HIG2,* and a combination thereof.

In addition, it may further comprise a probe to which biotin is bound complementarily binding to at least any one gene selected from the group consisting of *ENO2*, *ACPP, TYRO3*, *CPT1A*, *IFNG*, *CD279, CD274, ERBB2, EGFR, FOLR1, EPCAM,* and a combination thereof.

### <Bladder Cancer>

### Method for Diagnosing Bladder cancer

One aspect of the present invention provides a method for diagnosing bladder cancer by detecting a gene derived from bladder cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a bladder cancer biomarker. In this case, a gene known as a bladder cancer biomarker may be a gene encoding a protein overexpressed in bladder cancer.

Specifically, the probe having a sequence complementary to cfDNA may complementarily bind to at least any one gene selected from the group consisting of *OGT, FGFR3, TP53, NUMA1*, *COCH, CELSR3, HMOX1*, *KIF1A*, *MGC17624*, *MTAP, PFKFB4*, *S100A8*, *RSPH9, FOXM1*, *FANCB, FANCC, FANCD2, RUSC1-AS1*, *CACNA1B*, *IMP-1*, *PDE3A, POU3F4, SOX3, DMC1*, *PLXDC2, ZNF312*, *SYCP2L*, *HOXA9, ISL1, ALDH1A3*, and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin- streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of bladder cancer cells. Specifically, cfDNA may include a nucleic acid sequence overexpressed in bladder cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from bladder cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in bladder cancer cells may be any one selected from the group consisting of *OGT(NCBI Gene ID: 8473), FGFR3(NCBI Gene ID: 2261), TP53(NCBI Gene ID: 7157), NUMA1(NCBI Gene ID: 4926), KRT19(NCBI Gene ID: 3880), COCH(NCBI Gene ID: 1690), CELSR3(NCBI Gene ID: 1951), HMOX1(NCBI Gene ID: 3162), KIF1A(NCBI Gene ID: 547), MGC17624(NCBI Gene ID: 404550), MTAP(NCBI Gene ID: 4507), PFKFB4(NCBI Gene ID: 5210), S100A8(NCBI Gene ID: 6279), RSPH9(NCBI Gene ID: 221421), CCNB1(NCBI Gene ID: 891), FOXM1(NCBI Gene ID: 2305), FANCB(NCBI Gene ID: 2187), FANCC(NCBI Gene ID: 2176), FANCD2(NCBI Gene ID: 2177), RUSC1-AS1(NCBI Gene ID: 284618), CACNA1B(NCBI Gene ID: 774), IMP-1(NCBI Gene ID: 10642), PDE3A(NCBI Gene ID: 5139), POU3F4(NCBI Gene ID: 5456), SOX3(NCBI Gene ID: 6658), DMC1(NCBI Gene ID: 11144), PLXDC2(NCBI Gene ID: 84898), ZNF312(NCBI Gene ID: 55079), SYCP2L(NCBI Gene ID: 221711), HOXA9(NCBI Gene ID: 3205), ISL1(NCBI Gene ID: 3670), ALDHIA3(NCBI Gene ID: 220), CPT1A(NCBI Gene ID: 1374), IFNG(NCBI Gene ID: 3458), CD274(NCBI Gene ID: 29126), FOLR1(NCBI Gene ID: 2348), EPCAM(NCBI Gene ID: 4072),* and a combination thereof.

In one embodiment, the gene specifically present in bladder cancer may be *OGT, FGFR3, TP53, NUMA1, COCH, CELSR3*, *HMOX1*, *KIF1A*, *MGC17624*, *MTAP, PFKFB4*, *S100A8*, *RSPH9, FOXM1*, *FANCB, FANCC, FANCD2, RUSC1-AS1*, *CACNA1B*, *IMP-1*, *PDE3A, POU3F4, SOX3, DMC1, PLXDC2*, *ZNF312*, *SYCP2L*, *HOXA9*, *ISL1*, *ALDH1A3* genes, and additionally, *KRT19*, *CCNB1, CPT1A*, *IFNG*, *CD279, CD274, ERBB2, EGFR, FOLR1,* or *EPCAM* gene may be additionally detected to diagnose bladder cancer.

As used herein, the term *"OGT'* refers to a gene encoding O-G1cNAc transferase.

As used herein, the term *"FGFR1*" refers to a gene encoding fibroblast growth factor receptor 1.

As used herein, the term "*NUMA1*" refers to a gene encoding NMP22 (nuclear matrix protein-22). It was found that the level of the NMP22 in urine of patients with some types of cancer, including bladder cancer, is higher than a normal level, and thus the NMP22 is widely used as a bladder cancer marker.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from bladder cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Bladder Cancer Diagnostic Kit

Other aspect of the present invention provides a diagnostic kit for diagnosing bladder cancer, comprising a probe to which biotin is bound complementarily binding to a gene specifically expressed in bladder cancer; a positively charged material; a marker to which an avidin-based protein is bound; and a manual.

In this case, the gene specifically expressed in bladder cancer may be any one or more selected from the group consisting of *OGT, FGFR3, TP53, NUMA1, COCH, CELSR3*, *HMOX1*, *KIF1A*, *MGC17624*, *MTAP, PFKFB4, S100A8*, *RSPH9, FOXM1*, *FANCB, FANCC, FANCD2, RUSC1-AS1*, *CACNA1B*, *IMP-1*, *PDE3A, POU3F4, SOX3, DMC1, PLXDC2*, *ZNF312*, *SYCP2L*, *HOXA9, ISL1, ALDH1A3*, and a combination thereof.

In addition, the manual may describe that the kit is configured to be capable of diagnosing bladder cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *KRT19*, *CCNB1, CPT1A*, *IFNG, CD279, CD274, ERBB2, EGFR, FOLR1*, *EPCAM,* and a combination thereof.

In addition, the probe, the positively charged material, and the marker are as described above.

### Bladder Cancer Diagnostic Device

Other aspect of the present invention provides a device for diagnosing bladder cancer by detecting a gene derived from bladder cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in bladder cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from bladder cancer in the sample depending on whether the marker is detected.

In this case, the gene specifically expressed in bladder cancer may be any one or more selected from the group consisting of *OGT, FGFR3, TP53, NUMA1*, *COCH, CELSR3*, *HMOX1*, *KIF1A*, *MGC17624*, *MTAP, PFKFB4, S100A8*, *RSPH9, FOXM1*, *FANCB, FANCC, FANCD2, RUSC1-AS1*, *CACNA1B*, *IMP-1*, *PDE3A, POU3F4, SOX3, DMC1, PLXDC2, ZNF312*, *SYCP2L*, *HOXA9, ISL1, ALDH1A3*, and a combination thereof.

In addition, it may further comprise a probe to which biotin is bound complementarily binding to at least any one gene selected from the group consisting of *KRT19*, *CCNB1, CPT1A*, *IFNG, CD279, CD274, ERBB2, EGFR, FOLR1, EPCAM,* and a combination thereof.

### <Breast Cancer>

### Method for Diagnosing Breast cancer

One aspect of the present invention provides a method for diagnosing breast cancer by detecting a gene derived from breast cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a breast cancer biomarker. In this case, a gene known as a breast cancer biomarker may be a gene encoding a protein overexpressed in breast cancer.

Specifically, the probe having a sequence complementary to cfDNA may complementarily bind to at least any one gene selected from the group consisting of *MEST, NR1D1*, *BIRC5*, *RACGAP1*, *DHCR7, STC2, AZGP1*, *RBBP8, IL6ST, MGP, TRBC1, MMP11*, *COL10A1*, *C10orf64*, *COL11A1*, *POTEG, FSIP1*, *HER2,* and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin- streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of breast cancer cells. Specifically, cfDNA may include a nucleic acid sequence overexpressed in breast cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from breast cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, **DMSO,** a surfactant, and the like).

The gene overexpressed in breast cancer cells may be any one selected from the group consisting of *MUC1 (NCBI Gene ID: 4582), ACPP(NCBI Gene ID: 55), MEST(NCBI Gene ID: 4232), TYRO3(NCBI Gene ID: 7301), NR1D1(NCBI Gene ID: 9572), UBE2C(NCBI Gene ID: 11065), BIRC5(NCBI Gene ID: 332), RACGAP1(NCBI Gene ID: 29127), DHCR7(NCBI Gene ID: 1717), STC2(NCBI Gene ID: 8614), AZGP1(NCBI Gene ID: 563), RBBP8(NCBI Gene ID: 5932), IL6ST(NCBI Gene ID: 3572), MGP(NCBI Gene ID: 4256), TRBC1(NCBI Gene ID: 28639), MMP11(NCBI Gene ID: 4320), COL10A1(NCBI Gene ID: 1300), C10orf64(NCBI Gene ID: 57705), COL11A1(NCBI Gene ID: 1301), POTEG(NCBI Gene ID: 404785), FSIP1(NCBI Gene ID: 161835), HER2(NCBI Gene ID: 2064), CPT1A(NCBI Gene ID: 1374), IFNG(NCBI Gene ID: 3458), CD274(NCBI Gene ID: 29126), FOLR1(NCBI Gene ID: 2348), EPCAM(NCBI Gene ID: 4072),* and a combination thereof.

In one embodiment, the gene specifically present in breast cancer may be *MEST, NR1D1*, *BIRC5, RACGAP1*, *DHCR7, STC2, AZGP1*, *RBBP8, IL6ST, MGP, TRBC1, MMP11*, *COL10A1*, *C10orf64*, *COL11A1*, *POTEG, FSIP1*, *HER2* genes, and additionally, *MUC1*, *ACPP, TYRO3*, *UBE2C, CPT1A*, *IFNG, CD279, CD274, ERBB2, EGFR, FOLR1,* or *EPCAM* gene may be additionally detected to diagnose breast cancer.

As used herein, the term "*MUC1*" refers to a breast cancer-related gene encoding a protein including CA 15-3 (carcinoma antigen 15-3) and CA 27-29. The CA15-3 has been shown to increase the possibility of early recurrence in breast cancer and is used as a breast cancer marker.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from breast cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Breast Cancer Diagnostic Kit

Other aspect of the present invention provides a diagnostic kit for diagnosing breast cancer, comprising a probe to which biotin is bound complementarily binding to a gene specifically expressed in breast cancer; a positively charged material; a marker to which an avidin-based protein is bound; and a manual.

In this case, the gene specifically expressed in breast cancer may be any one or more selected from the group consisting of *MEST, NR1D1*, *BIRC5, RACGAP1*, *DHCR7, STC2, AZGP1*, *RBBP8, IL6ST, MGP, TREC1*, *MMP11*, *COL10A1*, *C10orf64*, *COL11A1*, *POTEG, FSIP1, HER2,* and a combination thereof.

In addition, the manual may describe that the kit is configured to be capable of diagnosing breast cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting *of MUC1*, *ACPP, TYRO3*, *UBE2C, CPT1A*, *IFNG, CD279, CD274, ERBB2, EGFR, FOLR1, EPCAM,* and a combination thereof.

In addition, the probe, the positively charged material, and the marker are as described above.

### Breast Cancer Diagnostic Device

Other aspect of the present invention provides a device for diagnosing breast cancer by detecting a gene derived from breast cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in breast cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from breast cancer in the sample depending on whether the marker is detected.

In this case, the gene specifically expressed in breast cancer may be any one or more selected from the group consisting of *MEST, NR1D1*, *BIRC5, RACGAP1*, *DHCR7, STC2, AZGP1*, *RBBP8, IL6ST, MGP, TREC1*, *MMP11*, *COL10A1*, *C10orf64*, *COL11A1*, *POTEG, FSIP1, HER2,* and a combination thereof.

In addition, it may further comprise a probe to which biotin is bound complementarily binding to at least any one gene selected from the group consisting of *MUC1*, *ACPP, TYRO3*, *UBE2C, CPT1A*, *IFNG, CD279, CD274, ERBB2, EGFR, FOLR1, EPCAM,* and a combination thereof.

### <Colorectal Cancer>

### Method for Diagnosing Colorectal cancer

One aspect of the present invention provides a method for diagnosing colorectal cancer by detecting a gene derived from colorectal cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a colorectal cancer biomarker. In this case, a gene known as a colorectal cancer biomarker may be a gene encoding a protein overexpressed in colorectal cancer.

Specifically, the probe having a sequence complementary to cfDNA may complementarily bind to at least any one gene selected from the group consisting of *NCKAP1*, *AUNIP, NOTUM, KRT5, TUBB, COL6A1*, *JUP, CDX2, MELTF, EFEMP2, DEFA5, CHEK1*, *MAD2L1*, *ENC1, CSE1L*, *RAD51AP1*, *ERICH3*, *SLC7A11*, *KRT23, PLAU*, *CDCA1, KLK6, DPEP1, CDH3, ANLN, CXCL1*, *CTHRC1*, *LCN2, HS6ST2, EGFL6, CXCL3, CA9, PROX1*, *SPP1, CST1*, *CXCL2, TSTA3, RRM2, MMP3, MMP7, MMP10*, *CXCL5*, *SERPINB5*, *TEAD4, BUB1*, *CDC2, CLDN2, HSPH1*, *LY6G6D, PRC1*, *PUS1*, *SQLE, TTK, ECT2, RNF183*, *FBXO39*, *TEX38, TTLL2, PRR7, CANP, KIAA010*, and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin protein with a solution of PEI to which biotin is bound, cationic branched polyethyleneimine (cationic branched PEI) may be further bound to the nanowires through the interaction of biotin- streptavidin protein. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of colorectal cancer cells. Specifically, cfDNA may include a nucleic acid sequence overexpressed in colorectal cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from colorectal cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in colorectal cancer cells may be any one selected from the group consisting of *ACPP(NCBI Gene ID: 55), FLU3(NCBI Gene ID: 837968), TYRO3(NCBI Gene ID: 7301), NCKAPI(NCBI Gene ID: 10787), AUNIP(NCBI Gene ID: 79000), NOTUM(NCBI Gene ID: 147111), KRT5(NCBI Gene ID: 3852), TUBB(NCBI Gene ID: 203068), COL6A1(NCBI Gene ID: 1291), JUP(NCBI Gene ID: 3728), COTL1(NCBI Gene ID: 23406), CK7(NCBI Gene ID: 3855), CK20(NCBI Gene ID: 54474), CDX2(NCBI Gene ID: 1045), MUC2(NCBI Gene ID: 4583), MELTF(NCBI Gene ID: 4241), SDC2(NCBI Gene ID: 6383), EFEMP2(NCBI Gene ID: 30008), DEFA5(NCBI Gene ID: 1670), ASB9(NCBI Gene ID: 140462), CHEK1(NCBI Gene ID: 1111), MAD2L1(NCBI Gene ID: 4085), ENC1(NCBI Gene ID: 8507), CSE1L(NCBI Gene ID: 1434), RAD5]AP1(NCBI Gene ID: 10635), ERICH3(NCBI Gene ID: 127524), SLC7A11(NCBI Gene ID: 23657), KRT23(NCBI Gene ID: 25984), PLAU(NCBI Gene ID: 5328), CCNB1(NCBI Gene ID: 891), MELK(NCBI Gene ID: 9833), CDCA1(NCBI Gene ID: 83540), KLK6(NCBI Gene ID: 5653), CKS2(NCBI Gene ID: 1164), IFITM1(NCBI Gene ID: 8519), DPEP1(NCBI Gene ID: 1800), CDH3(NCBI Gene ID: 1001), ANLN(NCBI Gene ID: 54443), CXCL1(NCBI Gene ID: 2919), CTHRC1(NCBI Gene ID: 115908), CEACAM6(NCBI Gene ID: 4680), LCN2(NCBI Gene ID: 3934), HS6ST2(NCBI Gene ID: 90161), EGFL6(NCBI Gene ID: 25975), CXCL3(NCBI Gene ID: 2921), CA9(NCBI Gene ID: 768), ATAD2(NCBI Gene ID: 29028), PROX1 (NCBI Gene ID: 5629), SPP1(NCBI Gene ID: 6696), CST1(NCBI Gene ID: 1469), CXCL2(NCBI Gene ID: 2920), TSTA3(NCBI Gene ID: 7264), RRM2(NCBI Gene ID: 6241), MMP3(NCBI Gene ID: 4314), MMP7(NCBI Gene ID: 4316), MMP10(NCBI Gene ID: 4319), CXCL5(NCBI Gene ID: 6374), SERPINB5(NCBI Gene ID: 5268), TEAD4(NCBI Gene ID: 7004), BUB1(NCBI Gene ID: 699), CDC2(NCBI Gene ID: 983), CLDN2(NCBI Gene ID: 9075), HSPH1(NCBI Gene ID: 10808), LY6G6D(NCBI Gene ID: 58530)*, *PRC1(NCBI Gene ID: 9055), PUS1(NCBI Gene ID: 80324), SQLE(NCBI Gene ID: 6713), TOP2A(NCBI Gene ID: 7153), TTK(NCBI Gene ID: 7272), DSCC1(NCBI Gene ID: 79075), ECT2(NCBI Gene ID: 1894), RNF183(NCBI Gene ID: 138065), FBX039(NCBI Gene ID: 162517), TEX38(NCBI Gene ID: 374973)*, *TTLL2(NCBI Gene ID: 83887), PRR7(NCBI Gene ID: 80758), CANP(NCBI Gene ID: 823), KIAA0101(NCBI Gene ID: 9768), CPT1A(NCBI Gene ID: 1374), IFNG(NCBI Gene ID: 3458), CD274(NCBI Gene ID: 29126), FOLR1(NCBI Gene ID: 2348), EPCAM(NCBI Gene ID: 4072), KRAS(NCBI Gene ID: 3845),* and a combination thereof.

In one embodiment, the gene specifically present in colorectal cancer may be *NCKAP1*, *AUNIP, NOTUM, KRT5, TUBB, COL6A1*, *JUP, CDX2, MELTF, EFEMP2, DEFA5, CHEK1*, *MAD2L1*, *ENC1*, *CSE1L*, *RAD51AP1*, *ERICH3*, *SLC7A11*, *KRT23, PLAU*, *CDCA1, KLK6, DPEP1, CDH3, ANLN, CXCL1*, *CTHRC1*, *LCN2, HS6ST2*, *EGFL6, CXCL3, CA9, PROX1*, *SPP1*, *CST1*, *CXCL2, TSTA3, RRM2, MMP3, MMP7, MMP10*, *CXCL5*, *SERPINB5*, *TEAD4, BUB1*, *CDC2, CLDN2, HSPH1*, *LY6G6D, PRC1*, *PUS1, SQLE, TTK, ECT2, RNF183*, *FBXO39*, *TEX38, TTLL2, PRR7, CANP, KIAA010* genes, and additionally, *ACPP, FLU3, TYRO3*, *COTL1*, *CK7, CK20, MUC2, SDC2, ASB9, CCNB1, MELK, CKS2, IFITM1*, *CEACAM6, ATAD2, TOP2A*, *CPT1A*, *DSCC1, IFNG, CD279, CD274, ERBB2, EGFR, FOLR1*, or *EPCAM* gene may be additionally detected to diagnose colorectal cancer.

As used herein, the term "FLU3" refers to a gene encoding a protein including CA19-9(Carcinoma Antigen 19-9).

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from colorectal cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Colorectal Cancer Diagnostic Kit

Other aspect of the present invention provides a diagnostic kit for diagnosing colorectal cancer, comprising a probe to which biotin is bound complementarily binding to a gene specifically expressed in colorectal cancer; a positively charged material; a marker to which an avidin-based protein is bound; and a manual.

In this case, the gene specifically expressed in colorectal cancer may be any one or more selected from the group consisting *of NCKAP1*, *AUNIP, NOTUM, KRT5, TUBB, COL6A1*, *JUP, CDX2, MELTF, EFEMP2, DEFA5, CHEK1*, *MAD2L1*, *ENC1*, *CSE1L*, *RAD51AP1*, *ERICH3*, *SLC7A11*, *KRT23, PLAU, CDCA1, KLK6, DPEP1, CDH3, ANLN, CXCL1*, *CTHRC1*, *LCN2, HS6ST2, EGFL6, CXCL3, CA9, PROX1*, *SPP1, CST1*, *CXCL2, TSTA3, RRM2, MMP3, MMP7, MMP10*, *CXCL5, SERPINB5*, *TEAD4, BUB1, CDC2, CLDN2, HSPH1*, *LY6G6D, PRC1*, *PUS1, SQLE, TTK, ECT2, RNF183*, *FBXO39*, *TEX38, TTLL2, PRR7, CANP, KIAA0101*, and a combination thereof.

In addition, the manual may describe that the kit is configured to be capable of diagnosing colorectal cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *ACPP, FLU3, TYRO3*, *COTL1*, *CK7, CK20, MUC2, SDC2, ASB9, CCNB1, MELK, CKS2, IFITM1*, *CEACAM6, ATAD2, TOP2A, CPT1A*, *DSCC1*, *IFNG*, *CD279, CD274, ERBB2, EGFR, FOLR1*, *EPCAM,* and a combination thereof.

In addition, the probe, the positively charged material, and the marker are as described above.

### Colorectal Cancer Diagnostic Device

Other aspect of the present invention provides a device for diagnosing colorectal cancer by detecting a gene derived from colorectal cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in colorectal cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from colorectal cancer in the sample depending on whether the marker is detected.

In this case, the gene specifically expressed in colorectal cancer may be any one or more selected from the group consisting *of NCKAP1*, *AUNIP, NOTUM, KRT5, TUBB, COL6A1*, *JUP, CDX2, MELTF, EFEMP2, DEFA5, CHEK1*, *MAD2L1*, *ENC1*, *CSE1L*, *RAD51AP1*, *ERICH3*, *SLC7A11*, *KRT23, PLAU*, *CDCA1, KLK6, DPEP1, CDH3, ANLN, CXCL1*, *CTHRC1*, *LCN2, HS6ST2, EGFL6, CXCL3, CA9, PROX1*, *SPP1, CST1*, *CXCL2, TSTA3, RRM2, MMP3, MMP7, MMP10*, *CXCL5*, *SERPINB5*, *TEAD4, BUB1, CDC2, CLDN2, HSPH1*, *LY6G6D, PRC1*, *PUS1, SQLE, TTK, ECT2, RNF183*, *FBXO39*, *TEX38, TTLL2, PRR7, CANP, KIAA0101*, and a combination thereof.

In addition, it may further comprise a probe to which biotin is bound complementarily binding to at least any one gene selected from the group consisting of *ACPP, FLU3, TYRO3*, *COTL1*, *CK7, CK20, MUC2, SDC2, ASB9, CCNB1, MELK, CKS2, IFITM1*, *CEACAM6, ATAD2, TOP2A, CPT1A*, *DSCC1*, *IFNG*, *CD279, CD274, ERBB2, EGFR, FOLR1*, *EPCAM,* and a combination thereof.

### <Biliary Tract Cancer>

### Method for Diagnosing Biliary Tract Cancer

One aspect of the present invention provides a method for diagnosing biliary tract cancer by detecting a gene derived from biliary tract cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a biliary tract cancer biomarker. In this case, a gene known as a biliary tract cancer biomarker may be a gene encoding a protein overexpressed in biliary tract cancer.

Specifically, the probe having a sequence complementary to cfDNA may complementarily bind to at least any one gene selected from the group consisting of *MUC16*, *ASH1L*, *DOCK70,* and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin- streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of biliary tract cancer cells. Specifically, cfDNA may include a nucleic acid sequence overexpressed in biliary tract cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring a absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from biliary tract cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in biliary tract cancer cells may be any one selected from the group consisting of *ACPP(NCBI Gene ID: 55), FLU3(NCBI Gene ID: 837968), MUC16(NCBI Gene ID: 94025), ASH1L(NCBI Gene ID: 55870)*, *DOCK7(NCBI Gene ID: 85440), CPT1A(NCBI Gene ID: 1374), IFNG(NCBI Gene ID: 3458), CD274(NCBI Gene ID: 29126), FOLR1(NCBI Gene ID: 2348)*, *EPCAM(NCBI Gene ID: 4072), CA125(NCBI Gene ID: 94025), CEACAM5(NCBI Gene ID: 1048),* and a combination thereof.

In one embodiment, the gene specifically present in biliary tract cancer may be *MUC16*, *ASH1L*, *DOCK70* genes, and additionally, *ACPP, FLU3, CPT1A*, *DSCC1, IFNG, CD279, CD274, ERBB2, EGFR, FOLR1*, or *EPCAM* gene may be additionally detected to diagnose biliary tract cancer.

As used herein, the term "*MUC16*" refers to a gene encoding CA-125 (carcinoma antigen 125). The CA-125 is used as a tumor marker or a biomarker that is positive in the blood of some patients with certain types of cancer.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from biliary tract cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Biliary Tract Cancer Diagnostic Kit

Other aspect of the present invention provides a diagnostic kit for diagnosing biliary tract cancer, comprising a probe to which biotin is bound complementarily binding to a gene specifically expressed in biliary tract cancer; a positively charged material; a marker to which an avidin-based protein is bound; and a manual.

In this case, the gene specifically expressed in biliary tract cancer may be any one or more selected from the group consisting of *MUC16*, *ASH1L*, *DOCK7*, and a combination thereof.

In addition, the manual may describe that the kit is configured to be capable of diagnosing biliary tract cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *ACPP*, *FLU3, CPT1A*, *DSCC1*, *IFNG*, *CD279, CD274, ERBB2, EGFR, FOLR1, EPCAM,* and a combination thereof.

In addition, the probe, the positively charged material, and the marker are as described above.

### Biliary Tract Cancer Diagnostic Device

Other aspect of the present invention provides a device for diagnosing biliary tract cancer by detecting a gene derived from biliary tract cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in biliary tract cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from biliary tract cancer in the sample depending on whether the marker is detected.

In this case, the gene specifically expressed in biliary tract cancer may be any one or more selected from the group consisting of *ACPP, FLU3, CPT1A*, *DSCC1, IFNG, CD279, CD274, ERBB2, EGFR, FOLR1, EPCAM,* and a combination thereof.

In addition, it may further comprise a probe to which biotin is bound specifically binding to at least any one gene selected from the group consisting of *ACPP*, *FLU3, CPT1A*, *DSCC1, IFNG, CD279, CD274, ERBB2, EGFR, FOLR1, EPCAM,* and a combination thereof.

### <Gastric Cancer>

### Method for Diagnosing Gastric cancer

One aspect of the present invention provides a method for diagnosing gastric cancer by detecting a gene derived from gastric cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a gastric cancer biomarker. In this case, a gene known as a gastric cancer biomarker may be a gene encoding a protein overexpressed in gastric cancer.

Specifically, the probe having a sequence complementary to cfDNA may complementarily bind to at least any one gene selected from the group consisting of *CGB, PARP1, FOXO3A, MED30, CCNE1, MYC, TFF1, FABP1, LAMP5, MATN3, CLIP4, NOX4, ADRA2C, CSK, FZD9, GALR1, GRM6, INSR, LPHN1, LYN, MRGPRX3, ADCY3, HDAC2, CFL1, NRP2, ANXA10, TFF2, CDCA5, NUSAP1,* and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin- streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of gastric cancer cells. Specifically, cfDNA may include a nucleic acid sequence overexpressed in gastric cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from gastric cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in gastric cancer cells may be any one selected from the group consisting of *ACPP(NCBI Gene ID: 55), FLU3(Gene ID: 837968), CGB(NCBI Gene ID: 1082), KRT19(NCBI Gene ID: 3880), PARP1(NCBI Gene ID: 142), FOXO3A(NCBI Gene ID: 2309), MED30(NCBI Gene ID: 90390), ERBB2(NCBI Gene ID: 2064), CCNE1(NCBI Gene ID: 898), MYC(NCBI Gene ID: 4609), EGFR(NCBI Gene ID: 1956), KRAS(NCBI Gene ID: 3845), TFF1(NCBI Gene ID: 7031), FABP1(NCBI Gene ID: 2168), CK20(NCBI Gene ID: 54474), MUC2(NCBI Gene ID: 4583), SDC2(NCBI Gene ID: 6383), LAMP5(NCBI Gene ID: 24141), MATN3(NCBI Gene ID: 4148), CLIP4(NCBI Gene ID: 79745), NOX4(NCBI Gene ID: 50507), ADRA2C(NCBI Gene ID: 152), CSK(NCBI Gene ID: 1445), FZD9(NCBI Gene ID: 8326), GALR1(NCBI Gene ID: 2587), GRM6(NCBI Gene ID: 2916), INSR(NCBI Gene ID: 3643), LPHN1(NCBI Gene ID: 22859), LYN(NCBI Gene ID: 4067), MRGPRX3(NCBI Gene ID: 117195), ADCY3(NCBI Gene ID: 109), HDAC2(NCBI Gene ID: 3066), CFL1(NCBI Gene ID: 1072), COTL1(NCBI Gene ID: 23406), NRP2(NCBI Gene ID: 8828), ANXA10(NCBI Gene ID: 11199), TFF2(NCBI Gene ID: 7032), CDCA5(NCBI Gene ID: 113130), ATAD2(NCBI Gene ID: 29028), ASB9(NCBI Gene ID: 140462), MMP1(NCBI Gene ID: 4312), CEACAM6(NCBI Gene ID: 4680), DSCC1(NCBI Gene ID: 79075), CKS2(NCBI Gene ID: 1164), CST1(NCBI Gene ID: 1469), IFITM1(NCBI Gene ID: 8519), NUSAP1(NCBI Gene ID: 51203), MELK(NCBI Gene ID: 9833), LGALS3BP(NCBI Gene ID: 3959), CPT1A(NCBI Gene ID: 1374), IFNG(NCBI Gene ID: 3458), CD274(NCBI Gene ID: 29126), FOLR1(NCBI Gene ID: 2348), EPCAM(NCBI Gene ID: 4072), CEACAM5(NCBI Gene ID: 1048),* and a combination thereof.

In one embodiment, the gene specifically present in gastric cancer may be *CGB, PARP1, FOXO3A, MED30, CCNE1, MYC, TFF1, FABP1, LAMP5, MATN3, CLIP4, NOX4, ADRA2C, CSK, FZD9, GALR1, GRM6, INSR, LPHN1, LYN, MRGPRX3, ADCY3, HDAC2, CFL1, NRP2, ANXA10, TFF2, CDCA5, NUSAP1* genes, and additionally, *ACPP, FLU3, KRT19, ERBB2, EGFR, KRAS, DSCC1, CK20, MUC2, SDC2, COTL1, ATAD2, ASB9, MMP1, CEACAM6, DSCC1, CKS2, CST1, IFITM1, MELK, LGALS3BP, CPT1A, IFNG, CD279, CD274, ERBB2, EGFR, FOLR1,* or *EPCAM* gene may be additionally detected to diagnose gastric cancer.

As used herein, the term *"CGB"* refers to a gene encoding a hCG (human chorionic gonadotropin) hormone. Some cancers also produce a hCG hormone. Therefore, if the hCG hormone level measured when the patient is not pregnant is high, it can be diagnosed with cancer.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from gastric cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Gastric Cancer Diagnostic Kit

Other aspect of the present invention provides a diagnostic kit for diagnosing gastric cancer, comprising a probe to which biotin is bound complementarily binding to a gene specifically expressed in gastric cancer; a positively charged material; a marker to which an avidin-based protein is bound; and a manual.

In this case, the gene specifically expressed in gastric cancer may be any one or more selected from the group consisting of *CGB, PARP1, FOXO3A, MED30, CCNE1, MYC, TFF1, FABP1, LAMP5, MATN3, CLIP4, NOX4, ADRA2C, CSK, FZD9, GALR1, GRM6, INSR, LPHN1, LYN, MRGPRX3, ADCY3, HDAC2, CFL1, NRP2, ANXA10, TFF2, CDCA5, NUSAP1,* and a combination thereof.

In addition, the manual may describe that the kit is configured to be capable of diagnosing gastric cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *ACPP, FLU3, KRT19, ERBB2, EGFR, KRAS, DSCC1, CK20, MUC2, SDC2, COTL1, ATAD2, ASB9, MMP1, CEACAM6, DSCC1, CKS2, CST1, IFITM1, MELK, LGALS3BP, CPT1A, IFNG, CD279, CD274, ERBB2, EGFR, FOLR1, EPCAM,* and a combination thereof.

In addition, the probe, the positively charged material, and the marker are as described above.

### Gastric Cancer Diagnostic Device

Other aspect of the present invention provides a device for diagnosing gastric cancer by detecting a gene derived from gastric cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in gastric cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from gastric cancer in the sample depending on whether the marker is detected.

In this case, the gene specifically expressed in gastric cancer may be any one or more selected from the group consisting of *CGB, PARP1, FOXO3A, MED30, CCNE1, MYC, TFF1, FABP1, LAMP5, MATN3, CLIP4, NOX4, ADRA2C, CSK, FZD9, GALR1, GRM6, INSR, LPHN1, LYN, MRGPRX3, ADCY3, HDAC2, CFL1, NRP2, ANXA10, TFF2, CDCA5, NUSAP1,* and a combination thereof.

In addition, it may further comprise a probe to which biotin is bound complementarily binding to at least any one gene selected from the group consisting of *ACPP, FLU3, KRT19, ERBB2, EGFR, KRAS, DSCC1, CK20, MUC2, SDC2, COTL1, ATAD2, ASB9, MWP1, CEACAM6, DSCC1, CKS2, CST1, IFITM1, MELK, LGALS3BP, CPT1A, IFNG, CD279, CD274, ERBB2, EGFR, FOLR1, EPCAM,* and a combination thereof.

### <Pancreatic Cancer>

### Method for Diagnosing Pancreatic Cancer

One aspect of the present invention provides a method for diagnosing pancreatic cancer by detecting a gene derived from pancreatic cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a pancreatic cancer biomarker. In this case, a gene known as a pancreatic cancer biomarker may be a gene encoding a protein overexpressed in pancreatic cancer.

Specifically, the probe having a sequence complementary to cfDNA may complementarily bind to at least any one gene selected from the group consisting of *SMAD4, APC, GNAS,* and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin- streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of pancreatic cancer cells. Specifically, cfDNA may include a nucleic acid sequence overexpressed in pancreatic cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from pancreatic cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in pancreatic cancer cells may be any one selected from the group consisting of *KRAS(NCBI Gene ID: 3845), SMADA4(NCBI Gene ID: 4089), APC(NCBI Gene ID: 324), GNAS(NCBI Gene ID: 2788), MUC1(NCBI Gene ID: 4582), CEACAM5(NCBI Gene ID: 1048), CEACAM1(NCBI Gene ID: 634), MUC16(NCBI Gene ID: 94025),* and a combination thereof.

In one embodiment, the gene specifically present in pancreatic cancer may be *SMAD4, APC, GNAS* genes, and additionally, *KRAS, MUC1, MSLN, CEACAM1, CEACAM5* or *MUC16* gene may be additionally detected to diagnose pancreatic cancer.

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from pancreatic cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Pancreatic Cancer Diagnostic Kit

Other aspect of the present invention provides a diagnostic kit for diagnosing pancreatic cancer, comprising a probe to which biotin is bound complementarily binding to a gene specifically expressed in pancreatic cancer; a positively charged material; a marker to which an avidin-based protein is bound; and a manual.

In this case, the gene specifically expressed in pancreatic cancer may be any one or more selected from the group consisting of *SMAD4, APC, GNAS,* and a combination thereof.

In addition, the manual may describe that the kit is configured to be capable of diagnosing pancreatic cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *KRAS, MUC1, MSLN, CEACAM1, CEACAM5* and *MUC16*, and a combination thereof.

In addition, the probe, the positively charged material, and the marker are as described above.

### Pancreatic Cancer Diagnostic Device

Other aspect of the present invention provides a device for diagnosing pancreatic cancer by detecting a gene derived from pancreatic cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in pancreatic cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from pancreatic cancer in the sample depending on whether the marker is detected.

In this case, the gene specifically expressed in pancreatic cancer may be any one or more selected from the group consisting of *SMAD4, APC, GNAS,* and a combination thereof.

In addition, it may further comprise a probe to which biotin is bound complementarily binding to at least any one gene selected from the group consisting of *KRAS, MUC1, MSLN, CEACAM1, CEACAM5* and *MUC16*, and a combination thereof.

### <Early Diagnosis and Prognosis Prediction>

### Method for Diagnosing Cancer

One aspect of the present invention provides a method for early diagnosing cancer or predicting the prognosis of cancer by detecting a gene derived from cancer cells from a sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the probe having a sequence complementary to cfDNA complementarily binds to a gene known as a cancer biomarker.

Specifically, the probe having a sequence complementary to cfDNA may complementary bind to at least any one gene selected from the group consisting of *CPT1A, IFNG, IFNGR1, CD279, CD274,* and a combination thereof. In this case, preferably, two or more genes selected from the above group may be combined.

The cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, lymphoma, acute leukemia, multiple myeloma, and a combination thereof.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin-streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of cancer cells. Specifically, cfDNA may include a nucleic acid sequence overexpressed in cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value may be about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

The gene overexpressed in cancer cells may be any one selected from the group consisting of *FNG* (NCBI Gene ID: 3458), *IFNGR1* (NCBI Gene ID: 3459), *CD279* (NCBI Gene ID: 5133) and *CD274* (NCBI Gene ID: 29126), and a combination thereof. In one embodiment, when two genes overexpressed in cancer cells are combined, the combination may be IFNG/ IFNGR1, IFNG/CD274, or IFNG/CD279. When three genes overexpressed in cancer cells are combined, the combination may be IFNG/IFNGR1/CD274, IFNG/CD274/CD279, or IFNGR1/CD274/CD279. When four genes overexpressed in cancer cells are combined, the combination may be INFG/IFNGR1/CD274/CD279. When two or more genes overexpressed in cancer cells are analyzed, the reliability of the analysis results may be enhanced.

As used herein, the term "IFNG" refers to a gene encoding interferon gamma.

As used herein, the term "*IFNGR1*" refers to a gene encoding interferon gamma receptor 1.

As used herein, the term "*CD274*" refers to a gene encoding PD-L1 (programmed death-ligand 1).

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Cancer Diagnostic Kit

Other aspect of the present invention provides a diagnostic kit for early diagnosing cancer or predicting the prognosis of cancer, comprising a probe to which biotin is bound complementarily binding to a gene specifically expressed in cancer; a positively charged material; a marker to which an avidin-based protein is bound; and a manual.

In this case, the gene specifically expressed in cancer may be any one or more selected from the group consisting of *CPT1A, IFNG, IFNGR1, CD279, CD274,* and a combination thereof.

In addition, the manual may describe that the kit is configured to be capable of early diagnosing cancer or predicting the prognosis of cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, the probe, the positively charged material, and the marker are as described above.

### Cancer Diagnostic Device

Other aspect of the present invention provides a device for early diagnosing cancer or predicting the prognosis of cancer by detecting a gene derived from cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from cancer in the sample depending on whether the marker is detected.

In this case, the gene specifically expressed in cancer may be any one or more selected from the group consisting of *CPT1A, IFNG, IFNGR1, CD279, CD274,* and a combination thereof.

### <Cancer>

### Method for Confirming Cancer, Metastasis of Cancer, and Resistance of Cancer to Drugs

One aspect of the present invention is a method for determining the presence or absence of cancer, metastasis state of cancer, and/or resistance by detecting a gene derived from cancer cells from the sample without amplification, wherein the method comprises a) a step of mixing a biological sample isolated from an individual comprising cell-free DNA (hereinafter cfDNA) and a positively charged material; b) a step of isolating the positively charged material to which cfDNA is bound; c) a step of sequentially or simultaneously mixing a probe having a sequence complementary to the cfDNA and a marker in the mixture; d) a step of removing the probe that does not bind to cfDNA and the marker; and e) a step of detecting the marker, wherein the cfDNA is derived from cancer cells, and the probe having a sequence complementary to cfDNA complementarily binds to a gene known as an indicator of the presence or absence of cancer and metastasis of cancer or a resistance biomarker, and the method determines the presence or absence of metastasis state or resistance of cancer. In this case, the gene known as an indicator of the presence or absence of cancer and metastasis of cancer or a resistance biomarker may be a gene including single nucleotide polymorphism (snp) in cancer (see Example 6 and Example 7).

Specifically, the probe having a sequence complementary to cfDNA may complementarily bind to at least any one gene selected from the group consisting of *CPT1A, IFNG, IFNGR1, CD279, CD274,* and a combination thereof.

The probe having a sequence complementary to cfDNA may complementarily bind to a gene overexpressed in cancer cells, a gene specifically present in cancer, a gene related to metastasis, or a gene related to drug resistance.

In one embodiment, the gene overexpressed in cancer cells may be any one selected from the group consisting of *CPT1A, IFNG, IFNGR1, CD279, CD274, NSE, SCC, CEA, cyfra21-1, TPA, NMP22, OGT, Thyroglobulin(TG), Calcitonin(CALCA), BRAF V600E, TERT C228T*/*C250T, AFP, β-HCG(CGB). CA19-9, PSA, PSMA, PAP, PCA3, TMPRSS2-ERG, CA125, HIF-1a, VEGF, CA15-3, HER2, SCC(SART3), TOP2A, MCM2, p16INK4a(CDKN2A), Ki-67(MKI167), HE4(WEDC2),* and a combination thereof.

In one embodiment, the gene specifically present in cancer may be *CPT1A, IFNG, IFNGR1, CD279, CD274* genes, and additionally, *NSE, SCC, CEA, cyfra21-1, TPA, NMP22, OGT, Thyroglobulin(TG), Calcitonin(CALCA), BRAF V600E, TERT C228T*/*C250T, AFP, β-HCG(CGB). CA19-9, PSA, PSMA, PAP, PCA3, TMPRSS2-ERG, CA125, HIF-1a, VEGF, CA15-3, HER2, SCC(SART3), TOP2A, MCM2, p16INK4a(CDKN2A), Ki-67(MKI167),* or *HE4(WEDC2)* gene may be additionally detected to diagnose cancer.

In one embodiment, the gene related to the metastasis of cancer may be genes related to "proliferation" and "invasion", and may be, for example, Ki67, STK15, Survivin, Cyclin B1, MYBL2, Stromelysin3, or Cathepsin L2.

In one embodiment, the gene related to the drug resistance may be determined depending on the drug and the type of cancer. In one embodiment, the gene related to acquired resistance to EGFR-TKI may be any one selected from the group consisting of EGFR T790M, PI3K, BRAF, MAPK1, HER2, KRAS, NRAS, RB deletion, p53 deletion, PTEN, and NFkB.

In addition, the purpose of using the isolated biological sample is to detect cfDNA present in the sample. Therefore, cfDNA in a sample may be used by being isolated and/or concentrated in various ways. In one embodiment, a nitrocellulose membrane having a strong affinity for nucleic acid may be used. In addition, in one embodiment, a positively charged material may be used to capture a negatively charged cfDNA. The positively charged material may be a nanoparticle, a nanowire, a network, or a positively charged filter, but is not limited thereto. In one embodiment of the "positively charged material", it may be a positively charged nanostructure or a positively charged membrane.

In one example of the nanostructure, it may comprise a cationic polymer. The kind of the cationic polymer is not limited. One embodiment of the cationic polymer may be polyethyleneimine (PEI), and may be a cationic branched polymer polyethyleneimine.

In addition, by mixing nanowires labeled with streptavidin with a solution of PEI to which biotin is bound, cationic branched polyethyleneimine (cationic branched PEI) may be further bound to the nanowires through the interaction of biotin- streptavidin. As a result, the nanoparticles in the nanostructures (PEI/mPpy NWs) with polyethyleneimine, a cationic polymer, bound to the surface of the nanostructures may be incorporated with high density and irregular distribution.

These nanowires can successfully capture genomic DNA and cfDNA with high efficiency even at a low concentration. In particular, due to the characteristics of the nanowires such as the large surface area for binding with target molecules such as DNA and the enhanced mobility for promoting interaction with DNA, it is possible to efficiently and effectively capture the target cfDNA.

In this case, the target cfDNA refers to the desired cfDNA to be detected. In the present specification, cfDNA has double strands. In this case, in a portion of the cfDNA, double strands may be unwound. In addition, the cfDNA may be derived from a gene of cancer cells. Specifically, cfDNA may include a nucleic acid sequence overexpressed in cancer cells. The nucleic acid sequence that is overexpressed in the cancer cells refers to a nucleic acid sequence that is overexpressed in specific cancer cells, although it has an appropriate expression level in normal cells.

Specifically, the level or reference (cutoff) of the nucleic acid sequence overexpressed in cancer cells may be a case where the OD value is 0.010 or more when the absorbance (optical density) is measured using a marker. More specifically, in the level or reference of the nucleic acid sequence overexpressed in cancer cells, an OD value is about 0.012 or about 0.015 or more by measuring an absorbance using a marker. In this case, the wavelength irradiated for measuring the absorbance may be appropriately determined according to a marker. The cfDNA may be DNA in which double strands are unwound (unwinding of DNA). In addition, cfDNA may be appropriately determined according to the purpose.

In addition, the cfDNA derived from cancer cells may be characterized by i) having a lower Tm value than that of cfDNA having a double helix structure derived from normal cells, or ii) being denatured in a condition in which the cfDNA having a double helix structure derived from normal cells is not denatured.

In addition, the cfDNA is capable of binding with a about 15-mer to about 30-mer probe that can complementarily bind to cfDNA in any one of the following conditions: i) a condition of allowing to stand for about 1 minute to about 120 minutes at ambient temperature; ii) a condition of heating at about 90 °C to about 95 °C for about 1 second to about 3 minutes; iii) a condition of heating at about 75 °C to about 90 °C for about 1 second to about 5 minutes; iv) a condition of heating at about 60 °C to about 75 °C for about 30 seconds to about 30 minutes; v) a condition of heating at about 25 °C to about 40 °C for about 10 minutes to about 120 minutes; vi) a condition of treating with protease for about 1 minute to about 30 minutes; vii) a condition of treating with DNase for about 1 minute to about 30 minutes; and viii) a condition of treating with a chemical substance (for example, sodium hydroxide, DMSO, a surfactant, and the like).

As used herein, the term "probe" refers to DNA or RNA for detecting cfDNA. The probe may have a specific sequence to be capable of complementarily binding to cfDNA. The probe having a sequence complementary to cfDNA refers to a probe having a nucleic acid sequence capable of complementarily binding to a desired double-stranded cfDNA to be detected present in plasma. In this case, the probe may be one to which biotin is bound. The probe may be bound to a marker that is bound to a biotin-binding protein.

As used herein, the term "marker" refers to a material that is used for detecting a probe bound to cfDNA. The marker may be a nanoparticle, a fluorescent dye, a fluorescent protein, or an enzyme. Specifically, the marker may be any one selected from the group consisting of a quantum dot, an HRP and a fluorescent protein. In one embodiment, the marker may be GFP (green fluorescent protein), BFP (blue fluorescent protein), CFP (cyan fluorescent protein), YFP (yellow fluorescent protein), or HRP (horse radish peroxidase), but is not limited thereto.

The marker may be one to which a biotin-binding protein is bound. The biotin-binding protein may be an avidin-based protein, avidin, streptavidin, traptavidin, or neutravidin, but may be used without limit as long as it is a protein that can specifically bind to biotin. In one embodiment, the marker may be one to which streptavidin is bound.

As used herein, the term "streptavidin" is a tetrameric biotin-binding protein with a molecular weight of about 60 kDa isolated from Streptomyces avidinii. It has very low homology to the avidin, but its structure is very similar to the avidin. Like avidin, it has an antibacterial activity and a very high binding capacity to biotin. On the other hand, unlike avidin, it does not contain carbohydrates, has an acidic isoelectric point (pI=5), and has a significantly lower solubility than avidin. A commercially available streptavidin such as Thermo Scientific Pierce Streptavidin is in a recombinant form of streptavidin having a molecular weight of about 53 kDa and has an isoelectric point which is near neutral (pI=6.8 to 7.5). The streptavidin having the lack of glycosylation and low pI has a low level of non-specific binding (in particular, lectin binding) as compared to avidin. These properties of streptavidin allow it to be selected as an ideal reagent for many detection systems.

As used herein, the term "traptavidin" refers to a mutant (variant or mutein) of streptavidin, and is a protein having a dissociation rate for biotin that is slower by about 10 times and having an increased mechanical strength and enhanced thermal stability. In addition, the traptavidin specifically binds to biotin.

The term "neutravidin" of the present invention is also referred to as "deglycosylated avidin" and is prepared to avoid the main disadvantages of naturally occurring avidin and streptavidin. As shown in the name, neutravidin is produced by deglycosylating avidin. It is a protein that has a reduced molecular weight (about 60 kDa) and maintains a high biotin binding capacity as compared to avidin. The deglycosylation of the avidin reduces the binding of lectin to an undetectable level and lowers an isoelectric point (pI= about 6.3) to effectively eliminate the main cause of non-specific binding to avidin. A lysine residue remains usable, so it can be easily derivatized or complexed like streptavidin. In addition, since it exhibits high biotin binding capacity and low non-specific binding, it can be used in various ways as an ideal biotin-binding protein.

As used herein, the term "a step of detecting the marker" refers to a step of detecting a marker that is bound to a probe through a reaction of biotin-avidin. The detection of the marker can be measured by a color change, a UV absorbance change, the presence or absence of bioluminescence, a fluorescence change, or an electrochemical change. Specifically, the method for detecting the marker may be performed differently depending on the marker used. For example, when HRP is used as a marker, the marker can be detected by observing the color reaction through the reaction between hydrogen peroxide and a substrate. In addition, when the marker is a fluorescent protein such as GFP, the presence or absence of the marker can be detected by observing the detected light after irradiating light of a specific wavelength. In addition, when the marker is luciferase, the presence of the marker can be detected by measuring the bioluminescence that is exhibited after adding a substrate such as luciferin by bioluminometer.

In addition, the diagnostic method of the present invention may further comprise a step of denaturing cfDNA. In this case, the denaturation step can be performed to selectively denature only cfDNA derived from cancer, but not to denature normal double-stranded cfDNA. To this end, the conditions of the denaturation step may be performed at about 50 °C to about 100 °C for about 0.1 second to about 5 minutes. One embodiment of the denaturation temperature may be about 95 °C, and the denaturation time may be usually about 0.1 second to about 8 minutes. In addition, it may be denatured for about 1 second, about 5 seconds, about 10 seconds, about 30 seconds, about 60 seconds, or about 90 seconds. In one embodiment, the step of denaturing cfDNA may be performed prior to step c) above.

Specifically, the method may further comprise a step of denaturing the sample or cfDNA bound to the positively charged material prior to step c) above in any one condition selected from the group consisting of i) a condition of allowing to stand for about 1 minute to about 10 minutes at ambient temperature; ii) a condition of heating at about 90 °C to about 95 °C for 1 second to 1 minute; iii) a condition of heating at about 75 °C to about 90 °C for about 10 seconds to about 3 minutes; iv) a condition of heating at about 60 °C to about 75 °C for about 1 minute to about 30 minutes; v) a condition of heating at about 25 °C to about 40 °C for about 5 minutes to about 60 minutes; vi) a condition of treating with protease for about 1 minute to about 10 minutes; and vii) a condition of treating with DNaseI for about 1 minute to about 10 minutes. This denaturation step does not denature the double-stranded cfDNA derived from normal cells, but selectively denatures only cfDNA derived from cancer cells, thereby making it easier to bind with the probe. The denaturation conditions of i) to vii) may be performed after obtaining a sample. In addition, the denaturation conditions of i) to vii) may be performed after obtaining cfDNA bound to the positively charged material. In addition, the temperature, protease, and DNase treatment time of the denaturation conditions of i) to vii) may be appropriately adjusted as long as they do not denature the stable cfDNA.

### Cancer Diagnostic Kit

Other aspect of the present invention provides a diagnostic kit for diagnosing cancer, comprising a probe to which biotin is bound complementarily binding to a gene specifically expressed in cancer; a positively charged material; a marker to which an avidin-based protein is bound; and a manual.

In this case, the gene specifically expressed in cancer may be any one or more selected from the group consisting of *CPT1A, IFNG, IFNGR1, CD279, CD274G,* and a combination thereof.

In addition, the manual may describe that the kit is configured to be capable of diagnosing cancer by the following protocol: a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

In addition, it may further comprise a probe to which biotin is bound complementarily binding to at least any one gene selected from the group consisting of *NSE, SCC, CEA, cyfra21-1, TPA, NMP22, OGT, Thyroglobulin(TG), Calcitonin(CALCA), BRAF V600E, TERT C228T*/*C250T, AFP, β-HCG(CGB). CA19-9, PSA, PSMA, PAP, PCA3, TMPRSS2-ERG, CA125, HIF-1a, VEGF, CA15-3, HER2, SCC(SART3), TOP2A, MCM2, p16INK4a(CDKN2A), Ki-67(MKI167), HE4(WEDC2),* and a combination thereof.

In addition, the probe, the positively charged material, and the marker are as described above.

### Cancer Diagnostic Device

Other aspect of the present invention provides a device for diagnosing cancer by detecting a gene derived from cancer cells from a sample without amplification, wherein the device comprises a) a mixing part for mixing a biological sample isolated from an individual comprising cfDNA and a positively charged material; b) an obtaining part for removing the sample except for the positively charged material to which cfDNA is bound; c) a reaction part for adding sequentially or simultaneously a probe to which biotin is bound capable of complementarily binding to the gene specifically expressed in cancer; and a nanoparticle comprising streptavidin and a marker to the positively charged material to which cfDNA is bound; d) a detection part for detecting the marker; and e) an information processing part for determining whether there is cfDNA having a sequence complementary to the probe and derived from cancer in the sample depending on whether the marker is detected.

In this case, the gene specifically expressed in cancer may be any one or more selected from the group consisting of *CPT1A, IFNG, IFNGR1, CD279, CD274,* and a combination thereof.

In addition, it may further comprise a probe to which biotin is bound complementarily binding to at least any one gene selected from the group consisting of *NSE, SCC, CEA, cyfra21-1, TPA, NMP22, OGT, Thyroglobulin(TG), Calcitonin(CALCA), BRAF V600E, TERT C228T*/*C250T, AFP, β-HCG(CGB). CA19-9, PSA, PSMA, PAP, PCA3, TMPRSS2-ERG, CA125, HIF-1a, VEGF, CA15-3, HER2, SCC(SART3), TOP2A, MCM2, p16INK4a(CDKN2A), Ki-67(MKI167), HE4(WEDC2),* and a combination thereof.

### Mode for Carrying out the Invention

Hereinafter, the present invention will be described in more detail through the working examples below. However, the following examples are provided only for the purpose of illustration of the present invention, but the scope of the present invention is not limited to the following examples.

### Experimental Method

### 1. Method for Detecting cfDNA Derived from Tumor Specific Gene

### Step 1: Sample Preparation and Nanowire Addition

Immediately after obtaining plasma, urine, saliva, sputum, or the like of patients, it was centrifuged at 4 °C, 3000 × g, for 10 minutes. The plasma, urine, saliva, sputum, or the like of patients was diluted in DPBS at a given ratio. In the case of plasma, 1 µL or 30 µL of plasma was mixed in 150 µL of DW and put in a spin column (Type G or Type Q), and PEI/Ppy nanowire (150 µL) was added and mixed for 20 minutes at a speed of 1,200 rpm at room temperature using a thermomixer.

### Step 2: Vacuum/Washing/Temperature Denaturation

The spin column was mounted on a vacuum suction device, and then suction was performed at 550 mBar. 400 µL of 1x DPBS was added and suction was performed again. The same process was repeated once more. Only the nanowire-DNA complex obtained through the 2-step step was captured in the spin column. When temperature denaturation was required, the spin column in which suction was completed was put in a heating block preheated to 95 °C, and incubation was performed at 95 °C for 1 minute, and then it was taken out immediately. Samples that do not require a temperature denaturation step were not subjected to this process.

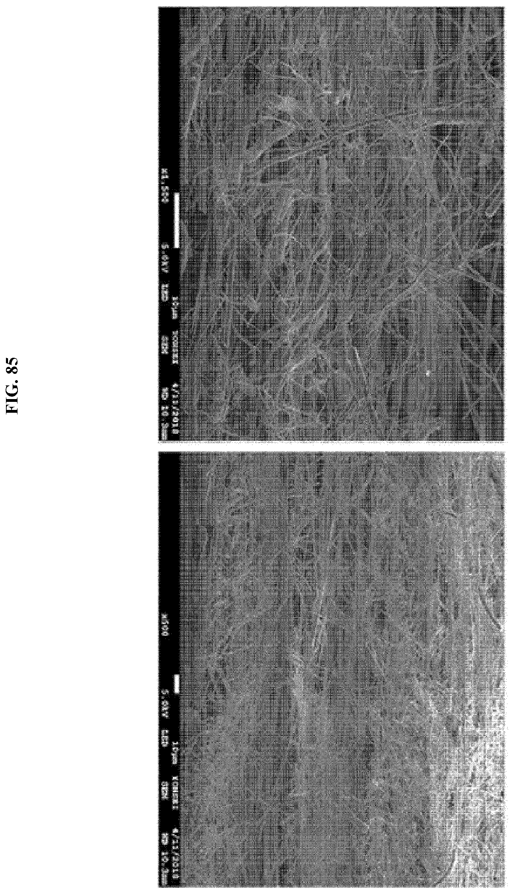

Figure 85 shows the separation of cfDNA through a spin column using a nanowire that did not contain magnetic nanoparticles (Figure 85). The upper picture is the SEM image of the spin column before centrifugation, and the lower picture is the SEM image of the spin column with cfDNA separated after centrifugation.

### Step 3: Addition of Probe and HRP/STR NPs

A probe (200 µL) which was compatible to the experiment and HRP/STR NPs solution (200 µL) were added to a spin column, respectively. It was mixed for 30 minutes at a speed of 850 rpm to 1,000 rpm at room temperature using a thermomixer. The spin column was mounted on a vacuum device, and then suction was performed. 400 µL of 1x DPBS was added and suction was performed again. The same process was repeated once more.

### Step 4: TMB Reaction for Detecting Gene Mutation

After replacing the collection tube with a new one, 200 µL of sodium acetate buffer (0.2 M, pH 7.0), 50 µL of H2O2 (0.1 M) were sequentially added to a spin column using a syringe pump, and then incubation was performed for 3 minutes. At the end of the incubation, the spin column was centrifuged at a speed of 3,500 rpm to 5,000 rpm for 30 seconds. 200 µL of the solution collected in the collection tube was transferred to each of 96 wells, and then the absorbance was measured in a wavelength range of 500 nm to 850 nm using a UV/VIS spectrophotometer.

### Overview of Detection Method

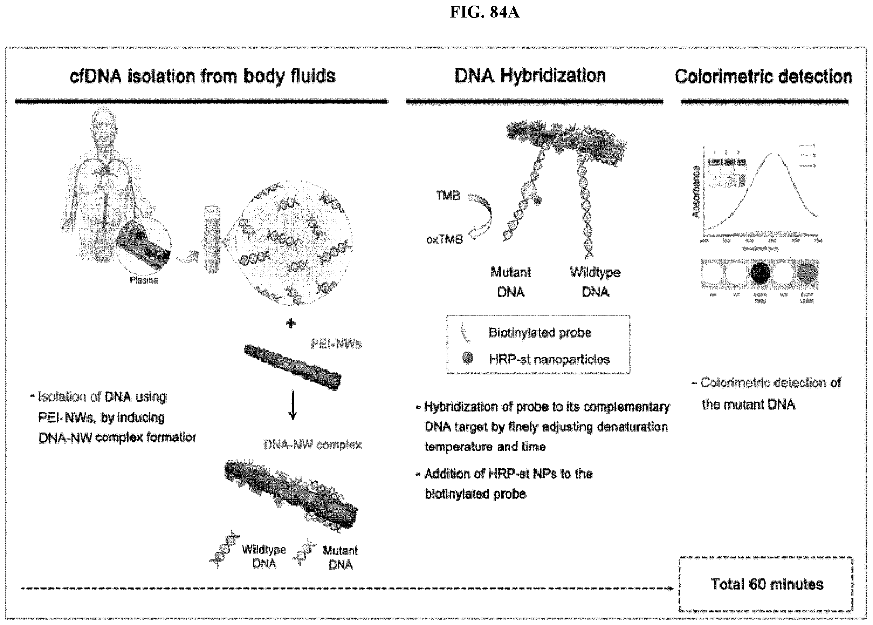

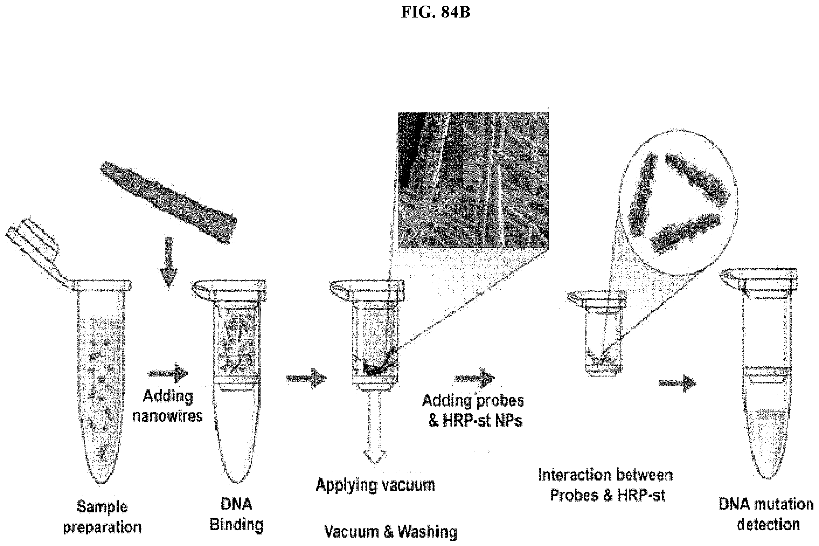

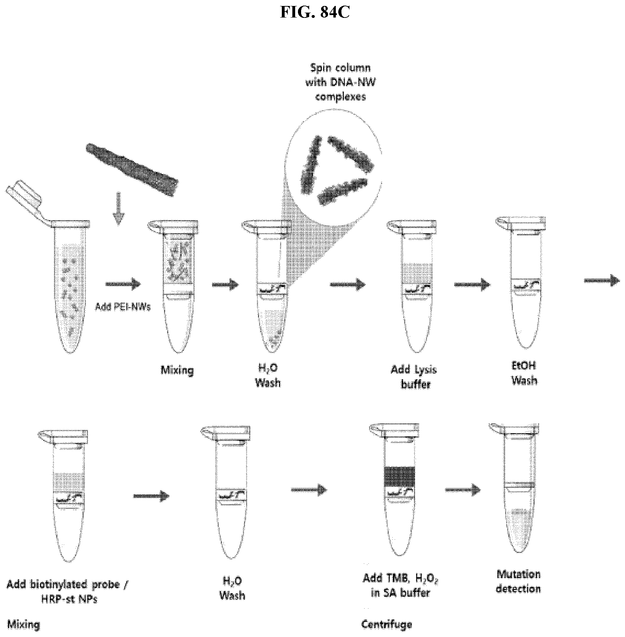

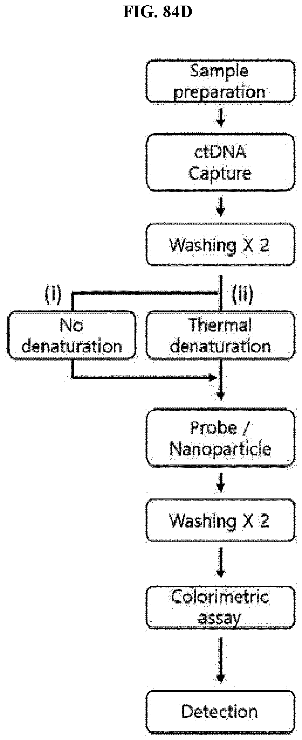

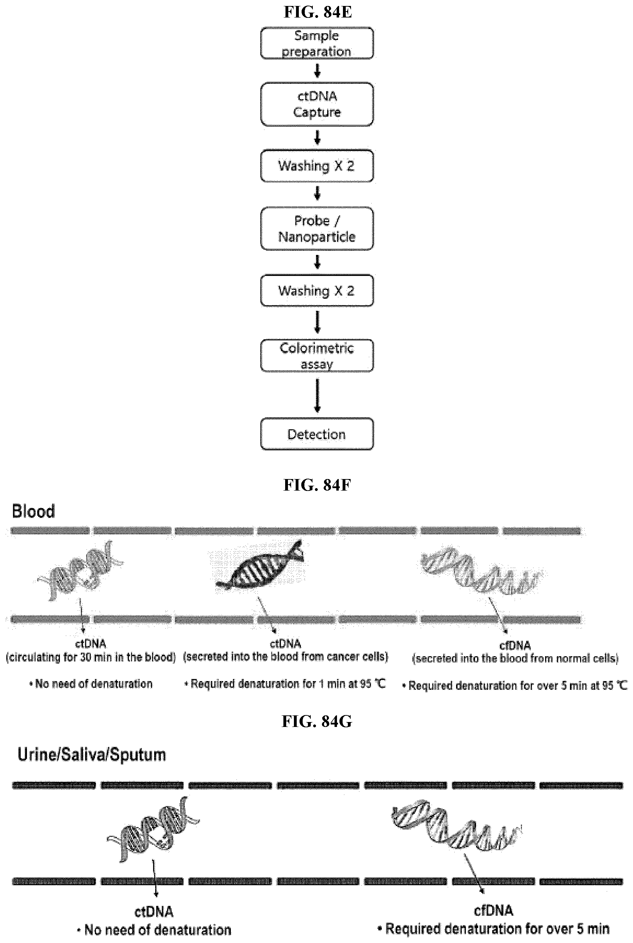

The detection step of the present invention is illustrated in Figures 84a to 85g. Figure 84A shows a schematic diagram of the method for analyzing a gene derived from cancer cells within 60 minutes through the reaction with a probe complementarily binding to a target cfDNA and HRP/streptavidin-nanoparticle (HRP/st-tagged NP) after obtaining cfDNA from the patient's body fluid, using the polypyrrole nanowires (PEI/Ppy NWs) with polyethyleneimine (PEI) bound to the surface of the nanowires. Figure 84B is a schematic view showing a method for detecting cfDNA derived from cancer cells using the nanowires, the probe, and the HRP/streptavidin nanoparticles (Figure 84B). Figure 84C is a figure showing a process of detecting a gene derived from cancer cells through a spin column using the nanowires (Figure 84C). In addition, in one embodiment of the present invention, a step of treating with a lysis buffer may be further included. Figure 84D shows a time series flow diagram of the method for detecting cfDNA derived from cancer cells in a sample such as blood, cerebrospinal fluid, or pleural fluid (Figure 84D). Figure 84E shows a time series flow diagram of the method for detecting cfDNA derived from cancer cells in a sample such as urine (Figure 84E). Figure 84F is a schematic view showing the difference in denaturation conditions according to the state of cfDNA obtained from the blood (Figure 84F). Figure 84G is a schematic view showing the difference in denaturation conditions according to the state of cfDNA obtained from the urine, saliva, and sputum (Figure 84G).

### Preparation Example 1. Preparation of Nanowires Surface-Treated with Cationic Polymers

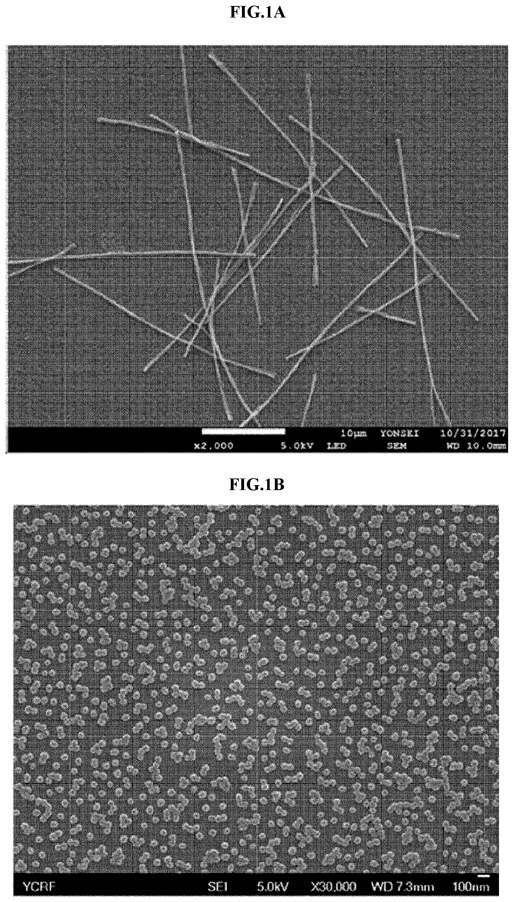

As shown in Figure 1A, a nanowire with polyethyleneimine (PEI), a cationic polymer, conjugated to the surface of the nanowire, was prepared. One surface of an anodic aluminium oxide (AAO) was coated with a gold (Au) layer (a thickness of about 150 nm) for 600 seconds at 5×10⁻³ mbar and 50 mA using the Q150T Modular Coating System (Quorum Technologies, UK). All electrochemical experiments were measured on a gold (Au)-coated AAO template by using the potentiostat/galvanostat (BioLogic SP-150) equipped with a platinum wire counter electrode and an Ag/AgCl (3.0 M NaCl type) reference electrode.

In order to prepare nanowires (PEI/Ppy NWs) surface-treated with cationic polymers, along with 0.01 M poly(4-styrene sulfonic acid) and a 0.01 M pyrrole solution containing 1 mg/ml biotin, chronoamperometry at 1.0 V (vs. Ag/AgCl) was applied into the pores of the AAO template for 7 minutes to perform an electrochemical deposition.

The resulting AAO template was washed several times with distilled water, dipped in a 2 M sodium hydroxide (NaOH) solution for 3 hours, and then put in Bioruptor UCD-200 (Diagenode) for sonication to obtain free-standing poly(pyrrole) nanowire (free-standing Ppy NWs) doped with biotin molecules. Then, 30 mM N-(3-dimethylaminopropyl)-N-ethylcarbodiimide hydrochloride (EDC) and 6 mM N-hydroxysuccinimide (NHS) were added to the resulting nanowire to activate a carboxylic acid group (-COOH). Thereafter, a PEI solution was added, then reacted for 1 hour at ambient temperature, and washed with water to obtain nanostructures (PEI/Ppy NW) on the surface of which polyethyleneimine was conjugated. The obtained nanostructures (PEI/Ppy NW) were dispersed in deionized water and stored at room temperature until use.

Through such preparation method, each of poly(pyrrole) (Ppy) nanowires was released from the AAO template after the AAO template was selectively dissolved, and cationic branched polyethyleneimine (cationic branched PEI, 25 kDa) was additionally conjugated to the nanowire through a biotin-streptavidin interaction.

### Preparation Example 2. Preparation of Nanowires Surface-Treated with Cationic Polymer

According to the method substantially the same as Preparation Example 1 above, the nanostructures (PL/Ppy NW) with polylysine conjugated to the surface of the nanostructures instead of polyethyleneimine were obtained.

### Preparation Example 3. Preparation of Poly(pyrrole) Nanoparticle Labeled with HRP and Streptavidin

In order to prepare nanoparticles to which HRP and streptavidin are bound, 0.5 g of polyvinylpyrrolidone (PVP) was added to 12.5 mL of tertiary distilled water, stirred for 30 minutes, and then 65 µL of pyrrole was added and further stirred for 10 minutes. Thereafter, 0.5 mL of a FeCl₃ solution at a concentration of 0.75 g/mL was added and reacted for 3 hours. Thereafter, 20 mL of an aqueous hyaluronic acid solution (400 mg/20 mL) was added and stirred for 3 hours to prepare poly(pyrrole)-hyaluronic acid nanoparticles (Ppy-HA-NPs).

MWCO: Dialysis in tertiary distilled water for 2 days using a 50,000 pore-sized membrane was performed. The large-sized particles aggregates were removed by centrifugation at 1,200 rpm for 3 minutes and then lyophilized. 200 µg of Ppy-HA-NPs prepared above were added to 1 mL of tertiary distilled water, and then a 100 mM EDC/50 mM NHS solution was added and reacted for 45 minutes to activate a carboxy group of hyaluronic acid. While the supernatant was removed by centrifugation at 15,000 rpm for 10 minutes, the washing was performed twice. 1 mg of HRP and 1 mg of streptavidin were added to Ppy-HA-NPs and mixed at 4 °C temperature. Thereafter, the supernatant was removed by centrifugation at 15,000 rpm for 10 minutes and then stored in tertiary distilled water. The morphology of the nanoparticle (HRP/st-tagged NP) with HRP and streptavidin conjugated to the surface of the nanoparticles was observed using a scanning electron microscope (Figure 1B).

### Preparation Example 4. Probe Construction

The probes were constructed to detect cfDNA having an unstable double helix structure. The probe constructs were differently constructed depending on cfDNA of the desired cancer type to be detected. In this case, biotin was bound to the probes. The specific nucleic acid sequence information of the probes is as shown in Tables 1, 5, 9 and 11 to 28 depending on the type of cancer to be diagnosed.

### I. Confirmation of Accuracy for Detection of Gene Derived from Cancer Cell Line

### Example 1. Detection of Gene Expressed in Cancer Cell Line

### Example 1.1. Confirmation of Accuracy for Detection of PD-L1

The accuracy for detection of PD-L1 was confirmed using MDA-MB-231, HCC827, H1975, PC9, and H460 cancer cell lines known as PD-L1 positive cancer cell lines and A549, MDA-MB-468, HeLa, and MCF7 cancer cell lines known as PD-L1 negative cancer cell lines obtained from ATCC and Korean Cell Line Bank. In this case, the PD-L1 positive cancer cell lines and PD-L1 negative cancer cell lines were classified based on the mRNA level of PD-L1 of each cancer cell line provided by CCLE (Cancer Cell Line Encyclopedia).

Specifically, genomic DNA was extracted from each of the PD-L1 positive cancer cell line and PD-L1 negative cancer cell line, followed by sonication to produce fDNA (fragmented DNA). 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated PD-L1 probe was added and reacted further for 20 minutes. In this case, the probes used are shown in Table 1 below.

**[Table 1]**

| | Sequence(5'-> 3') | Sequence ID NO. |
|---|---|---|
| biotinylated PD-L1 probe 1(Exon5) | TGGGAGCCATCTTATTATGCC | 1 |
| biotinylated PD-L1 probe 2(Exon5) | CGGAAGATGAATGTCAGTGC | 2 |
| biotinylated PD-L1 probe 3(Exon2) | CCTACTGGCATTTGCTGAACG | 3 |
| biotinylated PD-L1 probe 4(Exon3) | ACCATAGCTGATCATGCAGCG | 4 |

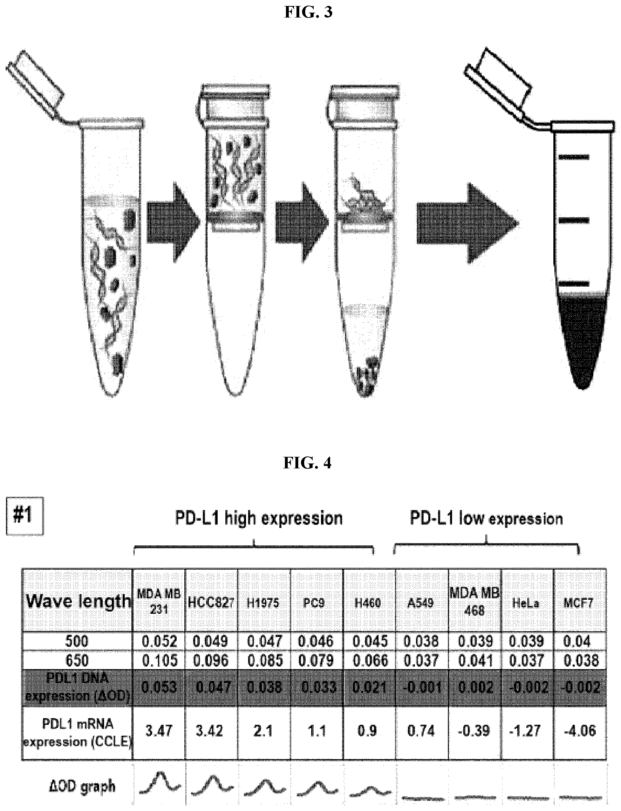

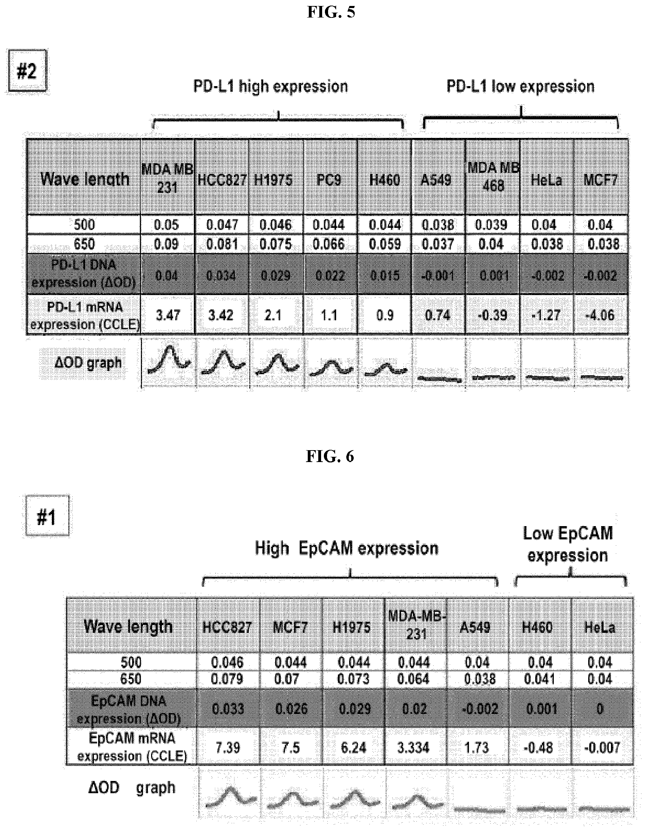

For colorimetric detection, it was treated with the solution in which TMB (3,3',5,5'-tetramethylbenzidine) and H₂O₂ were added to a sodium acetate buffer to confirm detection results. As a result of analyzing DNA-based PD-L1 expression (ΔOD) of each cancer cell line, in two repeated experiments, clear PD-L1 DNA expression (ΔOD; cutoff OD > 0.012) was observed only in fDNA of MDA-MB-231, HCC827, H1975, PC9, and H460 cancer cell lines. On the other hand, in two repeated experiments, PD-L1 DNA expression was not observed in A549, MDA-MB-461, HeLa, and MCF7 cancer cell lines (Figure 4 and Figure 5). In addition, the PD-L1 DNA expression result of each cancer cell line was compared to the PD-L1 (CD274) mRNA expression result of each cancer cell line obtained from CCLE (cancer cell line encyclopedia). The PD-L1 (CD274) mRNA value of each cancer cell line is shown in Table 2 below.

**[Table 2]**

| Cancer cell line | CD274 |
|---|---|
| MDAMB231_BREAST | 3.474506 |
| HCC827_LUNG | 3.426417 |
| NCIH1975_LUNG | 2.513606 |
| PC9_LUNG | 1.098854 |
| NCIH460_LUNG | 0.903786 |
| A549_LUNG | 0.735843 |
| MDAMB468_BREAST | -0.38864 |
| MCF7_BREAST | -4.06132 |

As a result, high mRNA expression was shown in MDA-MB-231, HCC827, H1975, PC9, and H460 cancer cell lines. On the other hand, mRNA expression was hardly shown in A549, MDA-MB-461, HeLa, and MCF7 cancer cell lines.

### Example 1.2. Confirmation of Accuracy for Detection of EpCAM

The accuracy for detection of EpCAM was confirmed using MDA-MB468, HCC827, MCF7, H1975, and MDA-MB-231 cancer cell lines known as EpCAM positive cancer cell lines and A549, H460, and HeLa cancer cell lines known as EpCAM negative cancer cell lines obtained from ATCC and Korean Cell Line Bank. In this case, the EpCAM positive cancer cell lines and EpCAM negative cancer cell lines were classified based on the mRNA level of EpCAM of each cancer cell line provided by CCLE (Cancer Cell Line Encyclopedia).

Specifically, genomic DNA was extracted from each of the EpCAM positive cancer cell line and EpCAM negative cancer cell line, followed by sonication to produce fDNA. 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated EpCAM probe was added and reacted further for 20 minutes. In this case, the probes used are shown in Table 3 below.

**[Table 3]**

| | Sequence(5'->3') | Sequence ID NO. |
|---|---|---|
| biotinylated EpCAM probe 1(Exon1) | ACAGAGCGCTAGTCCTTCGGC | 5 |
| biotinylated EpCAM probe 2(Exon4) | AACCTTATGATAGTAAAAGTT | 6 |
| biotinylated EpCAM probe 3(Exon7) | GGTCTAAAAGCTGGTGTTATT | 7 |
| biotinylated EpCAM probe 4(Exon9) | GAAACTGGCTTTACCAATCTT | 8 |

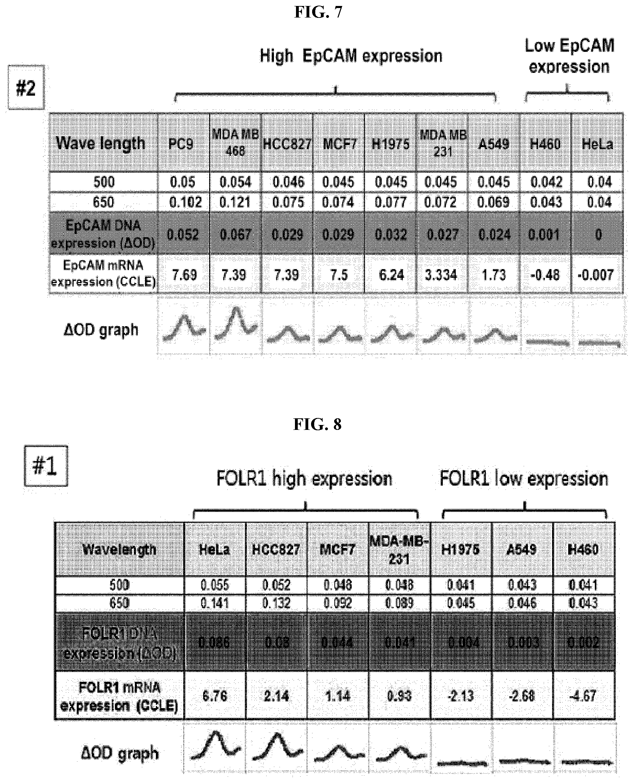

For colorimetric detection, it was treated with the solution in which TMB and H₂O₂ were added to a sodium acetate buffer to confirm detection results. As a result of analyzing DNA-based EpCAM expression (ΔOD) of each cancer cell line, in two repeated experiments, clear EpCAM DNA expression (ΔOD; cutoff OD > 0.012) was observed only in fDNA of PC9, MDA-MB468, HCC827, MCF7, H1975, and MDA-MB-231 cancer cell lines. On the other hand, in two repeated experiments, EpCAM DNA expression was not observed in H460 and HeLa cancer cell lines (Figure 6 and Figure 7). In addition, the EpCAM DNA expression result of each cancer cell line was compared to the EpCAM mRNA expression result of each cancer cell line obtained from CCLE (cancer cell line encyclopedia). The EpCAM mRNA value of each cancer cell line is shown in Table 4 below.

**[Table 4]**

| Gene | EpCAM |
|---|---|
| MDAMB468_BREAST | 7.391754 |
| HCC827_LUNG | 7.394143 |
| MCF7_BREAST | 7.506349 |
| NCIH1975_LUNG | 6.241641 |
| MDAMB231_BREAST | 3.334 |
| A549_LUNG | 1.727828 |
| NCIH460_LUNG | -0.448733 |
| HRLA_CERVIX | -0.00781 |

As a result, high mRNA expression was shown in PC9, MDA-MB468, HCC827, MCF7, H1975, and MDA-MB-231 cancer cell lines. On the other hand, mRNA expression was hardly shown in H460 and HeLa cancer cell lines.

### Example 1.3. Confirmation of Accuracy for Detection of FOLR1

The accuracy for detection of FOLR1 was confirmed using HeLa, MDA-MB-468, HCC827, MCF7, and MDA-MB-231 cancer cell lines known as FOLR1 positive cancer cell lines and H460, PC9, H1975 and A549 cancer cell lines known as FOLR1 negative cancer cell lines obtained from ATCC and Korean Cell Line Bank. In this case, the FOLR1 positive cancer cell lines and FOLR1 negative cancer cell lines were classified based on the mRNA level of FOLR1 of each cancer cell line provided by CCLE (Cancer Cell Line Encyclopedia).

Specifically, genomic DNA was extracted from each of the FOLR1 positive cancer cell line and FOLR1 negative cancer cell line, followed by sonication to produce fDNA. 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated FOLR1 probe was added and reacted further for 20 minutes. In this case, the probes used are shown in Table 5 below.

**[Table 5]**

| | Sequence(5'-> 3') | Sequence ID NO. |
|---|---|---|
| biotinylated FOLR1 probe 1(Exon4) | TGTTCTACCAACACCAGCCA G | 9 |
| biotinylated FOLR1 probe 2(Exon2) | ACTGAACCCAAAGGATCAC CT | 10 |
| biotinylated FOLR1 probe 3(Exon1) | CCTAGGCCACTAAACCACAG C | 11 |
| biotinylated FOLR1 probe 4(Exon5) | GGCTGGGCCCTGGGCAGCCT G | 12 |

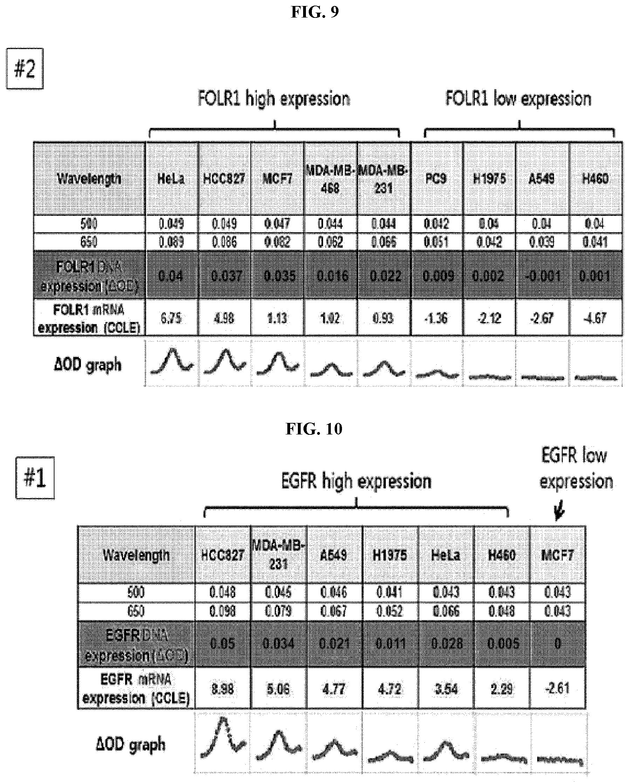

For colorimetric detection, it was treated with the solution in which TMB and H₂O₂ were added to a sodium acetate buffer to confirm detection results. As a result of analyzing DNA-based FOLR1 expression (ΔOD) of each cancer cell line, in two repeated experiments, clear FOLR1 DNA expression (ΔOD; cutoff OD > 0.012) was observed only in fDNA of HeLa, MDA-MB468, HCC827, MCF7, and MDA-MB-231 cancer cell lines. On the other hand, in two repeated experiments, FOLR1 DNA expression was not observed in H460, PC9, H1975 and A549 cancer cells (Figure 8 and Figure 9). In addition, the FOLR1 DNA expression result of each cancer cell line was compared to the FOLR1 mRNA expression result of each cancer cell line obtained from CCLE (cancer cell line encyclopedia). The FOLR1 mRNA value of each cancer cell line is shown in Table 6 below.

**[Table 6]**

| Cancer cell line | FOLR1 |
|---|---|
| NCIH460_LUNG | -4.67303 |
| A549_LUNG | -2.67569 |
| NCIH1975_LUNG | -2.1258 |
| PC9_LUNG | -1.3628 |
| MDAMB231_BREAST | 0.92787 |
| MDAMB468_BREAST | 1.029144 |
| MCF7_BREAST | 1.137905 |
| HCC827_LUNG | 2.13759 |
| HRLA_CERVIX | 6.756305 |

As a result, high mRNA expression was shown in HeLa, MDA-MB-468, HCC827, MCF7, and MDA-MB-231 cancer cell lines. On the other hand, mRNA expression was hardly shown in H460, PC9, H1975, and A549 cancer cell lines.

### Example 1.4. Confirmation of Accuracy for Detection of EGFR

The accuracy for detection of EGFR was confirmed using HeLa, PC9, A549, H1975, H460, MDA-MB-468, MCF7, and MDA-MB-231 cancer cell lines known as EGFR positive cancer cell lines and MCF7 cancer cell lines known as EGFR negative cancer cell lines obtained from ATCC and Korean Cell Line Bank. In this case, the EGFR positive cancer cell lines and EGFR negative cancer cell lines were classified based on the mRNA level of EGFR of each cancer cell line provided by CCLE (Cancer Cell Line Encyclopedia).

Specifically, genomic DNA was extracted from each of the EGFR positive cancer cell line and EGFR negative cancer cell line, followed by sonication to produce fDNA. 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated EGFR probe was added and reacted further for 20 minutes. In this case, the probes used are shown in Table 7 below.

**[Table 7]**

| | Sequence(5'-> 3') | Sequence ID NO. |
|---|---|---|
| biotinylated EGFR probe 1(Exon1) | AACGCCACAACCACCGCGC AC | 13 |
| biotinylated EGFR probe 2(Exon21) | GGACCGTCGCTTGGTGCAC CG | 14 |
| biotinylated EGFR probe 3 (Exon 19) | TCTGGACCCAGAAGGTGAG | 15 |
| | AA | |
| biotinylated EGFR probe 4(Exon20) | TGCTGGGCATCTGCCTCACC T | 16 |

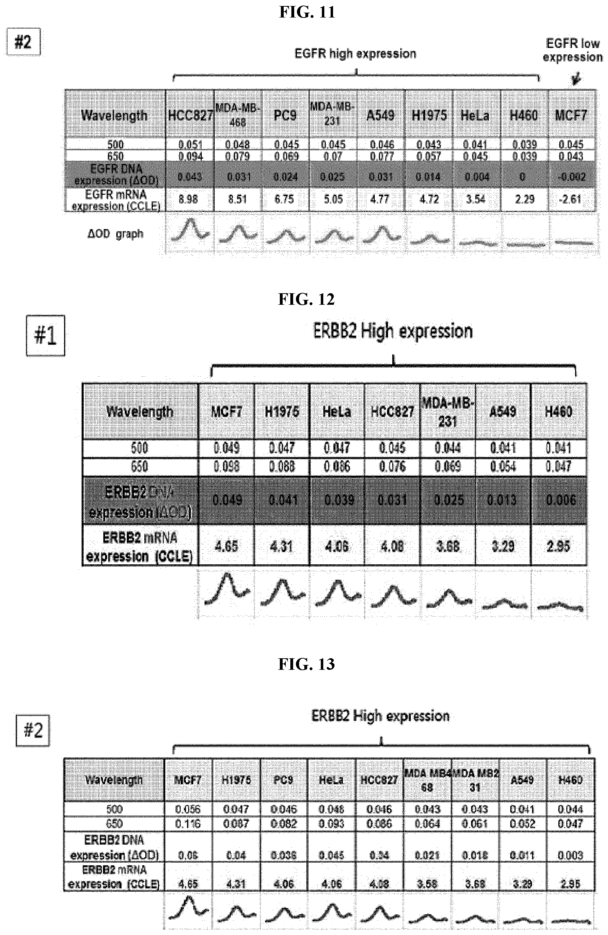

For colorimetric detection, it was treated with the solution in which TMB and H₂O₂ were added to a sodium acetate buffer to confirm detection results. As a result of analyzing DNA-based EGFR expression (ΔOD) of each cancer cell line, in two repeated experiments, clear EGFR DNA expression (ΔOD; cutoff OD > 0.012) was observed only in fDNA of HCC827, PC9, MDA-MB468, MCF7, and MDA-MB-231 cancer cell lines. On the other hand, in two repeated experiments, EGFR DNA expression was not observed in MCF7 cancer cell line (Figure 10 and Figure 11). In addition, the EGFR DNA expression result of each cancer cell line was compared to the EGFR mRNA expression result of each cancer cell line obtained from CCLE (cancer cell line encyclopedia). The EGFR mRNA value of each cancer cell line is shown in Table 8 below.

**[Table 8]**

| Gene | EGFR |
|---|---|
| MCF7_BREAST | -2.6082 |
| NCIH460_LUNG | 2.290725 |
| HRLA_CERVIX | 3.548315 |
| NCIH1975_LUNG | 4.722316 |
| A549_LUNG | 4.772873 |
| MDAMB231_BREAST | 5.060244 |
| PC9_LUNG | 6.4006 |
| MDAMB468_BREAST | 8.505156 |
| HCC827_LUNG | 8.98461 |

As a result, high mRNA expression was shown in HeLa, H460, PC9, H1975, MDA-MB-468, MCF7, and MDA-MB-231 cancer cell lines. On the other hand, mRNA expression was hardly shown in MCF7 cancer cell lines.

### Example 1.5. Confirmation of Accuracy for Detection of ERBB2(HER2)

The accuracy for detection of ERBB2 was confirmed using MCF7, PC9, A549, H1975, H460, MDA-MB468, MCF7, and MDA-MB-231 cancer cell lines known as ERBB2 positive cancer cell lines and H460 cancer cell lines known as ERBB2 negative cancer cell lines obtained from ATCC and Korean Cell Line Bank. In this case, the ERBB2 positive cancer cell lines and ERBB2 negative cancer cell lines were classified based on the mRNA level of ERBB2 of each cancer cell line provided by CCLE (Cancer Cell Line Encyclopedia).

Specifically, genomic DNA was extracted from each of the ERBB2 positive cancer cell line and ERBB2 negative cancer cell line, followed by sonication to produce fDNA. 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated ERBB2 probe was added and reacted further for 20 minutes. In this case, the probes used are shown in Table 9 below.

**[Table 9]**

| | Sequence(5'->3') | Sequence ID NO. |
|---|---|---|
| biotinylated ERBB2 probe 1(Exon1) | TCGCAGCACCCCGCGCCCCG C | 17 |
| biotinylated ERBB2 probe 2(Exon5) | CCTGTTCTCCGATGTGTAAG G | 18 |
| biotinylated ERBB2 probe 3(Exon10) | CCGCTCCAGCCAGAGCAGCT C | 19 |
| biotinylated ERBB2 probe 4(Exon17) | AAGATCCGGAAGTACACGA TG | 20 |

For colorimetric detection, it was treated with the solution in which TMB and H₂O₂ were added to a sodium acetate buffer to confirm detection results. As a result of analyzing DNA-based ERBB2 expression (ΔOD) of each cancer cell line, in two repeated experiments, clear ERBB2 DNA expression (ΔOD; cutoff OD > 0.010) was observed only in fDNA of MCF7, PC9, A549, H1975, MDA-MB468, MCF7, and MDA-MB-231 cancer cell lines. On the other hand, in two repeated experiments, ERBB2 DNA expression was not observed in H460 cancer cell line (Figure 12 and Figure 13). In addition, the ERBB2 DNA expression result of each cancer cell line was compared to the ERBB2 mRNA expression result of each cancer cell line obtained from CCLE (cancer cell line encyclopedia). The ERBB2 mRNA value of each cancer cell line is shown in Table 10 below.

**[Table 10]**

| Gene | ERBB2 |
|---|---|
| MCF7_BREAST | 4.64588 |
| NCIH1975_LUNG | 4.314726 |
| HRLA_CERVIX | 4.056006 |
| HCC827_LUNG | 4.077482 |
| MDAMB231_BREAST | 3.681573 |
| MDAMB468_BREAST | 3.576987 |
| A549_LUNG | 3.298639 |
| NCIH460_LUNG | 2.948514 |

As a result, high mRNA expression was shown in MCF7, PC9, A549, H1975, MDA-MB468, MCF7, H460, and MDA-MB-231 cancer cell lines.

### Example 1.6. Confirmation of Accuracy for Detection of OGT(O-linked β-N-acetylglucosamine transferase)

The accuracy for detection of OGT was confirmed using UMUC3, KU19-19, 253J, J82, T24, MBT2 cancer cell lines known as OGT positive cancer cell lines and RT4, MDCK, HBL EpC, Jurkat cancer cell lines known as OGT negative cancer cell lines obtained from ATCC and Korean Cell Line Bank. In this case, the OGT positive cancer cell lines and OGT negative cancer cell lines were classified based on the mRNA level of OGT of each cancer cell line provided by CCLE (Cancer Cell Line Encyclopedia).

Specifically, genomic DNA was extracted from each of the OGT positive cancer cell line and OGT negative cancer cell line, followed by sonication to produce fDNA. 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated OGT probe was added and reacted further for 20 minutes. In this case, the probes used are shown in Table11 below.

**[Table 11]**

| | Sequence(5'->3') | Sequence ID NO. |
|---|---|---|
| biotinylated OGT(O-GlcNAc transferase) probe 1 | ATGGCGTCTTCCGTGG GC | 21 |
| OGT_R biotinylated OGT(O-GlcNAc transferase) probe 2 | TGCCCTGTGGCCATGG AC | 22 |

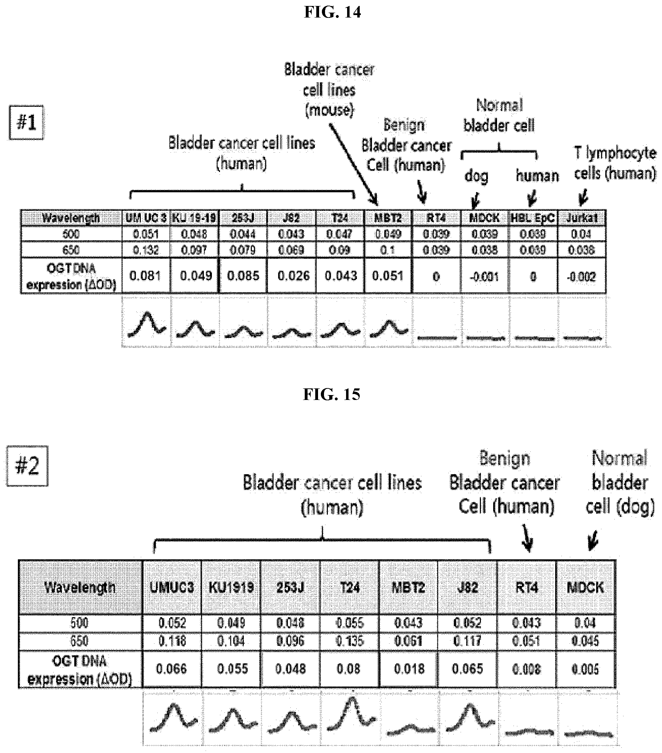

For colorimetric detection, it was treated with the solution in which TMB and H₂O₂ were added to a sodium acetate buffer to confirm detection results. As a result of analyzing DNA-based OGT expression (ΔOD) after detecting fDNA extracted from OGT positive cancer cell lines and OGT negative cancer cell lines with nanowires prepared in Preparation Example 1, in two repeated experiments, clear OGT DNA expression (ΔOD; cutoff OD > 0.010) was observed only in fDNA of UMUC3, KU19-19, 253J, J82, T24, MBT2 cancer cell lines. On the other hand, in two repeated experiments both, OGT DNA expression was not observed in RT4, a positive bladder cancer cell line, and MDCK and HBL_EpC, normal bladder cell lines, or Jurkat T-lymphocyte cells (Figure 14 and Figure 15).

### II. Tumor Marker Detection of Gene Derived from Cancer Cell Line

### Example 2. Detection of Biomarker Derived from Cancer Cell Line

### Example 2.1. Detection of CEA

CEA was detected using LoVo, MKN45, and SW1116 cancer cell lines known as CEA positive cancer cell lines and HCT8, HCT15, HeLa, and MDA-MB-231 cancer cell lines known as CEA negative cancer cell lines obtained from ATCC and Korean Cell Line Bank. In this case, the CEA positive cancer cell lines and CEA negative cancer cell lines were classified based on the mRNA level of CEA of each cancer cell line provided by CCLE (Cancer Cell Line Encyclopedia).

Specifically, genomic DNA was extracted from each of the CEA positive cancer cell line and CEA negative cancer cell line, followed by sonication to produce fDNA. 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated CEA probe was added and reacted further for 20 minutes. In this case, the probes used are shown in Table 12 below.

**[Table 12]**

| | Sequence(5'->3') | Sequence ID NO. |
|---|---|---|
| biotinylated CEA(Prostatic Acid Phosphatase; ACPP gene) probe 1 | AATCTGAACCTCTCCTGCCA C | 23 |
| biotinylated CEA(Prostatic Acid Phosphatase; ACPP gene) probe 2 | ACCCTTCATCACCAGCAACA A | 24 |
| biotinylated CEA(Prostatic Acid Phosphatase; ACPP gene) probe 3 | TATTCTTGGCTGATTGATGG G | 25 |
| biotinylated CEA(Prostatic Acid Phosphatase; ACPP gene) probe 4 | CAGCAACAACTCCAAACCC GT | 26 |

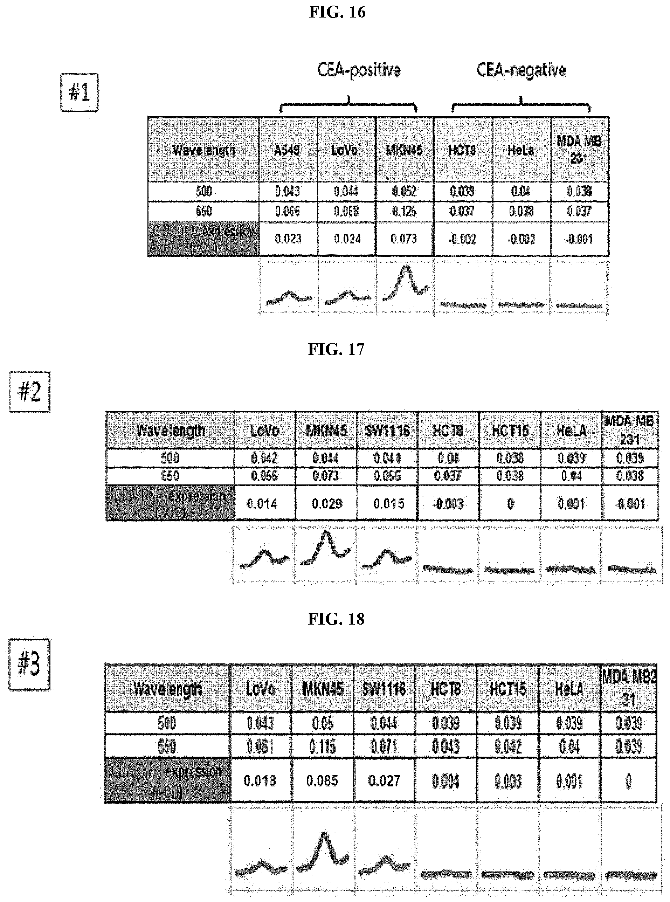

For colorimetric detection, it was treated with the solution in which TMB and H₂O₂ were added to a sodium acetate buffer to confirm detection results. As a result of analyzing DNA-based CEA expression (ΔOD) of each cancer cell line, in three repeated experiments, clear CEA DNA expression (ΔOD; cutoff OD > 0.010) was observed only in fDNA of LoVo, MKN45, and SW1116 cancer cell lines. On the other hand, in three repeated experiments, CEA DNA expression was not observed in HCT8, HCT15, HeLa, and MDA-MB-231 cancer cell lines (Figure 16 to Figure 18).

### Example 2.2. Detection of PSA

PSA was detected using LNE and LNCaP cancer cell lines known as PSA positive cancer cell lines and PC3, DU145, and MCF7 cancer cell lines known as PSA negative cancer cell lines obtained from ATCC and Korean Cell Line Bank. In this case, the PSA positive cancer cell lines and PSA negative cancer cell lines were classified based on the mRNA level of PSA of each cancer cell line provided by CCLE (Cancer Cell Line Encyclopedia).

Specifically, genomic DNA was extracted from each of the PSA positive cancer cell line and PSA negative cancer cell line, followed by sonication to produce fDNA. 50 ng/µL fDNA was added to PBS, and then the nanowires prepared in Preparation Example 1 were added and reacted for 20 minutes to isolate them. Thereafter, a temperature denaturation process was undergone at a temperature of 95 °C for 1 minute, and then a biotinylated PSA probe was added and reacted further for 20 minutes. In this case, the probes used are shown in Table 13 below.

**[Table 13]**

| | Sequence(5'-> 3') | Sequence ID NO. |
|---|---|---|
| biotinylated PSA(KLK3) probe 1 | CAGTCTGCGGCGGTGTTCTGG | 27 |
| biotinylated PSA(KLK3) probe 2 | GCAAGTTCACCCTCAGAAGGT | 28 |
| biotinylated PSA(KLK3) probe 3 | GAGGTCCAGGGTTGCTAGGAA | 29 |

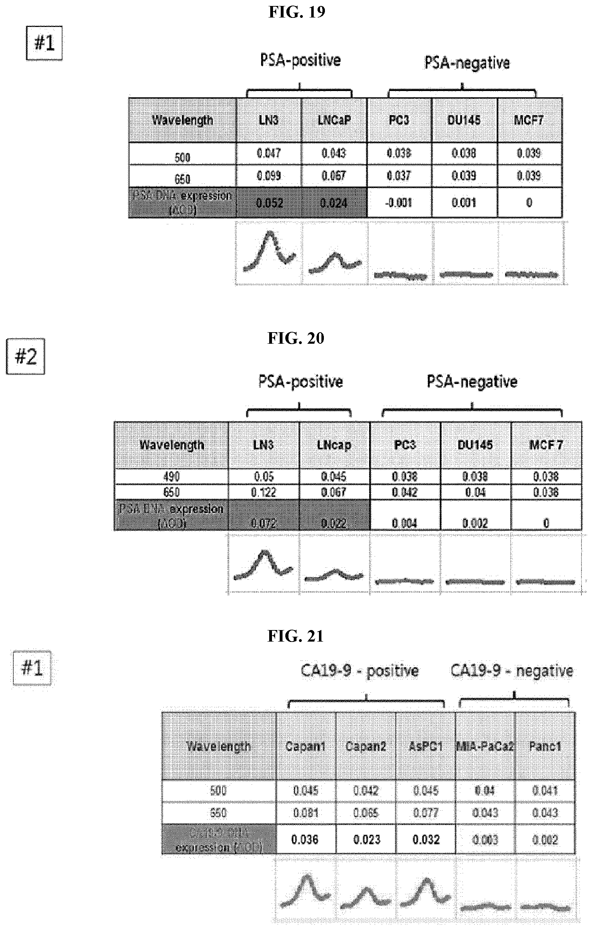

For colorimetric detection, it was treated with the solution in which TMB and H₂O₂ were added to a sodium acetate buffer to confirm detection results. As a result of analyzing DNA-based PSA expression (ΔOD) of each cancer cell line, in two repeated experiments, clear PSA DNA expression (ΔOD; cutoff OD > 0.010) was observed only in fDNA of LNE and LNCaP cancer cell lines. On the other hand, in two repeated experiments, PSA DNA expression was not observed in PC3, DU145, and MCF7 cancer cell lines (Figure 19 and Figure 20).

### Example 2.3. Detection of CA19-9

CA19-9 was detected using Capan1, Capn2, and AsPC1 cancer cell lines known as CA19-9 positive cancer cell lines and MIA-PaCa2 and Panel cancer cell lines known as CA19-9 negative cancer cell lines obtained from ATCC and Korean Cell Line Bank. In this case, the CA19-9 positive cancer cell lines and CA19-9 negative cancer cell lines were classified based on the mRNA level of CA19-9 of each cancer cell line provided by CCLE (Cancer Cell Line Encyclopedia).

Specifically, genomic DNA was extracted from each of the CA19-9 positive cancer cell line and CA19-9 negative cancer cell line, followed by sonication to produce fDNA. 50 ng/µL fDNA was added to PBS, and then the nanowires were added and reacted for 20 minutes to isolate them. Thereafter, a temperature denaturation process was undergone at a temperature of 95 °C for 1 minute, and then a biotinylated CA19-9 probe was added and reacted further for 20 minutes. In this case, the probes used are shown in Table 14 below.

**[Table 14]**

| | Sequence(5'-> 3') | Sequence ID NO. |
|---|---|---|
| biotinylated CA19-9(FLU3 gene) probe 1 | GATCATCACGGCACGGTC | 30 |
| biotinylated CA19-9(FLU3 gene) probe 2 | GTGCAGAGAGATCATCACGG C | 31 |
| biotinylated CA19-9(FLU3 gene) probe 3 | TCTCTCTTACCTGGGACCTCA | 32 |

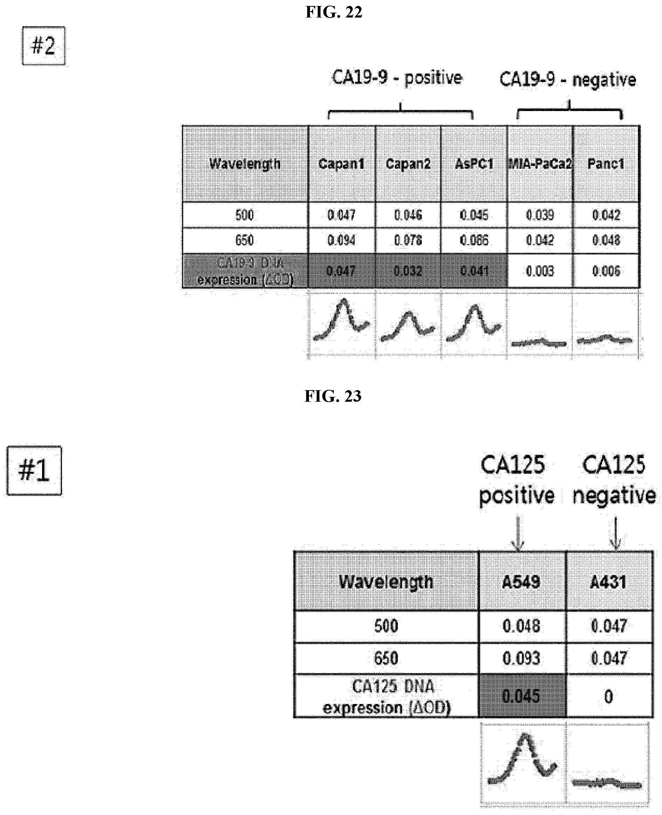

For colorimetric detection, it was treated with the solution in which TMB and H₂O₂ were added to a sodium acetate buffer to confirm detection results. As a result of analyzing DNA-based CA19-9 expression (ΔOD) of each cancer cell line, in two repeated experiments, clear CA19-9 DNA expression (ΔOD; cutoff OD > 0.010) was observed only in fDNA of Capan1, Capn2, and AsPC1 cancer cell lines. On the other hand, in two repeated experiments, CA19-9 DNA expression was not observed in MIA-PaCa2 and Panel cancer cell lines (Figure 21 and Figure 22).

### Example 2.4. Detection of CA125

CA125 was detected using A549 cancer cell line known as a CA125 positive cancer cell line and A431 cancer cell line known as a CA125 negative cancer cell line obtained from ATCC and Korean Cell Line Bank. In this case, the CA125 positive cancer cell line and CA125 negative cancer cell line were classified based on the mRNA level of CA125 of each cancer cell line provided by CCLE (Cancer Cell Line Encyclopedia).

Specifically, genomic DNA was extracted from each of the CA125 positive cancer cell line and CA125 negative cancer cell line, followed by sonication to produce fDNA. 50 ng/µL fDNA was added to PBS, and then the nanowires were added and reacted for 20 minutes to isolate them. Thereafter, a temperature denaturation process was undergone at a temperature of 95 °C for 1 minute, and then a biotinylated CA125 probe was added and reacted further for 20 minutes. In this case, the probes used are shown in Table 15 below.

**[Table 15]**

| | Sequence(5'->3') | Sequence ID NO. |
|---|---|---|
| biotinylated CA125(MUC16 gene) probe 1 | CTGGAACTGATTCTATCAA CC | 33 |
| biotinylated CA125(MUC16 gene) probe 2 | TGTGGCAGAAACCAGCTAT CC | 34 |
| biotinylated CA125(MUC16 gene) probe 3 | GAGGTCCTGAGGATGTGTC AT | 35 |

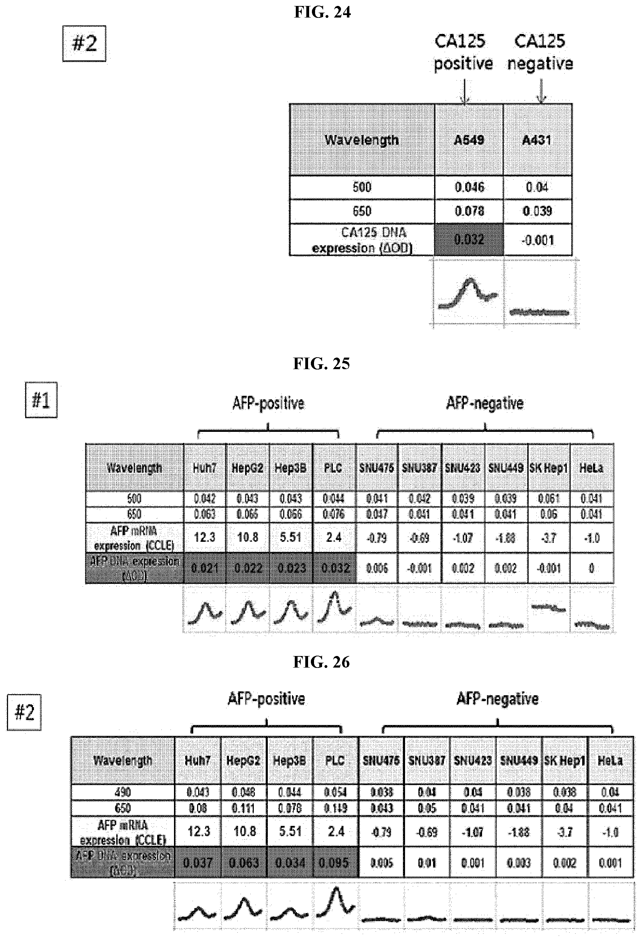

For colorimetric detection, it was treated with the solution in which TMB and H₂O₂ were added to a sodium acetate buffer to confirm detection results. As a result of analyzing DNA-based CA125 expression (ΔOD) of each cancer cell line, in two repeated experiments, clear CA125 DNA expression (ΔOD; cutoff OD > 0.010) was observed only in fDNA of A549 cancer cell line. On the other hand, in two repeated experiments, CA125 DNA expression was not observed in A431 cancer cell lines (Figure 23 and Figure 24).

### Example 2.5. Detection of AFP

AFP was detected using Huh7, HepG2, Hep3B, and PLC cancer cell lines known as AFP positive cancer cell lines and SNU475, SNU387, SNU423, SNU449, SK Hep1, and HeLa cancer cell lines known as AFP negative cancer cell lines obtained from ATCC and Korean Cell Line Bank. In this case, the AFP positive cancer cell lines and AFP negative cancer cell lines were classified based on the mRNA level of AFP of each cancer cell line provided by CCLE (Cancer Cell Line Encyclopedia).

Specifically, genomic DNA was extracted from each of the AFP positive cancer cell line and AFP negative cancer cell line, followed by sonication to produce fDNA. 50 ng/µL fDNA was added to PBS, and then the nanowires were added and reacted for 20 minutes to isolate them. Thereafter, a temperature denaturation process was undergone at a temperature of 95 °C for 1 minute, and then a biotinylated AFP probe was added and reacted further for 20 minutes. In this case, the probes used are shown in Table 16 below.

**[Table 16]**

| | Sequence(5'-> 3') | Sequence ID NO. |
|---|---|---|
| biotinylated AFP probe 1 | TGAGTCAGAAGTTTACCAAAG | 36 |
| biotinylated AFP probe 2 | TGCAAACTGACCACGCTGGAA | 37 |
| biotinylated AFP probe 3 | CTTGAATGCCAAGATAAAGGA | 38 |

For colorimetric detection, it was treated with the solution in which TMB and H₂O₂ were added to a sodium acetate buffer to confirm detection results. As a result of analyzing DNA-based AFP expression (ΔOD) of each cancer cell line, in two repeated experiments, clear AFP DNA expression (ΔOD; cutoff OD > 0.010) was observed only in fDNA of Huh7, HepG2, Hep3B, and PLC cancer cell lines. On the other hand, in two repeated experiments, AFP DNA expression was not observed in SNU475, SNU387, SNU423, SNU449, SK Hep1, and HeLa cancer cell lines (Figure 25 and Figure 26).

### III. Confirmation of Detection of Biomarker Using Plasma or Urine of Cancer Patients

### Example 3. Detection of Biomarker Derived from Cancer Patients

### Example 3.1. Confirmation of Detection of Tumor Marker Using Plasma of Prostate Cancer Patients

PSA, PSMA, PAP, and PCA3 prostate cancer tumor markers were detected from the plasma obtained from normal persons or prostate cancer patients. As a prostate cancer marker, PSA, PSMA, PAP, and PAC3 are highly expressed in prostate cancer patients, and are currently used for determining prostate cancer by confirming the level of prostate cancer antigen (PSA, PSMA, PAP, and PAC3) through cancer tissue and blood tests.

Specifically, plasma was obtained from normal persons or prostate cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 17 below.

**[Table 17]**

| | Sequence(5'-> 3') | Sequence ID NO. |
|---|---|---|
| biotinylated PSA(KLK3) probe 1 | CAGTCTGCGGCGGTGTTCTGG | 26 |
| biotinylated PSA(KLK3) probe 2 | GCAAGTTCACCCTCAGAAGGT | 27 |
| biotinylated PSA(KLK3) probe 3 | GAGGTCCAGGGTTGCTAGGA A | 28 |
| biotinylated PSMA(FOLH1) probe 1 | CATAGTGCTCCCTTTTGATTG | 39 |
| biotinylated PSMA(FOLH1) probe 2 | AGTGGAAAGAATTTGGCCTG G | 40 |
| biotinylated PSMA(FOLH1) probe 3 | ACCTAGAAGAACAATTTTGTT | 41 |
| biotinylated PAP(Prostatic Acid Phosphatase; ACPP gene) probe 1 | GCAGCATTATGAACTTGGAGA | 42 |
| biotinylated PAP(Prostatic Acid Phosphatase; ACPP gene) probe 2 | TCGGAATGAGACGCAGCACG A | 43 |
| biotinylated PCA3(Prostate cancer antigen 3; PCA3 gene) probe 1 | CTTAGTGTTTCAATGAACACC | 44 |
| biotinylated PCA3(Prostate cancer antigen 3; PCA3 gene) probe 2 | GCTTAGCCTTGTACTGAGGCT | 45 |

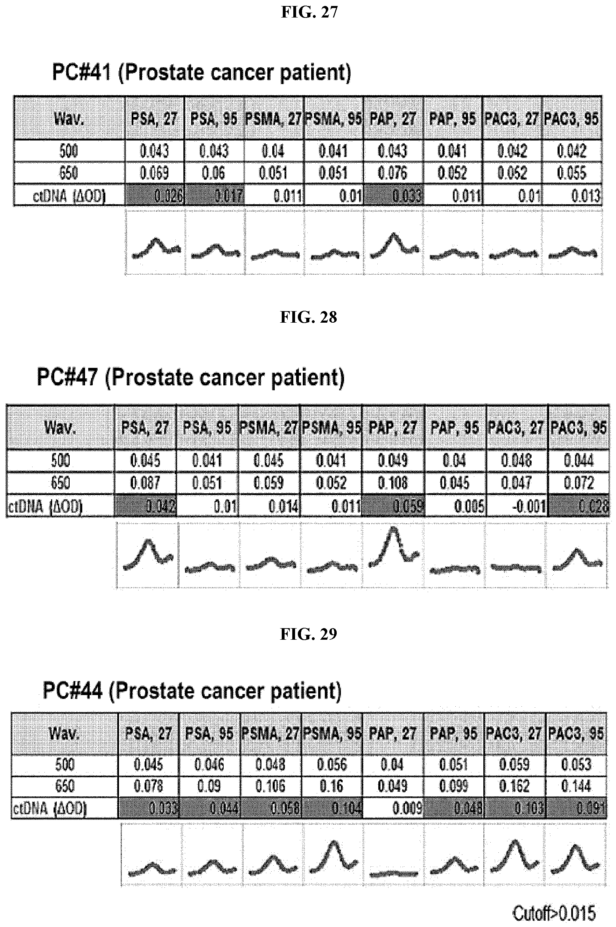

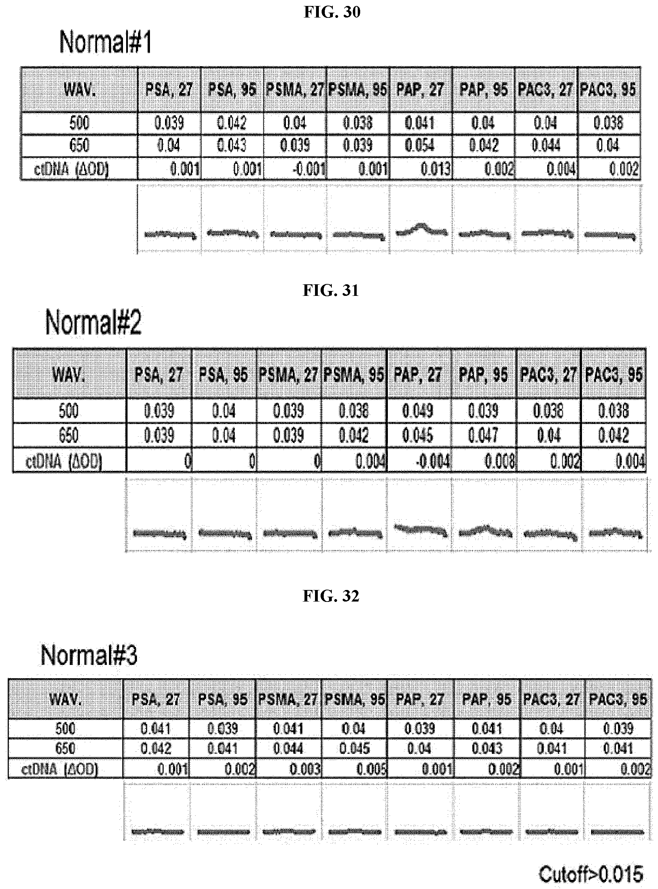

For colorimetric detection, it was treated with the solution in which TMB and H₂O₂ were added to a sodium acetate buffer to confirm expression of PSA, PSMA, PAP, and PAC3. As a result of analyzing PSA, PSMA, PAP, and PAC3 ctDNA expression (ΔOD), clear PSA, PSMA, PAP, and PAC3 ctDNA expression (ΔOD; cutoff OD > 0.015) was observed in prostate cancer patients when temperature denaturation was not undergone or when temperature denaturation process was undergone. On the other hand, PSA, PSMA, PAP, and PAC3 ctDNA expression (ΔOD; cutoff OD > 0.015) was not observed in normal persons both when temperature denaturation was not undergone and when temperature denaturation process was undergone (Figures 27 to 32).

### Example 3.2. Confirmation of Detection of Tumor Marker Using Plasma of Lung Cancer Patients

NSE, SCC, CEA, Cyfra21-1, TPA lung cancer tumor markers were detected from the plasma obtained from normal persons or lung cancer patients. As a lung cancer marker, NSE, SCC, CEA, Cyfra21-1, TPA are highly expressed in lung cancer patients, and are currently used for determining lung cancer by confirming the level of lung cancer antigen (NSE, SCC, CEA, Cyfra21-1, TPA) through cancer tissue and blood tests.

Specifically, plasma was obtained from normal persons or lung cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 18 below.

**[Table 18]**

| | Sequence(5'-> 3') | Sequence ID NO. |
|---|---|---|
| biotinylated NSE(Neuron-Specific Enolase; ENO2 gene) probe 1 | CCATAGAGAAGATCTGGGC CC | 46 |
| biotinylated NSE(Neuron-Specific Enolase; ENO2 gene) probe 2 | GATCCTCCCAGTGGGAGCTG A | 47 |
| biotinylated NSE(Neuron-Specific Enolase; ENO2 gene) probe 3 | GGTCATGGTGAGTCATCGCT C | 48 |
| biotinylated SCC(Squamous Cell Carcinoma Antigen; SART3 gene) probe 1 | GCTGGAGTGGCTGCATGACG A | 49 |
| biotinylated SCC(Squamous Cell Carcinoma Antigen; SART3 gene) probe 2 | GCGCCCTGGTCGAGAACTGC C | 50 |
| biotinylated SCC(Squamous Cell Carcinoma Antigen; SART3 gene) probe 3 | GTGGCTAGAGTATTACAACC T | 51 |
| biotinylated SCC(Squamous Cell Carcinoma Antigen; SART3 gene) probe 4 | TCGAGAACAGCATCCCTGCA G | 52 |
| biotinylated CEA(Prostatic Acid Phosphatase; ACPP gene) probe 1 | AATCTGAACCTCTCCTGCCA C | 23 |
| biotinylated CEA(Prostatic Acid Phosphatase; | ACCCTTCATCACCAGCAACA | 24 |
| ACPP gene) probe 2 | A | |
| biotinylated CEA(Prostatic Acid Phosphatase; ACPP gene) probe 3 | TATTCTTGGCTGATTGATGG G | 25 |
| biotinylated CEA(Prostatic Acid Phosphatase; ACPP gene) probe 4 | CAGCAACAACTCCAAACCC GT | 26 |
| biotinylated Cyfra21-1(cytokeratin 19-fragments; KRT19 gene) probe 1 | CTGGCCTCCTACCTGGACAA | 53 |
| biotinylated Cyfra21-1(cytokeratin 19-fragments; KRT19 gene) probe 2 | GACCTGGAGATGCAGATCG AA | 54 |
| biotinylated Cyfra21-1(cytokeratin 19-fragments; KRT19 gene) probe 3 | GTCGCTGGCCACACGGAGC AG | 55 |
| biotinylated TPA(Tissue plasminogen activator; PLAT gene) probe 1 | GCTGTGAAGCAATCATGGAT G | 56 |
| biotinylated TPA(Tissue plasminogen activator; PLAT gene) probe 2 | GCACCTGCCAGCAGGCCCTG T | 57 |
| biotinylated TPA(Tissue plasminogen activator; PLAT gene) probe 3 | ACTGGACGGAGTGTGAGCTC T | 58 |

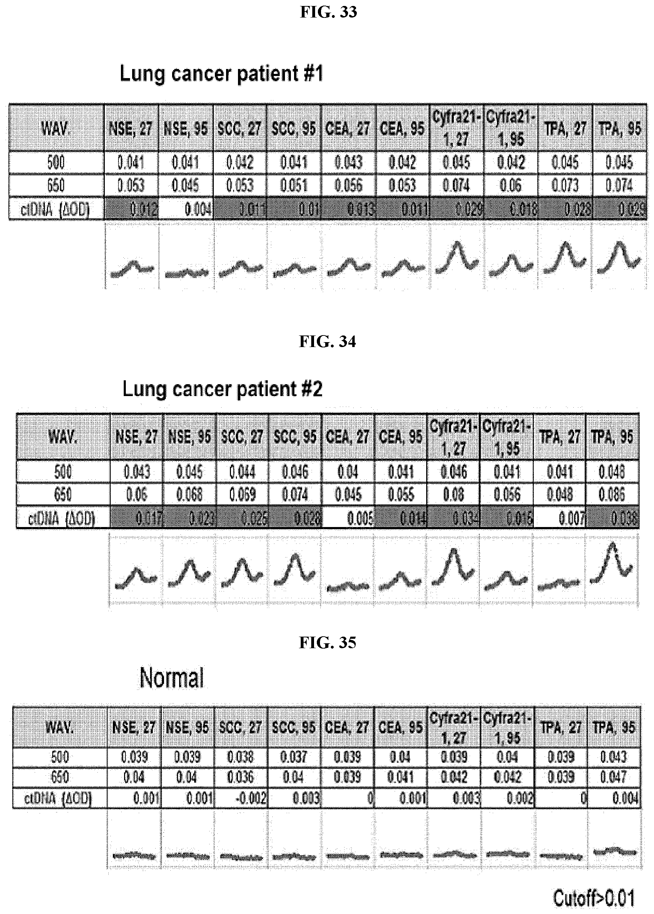

For colorimetric detection, it was treated with the solution in which TMB and H₂O₂ were added to a sodium acetate buffer to confirm expression of NSE, SCC, CEA, Cyfra21-1, TPA. As a result of analyzing ctDNA-based NSE, SCC, CEA, Cyfra21-1, TPA ctDNA expression (ΔOD), clear NSE, SCC, CEA, Cyfra21-1, TPA ctDNA expression (ΔOD; cutoff OD > 0.010) was observed in two lung cancer patients when temperature denaturation was not undergone or when temperature denaturation process was undergone. On the other hand, NSE, SCC, CEA, Cyfra21-1, TPA ctDNA expression (ΔOD; cutoff OD > 0.010) was not observed in normal persons both when temperature denaturation was not undergone and when temperature denaturation process was undergone (Figures 33 to 35).

### Example 3.3. Confirmation of Detection of Tumor Marker Using Plasma of Thyroid Cancer Patients

CEA, NSE, TG, CALCA thyroid cancer tumor markers were detected from the plasma obtained from normal persons or thyroid cancer patients. As a thyroid cancer marker, CEA, NSE, TG, CALCA are highly expressed in thyroid cancer patients, and are currently used for determining thyroid cancer by confirming the level of thyroid cancer antigen (CEA, NSE, TG, CALCA) through cancer tissue and blood tests.

Specifically, plasma was obtained from normal persons or thyroid cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 19 below.

**[Table 19]**

| | Sequence(5'-> 3') | Sequence ID NO. |
|---|---|---|
| biotinylated CEA(Prostatic Acid Phosphatase; ACPP gene) probe 1 | AATCTGAACCTCTCCTGCCA [?] C | 23 |
| biotinylated CEA(Prostatic Acid Phosphatase; ACPP gene) probe 2 | ACCCTTCATCACCAGCAACA [?] A | 24 |
| biotinylated CEA(Prostatic Acid Phosphatase; ACPP gene) probe 3 | TATTCTTGGCTGATTGATGG [?] G | 25 |
| biotinylated CEA(Prostatic Acid Phosphatase; ACPP gene) probe 4 | CAGCAACAACTCCAAACCC [?] GT | 26 |
| biotinylated NSE(Neuron-Specific Enolase; ENO2 gene) probe 1 | CCATAGAGAAGATCTGGGC [?] CC | 46 |
| biotinylated NSE(Neuron-Specific Enolase; ENO2 gene) probe 2 | GATCCTCCCAGTGGGAGCTG [?] A | 47 |
| biotinylated NSE(Neuron-Specific Enolase; ENO2 gene) probe 3 | GGTCATGGTGAGTCATCGCT [?] C | 48 |
| biotinylated Thyroglobulin(TG gene) probe 1 | CAACACCACAGACATGATG [?] AT | 59 |
| biotinylated Thyroglobulin(TG gene) probe 2 | TGCGGCCTGGCTCGAGCAGC ( A | 50 |
| biotinylated Thyroglobulin(TG gene) probe 3 | AATCAATCACTATCCAGCCA [?] G | 51 |
| biotinylated Thyroglobulin(TG gene) probe 4 | ACTCTCTGGAGCACTCCACG [?] G | 52 |
| biotinylated Calcitonin(Calcitonin Gene-Related Peptide; CALCA gene) probe 1 | GAGGTGTCATGGGCTTCCAA [?] A | 53 |
| biotinylated Calcitonin(Calcitonin Gene-Related Peptide; CALCA gene) probe 2 | GTAATCTGAGTACTTGCATG [?] C | 54 |

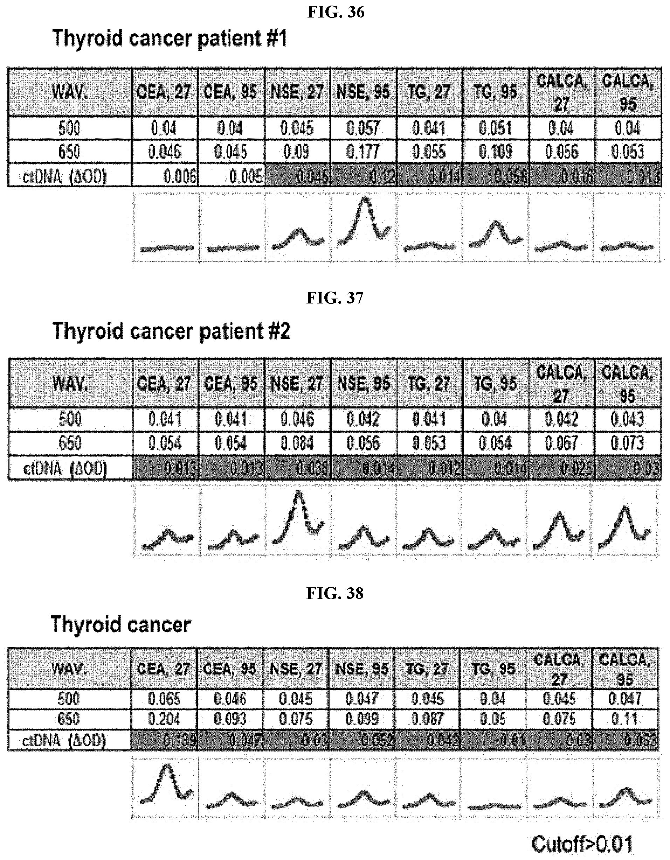

For colorimetric detection, it was treated with the solution in which TMB and H₂O₂ were added to a sodium acetate buffer to confirm expression of CEA, NSE, TG, CALCA. As a result of analyzing ctDNA-based CEA, NSE, TG, CALCA ctDNA expression (ΔOD), clear CEA, NSE, TG, CALCA ctDNA expression (ΔOD; cutoff OD > 0.010) was observed in thyroid cancer patients when temperature denaturation was not undergone or when temperature denaturation process was undergone. On the other hand, CEA, NSE, TG, CALCA ctDNA expression (ΔOD; cutoff OD > 0.010) was not observed in normal persons both when temperature denaturation was not undergone and when temperature denaturation process was undergone (Figures 36 to 40).

### Example 3.4. Confirmation of Detection of Tumor Marker Using Plasma of Bladder Cancer Patients

OGT, FGFR3, TP53, NMP22, Cyfra21-1 bladder cancer tumor markers were detected from the plasma obtained from normal persons or bladder cancer patients. As a bladder cancer marker, OGT, FGFR3, TP53, NMP22, Cyfra21-1 are highly expressed in the urine of bladder cancer patients, and can be used for determining bladder cancer.

Specifically, urine was obtained from normal persons or bladder cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 20 below.

**[Table 20]**

| | Sequence(5'-> 3') | Sequence ID NO. |
|---|---|---|
| biotinylated OGT(O-GlcNAc transferase) probe 1 | ATGGCGTCTTCCGTGGGC | 21 |
| biotinylated OGT(O-GlcNAc transferase) probe 2 | TGCCCTGTGGCCATGGAC | 22 |
| biotinylated FGFR3 probe 1 | AGTGGCGGTGGTGGTGAGGG AG | 65 |
| biotinylated FGFR3 probe 2 | CCCACAGCTTCTGCCCCCGA | 66 |
| biotinylated FGFR3 probe 3 | GGGCATCCATGGGAGCC | 67 |
| biotinylated FGFR3 probe 4 | CAGCGTGGGCCGAGGT | 68 |
| biotinylated FGFR3 probe 5 | CCCTGAGATGCTGGGAGCAG | 69 |
| biotinylated FGFR3 probe 6 | GTGTGGGAAGGCGGTGTTG | 70 |
| biotinylated TP53 probe 1 | GCCCTGACTTTCAACTCTG | 71 |
| biotinylated TP53 probe 2 | ACGACAGGGCTGGTTGCC | 72 |
| biotinylated TP53 probe 3 | TCCCCAGGCCTCTGATTCC | 73 |
| biotinylated TP53 probe 4 | CCTCCAGGTGAGCAGTAG | 74 |
| biotinylated TP53 probe 5 | CTTGGGCCTGTGTTATCTC | 75 |
| biotinylated TP53 probe 6 | CCCACCTCTTACCGATTT | 76 |
| biotinylated TP53 probe 7 | ACAAGGGTGGTTGGGAGTAG | 77 |
| biotinylated TP53 probe 8 | GGACAAGAAGCGGTGGAGG | 78 |
| biotinylated Cyfra21-1(cytokeratin 19-fragments; KRT19 gene) probe 1 | CTGGCCTCCTACCTGGACAA | 53 |
| biotinylated Cyfra21-1(cytokeratin 19-fragments; KRT19 gene) probe 2 | GACCTGGAGATGCAGATCGA A | 54 |
| biotinylated Cyfra21-1(cytokeratin 19-fragments; KRT19 gene) probe 3 | GTCGCTGGCCACACGGAGCA G | 55 |
| biotinylated NMP22(nuclear matrix protein-22; NUMA1 gene) probe 1 | ATGGCACTGAAGAGGGACA GC | 79 |
| biotinylated NMP22(nuclear matrix protein-22; NUMA1 gene) probe 2 | GCCAAGGAAGAGCTGGAGC AG | 80 |
| biotinylated NMP22(nuclear matrix protein-22; NUMA1 gene) probe 3 | GAGCAGCTAGAGGTATTTCA G | 81 |
| biotinylated NMP22(nuclear matrix protein-22; NUMA1 gene) probe 4 | AGGTAGAATCCCTGGAGAGT C | 82 |

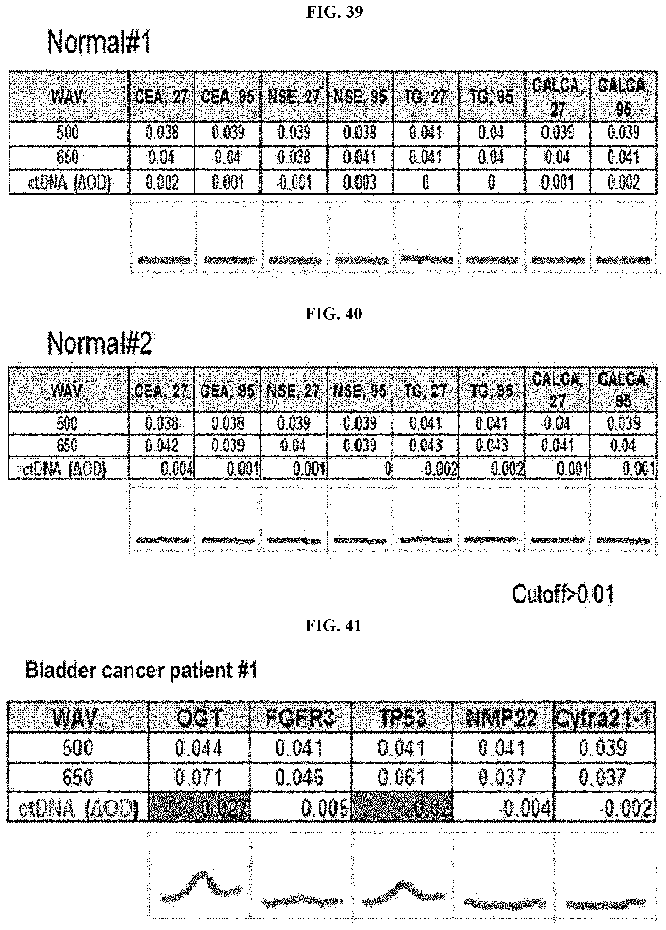

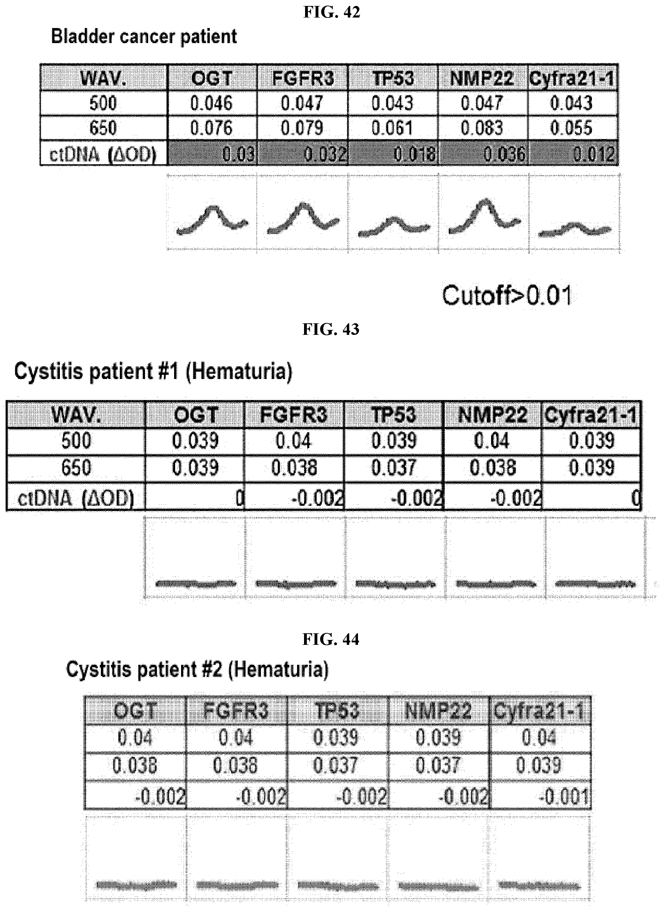

For colorimetric detection, it was treated with the solution in which TMB and H₂O₂ were added to a sodium acetate buffer to confirm expression of OGT, FGFR3, TP53, NMP22, Cyfra21-1. As a result of analyzing ctDNA-based OGT, FGFR3, TP53, NMP22, Cyfra21-1 ctDNA expression (ΔOD), clear OGT, FGFR3, TP53, NMP22, Cyfra21-1 ctDNA expression (ΔOD; cutoff OD > 0.010) was observed in bladder cancer patients when temperature denaturation was not undergone or when temperature denaturation process was undergone. On the other hand, OGT, FGFR3, TP53, NMP22, Cyfra21-1 ctDNA expression (ΔOD; cutoff OD > 0.010) was not observed in normal persons and cystitis patients both when temperature denaturation was not undergone and when temperature denaturation process was undergone (Figures 41 to 46).

### Example 3.5. Confirmation of Detection of Tumor Marker Using Plasma of Breast Cancer Patients

CA27-29, CA15-3, CEA breast cancer tumor markers were detected from the plasma obtained from normal persons or breast cancer patients. As a breast cancer marker, CA27-29, CA15-3*,* CEA are highly expressed in breast cancer patients, and are currently used for determining breast cancer by confirming the level of breast cancer antigen (CA27-29, CA15-3, CEA) through cancer tissue and blood tests.

Specifically, plasma was obtained from normal persons or breast cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 21 below.

**[Table 21]**

| | Sequence(5'-> 3') | Sequence ID NO. |
|---|---|---|
| biotinylated CA27.29/CA15-3(Carcinoma | GCGCCTGCCTGAATCTGTTCT | 83 |
| Antigen 15-3; MUC1 gene) probe 1 | | |
| biotinylated CA27.29/CA15-3 (Carcinoma Antigen 15-3; MUC1 gene) probe 2 | TTCTGGGCCTCTCCAATATTA | 84 |
| biotinylated CA27.29/CA15-3 (Carcinoma Antigen 15-3; MUC1 gene) probe 3 | GGATACCTACCATCCTATGAG | 85 |
| biotinylated CEA(Prostatic Acid Phosphatase; ACPP gene) probe 1 | AATCTGAACCTCTCCTGCCAC | 23 |
| biotinylated CEA(Prostatic Acid Phosphatase; ACPP gene) probe 2 | ACCCTTCATCACCAGCAACAA | 24 |
| biotinylated CEA(Prostatic Acid Phosphatase; ACPP gene) probe 3 | TATTCTTGGCTGATTGATGGG | 25 |
| biotinylated CEA(Prostatic Acid Phosphatase; ACPP gene) probe 4 | CAGCAACAACTCCAAACCCGT | 26 |

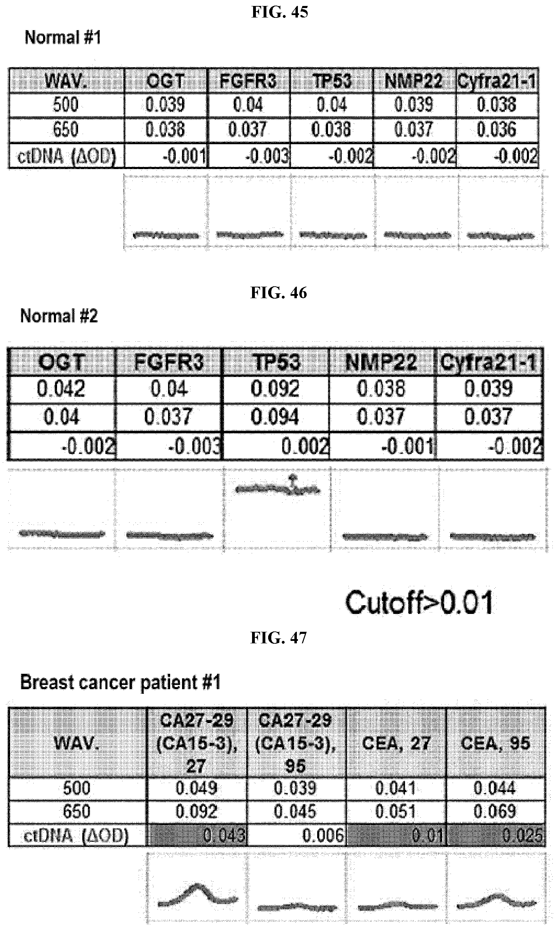

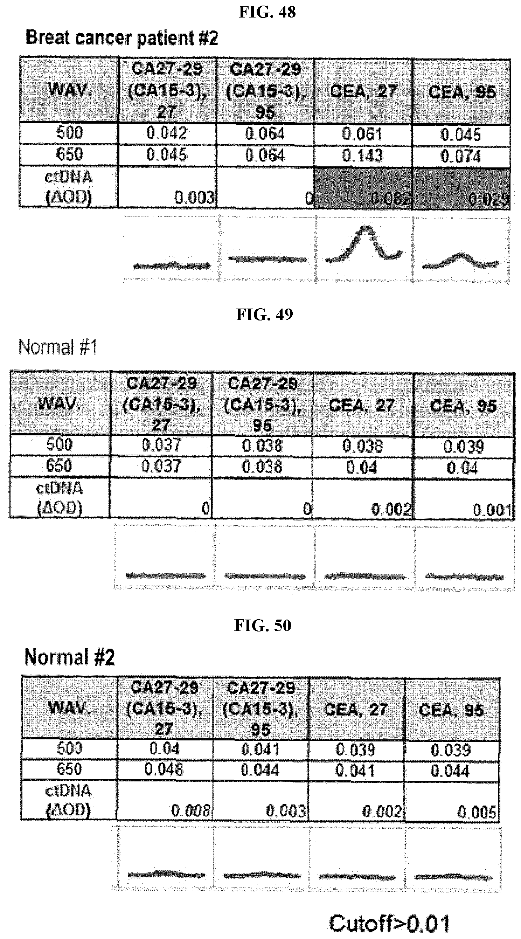

For colorimetric detection, it was treated with the solution in which TMB and H₂O₂ were added to a sodium acetate buffer to confirm expression of CA27-29, CA15-3*,* CEA. As a result of analyzing ctDNA-based CA27-29, CA15-3, CEA ctDNA expression (ΔOD), clear CA27-29, CA15-3, CEA ctDNA expression (ΔOD; cutoff OD > 0.010) was observed in breast cancer patients when temperature denaturation was not undergone or when temperature denaturation process was undergone. On the other hand, CA27-29, CA15-3, CEA ctDNA expression (ΔOD; cutoff OD > 0.010) was not observed in normal persons both when temperature denaturation was not undergone and when temperature denaturation process was undergone (Figures 47 to 50).

### Example 3.6. Confirmation of Detection of Tumor Marker Using Plasma of Colorectal Cancer Patients

CEA, CA19-9 colorectal cancer tumor markers were detected from the plasma obtained from normal persons or colorectal cancer patients. As a colorectal cancer marker, CEA, CA19-9 are highly expressed in colorectal cancer patients, and are currently used for determining colorectal cancer by confirming the level of colorectal cancer antigen (CEA, CA19-9) through cancer tissue and blood tests.

Specifically, plasma was obtained from normal persons or colorectal cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 22 below.

**[Table 22]**

| | **Sequence(5'-> 3')** | Sequence ID |
|---|---|---|
| | | NO. |
| biotinylated CEA(Prostatic Acid Phosphatase; ACPP gene) probe 1 | AATCTGAACCTCTCCTGCCA C | 23 |
| biotinylated CEA(Prostatic Acid Phosphatase; ACPP gene) probe 2 | ACCCTTCATCACCAGCAACA A | 24 |
| biotinylated CEA(Prostatic Acid Phosphatase; ACPP gene) probe 3 | TATTCTTGGCTGATTGATGG G | 25 |
| biotinylated CEA(Prostatic Acid Phosphatase; ACPP gene) probe 4 | CAGCAACAACTCCAAACCC GT | 26 |
| biotinylated CA19-9(FLU3 gene) probe 1 | GATCATCACGGCACGGTC | 30 |
| biotinylated CA19-9(FLU3 gene) probe 2 | GTGCAGAGAGATCATCACG GC | 31 |
| biotinylated CA19-9(FLU3 gene) probe 3 | TCTCTCTTACCTGGGACCTC A | 32 |

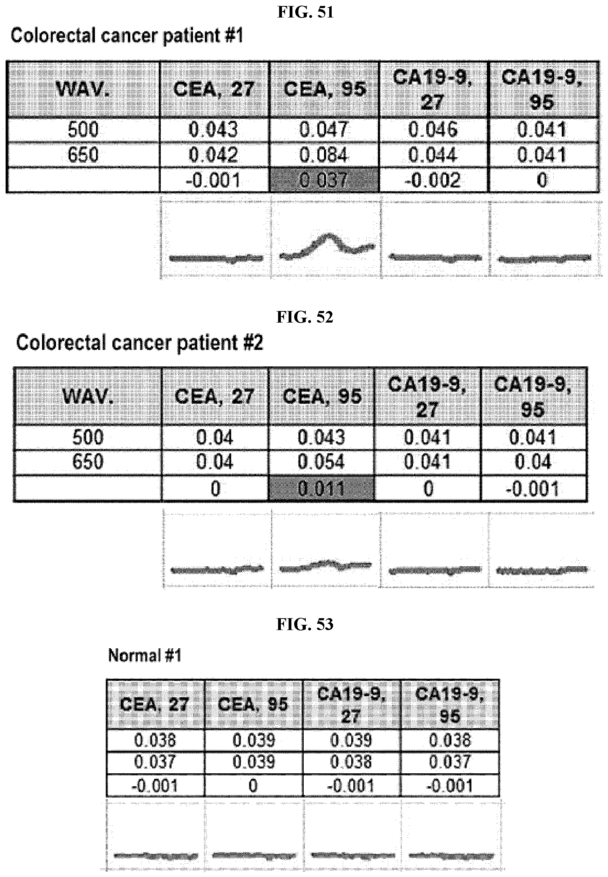

For colorimetric detection, it was treated with the solution in which TMB and H₂O₂ were added to a sodium acetate buffer to confirm expression of CEA, CA19-9. As a result of analyzing ctDNA-based CEA, CA19-9 ctDNA expression (ΔOD), clear CEA, CA19-9 ctDNA expression (ΔOD; cutoff OD > 0.010) was observed in colorectal cancer patients when temperature denaturation was not undergone or when temperature denaturation process was undergone. On the other hand, CEA, CA19-9 ctDNA expression (ΔOD; cutoff OD > 0.010) was not observed in normal persons both when temperature denaturation was not undergone and when temperature denaturation process was undergone (Figures 51 to 55).

### Example 3.7. Confirmation of Detection of Tumor Marker Using Plasma of Biliary Tract Cancer Patients

CEA, CA19-9, CA125 biliary tract cancer tumor markers were detected from the plasma obtained from normal persons or biliary tract cancer patients. As a biliary tract cancer marker, CEA, CA19-9, CA125 are highly expressed in biliary tract cancer patients, and are currently used for determining biliary tract cancer by confirming the level of biliary tract cancer antigen (CEA, CA19-9, CA125) through blood tests.

Specifically, plasma was obtained from normal persons or biliary tract cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 23 below.

**[Table 23]**

| | Sequence(5'->3') | Sequence ID NO. |
|---|---|---|
| biotinylated CEA(Prostatic Acid Phosphatase; ACPP gene) probe 1 | AATCTGAACCTCTCCTGCCAC | 23 |
| biotinylated CEA(Prostatic Acid Phosphatase; ACPP gene) probe 2 | ACCCTTCATCACCAGCAACAA | 24 |
| biotinylated CEA(Prostatic Acid Phosphatase; ACPP gene) probe 3 | TATTCTTGGCTGATTGATGGG | 25 |
| biotinylated CEA(Prostatic Acid Phosphatase; ACPP gene) probe 4 | CAGCAACAACTCCAAACCCGT | 26 |
| biotinylated CA19-9(FLU3 gene) probe 1 | GATCATCACGGCACGGTC | 30 |
| biotinylated CA19-9(FLU3 gene) probe 2 | GTGCAGAGAGATCATCACGGC | 31 |
| biotinylated CA19-9(FLU3 gene) probe 3 | TCTCTCTTACCTGGGACCTCA | 32 |
| biotinylated CA125(MUC16 gene) probe 1 | CTGGAACTGATTCTATCAACC | 33 |
| biotinylated CA125(MUC16 gene) probe 2 | TGTGGCAGAAACCAGCTATCC | 34 |
| biotinylated CA125(MUC16 gene) probe 3 | GAGGTCCTGAGGATGTGTCAT | 35 |

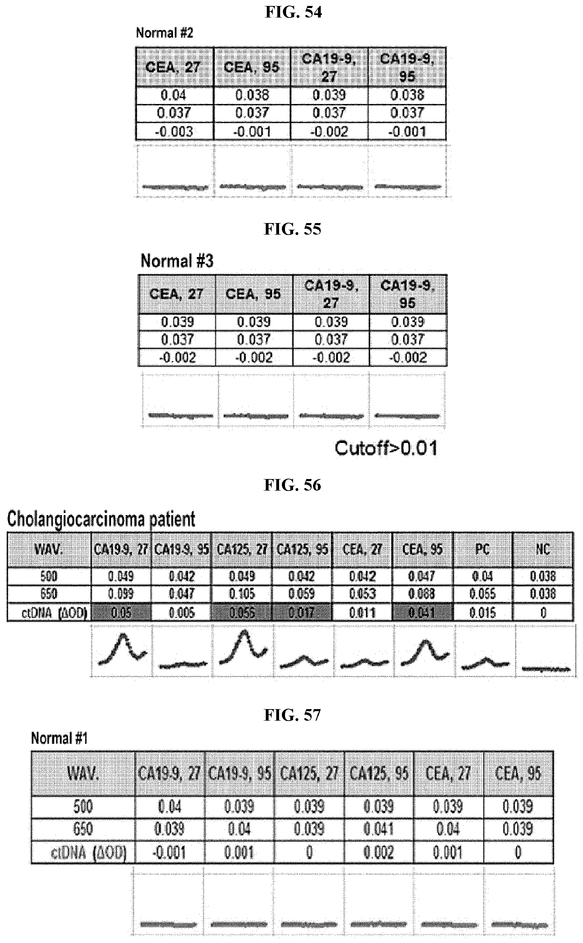

For colorimetric detection, it was treated with the solution in which TMB and H₂O₂ were added to a sodium acetate buffer to confirm expression of CEA, CA19-9, CA125. As a result of analyzing ctDNA-baseed CEA, CA19-9, CA125 ctDNA expression (ΔOD), clear CEA, CA19-9, CA125 ctDNA expression (ΔOD; cutoff OD > 0.010) was observed in biliary tract cancer patients when temperature denaturation was not undergone or when temperature denaturation process was undergone. On the other hand, CEA, CA19-9, CA125 ctDNA expression (ΔOD; cutoff OD > 0.010) was not observed in normal persons both when temperature denaturation was not undergone and when temperature denaturation process was undergone (Figures 56 to 58).

### Example 3.8. Confirmation of Detection of Tumor Marker Using Plasma of Gastric Cancer Patients

CEA, CA19-9, CGB, Cyfra21-1 gastric cancer tumor markers were detected from the plasma obtained from normal persons or gastric cancer patients. As a gastric cancer marker, CEA, CA19-9, CGB, Cyfra21-1 are highly expressed in gastric cancer patients, and are currently used for determining gastric cancer by confirming the level of gastric cancer antigen (CEA, CA19-9, CGB, Cyfra21-1) through blood tests.

Specifically, plasma was obtained from normal persons or gastric cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 24 below.

**[Table 24]**

| | Sequence(5'-> 3') | Sequence ID NO. |
|---|---|---|
| biotinylated CEA(Prostatic Acid Phosphatase; ACPP gene) probe 1 | AATCTGAACCTCTCCTGCCAC | 23 |
| biotinylated CEA(Prostatic Acid Phosphatase; ACPP gene) probe 2 | ACCCTTCATCACCAGCAACA A | 24 |
| biotinylated CEA(Prostatic Acid Phosphatase; ACPP gene) probe 3 | TATTCTTGGCTGATTGATGGG | 25 |
| biotinylated CEA(Prostatic Acid Phosphatase; ACPP gene) probe 4 | CAGCAACAACTCCAAACCCG T | 26 |
| biotinylated CA19-9(FLU3 gene) probe 1 | GATCATCACGGCACGGTC | 30 |
| biotinylated CA19-9(FLU3 gene) probe 2 | GTGCAGAGAGATCATCACGG C | 31 |
| biotinylated CA19-9(FLU3 gene) probe 3 | TCTCTCTTACCTGGGACCTCA | 32 |
| biotinylated Beta-HCG(human chorionic gonadotropin; CGB gene) probe 1 | TGCTGAGCATGGGCGGACA T | 86 |
| biotinylated Beta-HCG(human chorionic gonadotropin; CGB gene) probe 2 | TGGCTCTCAGCTGTCAATGTG | 87 |
| biotinylated Cyfra21-1(cytokeratin 19-fragments; KRT19 gene) probe 1 | CTGGCCTCCTACCTGGACAA | 53 |
| biotinylated Cyfra21-1(cytokeratin 19-fragments; KRT19 gene) probe 2 | GACCTGGAGATGCAGATCGA A | 54 |
| biotinylated Cyfra21-1(cytokeratin 19-fragments; KRT19 gene) probe 3 | GTCGCTGGCCACACGGAGCA G | 55 |

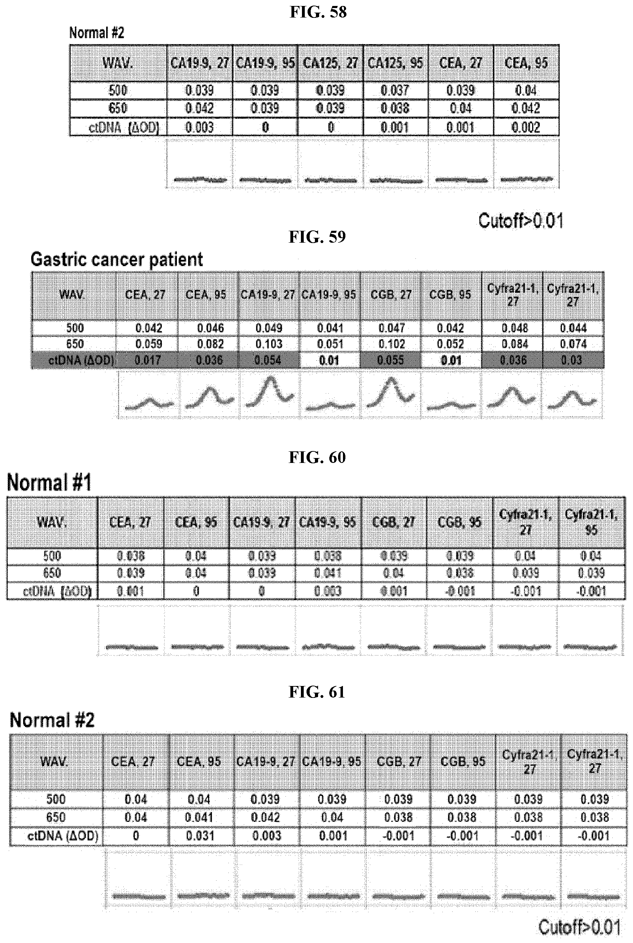

For colorimetric detection, it was treated with the solution in which TMB and H₂O₂ were added to a sodium acetate buffer to confirm expression of CEA, CA19-9, CGB, Cyfra21-1. As a result of analyzing ctDNA-based CEA, CA19-9, CGB, Cyfra21-1 ctDNA expression (ΔOD), clear CEA, CA19-9, CGB, Cyfra21-1 ctDNA expression (ΔOD; cutoff OD > 0.010) was observed in gastric cancer patients when temperature denaturation was not undergone or when temperature denaturation process was undergone. On the other hand, CEA, CA19-9, CGB, Cyfra21-1 ctDNA expression (ΔOD; cutoff OD > 0.010) was not observed in normal persons both when temperature denaturation was not undergone and when temperature denaturation process was undergone (Figures 59 to 61).

### Example 3.9. Confirmation of Detection of Tumor Marker Using Plasma of Pancreatic Cancer Patients

CA19-9, CA125, CEA pancreatic cancer tumor markers were detected from the plasma obtained from normal persons or pancreatic cancer patients.

Specifically, plasma was obtained from normal persons or pancreatic cancer patients, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 25 below.

**[Table 25]**

| | Sequence(5'->3') | Sequence ID NO. |
|---|---|---|
| biotinylated CEA(Prostatic Acid Phosphatase; ACPP gene) probe 1 | AATCTGAACCTCTCCTGCCAC | 23 |
| biotinylated CEA(Prostatic Acid Phosphatase; ACPP gene) probe 2 | ACCCTTCATCACCAGCAACAA | 24 |
| biotinylated CEA(Prostatic Acid Phosphatase; ACPP gene) probe 3 | TATTCTTGGCTGATTGATGGG | 25 |
| biotinylated CEA(Prostatic Acid Phosphatase; ACPP gene) probe 4 | CAGCAACAACTCCAAACCCGT | 26 |
| biotinylated CA19-9(FLU3 gene) probe 1 | GATCATCACGGCACGGTC | 30 |
| biotinylated CA19-9(FLU3 gene) probe 2 | GTGCAGAGAGATCATCACGGC | 31 |
| biotinylated CA19-9(FLU3 gene) probe 3 | TCTCTCTTACCTGGGACCTCA | 32 |

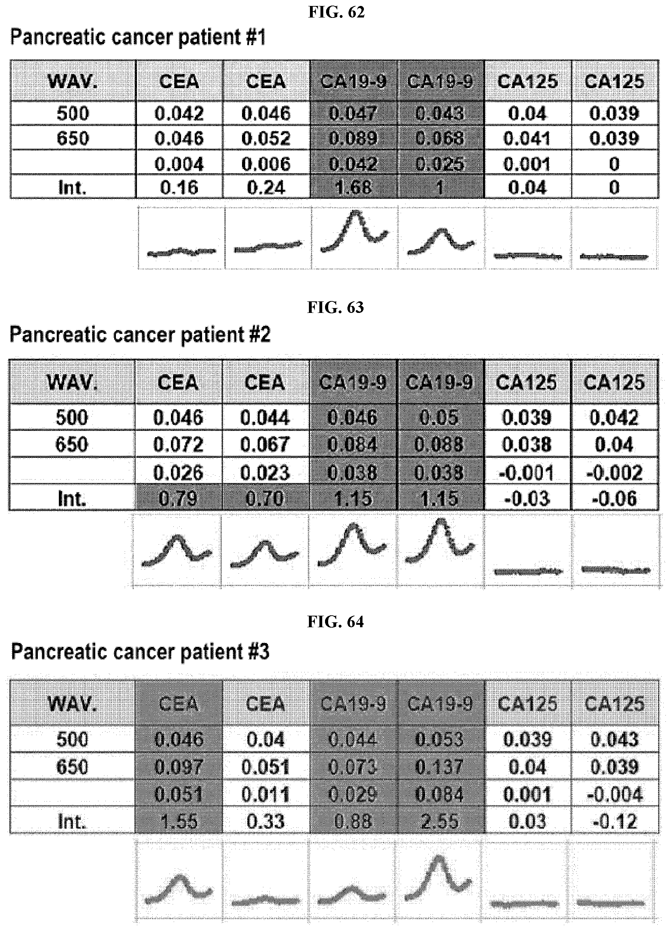

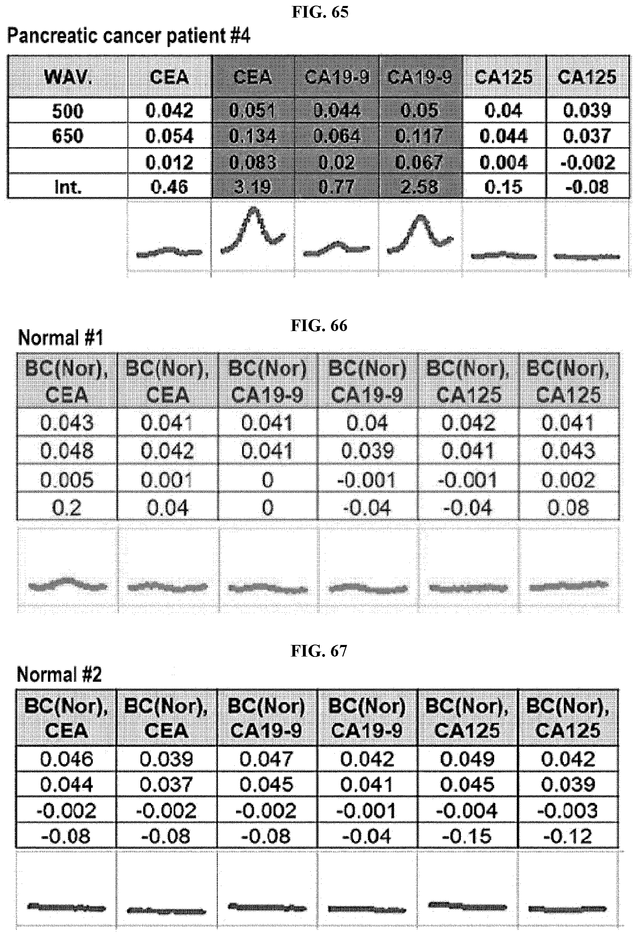

For colorimetric detection, it was treated with the solution in which TMB and H₂O₂ were added to a sodium acetate buffer to confirm expression of CA19-9, CA125, CEA. As a result of analyzing ctDNA-based CA19-9, CA125, CEA ctDNA expression (ΔOD), clear CA19-9, CA125, CEA ctDNA expression (ΔOD; cutoff OD > 0.010) was observed in pancreatic cancer patients when temperature denaturation was not undergone. On the other hand, CA19-9, CA125, CEA ctDNA expression (ΔOD; cutoff OD > 0.010) was not observed in normal persons when temperature denaturation was not undergone (Figures 62 to 69).

### IV. Evaluation of Tumor Marker Detection for Early Diagnosis and Prognosis Prediction of Cancer

### Example 4. Detection of Biomarker Derived from Cancer Patients

### Example 4.1. Detection of CPT1A Using Plasma or Urine of Cancer Patients

CPT1A(Carnitine palmitoyltransferase 1A) was detected from the plasma or urine obtained from normal persons or lung cancer patients. Also, CPT1A was detected from the urine obtained from bladder cancer patients. CPT1A can be used as a diagnostic target for cancer, since it is known that the expression of CPT1A in cancer tissue is significantly increased than in normal tissue.

Specifically, the plasma or urine was obtained, and then the nanowire was added and reacted for 20 minutes to isolate ctDNA (circulating tumor DNA). In this case, ctDNA was attached to the nanowire to form a complex. The experiments were performed i) when the complex of ctDNA and nanowire was used at a temperature of 27 °C without temperature denaturation, and ii) when the complex of ctDNA and nanowire was used after being subjected to a temperature denaturation process at a temperature of 95 °C for 1 minute, respectively. Thereafter, each biotinylated probe and HRP and streptavidin-labeled poly(pyrrole) nanoparticles (HRP/st-tagged NPs) were added and reacted further for 20 minutes. In this case, the probes used are shown in Table 26 below.

**[Table 26]**

| | Sequence(5'->3') | Sequence ID NO. |
|---|---|---|
| biotinylated CPT1A probe 1 | GAGCCATGAAGCTCTTAGACA | 88 |
| biotinylated CPT1A probe 2 | CTTCCAGACATCGCTGCCTCG | 89 |
| biotinylated CPT1A probe 3 | TGGACAATACCTCGGAGCCTC | 90 |
| biotinylated CPT1A probe 4 | CATGACCCGGCTCTTCCGAGA | 91 |

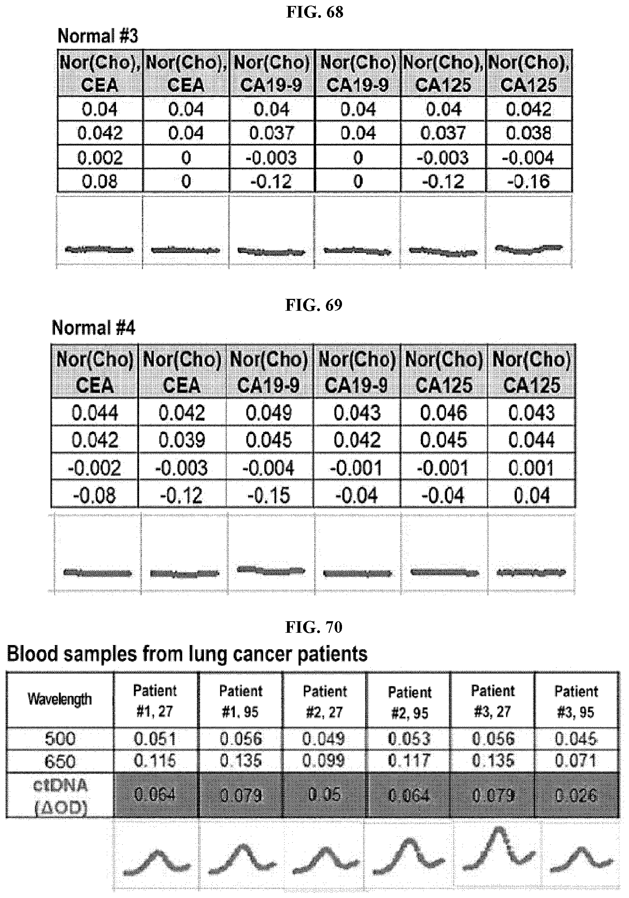

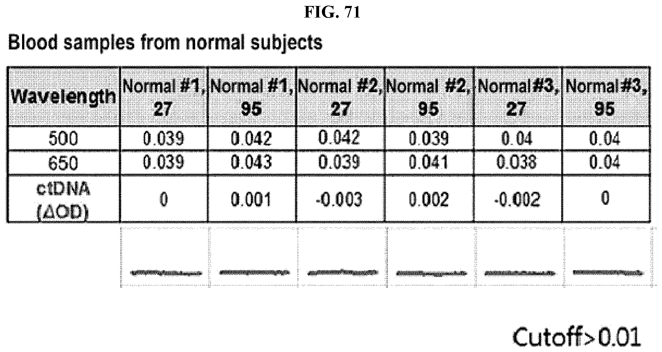

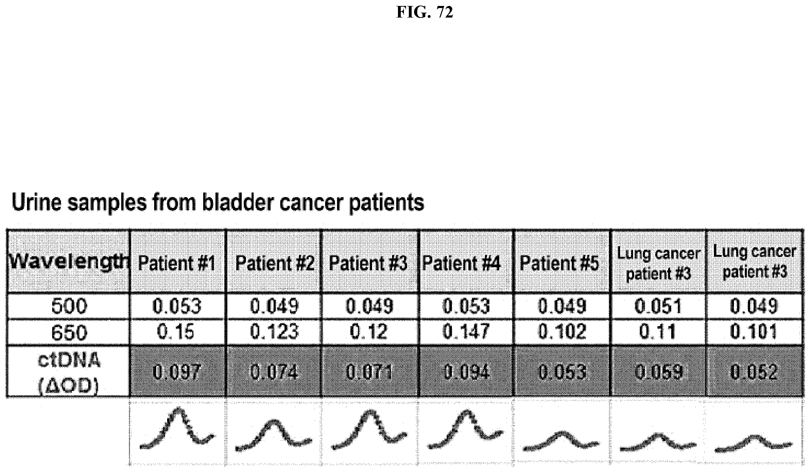

For colorimetric detection, it was treated with the solution in which TMB and H₂O₂ were added to a sodium acetate buffer to confirm expression of CPT1A. As a result of analyzing ctDNA-based CPT1A ctDNA expression (ΔOD), clear CPT1A ctDNA expression (ΔOD; cutoff OD > 0.010) was observed in lung or bladder cancer patients when temperature denaturation was not undergone or when temperature denaturation process was undergone. On the other hand, CPT1A ctDNA expression (ΔOD; cutoff OD > 0.010) was not observed in normal persons both when temperature denaturation was not undergone and when temperature denaturation process was undergone (Figures 70 to 73).

### Example 4.2. Confirmation of IFN-γ, IFN-γ Receptor, and PD-L1 Expression Using Cancer Cell Line

The accuracy for detection of IFN-γ, IFN-γ receptor, and PD-L1 was evaluated using MDA-MB-231, HCC827, H1975, PC9, and H460 cancer cell lines known as PD-L1 positive cancer cell lines and A549, MDA-MB-461, HeLa, and MCF7 cancer cell lines known as PD-L1 negative cancer cell lines obtained from ATCC and Korean Cell Line Bank. In this case, PD-L1 positive cancer cell lines and PD-L1 negative cancer cell lines were classified based on the mRNA level of PD-L1 of each cancer cell line provided by CCLE (Cancer Cell Line Encyclopedia). 1×10⁶ cells of the same cells were cultured in 100 mm dishes, and then 10 nmol INF-γ was added in one dish, and IFN-γ was not treated in the other dish, and they were cultured for one day, and then genomic DNA was extracted from the PD-L1 positive cancer cell line and the PD-L1 negative cancer cell line.

Thereafter, it was sonicated to produce fDNA of 200 bp or less. 50 ng/µL fDNA was added to PBS, and then the nanowires were added and reacted for 20 minutes to isolate them. Thereafter, a temperature denaturation process was undergone at a temperature of 95 °C for 1 minute, and then a biotinylated probe and streptavidin labeled poly(pyrrole) nanoparticles (HRP/st-tagged NPs) were added and reacted further for 20 minutes. In this case, the probes used are shown in Table 27 below.

**[Table 27]**

| | Sequence(5'->3') | Sequence ID NO. |
|---|---|---|
| Biotinylated PD-L1 probe 1 | TGGGAGCCATCTTATTATGCC | 1 |
| Biotinylated PD-L1 probe 2 | CGGAAGATGAATGTCAGTGC | 2 |
| Biotinylated PD-L1 probe 3 | CCTACTGGCATTTGCTGAACG | 3 |
| Biotinylated PD-L1 probe 4 | ACCATAGCTGATCATGCAGCG | 4 |
| Biotinylated IFN-γ probe 1 | ATCGTATATTGGGAGTACCAG | 92 |
| Biotinylated IFN-γ probe 2 | TAAATGGAGACGAGCAGGAAG | 93 |
| Biotinylated IFN-γ probe 3 | TAGTGCTTAGCCTGGTATTCA | 94 |
| Biotinylated PD1 probe 1 | GTACCGCATGAGCCCCAGCAA | 95 |
| Biotinylated PD1 probe 2 | GTTCCAAACCCTGGTGGTTGG | 96 |
| Biotinylated PD1 probe 3 | GAGCAGACGGAGTATGCCAC | 97 |
| Biotinylated IFN-γreceptor probe 1 | ATCGTATATTGGGAGTACCAG | 98 |
| Biotinylated IFN-γ receptor probe 2 | TAAATGGAGACGAGCAGGAAG | 99 |
| Biotinylated IFN-γ receptor probe 3 | TAGTGCTTAGCCTGGTATTCA | 100 |

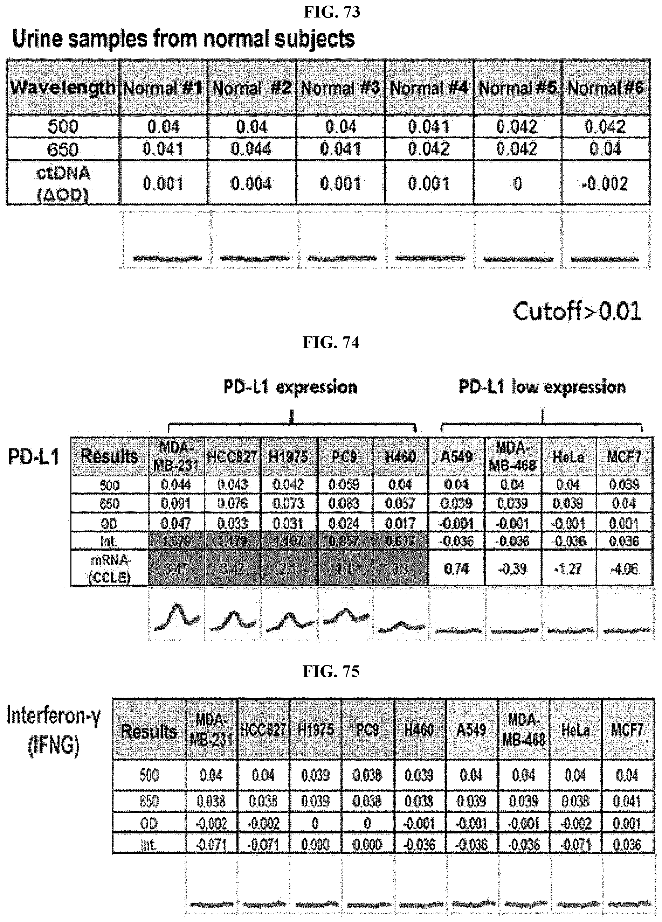

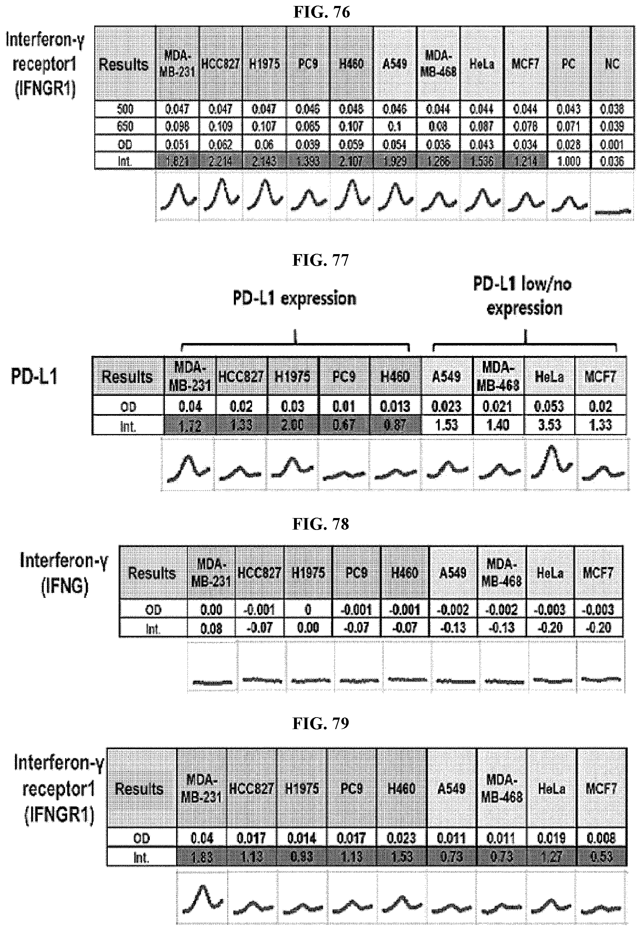

For colorimetric detection, it was treated with the solution in which TMB and H₂O₂ were added to a sodium acetate buffer to confirm the results of IFN-γ, IFN-γ receptor, and PD-L1 detection. First, when IFN-γ was not added, as a result of analyzing the DNA-based PD-L1 expression (ΔOD) of cancer cell lines, clear PD-L1 DNA expression (cutoff: OD > 0.6) in fDNA was observed only in MDA-MB-231, HCC827, H1975, PC9, and H460 cancer cell lines in all three experiments. On the other hand, the PD_L1 DNA expression was not observed in A549, MDA-MB-461, HeLa, and MCF7 cancer cell lines in all three experiments (Figures 74 to 76). However, when IFN-γ was added, as a result of analyzing DNA-based PD-L1 expression (ΔOD) of cancer cell lines, clear fDNA-based PD-L1 DNA expression (cutoff: OD > 0.6) was observed in MDA-MB-231, HCC827, H1975, PC9, and H460 PD-L1 positive cancer cell lines, as well as PD-L1 negative cancer cell line, i.e., A549, MDA-MB-461, HeLa, and MCF7 in all three experiments (Figure 77). In addition, as a result of confirming IFN-γ and IFN-γ receptor through fDNA of PD-L1 positive cancer cell lines of MDA-MB-231, HCC827, H1975, PC9, and H460 and PD-L1 negative cancer cell line, i.e., A549, MDA-MB-461, HeLa, and MCF7, IFN-γ was not confirmed regardless of whether IFN-γ was added in all three experiments, and the IFN-γ receptor was confirmed in all cell lines (Figure 78 and Figure 79).

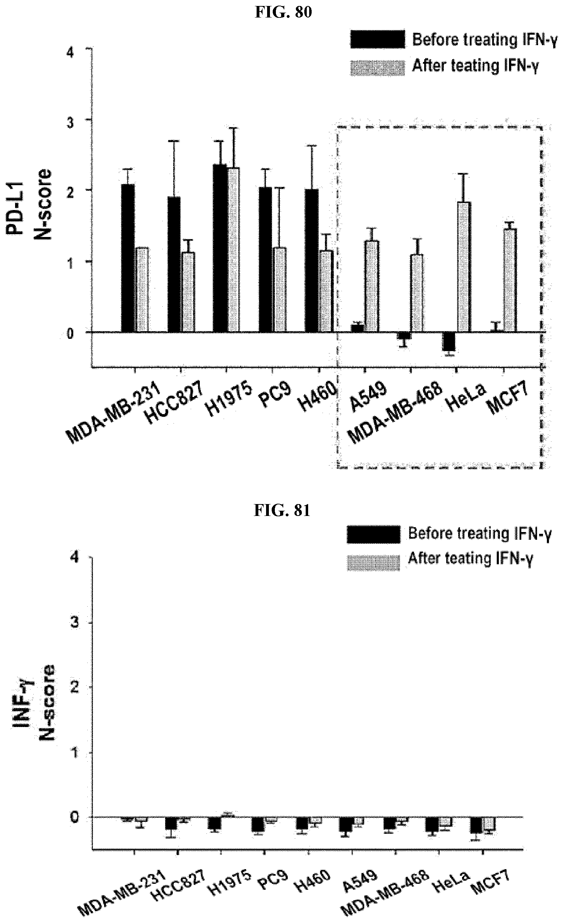

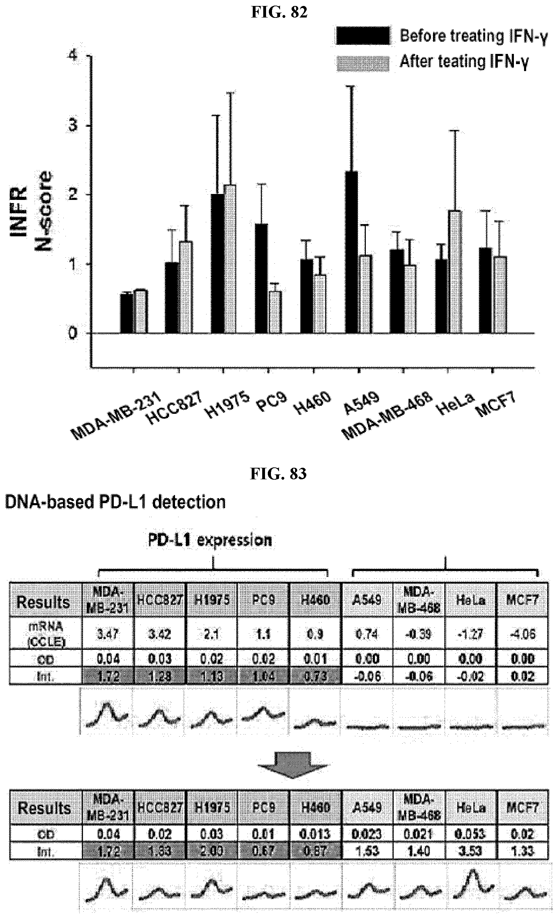

Furthermore, when IFN-γ was not added and when IFN-γ was added, in a graph of analyzing PD-L1, IFN-γ, and IFN-γ receptor through fDNA of PD-L1 positive cancer cell lines of MDA-MB-231, HCC827, H1975, PC9, and H460 and PD-L1 negative cancer cell lines of A549, MDA-MB-461, HeLa, and MCF7 in three repeated experiments, high PD-L1 expression was confirmed in PD-L1 negative cancer cell lines when IFN-γ was added, whereas IFN-γ was not confirmed regardless of whether IFN-γ was added or not, and IFN-γ receptor was confirmed in all cell lines (Figures 80 to 83).

Figures 80 to 83 show graphs for analyzing DNA-based PD-L1 expression (ΔOD) of cancer cell lines before and after adding IFN-γ to PD-L1 positive and negative cancer cell lines (Figures 80 to 83).

### V. Confirmation of Possibility of Detecting Cancer-Related cfDNA According to Obtaining Method

### Example 5. Confirmation of Biomarker Detection According to Method for Collecting Sample

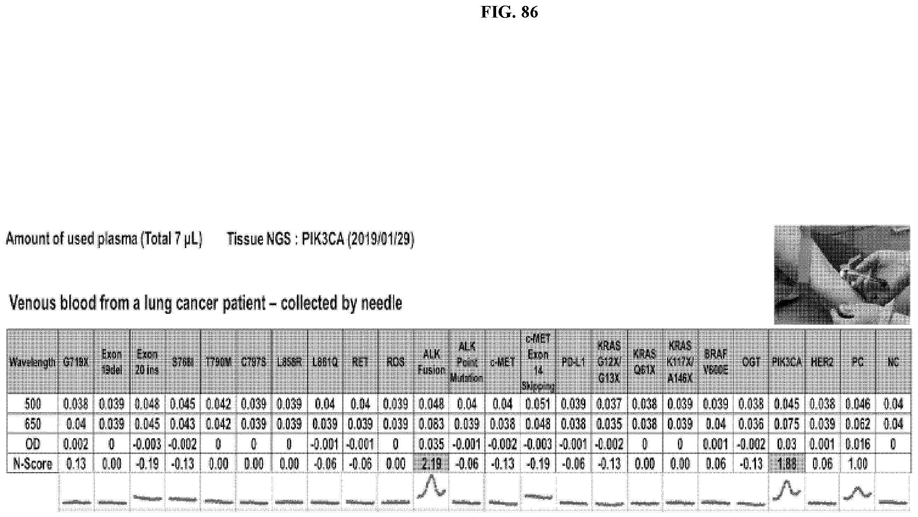

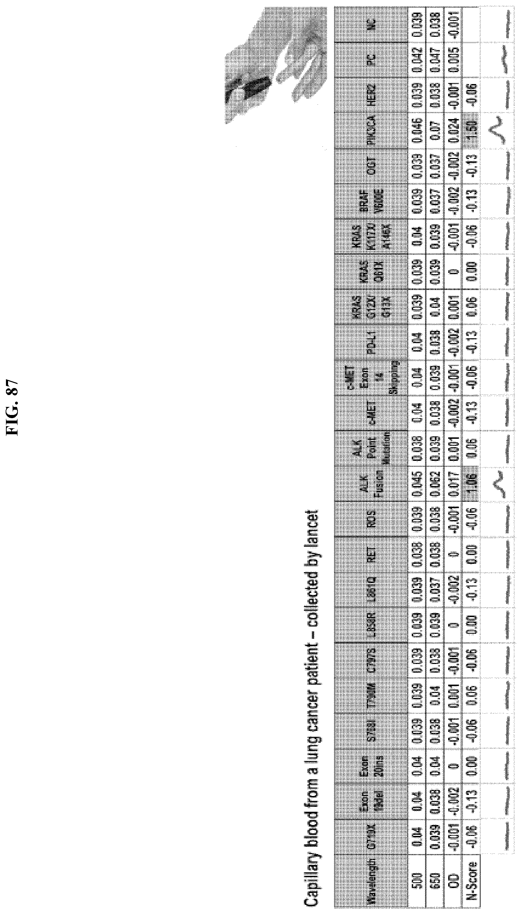

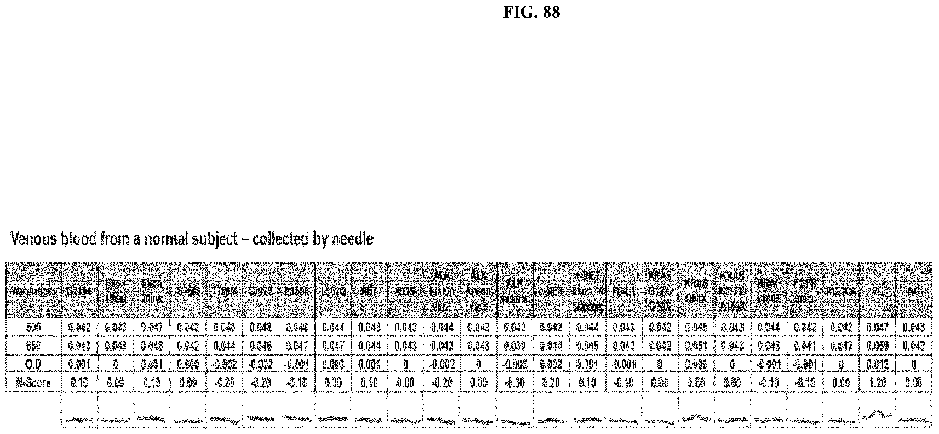

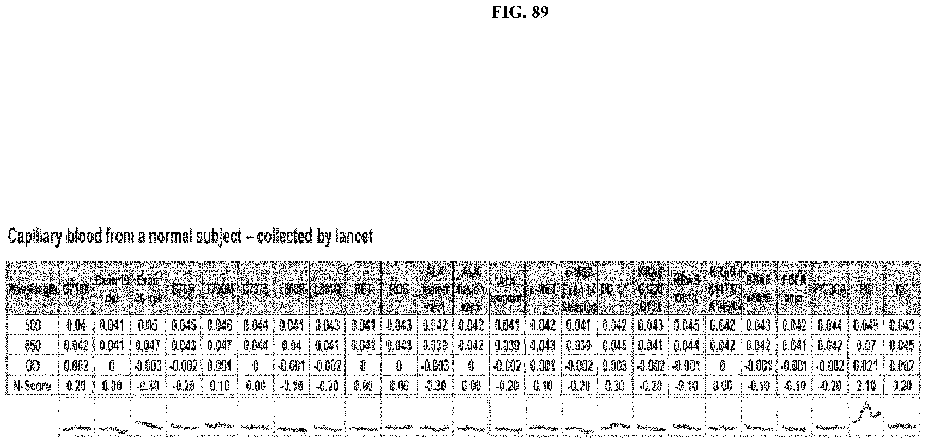

The cfDNA was detected in lung cancer patients and normal persons in the same manner as described above. However, in the collection method, it was collected from venous blood using an injection needle and it was collected from capillary blood using a lancet. The nanowires and probes used were those prepared according to Preparation Example 1 and Preparation Example 3. A level of DNA expression of a cancer-related biomarker such as AKL Fusion and PIK3CA from the blood obtained from lung cancer patients using an injection needle was shown (Figure 86). In addition, a level of DNA expression of a cancer-related biomarker such as AKL Fusion and PIK3CA from the blood obtained from lung cancer patients using a lancet was shown (Figure 87). A level of DNA expression of a cancer-related biomarker such as AKL Fusion and PIK3CA from the blood obtained from normal persons using an injection needle was shown (Figure 88). In addition, a level of DNA expression of a cancer-related biomarker such as AKL Fusion and PIK3CA from the blood obtained from normal persons using a lancet was measured (Figure 89). As a result, it was confirmed that cancer-derived biomarkers could be detected in venous blood and capillary blood in the same way.

### VI. Confirmation of Possibility of Detecting Lung Cancer-Related cfDNA

### Example 6. Confirmation of Detection of Mutation Biomarker in Lung Cancer Cell line

The presence or absence of a mutation (snp) occurring in a cancer-specific gene provides useful information to confirm whether there is resistance to a specific treatment method or whether it has resistance to a specific treatment method, and thus to find an appropriate treatment method.

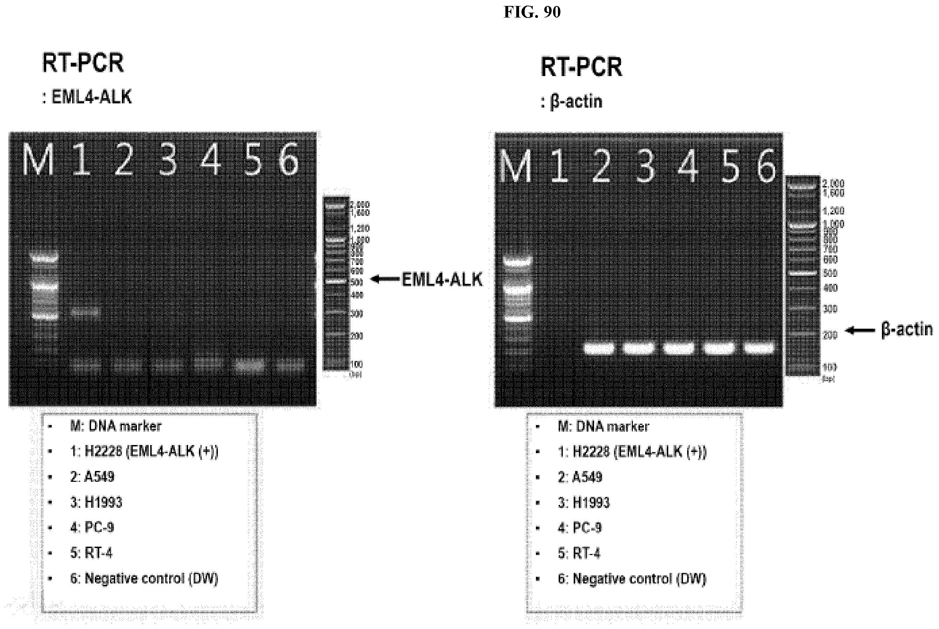

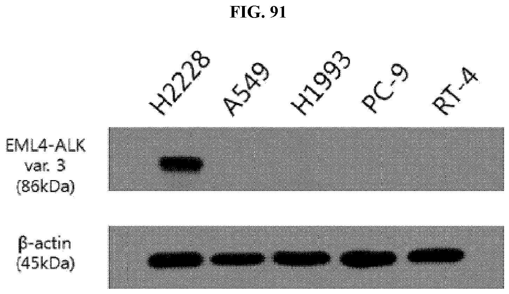

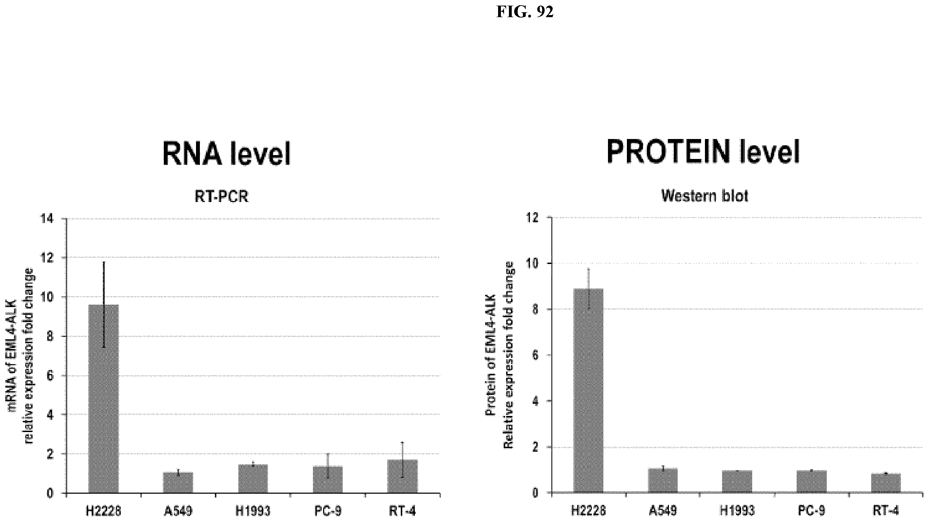

In order to confirm that a mutation of the lung cancer cell line was also detectable, the EML4-ALK gene was analyzed. First, in order to confirm the expression level, the expression level of the EML4-ALK gene was confirmed by RT-PCR. A level of EML4-ALK expression from cfDNA of EML4-ALK variant 3a/b positive cell (H2228) and EML4-ALK negative cell (A549, H1993, PC9, RT4) cancer cell lines was confirmed through RT-PCR (Figure 90). In addition, a level of EML4-ALK expression from cfDNA of EML4-ALK variant 3a/b positive cell (H2228) and EML4-ALK negative cell (A549, H1993, PC9, RT4) cancer cell lines was confirmed through Western blot (Figure 91). In addition, a result of confirming a level of EML4-ALK expression from cfDNA of EML4-ALK variant 3a/b positive cell (H2228) and EML4-ALK negative cell (A549, H1993, PC9, RT4) cancer cell lines through RT-PCR and Western blot was shown (Figure 92).

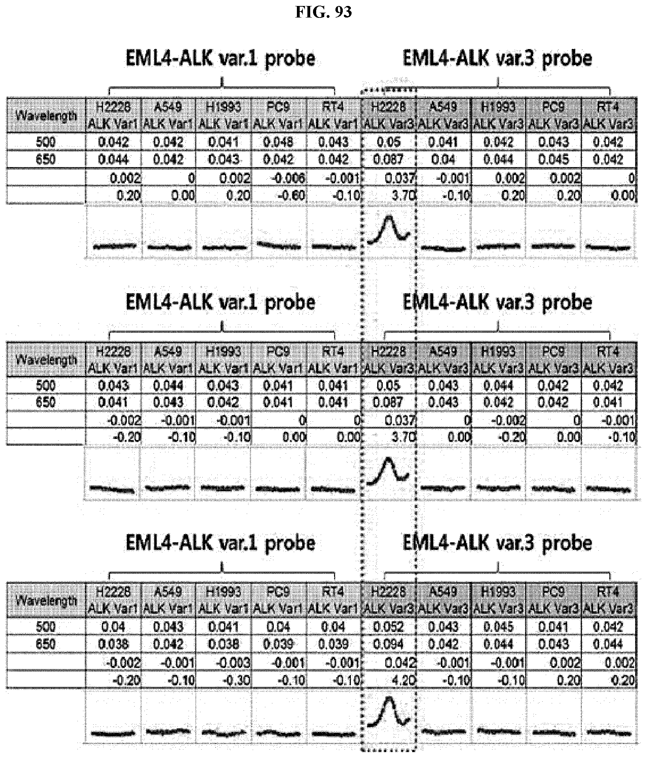

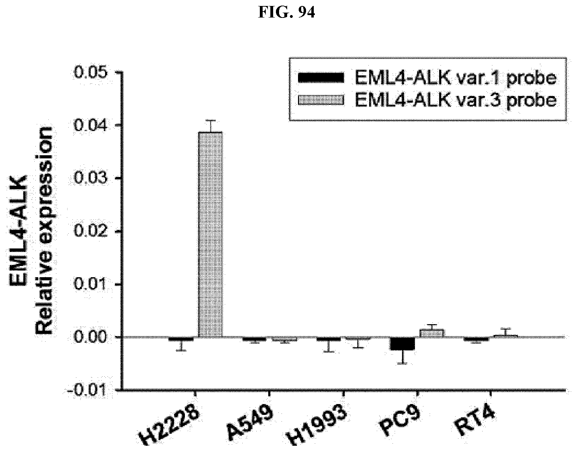

Thereafter, EML4-ALK was detected by a method using fDNA described herein. The detection method was performed in the same manner as described above. As a result, it was confirmed that EML4-ALK can be also detected using the fDNA detection method. A measured level of DNA expression of EML4-ALK fusion var.1 or EML4-ALK fusion var.3 from cfDNA of EML4-ALK variant 3a/b positive cell (H2228) and EML4-ALK negative cell (A549, H1993, PC9, RT4) cancer cell lines was shown (Figure 93). In addition, a measured level of DNA expression of EML4-ALK fusion var.1 or EML4-ALK fusion var.3 from cfDNA of EML4-ALK variant 3a/b positive cell (H2228) and EML4-ALK negative cell (A549, H1993, PC9, RT4) cancer cell lines was shown (Figure 94).

**[Table 28]**

| EGFR | Probe sequences |
|---|---|
| KRAS exon2-probe | CP1: AAATGACTGAATATAAACTTG (Sequence ID NO. 127) DP: GAGTGCCTTGACGATACAGCT (Sequence ID NO. 128) |
| ALK-EML4 variant 1-probe | CP2: TAGAGCCCACACCTGGGAAA (Sequence ID NO. 129) DP: CGGAGCTTGCTCAGCTTGTA (Sequence ID NO. 130) |
| ALK-EML4 variant 3 -probe | CP3: GCATAAAGATGTCATCATCAACCAAG (Sequence ID NO. 131) DP: CGGAGCTTGCTCAGCTTGTA (Sequence ID NO. 132) |

### Example 7. Confirmation of Detection of Mutation Biomarker in Lung Cancer Patients: Drug Resistance

Various mutations were detected in lung cancer patients in the same manner as described above. In this case, as a probe for detecting cfDNA of lung cancer patients, a nucleic acid sequence complementarily binding to the lung cancer biomarker gene described above was used. Patient information and analysis genes are as described in the figures.

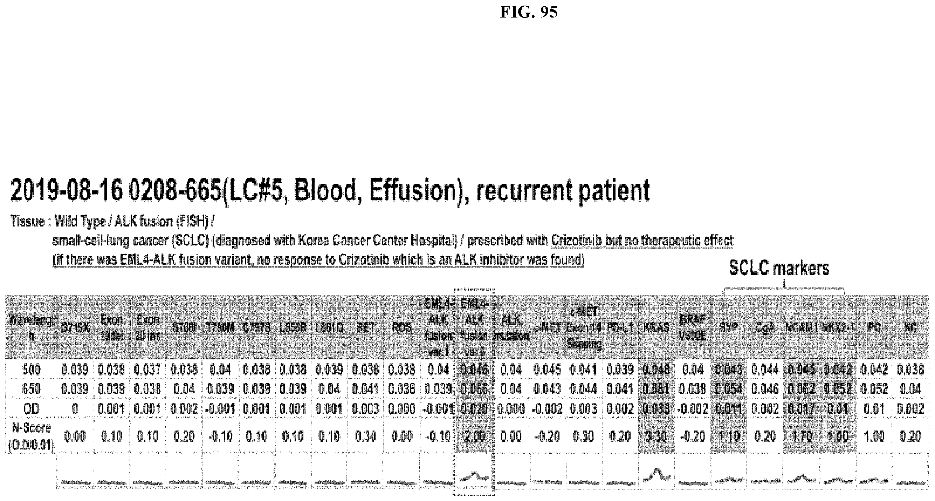

Specifically, a level of DNA expression of a cancer-related biomarker such as EML4-ALK fusion var.3, KRAS, SYP, NCAM1, and NKX2-1 from the blood obtained from small cell lung cancer patients was measured (Figure 95). As a result, EML4-ALK fusion was found to be the same in ctDNA in cancer tissue and blood, and as a result of ctDNA, EML4-ALK fusion was found to be var.3, not var.1, indicating that Crizotinib, an ALK TKI, was not well responsive.

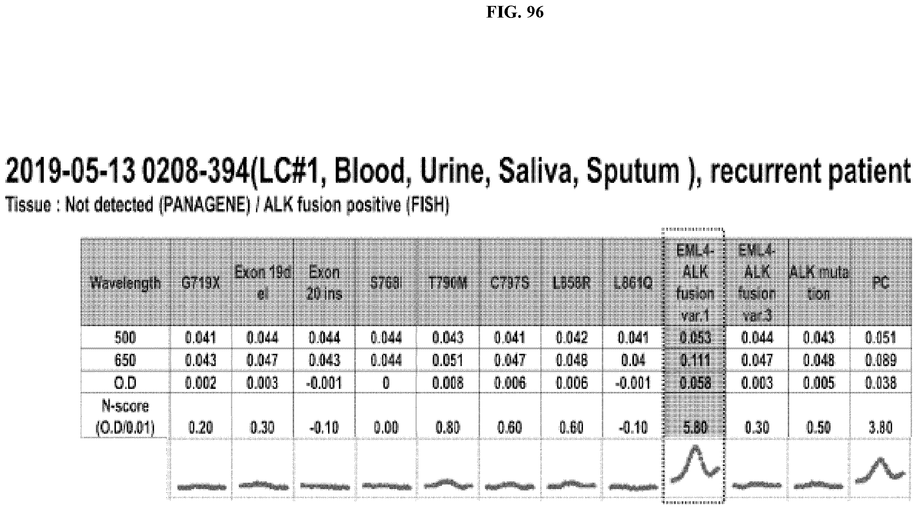

In addition, a level of DNA expression of a cancer-related biomarker such as EML4-ALK fusion var.1 from the blood obtained from cancer patients was measured (Figure 96). As a result, EML4-ALK fusion was found to be the same in ctDNA in cancer tissue and blood, and as a result of ctDNA, EML4-ALK fusion was found to be var.1, not var.3, indicating that Crizotinib, an ALK TKI, was well responsive and patient's response of partial response (PR) was obtained.

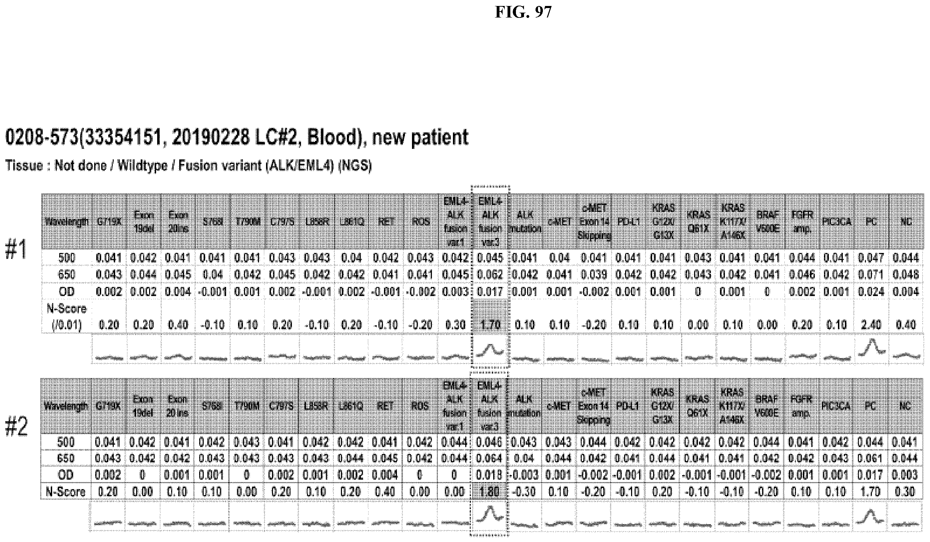

In addition, a level of DNA expression of a cancer-related biomarker such as EML4-ALK fusion var.3 from the blood obtained from cancer patients was measured (Figure 97). As a result, EML4-ALK fusion was found to be the same in ctDNA in cancer tissue and blood, and as a result of ctDNA, EML4-ALK fusion was found to be var.3, not var.1, indicating that Crizotinib, an ALK TKI, would not be well responsive, and thus alectinib was prescribed from the beginning to await patient's response.

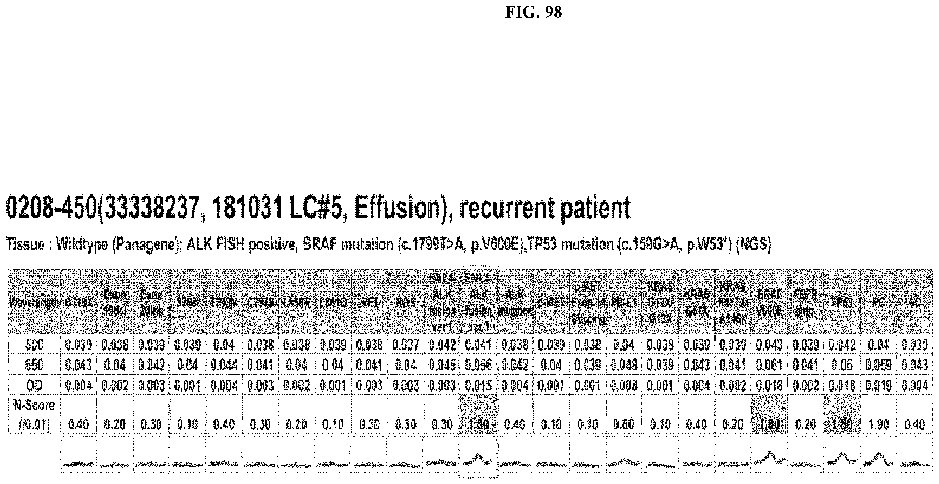

In addition, a level of DNA expression of a cancer-related biomarker such as EML4-ALK fusion var.3, BRAFV800E, and TP53 from the blood obtained from cancer patients was measured (Figure 98). As a result, EML4-ALK fusion was found to be the same in ctDNA in cancer tissue and blood, and as a result of ctDNA, EML4-ALK fusion was found to be var.3, not var.1, indicating that Crizotinib, an ALK TKI, was not well responsive (PD).

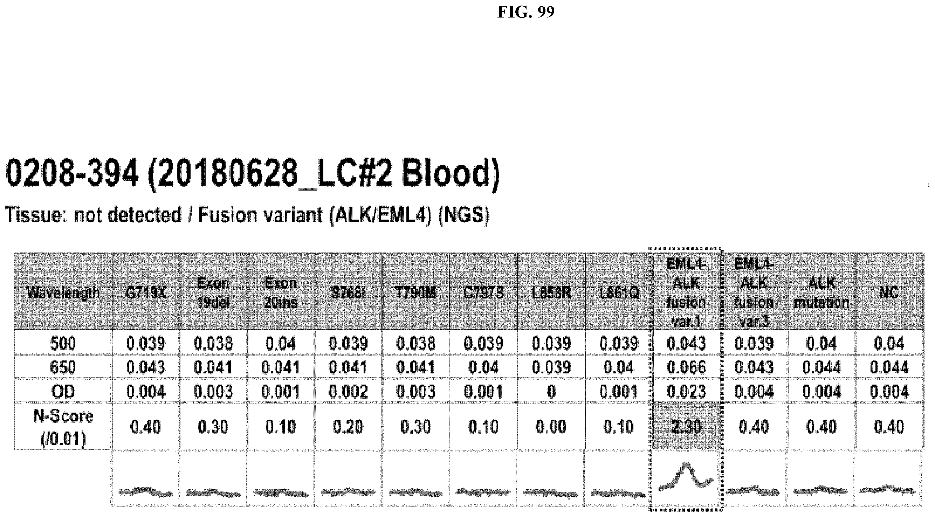

In addition, a level of DNA expression of a cancer-related biomarker such as EML4-ALK fusion var.1 from the blood obtained from cancer patients was measured (Figure 99). As a result, EML4-ALK fusion was found to be the same in ctDNA in cancer tissue and blood, and as a result of ctDNA, EML4-ALK fusion was found to be var.1, not var.3, indicating that Crizotinib, an ALK TKI, was well responsive and patient's response of partial response (PR) was obtained.

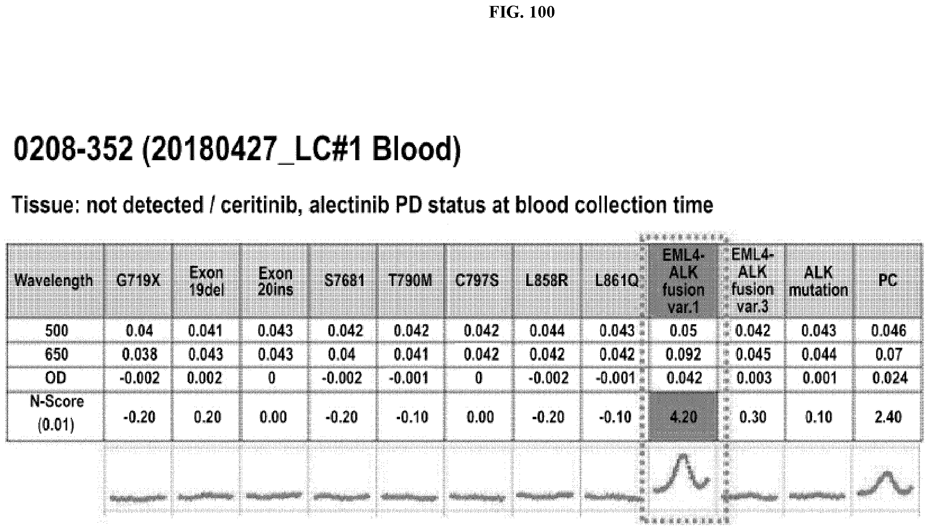

In addition, a level of DNA expression of a cancer-related biomarker such as EML4-ALK fusion var.1 from the blood obtained from cancer patients was measured (Figure 100). As a result, EML4-ALK fusion was found to be the same in ctDNA in cancer tissue and blood, and as a result of ctDNA, EML4-ALK fusion was found to be var.1, not var.3, indicating that an ALK TKI was well responsive and patient's response of partial response (PR) was obtained.

### VII. Confirmation of Possibility of Diagnosing Bladder Cancer through OGT Detection

### Example 8. Confirmation of Expression Level of OGT Derived from Cancer Cell Line and fDNA Detection Possibility

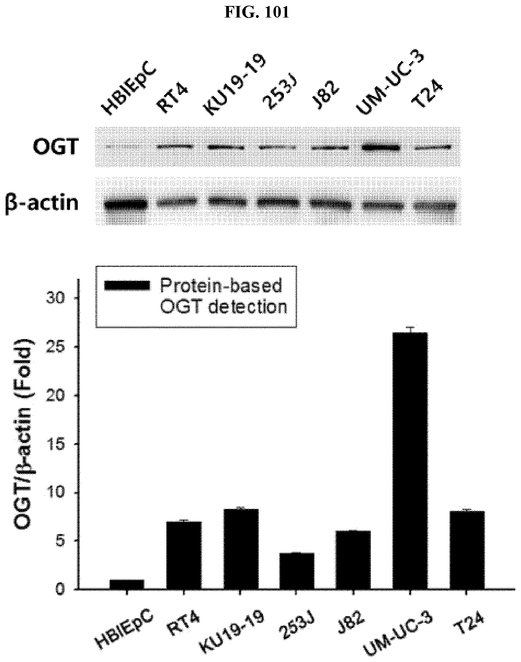

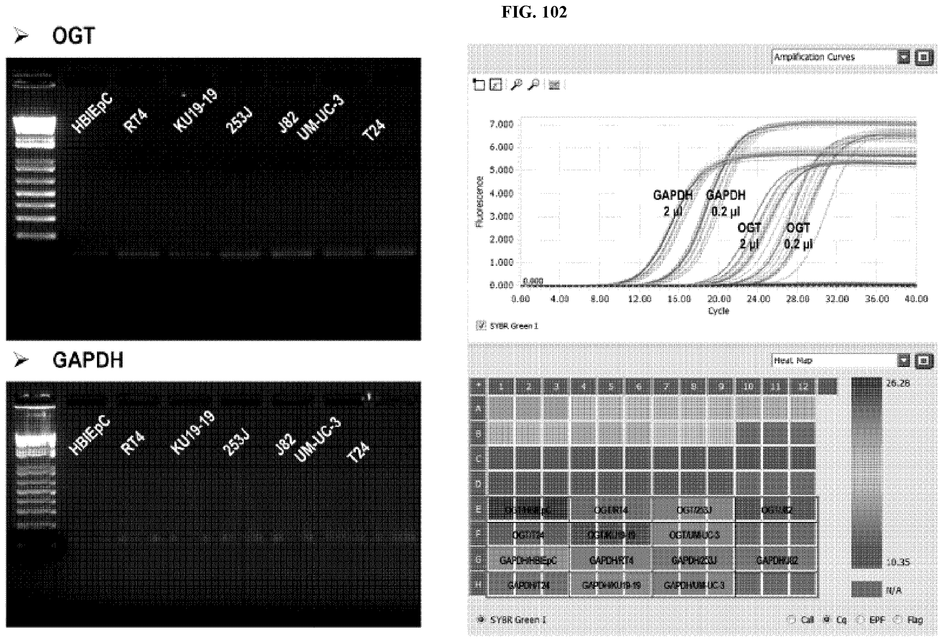

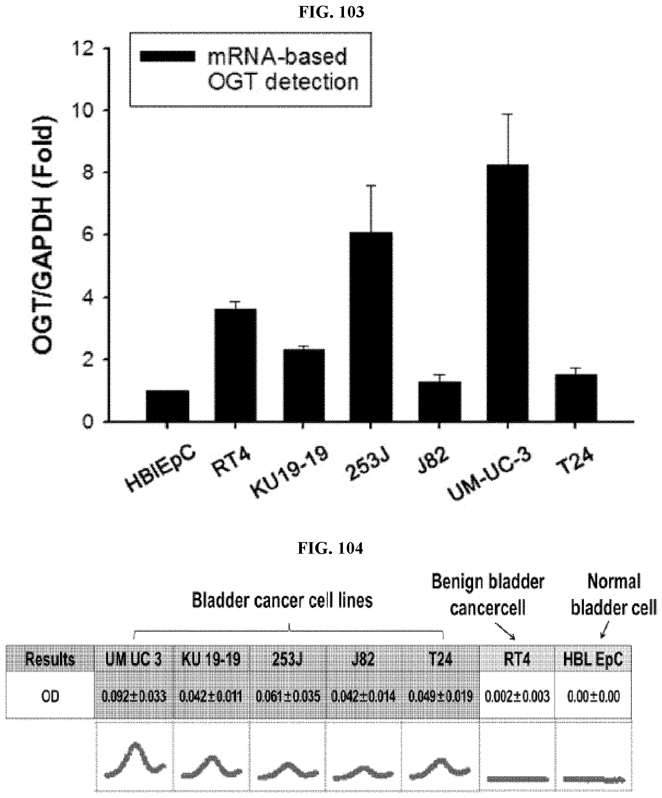

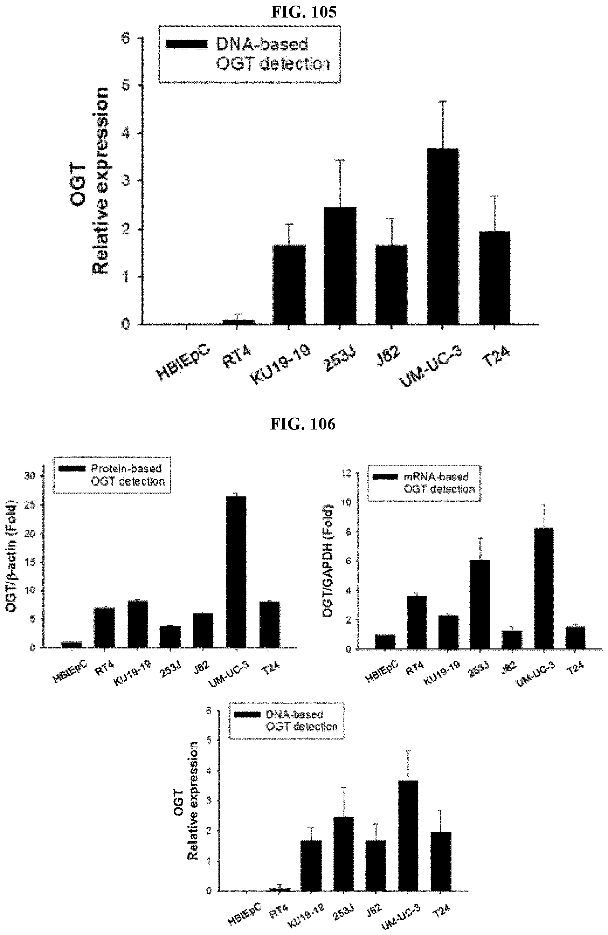

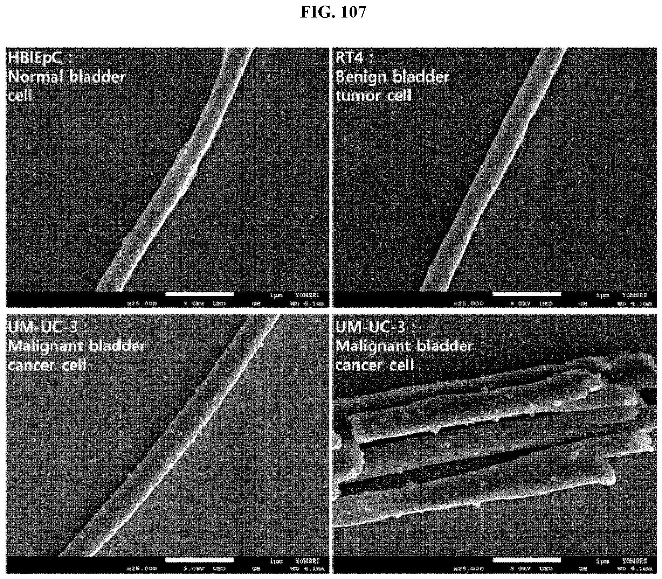

In order to confirm the expression level of OGT in the cancer cell line and the possibility of detection in patient's plasma, experiment was conducted using an in vitro experiment and a patient's blood sample in the same manner as described above. Figure 101 shows a result of confirming a level of protein expression of OGT from cfDNA of each cancer cell line *in vitro* through Western blot. In addition, Figure 102 shows a result of confirming a level of mRNA expression of OGT from cfDNA of each cancer cell line *in vitro* through RT-PCR. Figure 103 shows a result of confirming a level of mRNA expression of OGT from cfDNA of each cancer cell line *in vitro* through RT-PCR. Figure 104 shows a result of measuring a level of DNA expression of OGT from cfDNA of each cell line *in vitro.* Figure 105 shows a result of measuring a level of DNA expression of OGT from cfDNA of each cell line *in vitro.* Figure 106 shows a result of confirming a level of expression of OGT from each cell line *in vitro* through Western blot, RT-PCR, and cfDNA detection. Figure 107 shows a photograph of cfDNA of OGT detected in the nanowires that do not comprise magnetic nanoparticles from each cell line *in vitro.*

### Example 9. Confirmation of Detection of OGT-Derived cfDNA

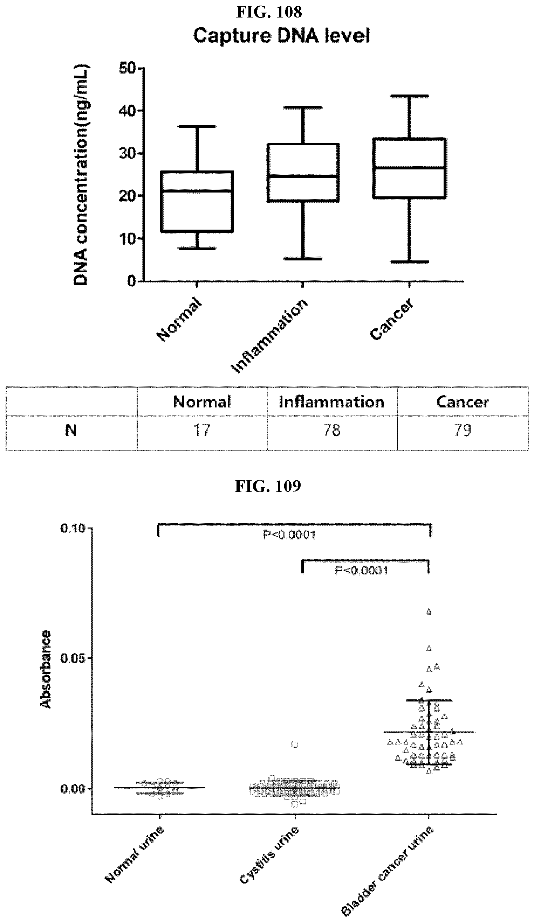

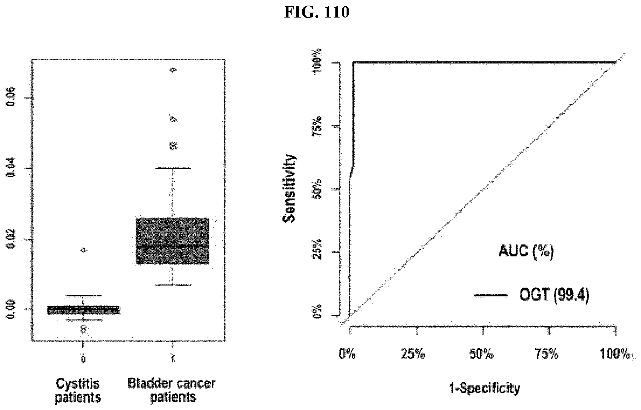

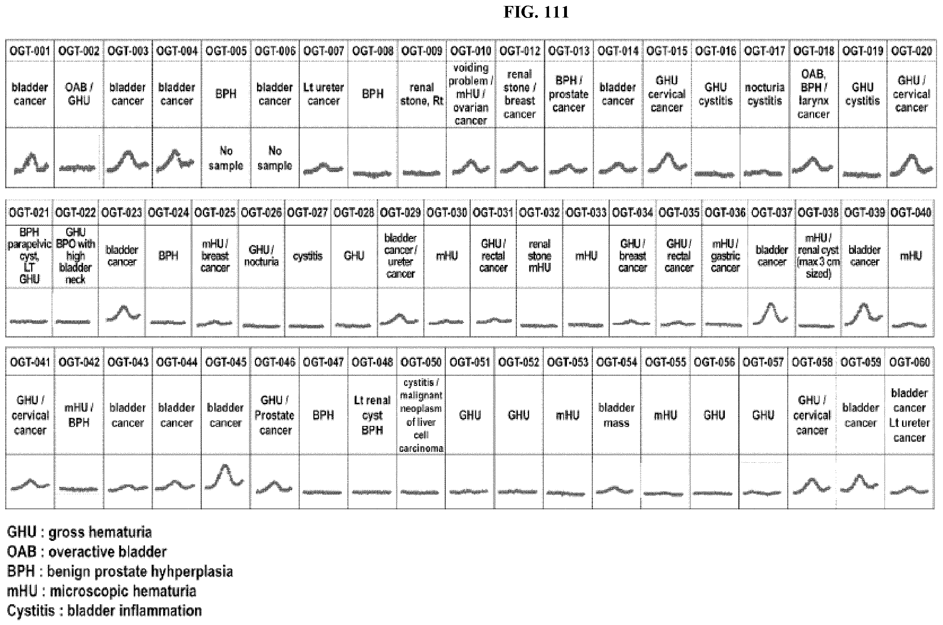

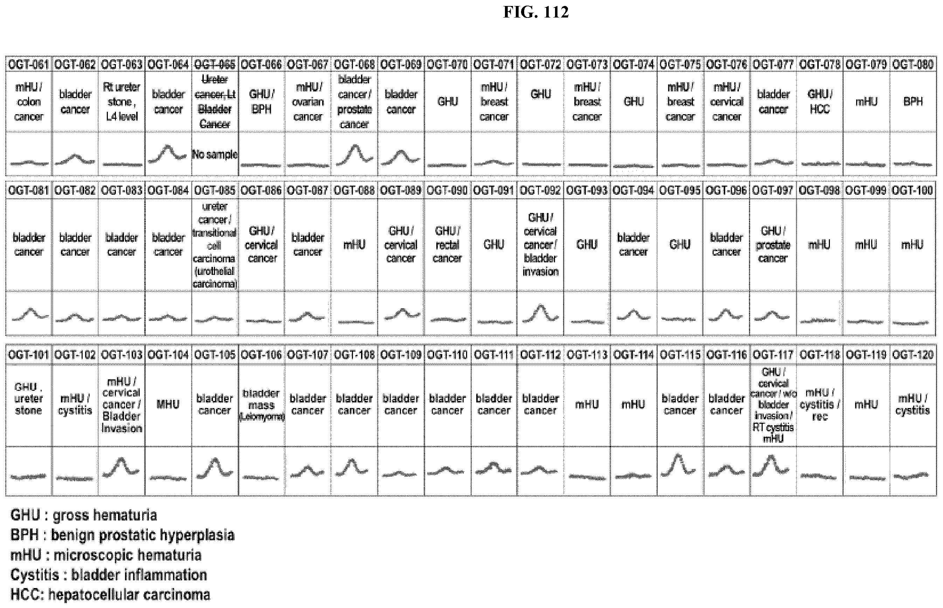

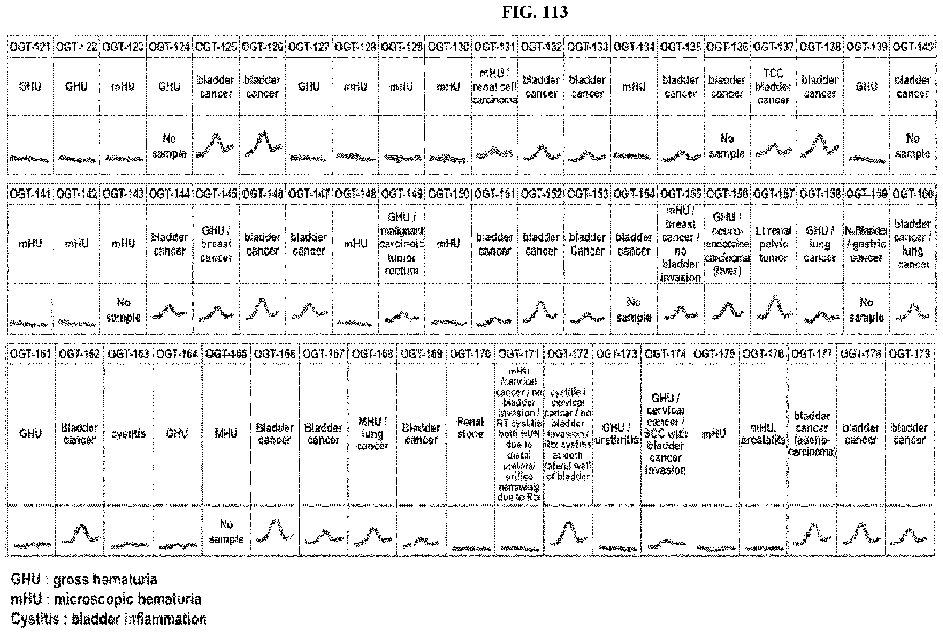

The possibility of diagnosis of bladder cancer patients was confirmed using the detection method of the present invention in the same manner as described above. The results are shown in Figures 108 to 113. Figure 108 shows a graph of quantifying cfDNA obtained from the urine of normal persons, cystitis patients, and bladder cancer patients. Figure 109 shows a result of analyzing a level of DNA expression of OGT from cfDNA obtained from the urine of normal persons, cystitis patients, and bladder cancer patients. Figure 110 shows a result of analyzing a level of DNA expression of OGT from cfDNA obtained from the urine of normal persons, cystitis patients, and bladder cancer patients. Figures 111 to 113 show results of analyzing a level of DNA expression of OGT from cfDNA obtained from the urine of several cancer patients as a blind test. As a result, it was confirmed that bladder cancer can be diagnosed by detecting OGT using the method described above.

### VIII. Confirmation of Possibility of Diagnosis of Various Cancer

### Example 10. Confirmation of Possibility of Diagnosis of Thyroid Cancer

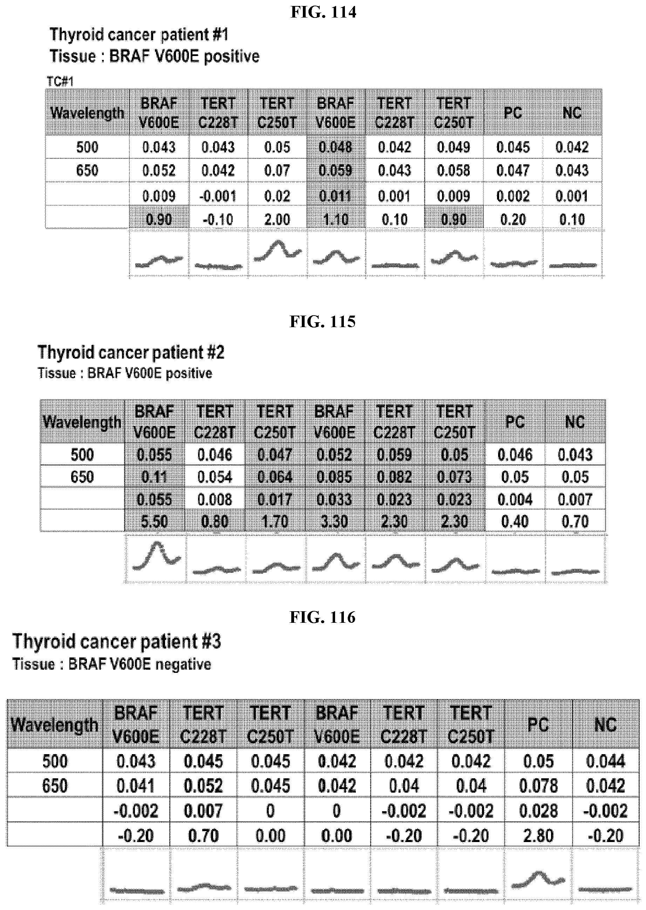

Using the same method as described above, the patient's cfDNA was detected to confirm whether it is possible to diagnose thyroid cancer. Figures 114 to 116 show results of analyzing a level of DNA expression of BRAF V600E and TERT C250T from cfDNA obtained from the tissue of thyroid cancer patients. As a result of the analysis, it was confirmed that the thyroid cancer can be accurately diagnosed through the analysis method of the present invention.

### Example 11. Confirmation of Possibility of Diagnosis of Small Cell Lung Cancer(SCLC)

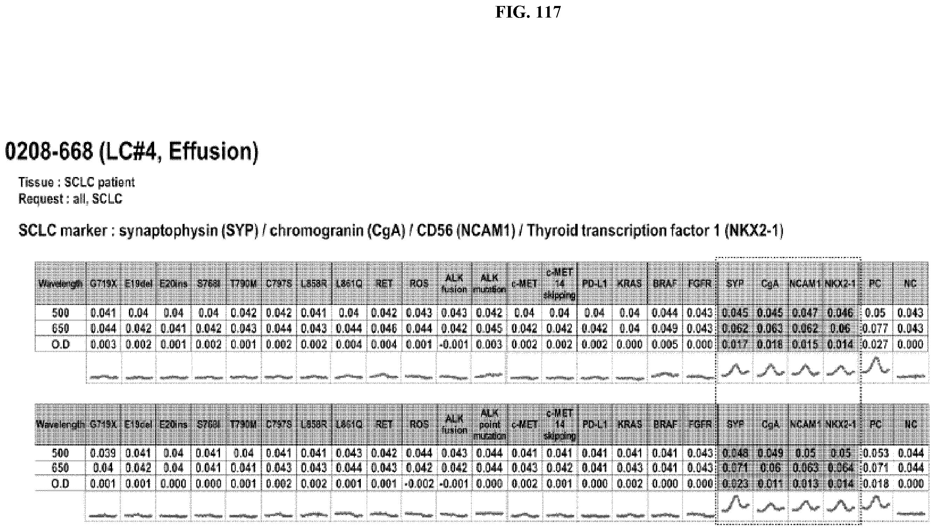

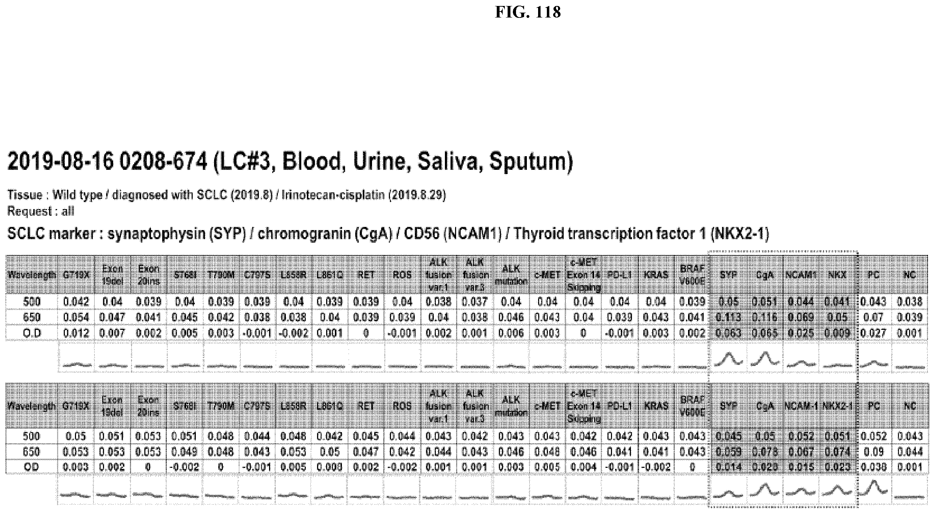

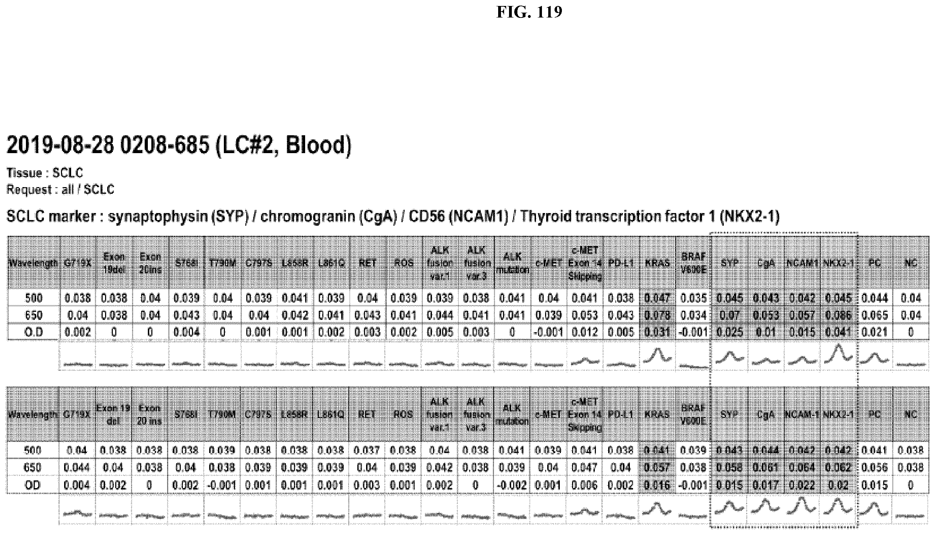

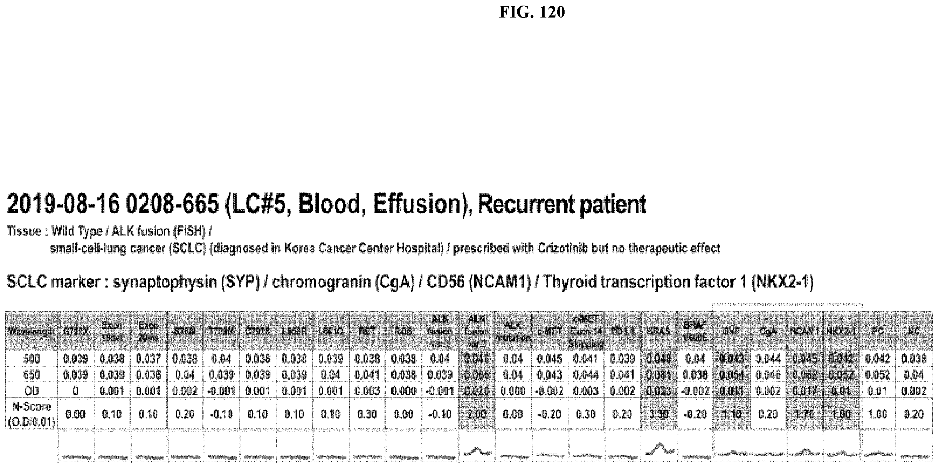

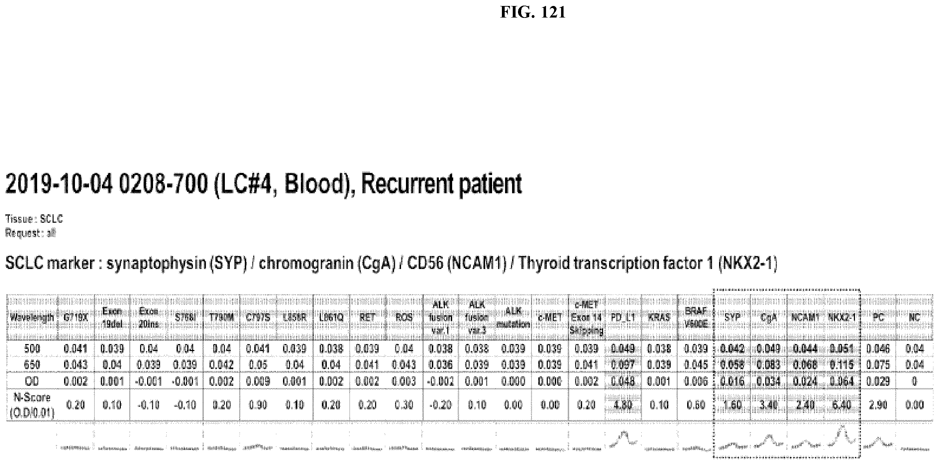

Using the same method as described above, cancer-related biomarkers such as SYP, CgA, NCAM1, and NKX2-1 were analyzed using blood obtained from small cell lung cancer patients(Figures 117 to 121). Patient information and sample information are as shown in the figures. As a result of the analysis, it was confirmed that the small cell lung cancer can be accurately diagnosed through the analysis method of the present invention.

### Example 12. Confirmation of Possibility of Diagnosis of Non Small Cell Lung Cancer(NSCLC)

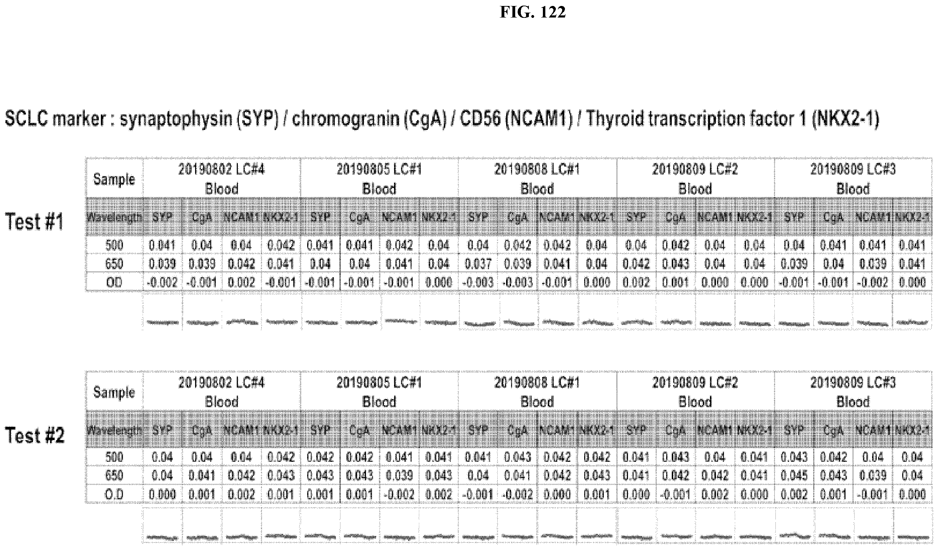

Using the same method as described above, biomarkers such as SYP, CgA, NCAM1, and NKX2-1 were analyzed using blood obtained from non-small cell lung cancer patients(Figure 122). Patient information and sample information are as shown in the figure. As a result of the analysis, it was confirmed that the non-small cell lung cancer can be accurately diagnosed through the analysis method of the present invention.

### Example 13. Comparison of Biomarker Detection Before and After Anti-cancer Treatment

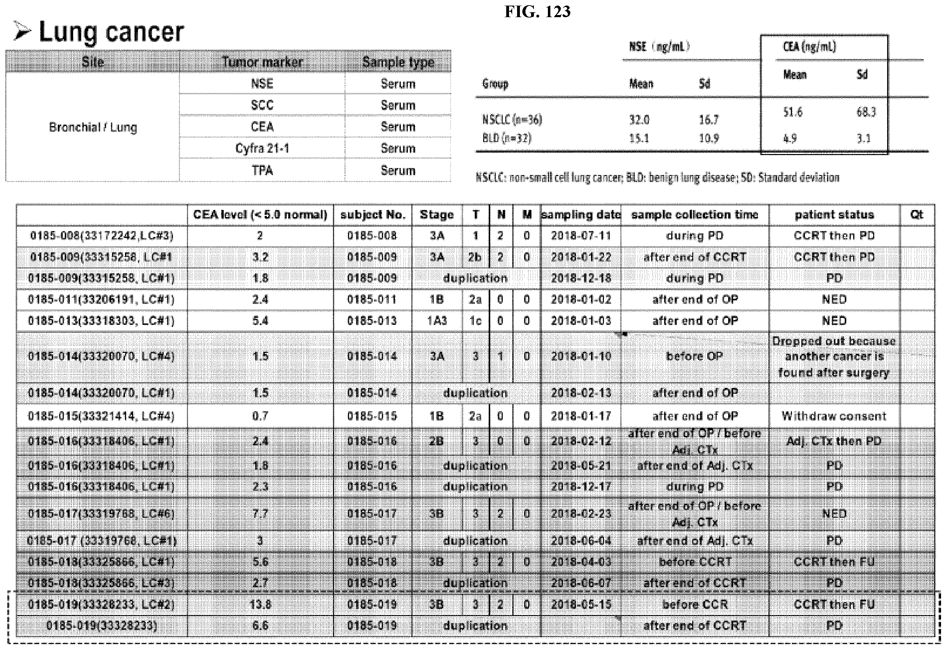

Using the same method as described above, it is a result showing a level of DNA expression of CEA using the blood obtained from lung cancer patients before and after anti-cancer treatment, and the prognosis of patients (Figure 123). As a result of the analysis, it was confirmed that the prognosis can be accurately confirmed after cancer treatment through the analysis method of the present invention.

### Example 14. Confirmation of Possibility of Diagnosis of Lung Cancer

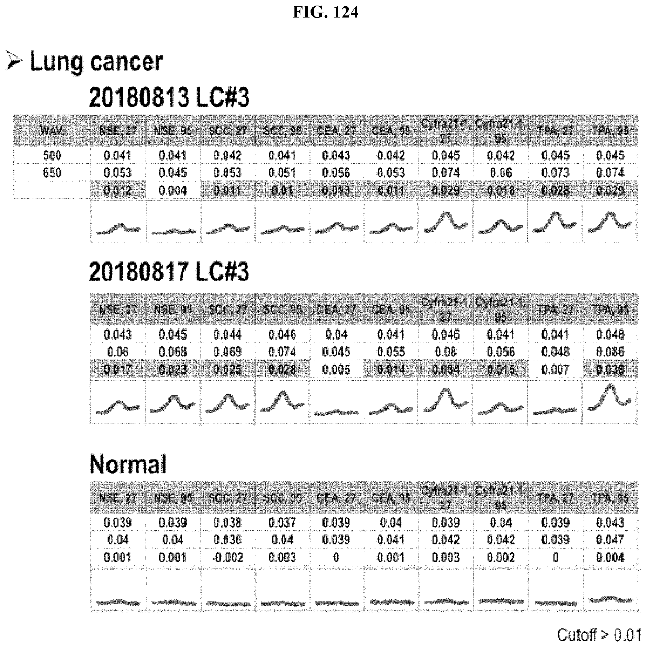

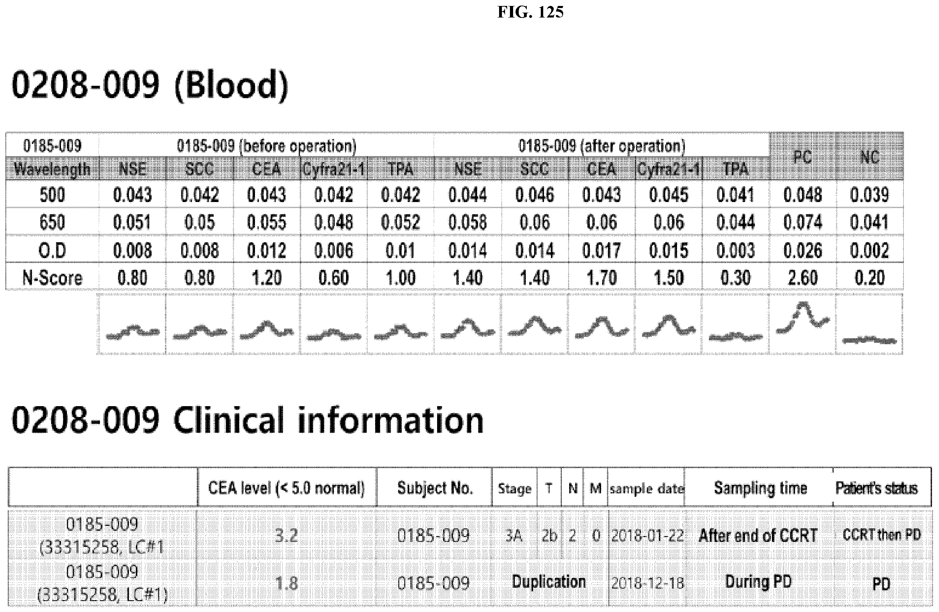

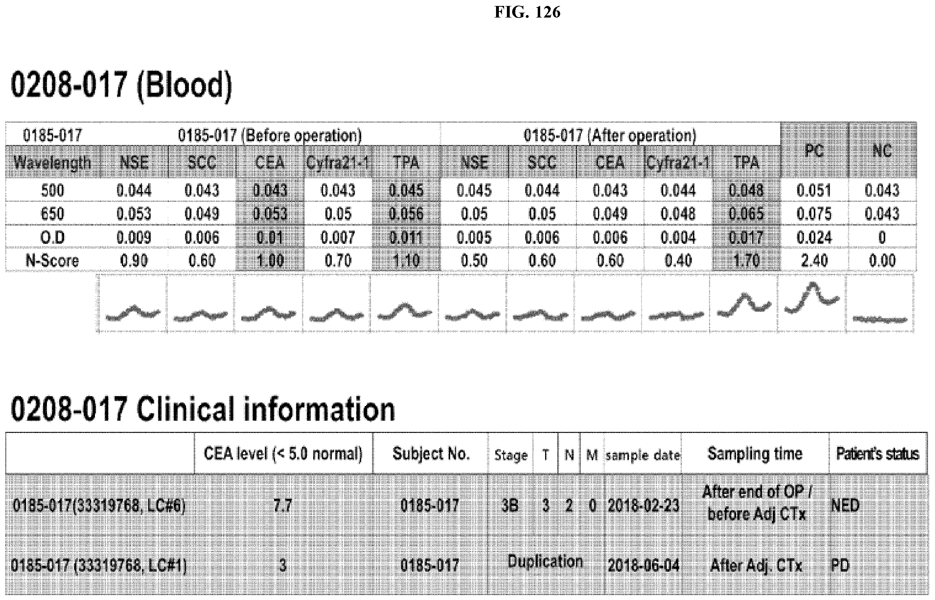

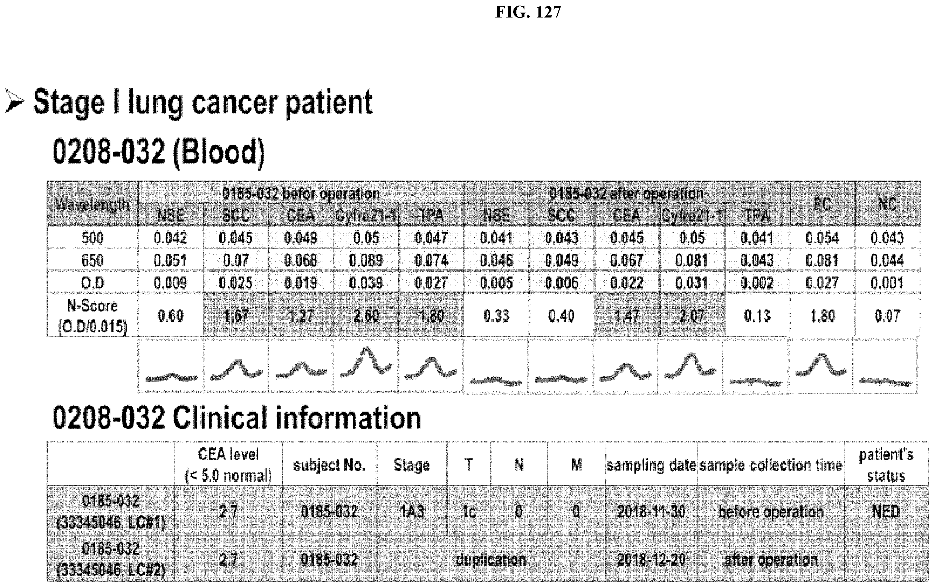

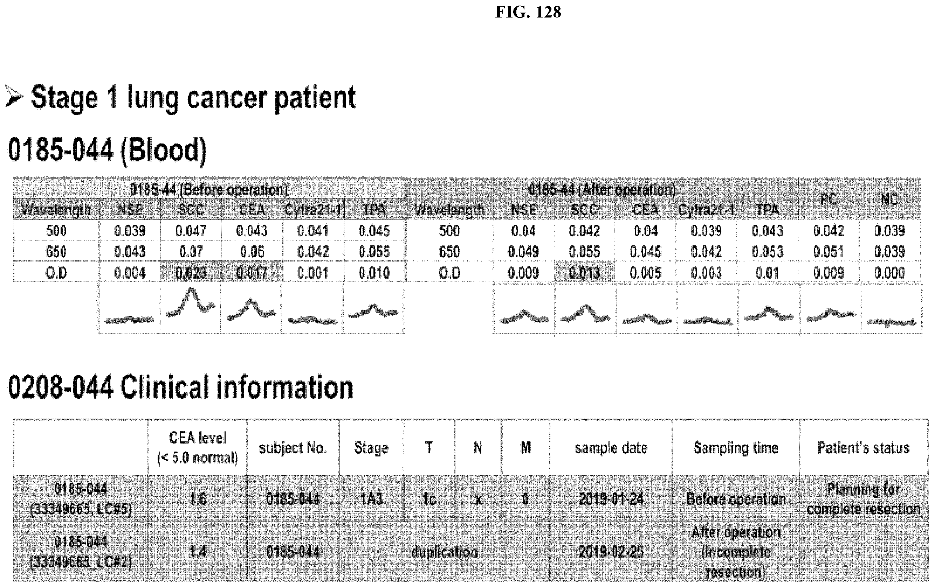

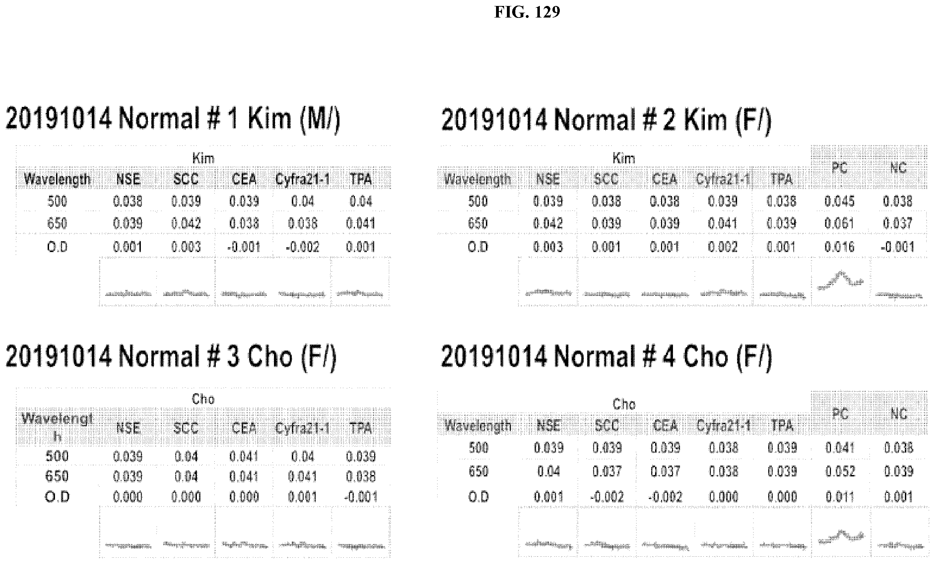

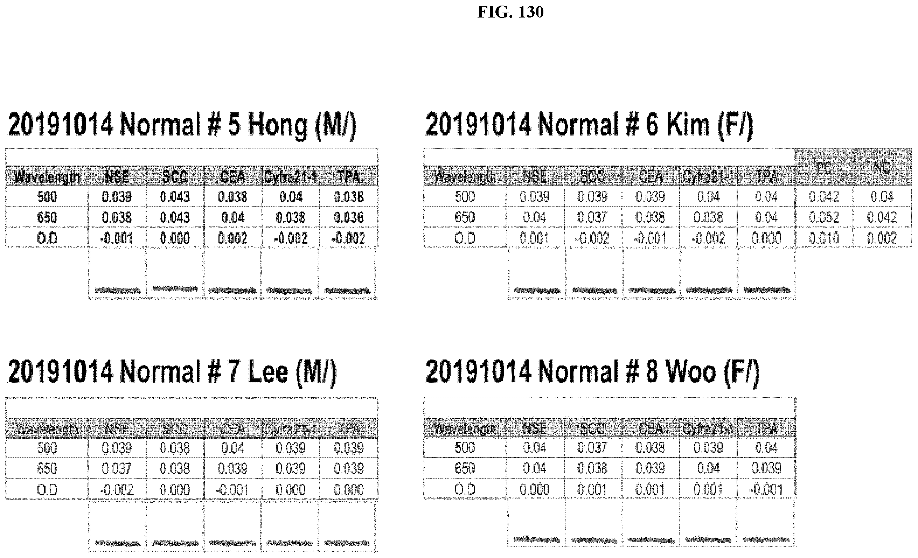

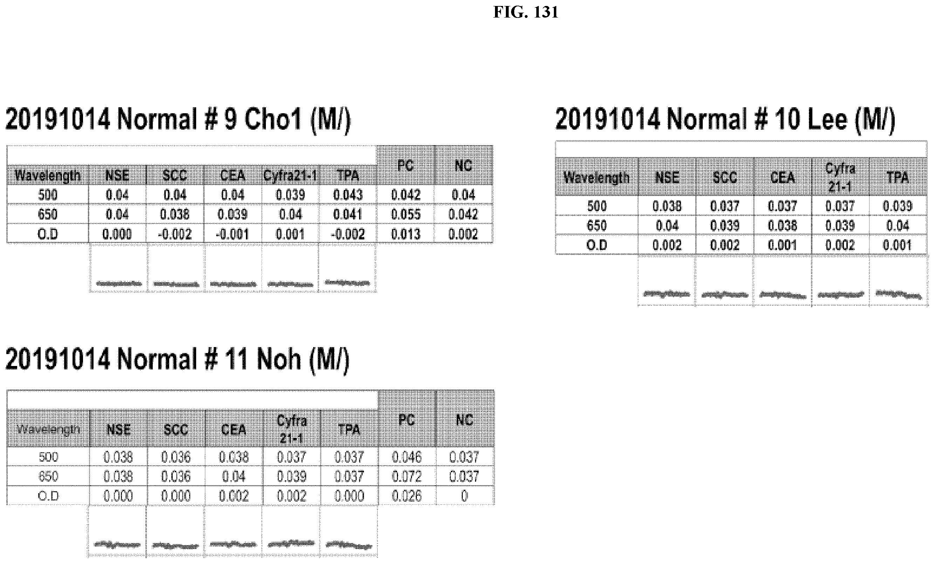

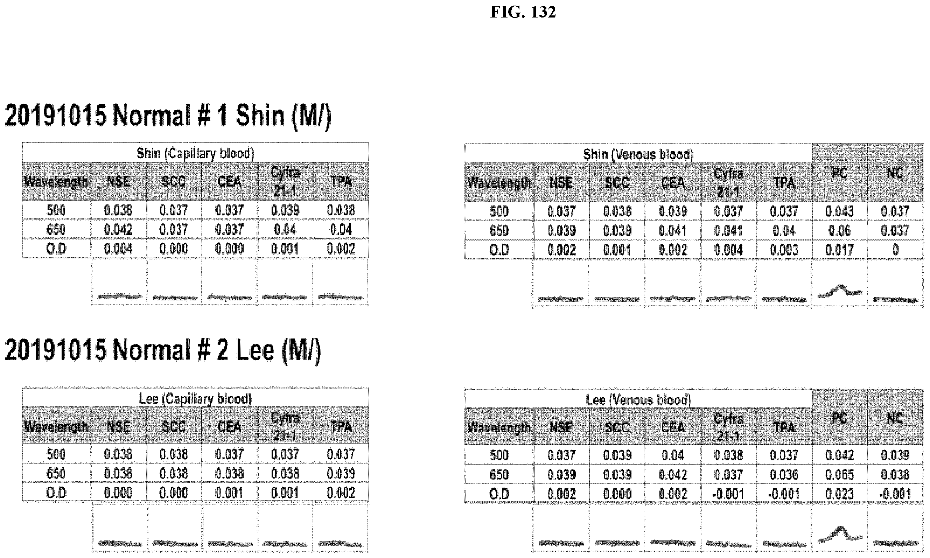

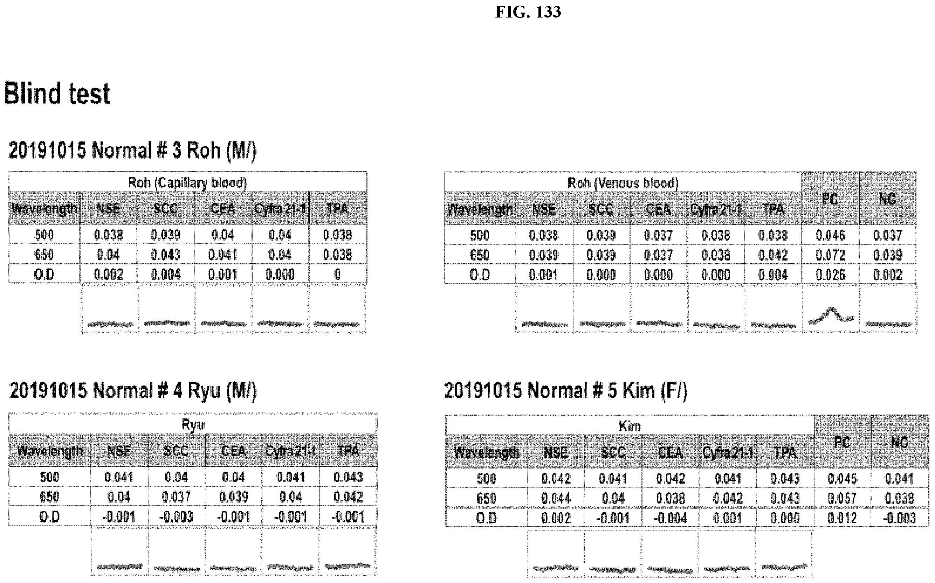

Using the same method as described above, cancer-related biomarkers such as NSE and CEA were analyzed using blood obtained from lung cancer patients(Figures 124 to 128). In addition, cancer-related biomarkers such as NSE and CEA were analyzed using blood obtained from normal persons(Figures 129 to 133). As a result of the analysis, it was confirmed that the lung cancer can be accurately diagnosed through the analysis method of the present invention.

### Example 15. Confirmation of Possibility of Diagnosis of Prostate Cancer

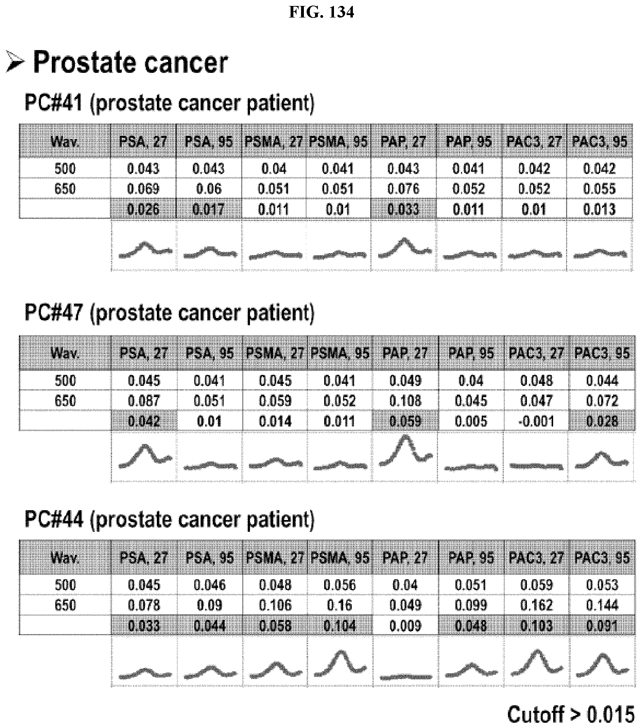

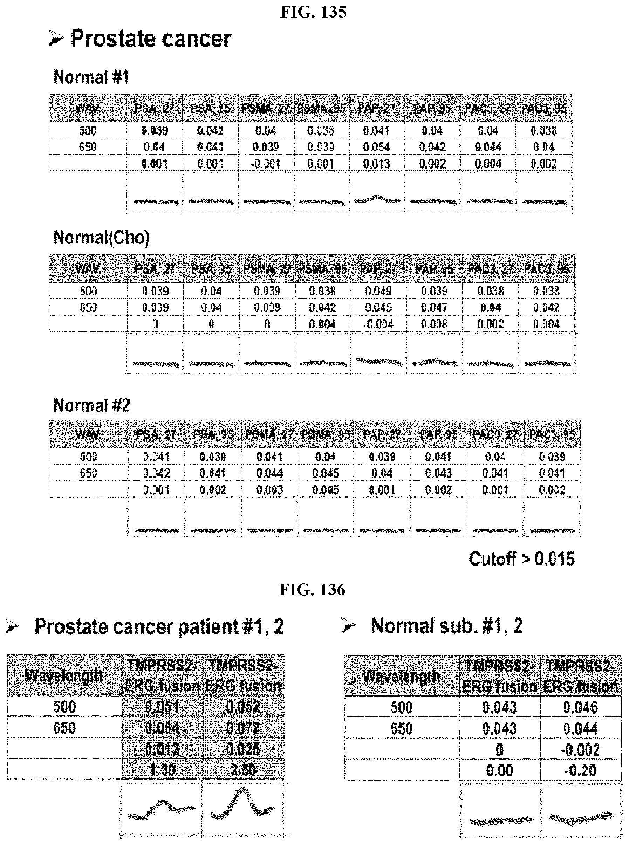

Using the same method as described above, a cancer-related biomarker such as PSA, PSMA, PAP, and PCA3 was analyzed using the blood obtained from prostate cancer patients (Figure 134). In addition, a cancer-related biomarker such as PSA, PSMA, PAP, and PCA3 was analyzed using the blood obtained from normal persons (Figure 135). In addition, a biomarker of TMPRSS2-ERG fusion was analyzed using the blood obtained from prostate cancer patients and normal persons (Figure 136). As a result of the analysis, it was confirmed that the prostate cancer can be accurately diagnosed through the analysis method of the present invention.

### Example 16. Confirmation of Possibility of Diagnosis of Thyroid Cancer

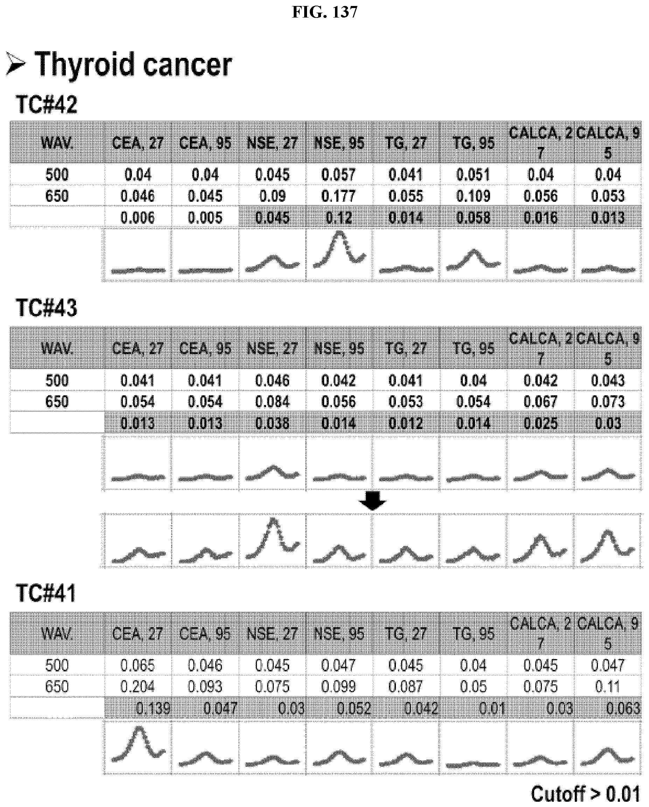

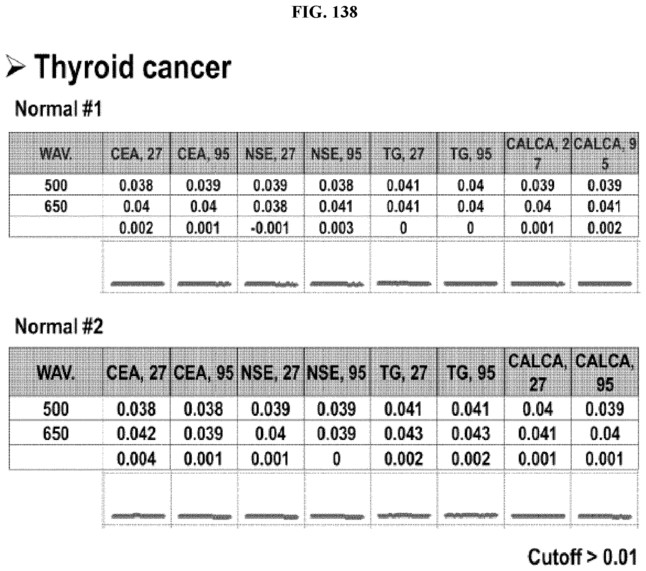

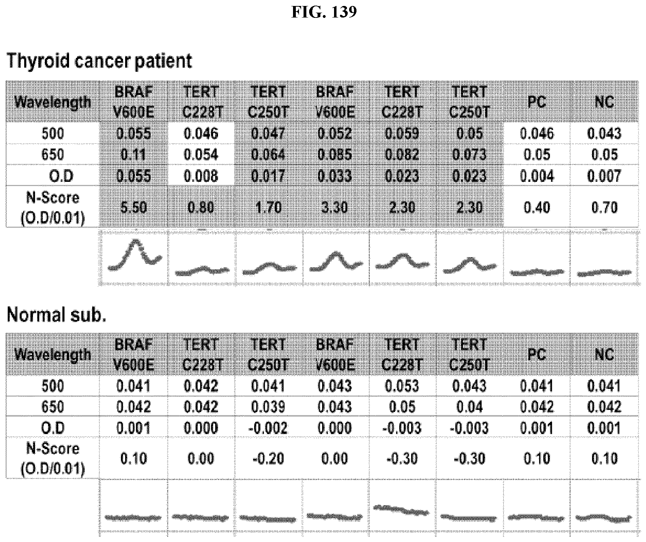

Using the same method as described above, a cancer-related biomarker such as CEA, NSE, TG(Thyroglobulin), and CALCAwas analyzed using the blood obtained from thyroid cancer patients (Figure 137). In addition, a cancer-related biomarker such as CEA, NSE, TG(Thyroglobulin), and CALCA was analyzed using the blood obtained from normal persons (Figure 138). In addition, the DNA expression level of BRAF mutation(V600E), TERT Promotor mutation(C228T, C250T) was analyzed using the blood obtained from thyroid cancer patients and normal persons (Figure 139). As a result of the analysis, it was confirmed that the thyroid cancer can be accurately diagnosed through the analysis method of the present invention.

### Example 17. Confirmation of Possibility of Diagnosis of Bladder Cancer

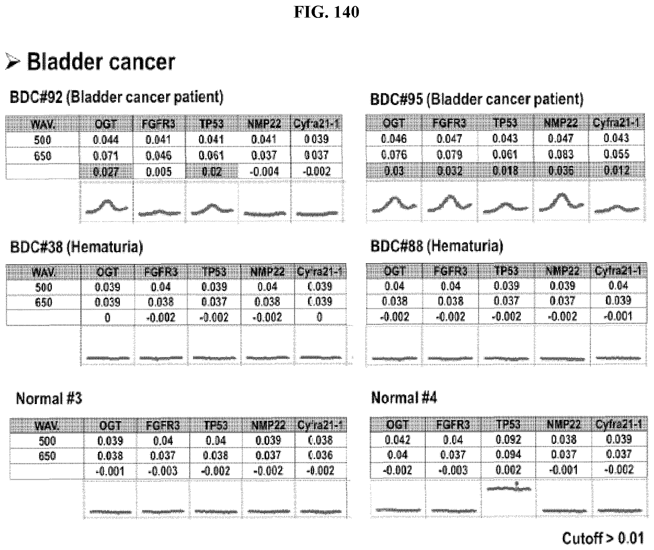

Using the same method as described above, the DNA expression level of OGT, FGFR3, TP53, NMP22, and Cyfra21-1was analyzed using the urine obtained from bladder cancer patients, hematuria patients, and normal persons(Figure 140). As a result of the analysis, it was confirmed that the bladder cancer can be accurately diagnosed through the analysis method of the present invention.

### Example 18. Confirmation of Possibility of Diagnosis of Breast Cancer

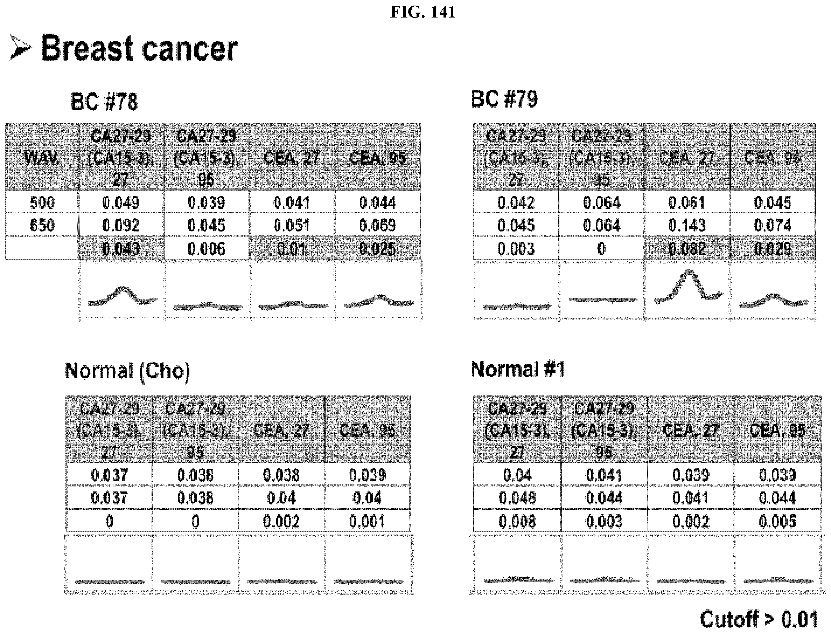

Using the same method as described above, the DNA expression level of CA27-29 and CEA was analyzed using the blood obtained from breast cancer patients and normal persons(Figure 141). As a result of the analysis, it was confirmed that the breast cancer can be accurately diagnosed through the analysis method of the present invention.

### Example 19. Confirmation of Possibility of Diagnosis of Colorectal Cancer

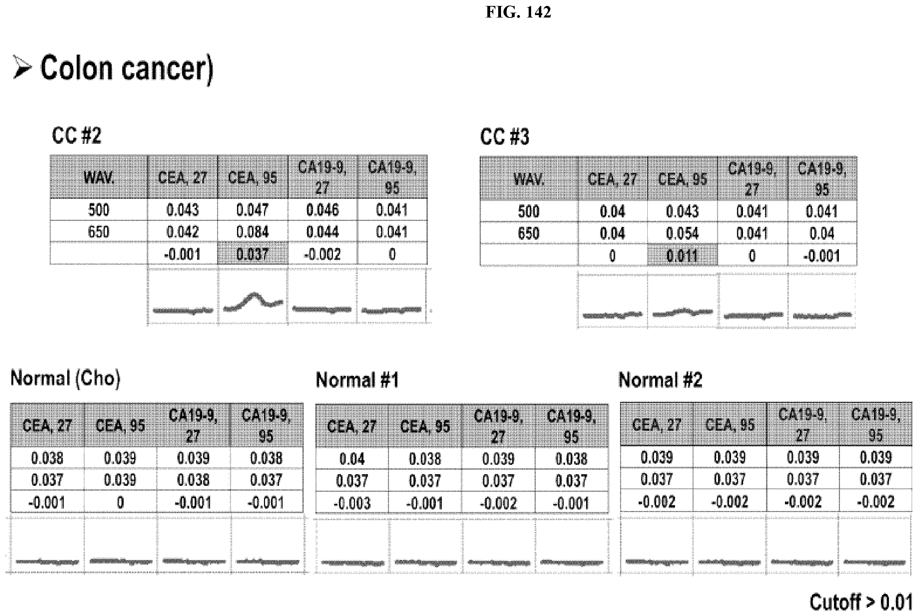

Using the same method as described above, the DNA expression level of CEA and CA19-9 was analyzed using the blood obtained from colorectal cancer patients and normal persons(Figure 142). As a result of the analysis, it was confirmed that the colorectal cancer can be accurately diagnosed through the analysis method of the present invention.

### Example 20. Confirmation of Possibility of Diagnosis of Biliary tract Cancer

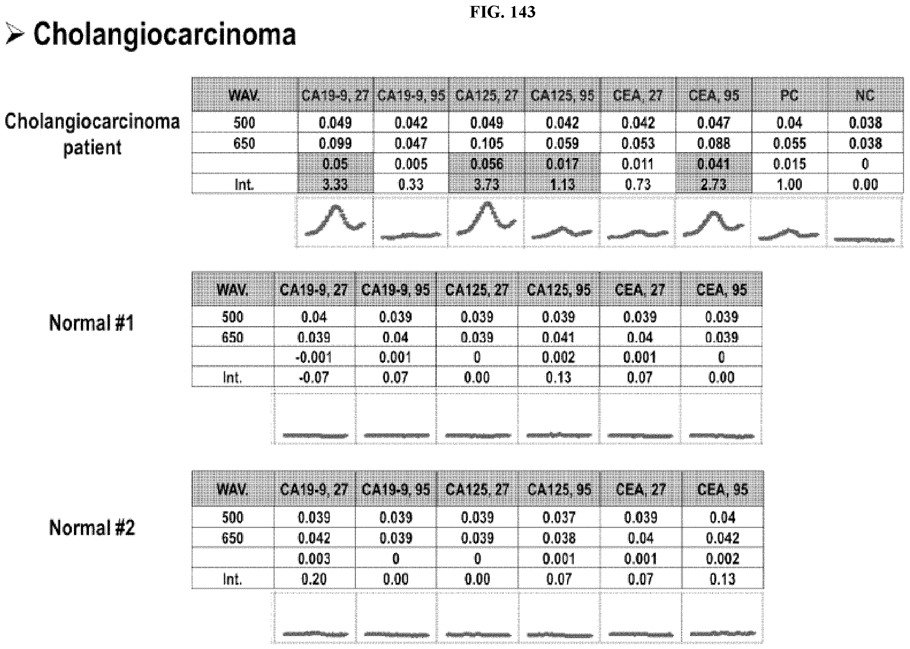

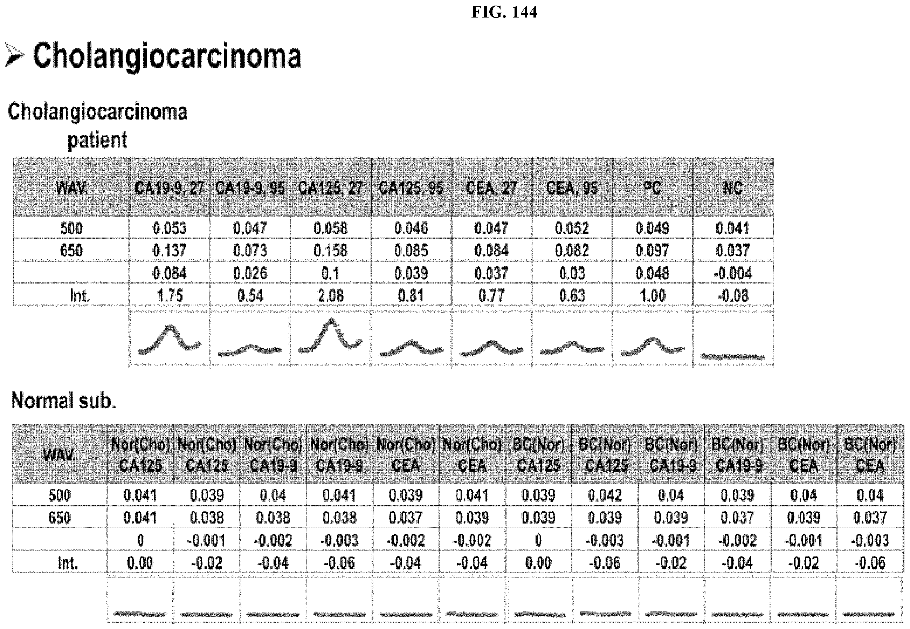

Using the same method as described above, the DNA expression level of CA19-9, CEA, and CA123 was analyzed using the blood obtained from biliary tract cancer patients and normal persons(Figure 143 to 144). As a result of the analysis, it was confirmed that the biliary tract cancer can be accurately diagnosed through the analysis method of the present invention.

### Example 21. Confirmation of Possibility of Diagnosis of Gastric Cancer

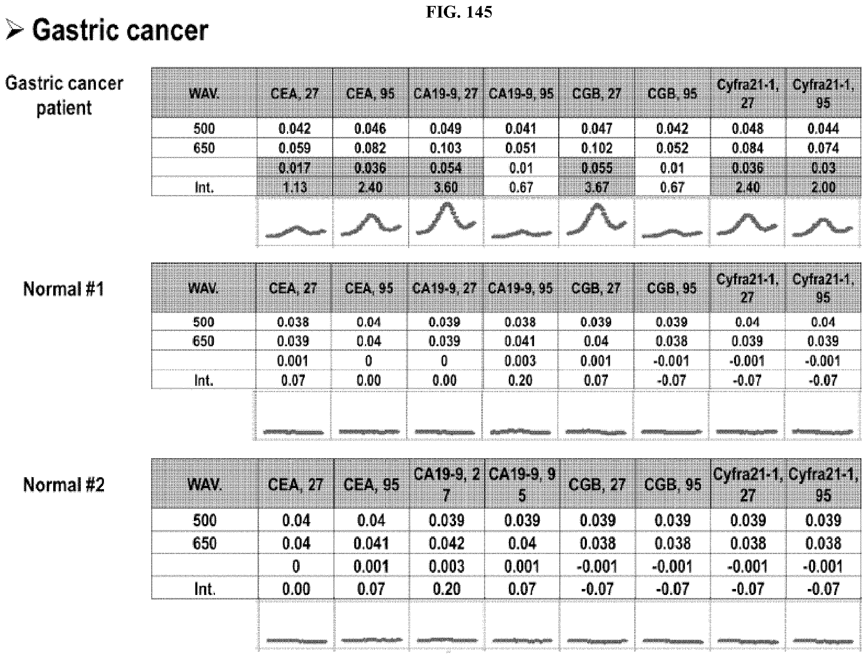

Using the same method as described above, the DNA expression level of CEA, CA19-9, CGB, and Cyfra21-1 was analyzed using the blood obtained from gastric cancer patients and normal persons(Figure 145). As a result of the analysis, it was confirmed that the gastric cancer can be accurately diagnosed through the analysis method of the present invention.

### Example 22. Confirmation of Possibility of Diagnosis of Ovarian Cancer

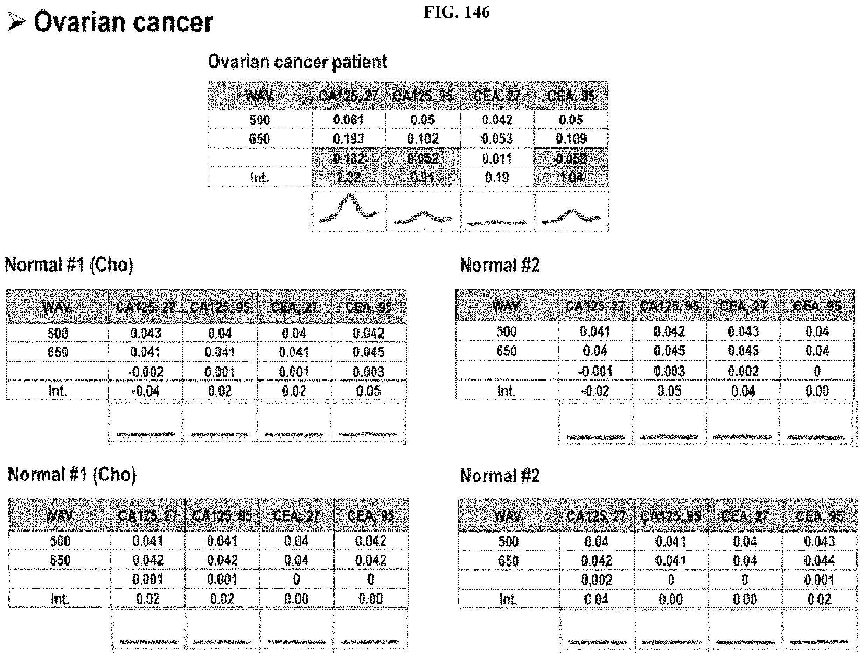

Using the same method as described above, the DNA expression level of CA125 and CEA was analyzed using the blood obtained from ovarian cancer patients and normal persons(Figure 146). As a result of the analysis, it was confirmed that the ovarian cancer can be accurately diagnosed through the analysis method of the present invention.

### Example 23. Confirmation of Possibility of Diagnosis of Pancreatic Cancer

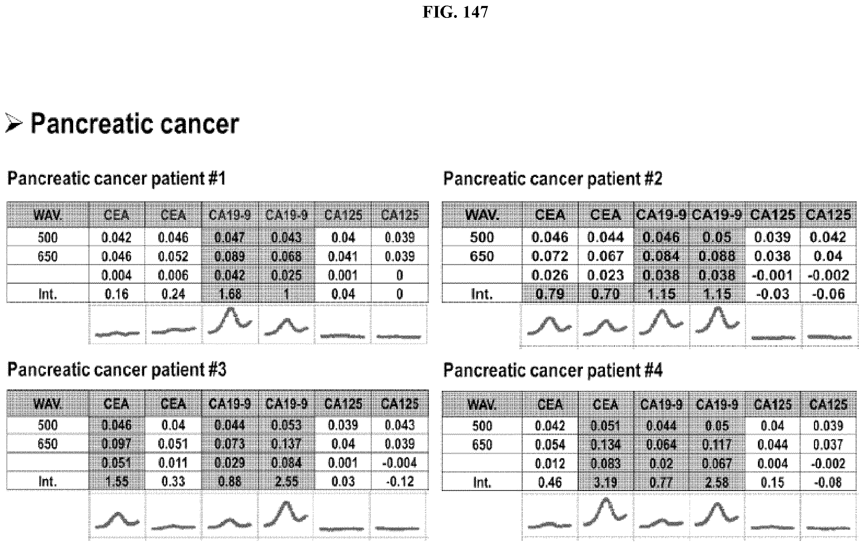

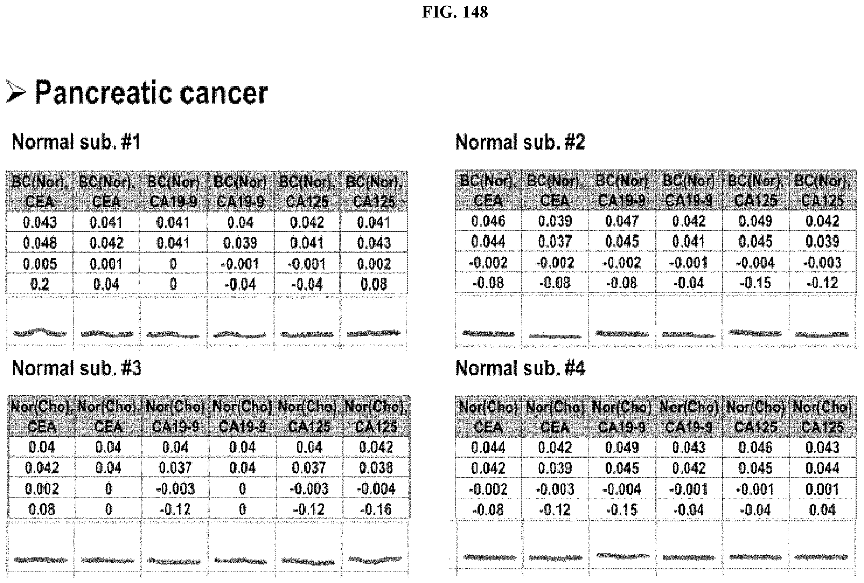

Using the same method as described above, the DNA expression level of CEA, CA19-9, and CA125 was analyzed using the blood obtained from pancreatic cancer patients(Figure 147). the DNA expression level of CEA, CA19-9, and CA125 was analyzed using the blood obtained from normal persons(Figure 148). As a result of the analysis, it was confirmed that the pancreatic cancer can be accurately diagnosed through the analysis method of the present invention.

### Example 24. Confirmation of Accuracy for Detection Method Using cfDNA Through Detection of Various Cancer Biomarkers in Sample of Normal Persons

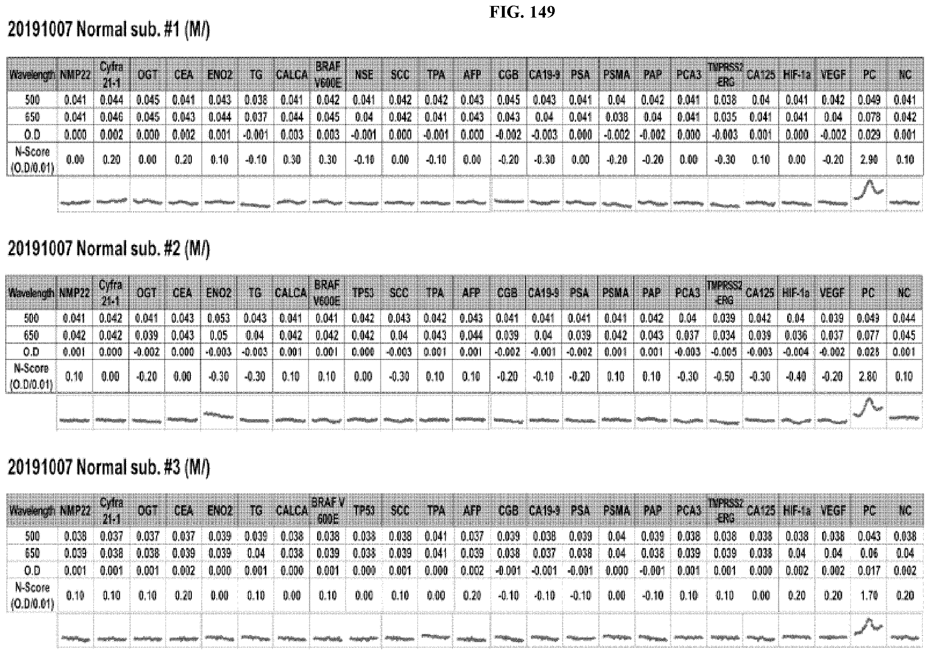

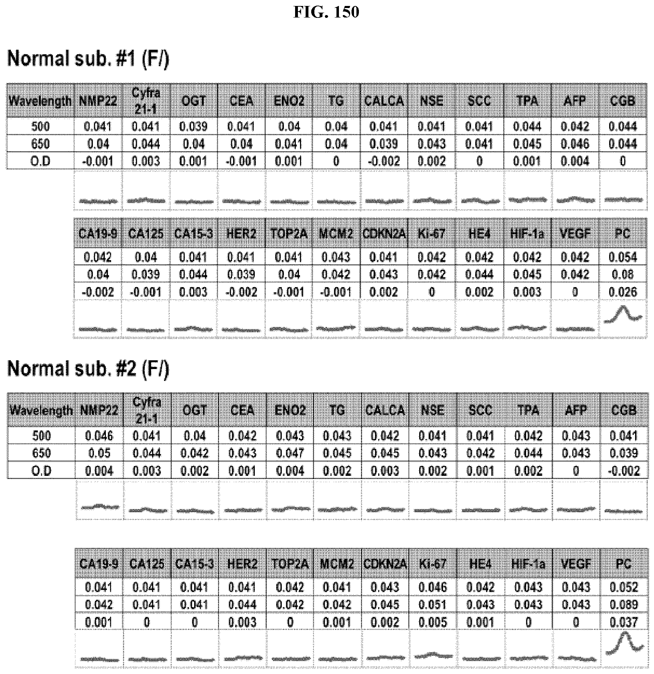

Using the same method as described above, by measuring a level of DNA expression of a cancer-related biomarker using the blood obtained from normal persons, it was confirmed that a cancer-related biomarker was not detected in normal persons (Figure 149 and Figure 150) (PC: positive control).

### Example 25. Confirmation of Possibility of Diagnosis of Cervical Cancer

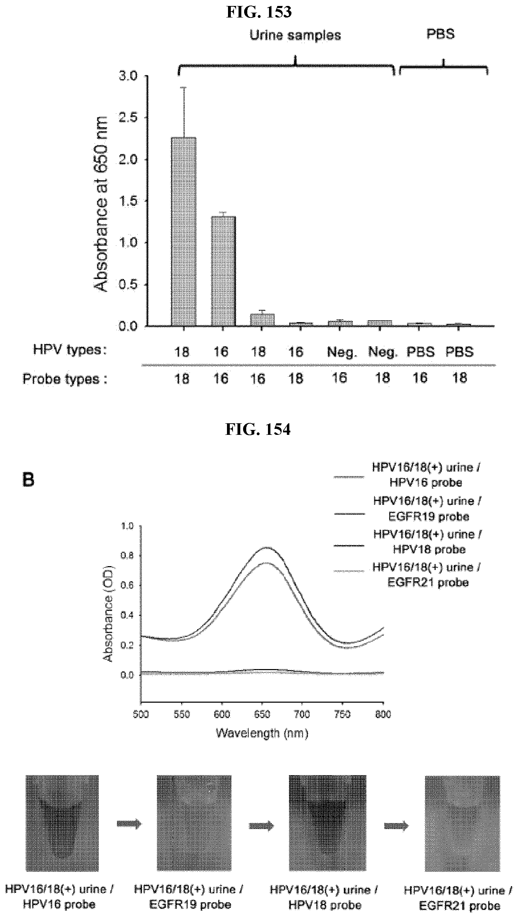

Using the same method as described above, the presence or absence of the binding of cfDNA present in the urine of HPV positive cervical cancer patients (HPV16 (+) and HPV18 (+)) and a HPV negative healthy control (HPV-) with a probe specific to HPV 18 or HPV 16 was confirmed through the absorbance (Figure 153). In addition, the presence or absence of the binding of cfDNA with each probe was confirmed after sequentially reacting cfDNA isolated from the urine of cervical cancer patients with probes specific to HPV 16, EGFR19 deletion, HPV 18, and EGFR 21 L858R (Figure 154). It was confirmed that the cervical cancer can be accurately diagnosed through the analysis method of the present invention.

### Example 26. Confirmation of Possibility of Diagnosis of Lung Cancer

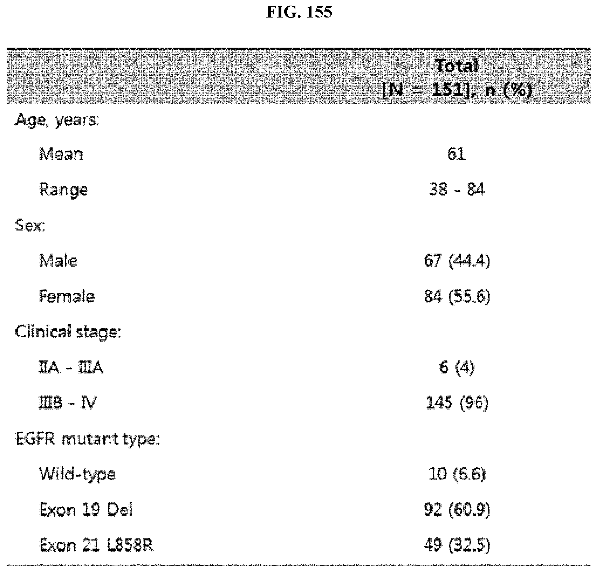

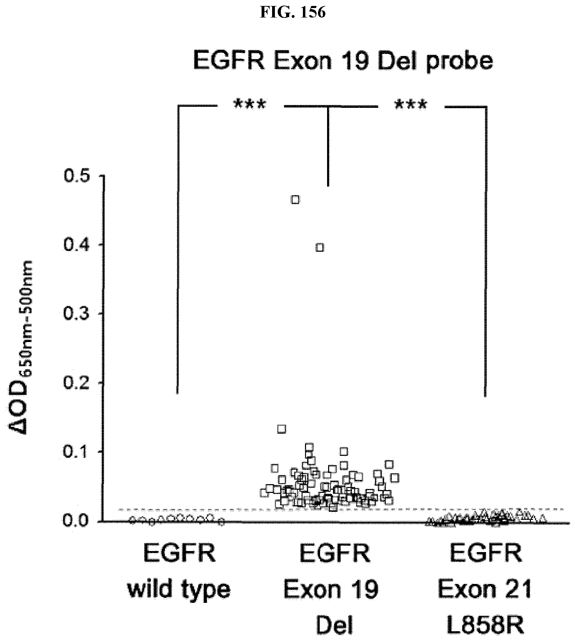

Using the same method as described above, a lung cancer biomarker was detected using the blood obtained from lung cancer patients. Figure 155 is a table of analyzing gene mutations of lung cancer patients using cfDNA obtained from the plasma of 151 lung cancer patients. In addition, gene mutations of lung cancer patients were confirmed by obtaining cfDNA from the plasma of patients without an EGFR mutation (wild type), patients with EGFR exon19 deletion, and lung cancer patients with EGFR exon 21 L858R, mixing with a probe specific to EGFR exon19 Del, and then analyzing the absorbance (ΔOD, 500 nm to 650 nm) values of UV spectrum (Figure 156).

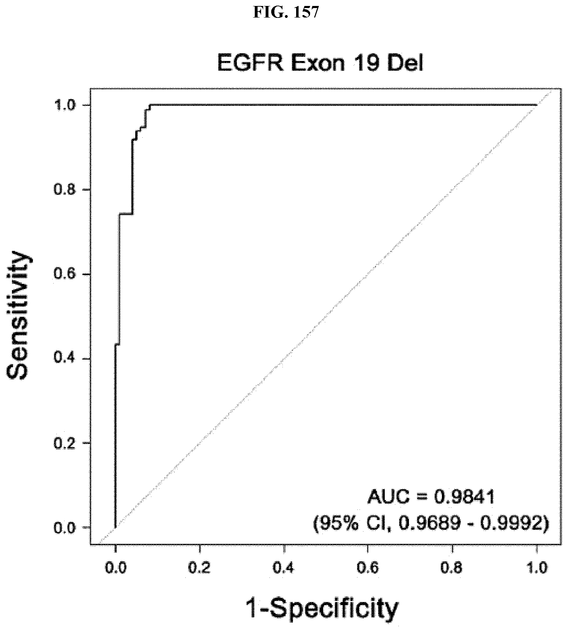

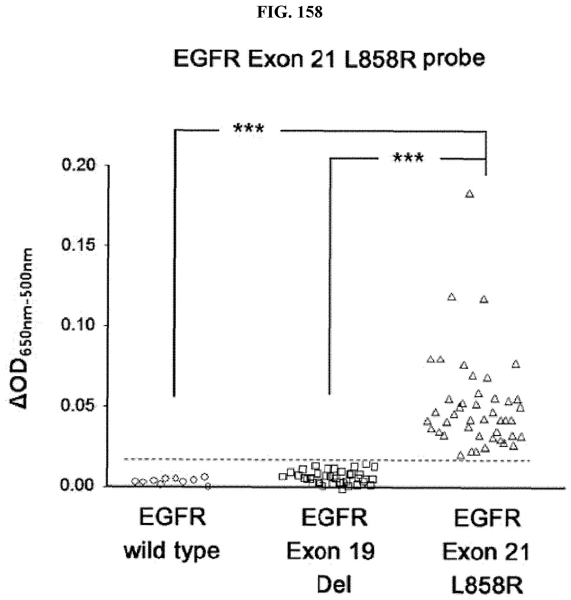

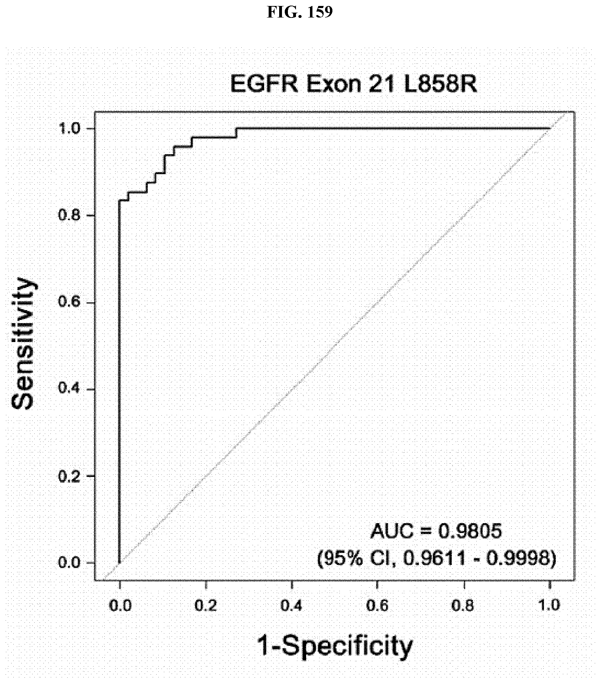

In addition, cfDNA was obtained from the plasma of lung cancer patients with EGFR exon19 deletion, and then it was mixed with a probe specific to EGFR exon19 Del, and then the specificity and sensitivity of gene mutations were analyzed (Figure 157). The gene mutations of patients were confirmed by obtaining cfDNA from the plasma of patients without EGFR mutation (wild type), patients with EGFR exon19 deletion, and lung cancer patients with EGFR exon 21 L858R, adding a probe specific to EGFR exon21 L858R, and then analyzing the absorbance (ΔOD, 500 nm to 650 nm) values of UV spectrum (Figure 158). In addition, cfDNA was obtained from the plasma of lung cancer patients with EGFR exon 21 L858R, a probe specific to EGFR exon 21 L858R was added, and then the specificity and sensitivity of gene mutations of patients were analyzed (Figure 159).

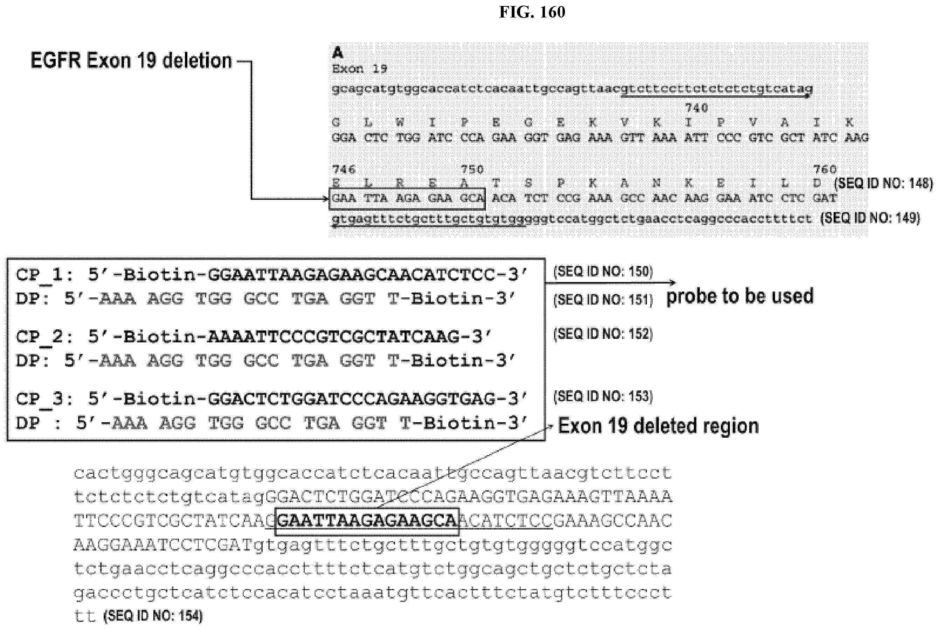

In addition, the sequences of CP and DP of EGFR exon 19 deletion are shown (Figure 160). In this study, CP_1 and DP were used to analyze the cfDNA gene mutation of lung cancer patients. In this case, CP is a probe that is designed to complementarily bind to a sequence that contains or is adjacent to a mutated portion, and DP means a probe that is designed to complementarily bind to a portion spaced apart a mutated sequence.

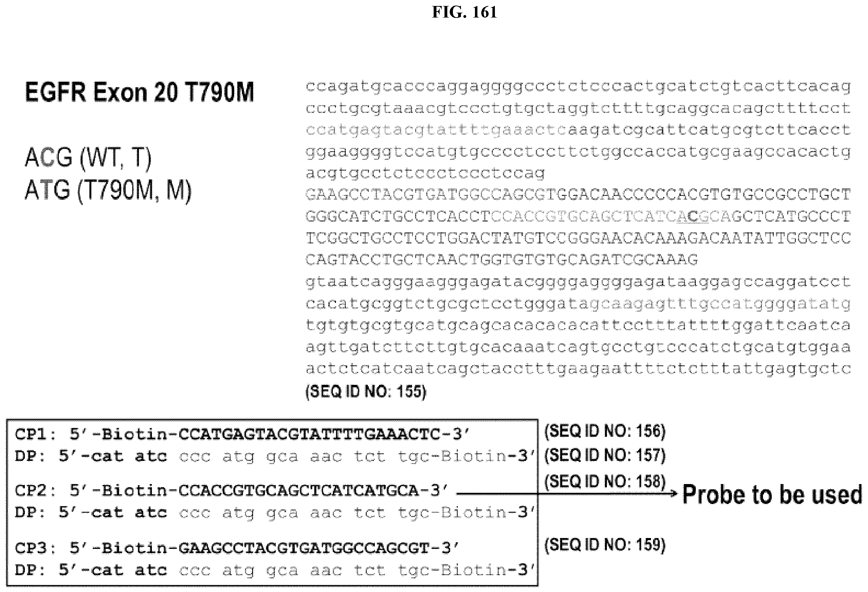

In addition, the sequences of CP and DP of EGFR exon 20 T790M are shown. In this study, CP2 and DP were used to analyze the cfDNA gene mutation of lung cancer patients (Figure 161).

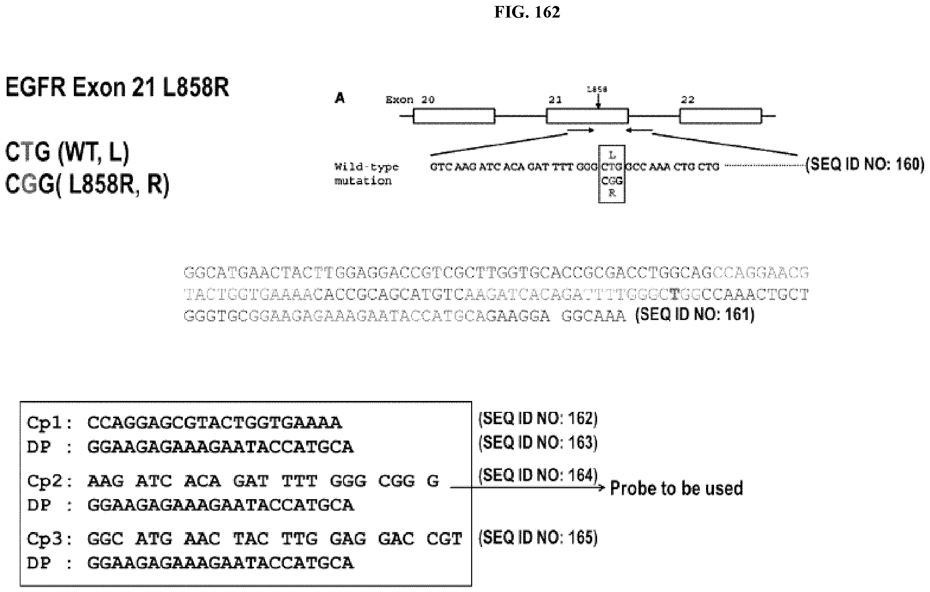

In addition, the sequences of CP and DP of EGFR exon 21 L858R are shown. In this study, CP2 and DP were used to analyze the cfDNA gene mutation of lung cancer patients (Figure 162).

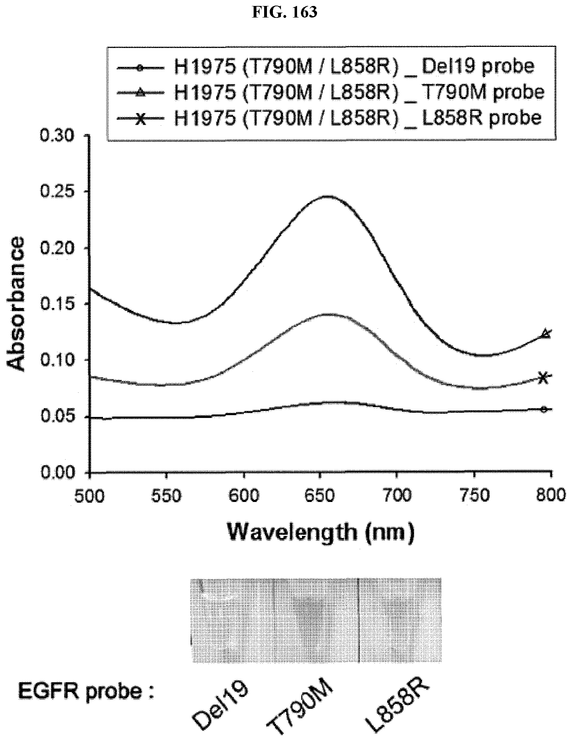

In addition, whether cfDNA was detected by the color change and UV absorbance was confirmed by reacting cfDNA obtained from the plasma of lung cancer patients with EGFR exon 19 deletion and EGFR exon 20 T790M gene mutations and a probe specific to EGFR exon 19 deletion (De119), EGFR exon 20 T790M, and EGFR exon 21 L858R, and then adding HRP/streptavidin nanoparticles (including a large amount of HRP) (Figure 163).

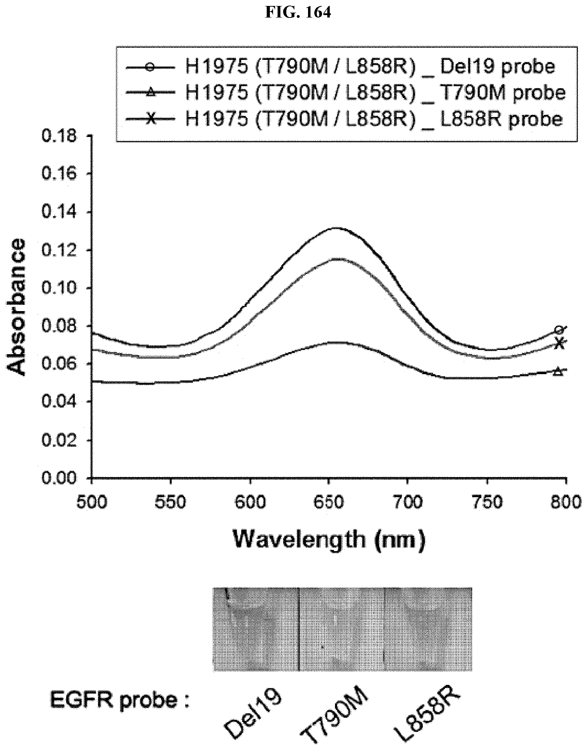

In addition, Figure 164 shows a result of confirming whether cfDNA was detected by the color change and UV absorbance by reacting cfDNA obtained from the plasma of lung cancer patients with EGFR exon 19 deletion and EGFR exon 20 T790M gene mutations that are the same as in Figure 47 and a probe specific to EGFR exon 19 deletion (Del19), EGFR exon 20 T790M, and EGFR exon 21 L858R, and then adding an HRP/streptavidin complex (a complex of HRP and streptavidin bound 1:1). It was confirmed that noise was generated in the case of the HRP/streptavidin complex compared to the HRP/streptavidin nanoparticles.

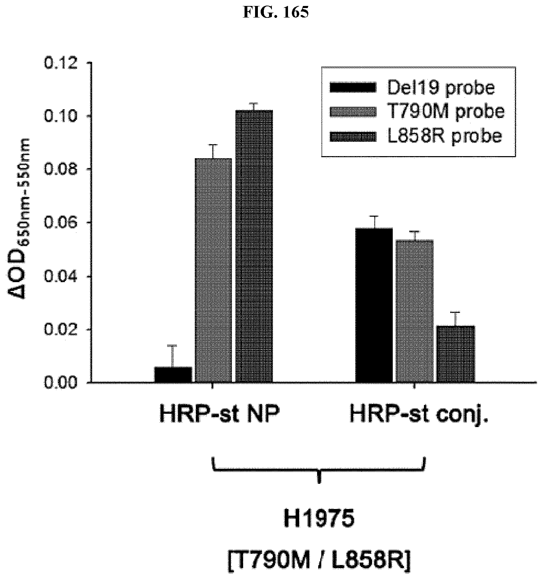

In addition, Figure 165 shows a result of confirming and comparing the consistency of the genotype with cancer tissue through the analysis of the result of extracting cfDNA from the plasma of 5 lung cancer patients with EGFR exon19 deletion and exon20 T790M gene mutations, and then reacting with a probe specific to EGFR exon 19 Del, EGFR exon 20 T790M, EGFR exon 21 L858R and HRP/streptavidin nanoparticles (HRP/st-tagged NPs) and the result of reacting with a probe specific to EGFR exon19 Del, EGFR exon 20 T790M, EGFR exon 21 L858R and an HRP/streptavidin complex (HRP and streptavidin bound 1:1).

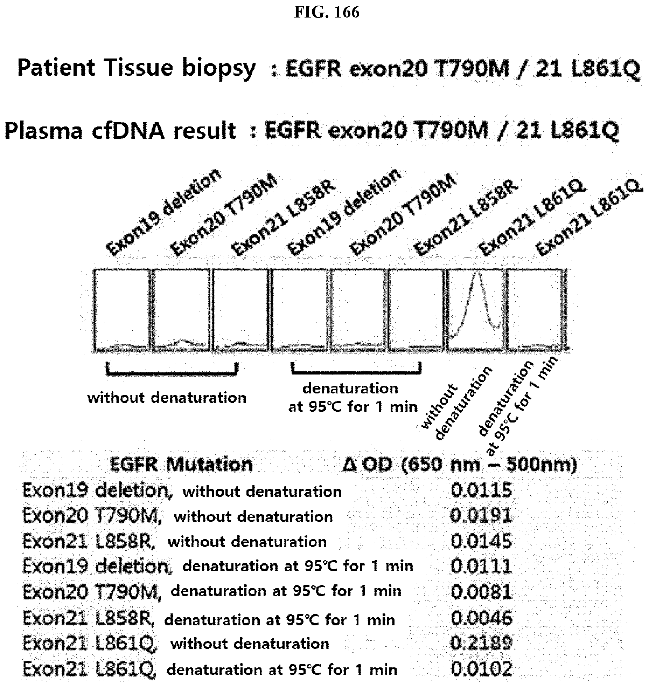

In addition, Figure 166 shows a result of confirming by the LTV absorbance that the gene mutation was observed only in EGFR exon 20 T790M and EGFR exon 21 L861Q in the same manner as a cancer tissue, when mixing a probe specific to EGFR exon 19 deletion (19 Del), EGFR exon 20 T790M, EGFR exon 21 L858R, and EGFR exon L861Q and HRP/st-tagged NP all at once in order to detect the gene mutation of cfDNA obtained from the plasma of lung cancer patients with EGFR exon 20 T790M and EGFR exon 21 L861Q gene mutations.

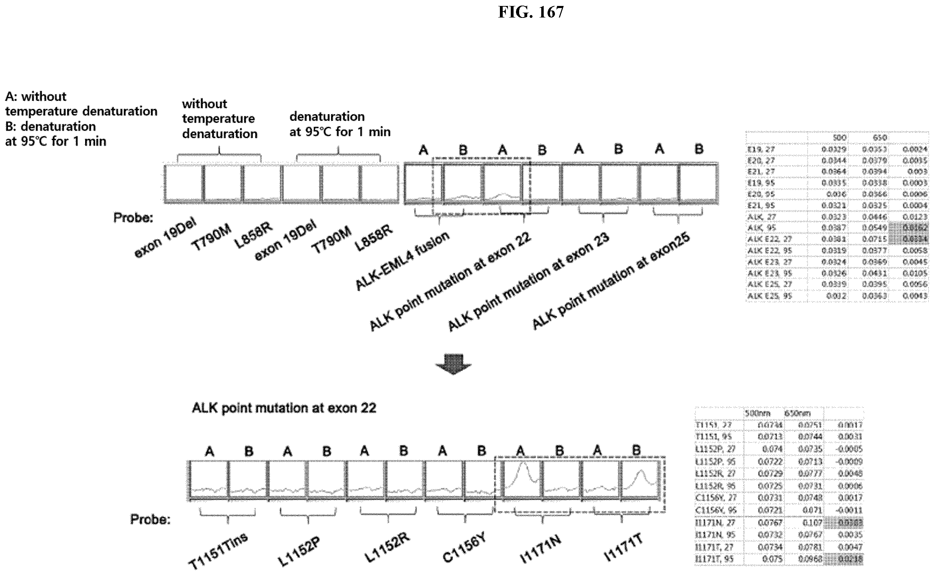

In addition, Figure 167 shows a result of confirming that the ALK-EML4 fusion and ALK point mutation (I1171N/T) genotypes were detected in the same manner as a cancer tissue, when mixing a probe specific to ALK-EML4 fusion and ALK point mutation (T1151, L1152P, L1152R, C1156Y, I1171N/T) and HRP/st-tagged NP all at once in order to detect the gene mutation of cfDNA obtained from the plasma of lung cancer patients with ALK-EML4 fusion and ALK point mutation (I1171N/T) gene mutations.

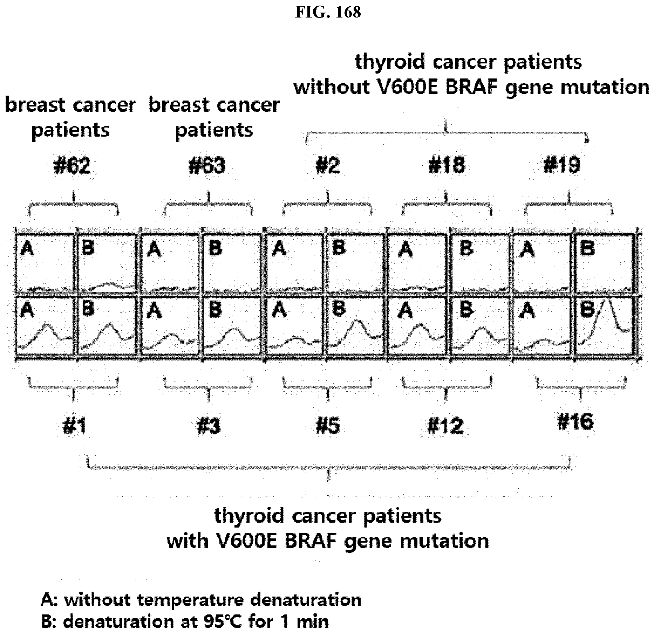

In addition, Figure 168 shows a result of confirming that the BRAF V600E gene mutation was detected in the same manner as the genotype of patients, by mixing a probe specific to BRAF V600E and HRP/st-tagged NP all at once in order to detect the gene mutation of cfDNA obtained from plasma of thyroid cancer patients with BRAF V600E gene mutation.

### IX. Detection of cfDNA Derived from Cancer According to Sample Denaturation Condition

### Example 27. Detection of cfDNA After Denaturation of Sample Obtained from Cancer Patients According to Temperature Condition

The Experiment was performed to confirm whether the cfDNA derived from cancer and the cfDNA derived from normal persons could be distinguished according to the sample denaturation conditions. Specifically, using a probe capable of detecting EGFR 19 deletion, it was confirmed that whether the unstable cfDNA and the stable cfDNA could be distinguished after applying various denaturation conditions to the plasma collected from normal persons and lung cancer patients (0208-343, 20190311_LC#1, Tissue result: E19del).

As a probe, ggaattaaga gaagcaacat ctcc (SEQ ID NO 105), a probe capable of detecting EGFR exon 19, deletion, was used. In this case, the probe to which biotin is bound was used. The PEI/Ppy nanowires were used, and the HRP/streptavidin-aggregated nanoparticles were used as markers.

Specifically, before cfDNA was isolated with the nanowires, the sample was treated under various conditions as follows. The sample was heated at 30 °C for 15 minutes and 0 minute. In addition, it was heated at 60 °C for 5 minutes and 0 minute. In addition, it was heated at 95 °C for 1 minute and 0 minute. The other steps were performed by the method described above.

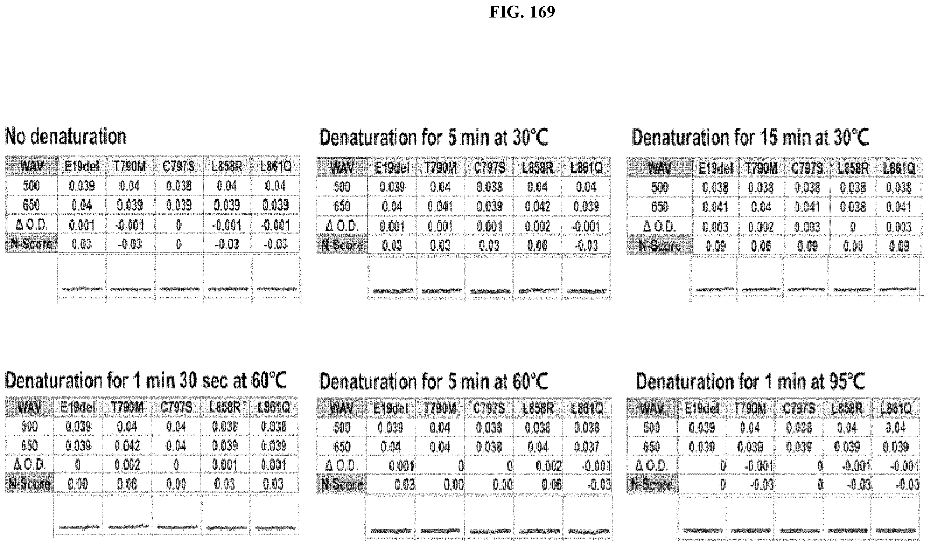

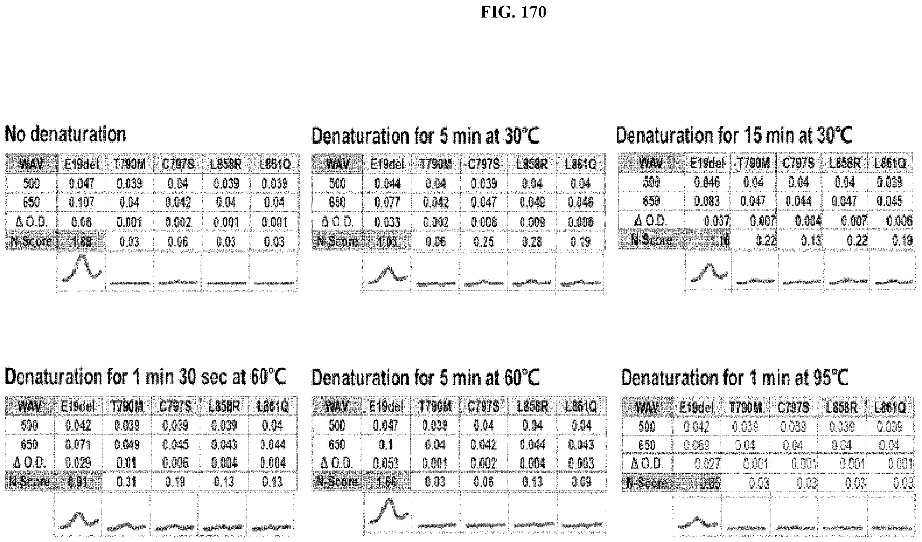

As a result, the unstable cfDNA was not detected under any denaturation condition in normal person without EGFR 19 deletion mutation (Figure 169). However, it was confirmed that unstable cfDNA was detected in all various denaturation conditions in E19del patients (Figure 170). Based on these results, it is possible to provide information that the lung cancer patient has E19del mutation through the presence or absence of the unstable cfDNA.

### Example 28. Detection of cfDNA Derived From Cancer Cell Line According to Temperature Condition

In addition to the samples obtained from the human body, the experiment was performed to confirm the locations of mutations present in the cell line. Specifically, fDNA having a size similar to cfDNA was obtained from HCC2279 (Exon19Del), HCC827 (Exon19Del), H1975 (T790M, L858R), and A549 (EGFR wildtype).

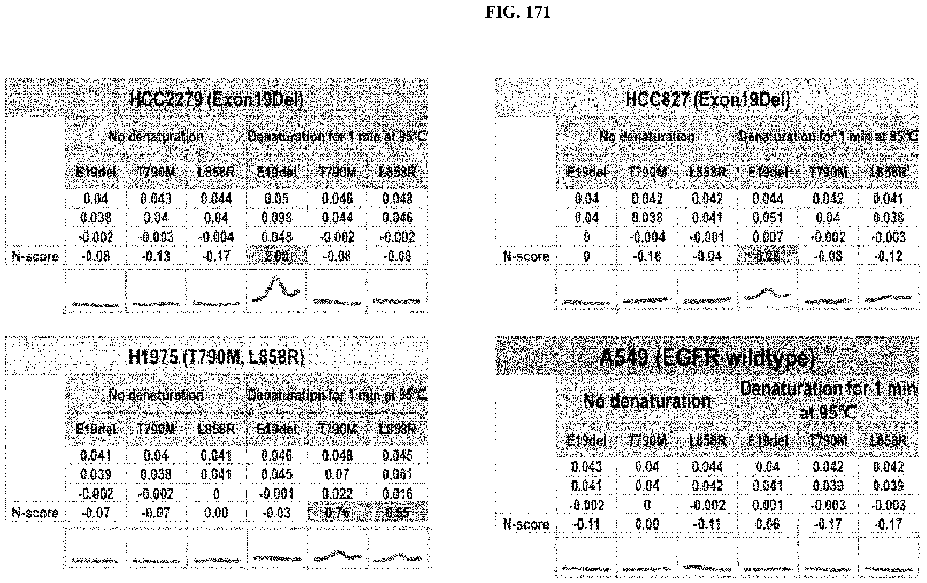

As a result, it was confirmed that in a denaturation condition of heating at 95 °C for 1 minute and 0 minute, only unstable cfDNA was detected by complementarily binding to the probe and binding to the marker (Figure 171). Based on these results, it was verified that the presence or absence of unstable cfDNA could be confirmed even in the sample obtained from the cell line.

### Example 29. Detection of cfDNA Derived from Cancer According to DNase Treatment

In order to confirm whether the unstable cfDNA derived from cancer cells and the stable cfDNA have a difference not only by the temperature conditions but also by DNase, the reactivity with the probe was confirmed after the unstable cfDNA and the stable cfDNA were treated with DNase. In this case, the sample was not subjected to the denaturation using high temperature.

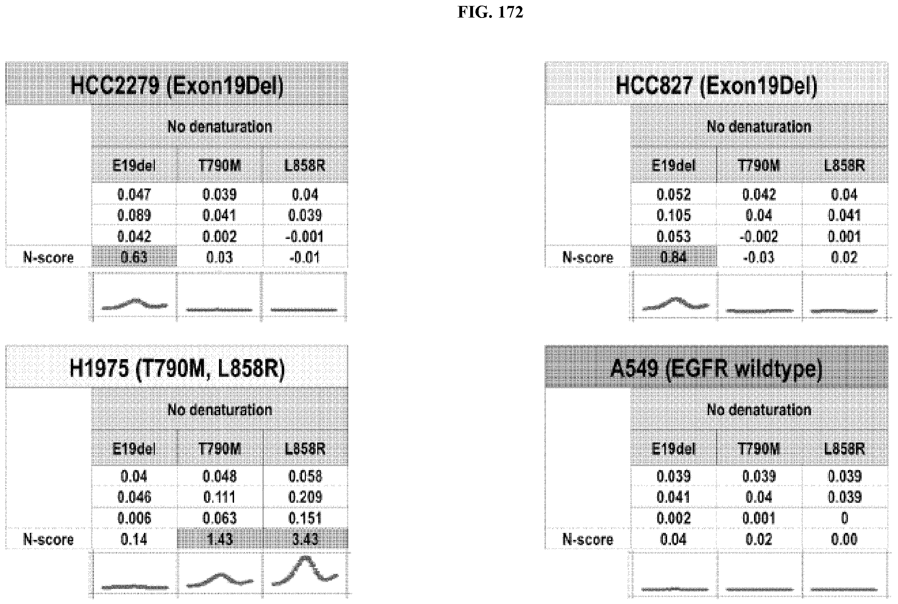

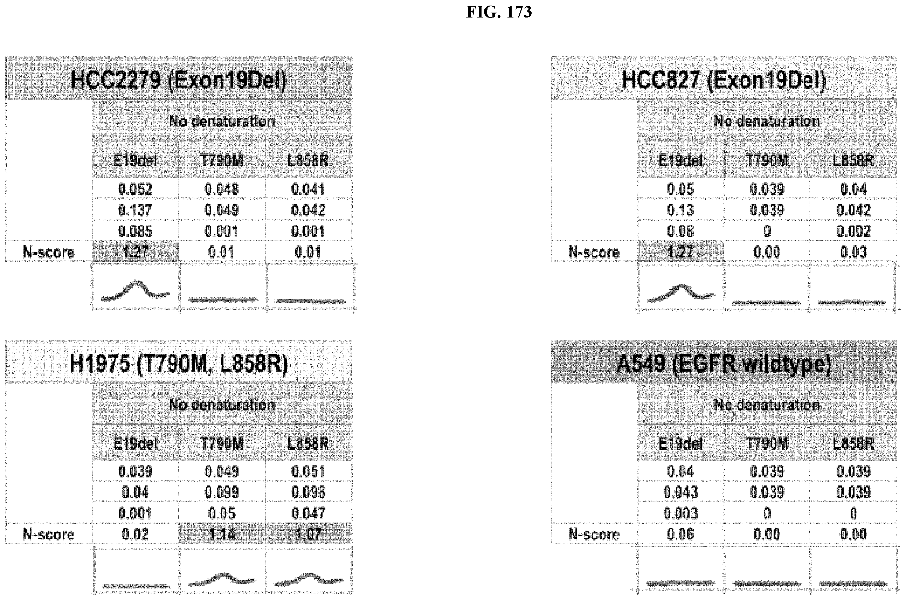

Specifically, fDNA obtained from HCC2279 (Exon19Del), HCC827 (Exon19Del), H1975 (T790M, L858R), and A549 (EGFR wildtype) using PEI/Ppy nanowires was suspended in PBS and then treated with 1 µL of DNase. As a result of treating at 37 °C for 60 minutes, it was confirmed that there was the difference between the unstable cfDNA and the stable cfDNA in terms of the reactivity with the probe (Figure 172). In addition, it was confirmed that the same effect was shown even when treating with DNase at 37 °C for 60 minute (Figure 173). Based on these results, it was confirmed that the stable cfDNA was not easily degraded by a DNase enzyme.

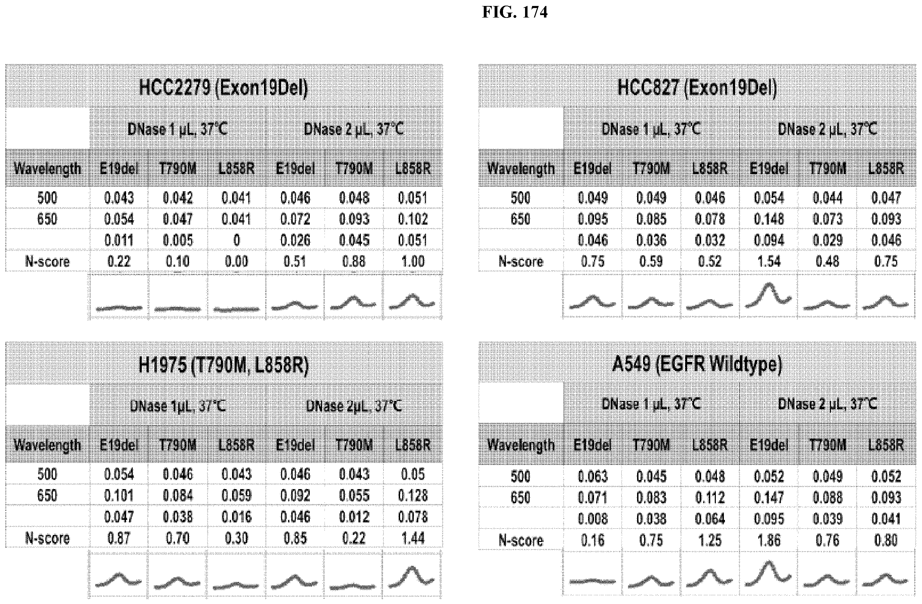

However, as a result of treating with 1 µL or 2 µL of DNase at 37 °C for 120 minutes, it was confirmed that the stable cfDNA also reacted with the probe when the treatment time was increased or the amount of DNase was increased (Figure 174). Based on these experimental results, the difference in the stability of the unstable cfDNA and the stable cfDNA could be confirmed.

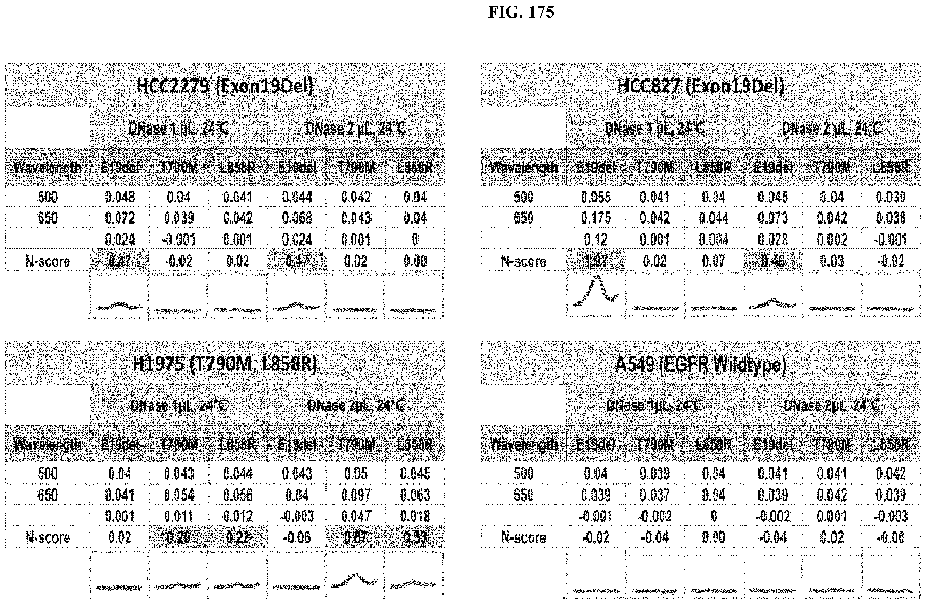

In addition, in order to confirm the difference between the unstable cfDNA and the stable cfDNA according to the activity of DNase, the results of treating with 1 µL or 2 µL of DNase at 24 °C for 120 minutes are shown in Figure 60. As a result, even though the activity of the enzyme was reduced at 24 °C, it was confirmed that there was the difference between the unstable cfDNA and the stable cfDNA in terms of the reactivity with the probe (Figure 175).

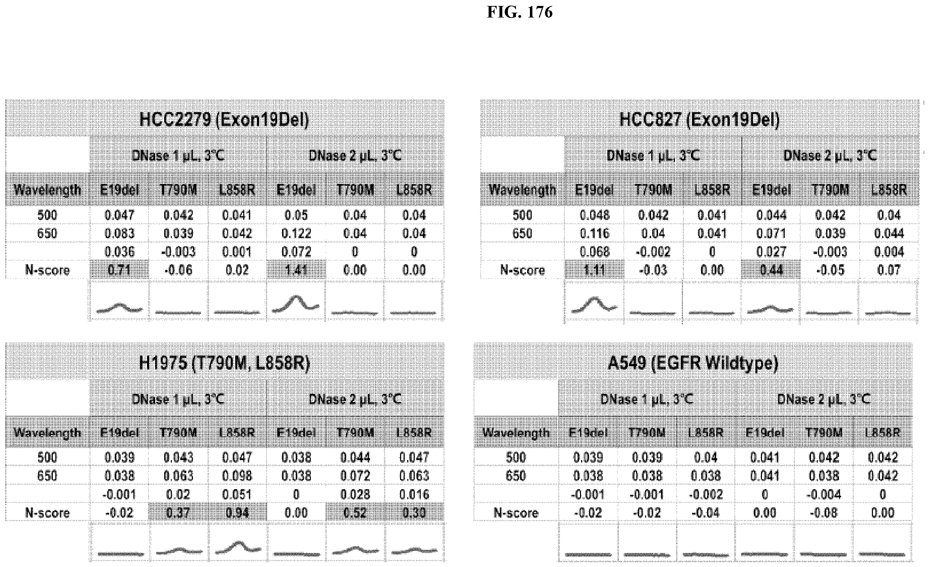

In addition, in order to confirm the difference between the unstable cfDNA and the stable cfDNA according to the activity of DNase, the results of treating with 1 µL or 2 µL of DNase at 3 °C for 120 minutes are shown in Figure 58. As a result, even though the activity of the enzyme was reduced at 3 °C, it was confirmed that there was the difference between the unstable cfDNA and the stable cfDNA in terms of the reactivity with the probe (Figure 176).

## Claims

1. A method for diagnosing prostate cancer by detecting a gene derived from prostate cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from prostate cancer cells, and
wherein the probe having a sequence complementary to cfDNA complementarily binds to a gene known as a prostate cancer biomarker.

2. The method for diagnosing prostate cancer according to claim 1, **characterized in that** the cfDNA derived from prostate cancer cells
i) has a lower Tm value than that of cfDNA having a double helix structure derived from normal cells, or
ii) is denatured in a condition in which the cfDNA having a double helix structure derived from normal cells is not denatured.

3. The method for diagnosing prostate cancer according to claim 1, wherein the cfDNA derived from prostate cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 30 minutes;
vii) a condition of treating with DNase for 1 minute to 30 minutes; and
viii) a condition of treating with a chemical substance (for example, sodium hydroxide, DMSO, a surfactant, and the like).

4. The method for diagnosing prostate cancer according to claim 1, wherein the cfDNA derived from prostate cancer cells is circulating tumor DNA derived from prostate cancer.

5. The method for diagnosing prostate cancer according to claim 4, wherein the probe having a sequence complementary to cfDNA complementarily binds to at least any one gene selected from the group consisting of *KLK3, FOLH1, PCA3, PDE4D7, SFMBT2, EFEMP1 , RETN, ACADL, AGR2, COL1A1, FAM13C, GPX8, GRHL2, HNF1A, HOXB13, KLK2, MYBPC1, NR0B1, PITX2, SFRP4, SLC01B3, TMEFF2, TMPRSS2-ERG,* and a combination thereof.

6. The method for diagnosing prostate cancer according to claim 1, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

7. The method for diagnosing prostate cancer according to claim 1, wherein the sample is any one selected from the group consisting of urine, cerebrospinal fluid, plasma, blood, pleural fluid, ascites, saliva, sputum, and body fluid.

8. The method for diagnosing prostate cancer according to claim 1, wherein the positively charged material is a positively charged nanowire.

9. The method for diagnosing prostate cancer according to claim 8, wherein the nanowire comprises a conductive polymer.

10. The method for diagnosing prostate cancer according to claim 9, wherein the nanowire further comprises biotin.

11. The method for diagnosing prostate cancer according to claim 9, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

12. The method for diagnosing prostate cancer according to claim 1, wherein the probe is composed of a 15-mer to 30-mer nucleotide.

13. The method for diagnosing prostate cancer according to claim 1, wherein at least one biotin is bound to the probe.

14. The method for diagnosing prostate cancer according to claim 1, wherein the marker is any one selected from the group consisting of a quantum dot, a HRP (horse-radish peroxidase), a fluorescent protein, a phosphor, an alkaline phosphatase, and a luciferase.

15. The method for diagnosing prostate cancer according to claim 14, wherein an avidin-based protein is bound to the marker.

16. The method for diagnosing prostate cancer according to claim 14, wherein the avidin-based protein is any one selected from the group consisting of avidin, streptavidin, and a combination thereof.

17. The method for diagnosing prostate cancer according to claim 1, wherein the marker is a nanoparticle comprising a conductive polymer; hyaluronic acid; avidin or streptavidin; and a HRP (horse-radish peroxidase) or a fluorescent protein.

18. The method for diagnosing prostate cancer according to claim 1, wherein the cfDNA further comprises one derived from at least any one gene selected from the group consisting *ofACPP, CPT1A, IFNG, CD274, FOLR1, EPCAM, OGT,* and a combination thereof.

19. The method for diagnosing prostate cancer according to claim 1, wherein the marker is detected in the step e) by a color change, a UV absorbance change, a fluorescence change, or an electrochemical change.

20. A diagnostic kit for diagnosing prostate cancer, comprising
a probe to which biotin is bound complementarily binding to a gene specifically expressed in prostate cancer;
a positively charged material;
a marker to which an avidin-based protein is bound; and
a manual,
wherein the manual describes that the kit is configured to be capable of diagnosing prostate cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

21. The diagnostic kit according to claim 20, wherein the gene specifically expressed in prostate cancer is any one or more gene selected from the group consisting of *KLK3, FOLH1, PCA3, PDE4D7, SFMBT2, EFEMP1, RETN, ACADL, AGR2, COL1A1, FAM13C, GPX8, GRHL2, HNF1A, HOXB13, KLK2, MYBPC1, NR0B1, PITX2, SFRP4, SLC01B3, TMEFF2, TMPRSS2-ERG,* and a combination thereof.

22. The diagnostic kit according to claim 20, wherein the kit further comprises a probe to which biotin is bound complementarily binding to at least any one gene selected from the group consisting of *ACPP, CPT1A, IFNG, CD274, FOLR1, EPCAM, OGT,* and a combination thereof.

23. The diagnostic kit according to claim 20, wherein the positively charged material is a positively charged nanowire.

24. The diagnostic kit according to claim 23, wherein the nanowire comprises a conductive polymer.

25. The diagnostic kit according to claim 24, wherein the nanowire further comprises biotin.

26. The diagnostic kit according to claim 24, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

27. The diagnostic kit according to claim 20, wherein the marker is a nanoparticle comprising a conductive polymer; hyaluronic acid; avidin or streptavidin; and a HRP (horse-radish peroxidase) or a fluorescent protein.

28. A device for diagnosing prostate cancer by detecting a gene derived from prostate cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in prostate cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from prostate cancer in the sample depending on whether the marker is detected.

29. The device for diagnosing prostate cancer according to claim 28, wherein the gene specifically expressed in prostate cancer further comprises a probe to which biotin is bound complementarily binding to at least any one gene selected from the group consisting of *KLK3, FOLH1, PCA3, PDE4D7, SFMBT2, EFEMP1, RETN, ACADL, AGR2, COL1A1, FAM13C, GPX8, GRHL2, HNF1A, HOXB13, KLK2, MYBPC1, NR0B1, PITX2, SFRP4, SLC01B3, TMEFF2, TMPRSS2-ERG,* and a combination thereof.

30. The device for diagnosing prostate cancer according to claim 29, wherein the device further comprises a probe to which biotin is bound complementarily binding to at least any one gene selected from the group consisting of *ACPP, CPT1A, IFNG, CD274, FOLR1, EPCAM, OGT,* and a combination thereof.

31. A method for diagnosing lung cancer by detecting a gene derived from lung cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from lung cancer cells, and
wherein the probe having a sequence complementary to cfDNA complementarily binds to a gene known as a lung cancer biomarker.

32. The method for diagnosing lung cancer according to claim 31, **characterized in that** the cfDNA derived from lung cancer cells
i) has a lower Tm value than that of cfDNA having a double helix structure derived from normal cells, or
ii) is denatured in a condition in which the cfDNA having a double helix structure derived from normal cells is not denatured.

33. The method for diagnosing lung cancer according to claim 31, wherein the cfDNA derived from lung cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 30 minutes;
vii) a condition of treating with DNase for 1 minute to 30 minutes; and
viii) a condition of treating with a chemical substance (for example, sodium hydroxide, DMSO, a surfactant, and the like).

34. The method for diagnosing lung cancer according to claim 31, wherein the cfDNA derived from lung cancer cells is circulating tumor DNA derived from lung cancer.

35. The method for diagnosing lung cancer according to claim 34, wherein the probe having a sequence complementary to cfDNA complementarily binds to at least any one gene selected from the group consisting of *SART3, PLAT, ALK, ROS1, PI3K, S100P, CDCA7, S100A2, ETV4,* and a combination thereof.

36. The method for diagnosing lung cancer according to claim 31, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

37. The method for diagnosing lung cancer according to claim 31, wherein the sample is any one selected from the group consisting of urine, cerebrospinal fluid, plasma, blood, pleural fluid, ascites, saliva, sputum, and body fluid.

38. The method for diagnosing lung cancer according to claim 31, wherein the positively charged material is a positively charged nanowire.

39. The method for diagnosing lung cancer according to claim 38, wherein the nanowire comprises a conductive polymer.

40. The method for diagnosing lung cancer according to claim 39, wherein the nanowire further comprises biotin.

41. The method for diagnosing lung cancer according to claim 39, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(paraphenylene vinylene), and polyaniline.

42. The method for diagnosing lung cancer according to claim 31, wherein the probe is composed of a 15-mer to 30-mer nucleotide.

43. The method for diagnosing lung cancer according to claim 31, wherein at least one biotin is bound to the probe.

44. The method for diagnosing lung cancer according to claim 31, wherein the marker is any one selected from the group consisting of a quantum dot, a HRP (horse-radish peroxidase), a fluorescent protein, a phosphor, an alkaline phosphatase, and a luciferase.

45. The method for diagnosing lung cancer according to claim 44, wherein an avidin-based protein is bound to the marker.

46. The method for diagnosing lung cancer according to claim 44, wherein the avidin-based protein is any one selected from the group consisting of avidin, streptavidin, and a combination thereof.

47. The method for diagnosing lung cancer according to claim 31, wherein the marker is a nanoparticle comprising a conductive polymer; hyaluronic acid; avidin or streptavidin; and a HRP (horse-radish peroxidase) or a fluorescent protein.

48. The method for diagnosing lung cancer according to claim 31, wherein the cfDNA further comprises one derived from at least any one gene selected from the group consisting of *ENO2, ACPP, KRT19, EGFR, KRAS, RET, ERBB2, MMP11, TOP2A, UBE2C, CPT1A, IFNG, CD279, CD274, ERBB2, EGFR, FOLR1, EPCAM,* and a combination thereof.

49. The method for diagnosing lung cancer according to claim 31, wherein the marker is detected in the step e) by a color change, a UV absorbance change, a fluorescence change, or an electrochemical change.

50. A diagnostic kit for diagnosing lung cancer, comprising
a probe to which biotin is bound complementarily binding to a gene specifically expressed in lung cancer;
a positively charged material;
a marker to which an avidin-based protein is bound; and
a manual,
wherein the manual describes that the kit is configured to be capable of diagnosing lung cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

51. The diagnostic kit according to claim 50, wherein the gene specifically expressed in lung cancer is any one or more gene selected from the group consisting of *SART3, PLAT, ALK, ROS1, PI3K, S100P, CDCA7, S100A2, ETV4,* and a combination thereof.

52. The diagnostic kit according to claim 50, wherein the kit further comprises a probe to which biotin is bound complementarily binding to at least any one gene selected from the group consisting of *ENO2, ACPP, KRT19, EGFR, KRAS, RET, ERBB2, MMP11, TOP2A, UBE2C, CPT1A, IFNG, CD279, CD274, ERBB2, EGFR, FOLR1, EPCAM,* and a combination thereof.

53. The diagnostic kit according to claim 50, wherein the positively charged material is a positively charged nanowire.

54. The diagnostic kit according to claim 53, wherein the nanowire comprises a conductive polymer.

55. The diagnostic kit according to claim 54, wherein the nanowire further comprises biotin.

56. The diagnostic kit according to claim 53, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

57. The diagnostic kit according to claim 50, wherein the marker is a nanoparticle comprising a conductive polymer; hyaluronic acid; avidin or streptavidin; and a HRP (horse-radish peroxidase) or a fluorescent protein.

58. A device for diagnosing lung cancer by detecting a gene derived from lung cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in lung cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from lung cancer in the sample depending on whether the
marker is detected.

59. The device for diagnosing lung cancer according to claim 58, wherein the gene specifically expressed in lung cancer further comprises a probe to which biotin is bound complementarily binding to at least any one gene selected from the group consisting of *SART3, PLAT, ALK, ROS1, PI3K, S100P, CDCA7, S100A2, ETV4G,* and a combination thereof.

60. The device for diagnosing lung cancer according to claim 59, wherein the device further comprises a probe to which biotin is bound complementarily binding to at least any one gene selected from the group consisting of *ENO2, ACPP, KRT19, EGFR, KRAS, RET, ERBB2, MMP11, TOP2A, UBE2C, CPT1A, IFNG, CD279, CD274, ERBB2, EGFR, FOLR1, EPCAM,* and a combination thereof.

61. A method for diagnosing thyroid cancer by detecting a gene derived from thyroid cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from thyroid cancer cells, and
wherein the probe having a sequence complementary to cfDNA complementarily binds to a gene known as a thyroid cancer biomarker.

62. The method for diagnosing thyroid cancer according to claim 61, **characterized in that** the cfDNA derived from thyroid cancer cells
i) has a lower Tm value than that of cfDNA having a double helix structure derived from normal cells, or
ii) is denatured in a condition in which the cfDNA having a double helix structure derived from normal cells is not denatured.

63. The method for diagnosing thyroid cancer according to claim 61, wherein the cfDNA derived from thyroid cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

64. The method for diagnosing thyroid cancer according to claim 61, wherein the cfDNA derived from thyroid cancer cells is circulating tumor DNA derived from thyroid cancer.

65. The method for diagnosing thyroid cancer according to claim 64, wherein the probe having a sequence complementary to cfDNA complementarily binds to at least any one gene selected from the group consisting *of TG, CALCA, APOC1, HIG2,* and a combination thereof.

66. The method for diagnosing thyroid cancer according to claim 61, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

67. The method for diagnosing thyroid cancer according to claim 61, wherein the sample is any one selected from the group consisting of urine, cerebrospinal fluid, plasma, blood, pleural fluid, ascites, saliva, sputum, and body fluid.

68. The method for diagnosing thyroid cancer according to claim 61, wherein the positively charged material is a positively charged nanowire.

69. The method for diagnosing thyroid cancer according to claim 68, wherein the nanowire comprises a conductive polymer.

70. The method for diagnosing thyroid cancer according to claim 69, wherein the nanowire further comprises biotin.

71. The method for diagnosing thyroid cancer according to claim 69, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

72. The method for diagnosing thyroid cancer according to claim 61, wherein the probe is composed of a 15-mer to 30-mer nucleotide.

73. The method for diagnosing thyroid cancer according to claim 61, wherein at least one biotin is bound to the probe.

74. The method for diagnosing thyroid cancer according to claim 61, wherein the marker is any one selected from the group consisting of a quantum dot, a HRP (horse-radish peroxidase), a fluorescent protein, a phosphor, an alkaline phosphatase, and a luciferase.

75. The method for diagnosing thyroid cancer according to claim 74, wherein an avidin-based protein is bound to the marker.

76. The method for diagnosing thyroid cancer according to claim 74, wherein the avidin-based protein is any one selected from the group consisting of avidin, streptavidin, and a combination thereof.

77. The method for diagnosing thyroid cancer according to claim 61, wherein the marker is a nanoparticle comprising a conductive polymer; hyaluronic acid; avidin or streptavidin; and a HRP (horse-radish peroxidase) or a fluorescent protein.

78. The method for diagnosing thyroid cancer according to claim 61, wherein the cfDNA further comprises one derived from at least any one gene selected from the group consisting of *ENO2, ACPP, TYRO3, CPT1A, IFNG, CD279, CD274, ERBB2, EGFR, FOLR1, EPCAM,* and a combination thereof.

79. The method for diagnosing thyroid cancer according to claim 61, wherein the marker is detected in the step e) by a color change, a UV absorbance change, a fluorescence change, or an electrochemical change.

80. A diagnostic kit for diagnosing thyroid cancer, comprising
a probe to which biotin is bound complementarily binding to a gene specifically expressed in thyroid cancer;
a positively charged material;
a marker to which an avidin-based protein is bound; and
a manual,
wherein the manual describes that the kit is configured to be capable of diagnosing thyroid cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

81. The diagnostic kit according to claim 80, wherein the gene specifically expressed in thyroid cancer is any one or more gene selected from the group consisting *of TG, CALCA, APOC1, HIG2,* and a combination thereof.

82. The diagnostic kit according to claim 80, wherein the kit further comprises a probe to which biotin is bound complementarily binding to at least any one gene selected from the group consisting of *EENO2 ACPP, TYRO3, CPT1A, IFNG, CD279, CD274, ERBB2, EGFR, FOLR1, EPCAM,* and a combination thereof.

83. The diagnostic kit according to claim 80, wherein the positively charged material is a positively charged nanowire.

84. The diagnostic kit according to claim 83, wherein the nanowire comprises a conductive polymer.

85. The diagnostic kit according to claim 84, wherein the nanowire further comprises biotin.

86. The diagnostic kit according to claim 83, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

87. The diagnostic kit according to claim 80, wherein the marker is a nanoparticle comprising a conductive polymer; hyaluronic acid; avidin or streptavidin; and a HRP (horse-radish peroxidase) or a fluorescent protein.

88. A device for diagnosing thyroid cancer by detecting a gene derived from thyroid cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in thyroid cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from thyroid cancer in the sample depending on whether the marker is detected.

89. The device for diagnosing thyroid cancer according to claim 88, wherein the gene specifically expressed in thyroid cancer further comprises a probe to which biotin is bound complementarily binding to at least any one gene selected from the group consisting of *TG, CALCA, APOC1, HIG2,* and a combination thereof.

90. The device for diagnosing thyroid cancer according to claim 89, wherein the device further comprises a probe to which biotin is bound complementarily binding to at least any one gene selected from the group consisting of *ENO2, ACPP, TYRO3, CPT1A, IFNG, CD279, CD274, ERBB2, EGFR, FOLR1, EPCAM,* and a combination thereof.

91. A method for diagnosing bladder cancer by detecting a gene derived from bladder cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from bladder cancer cells, and
wherein the probe having a sequence complementary to cfDNA complementarily binds to a gene known as a bladder cancer biomarker.

92. The method for diagnosing bladder cancer according to claim 91, **characterized in that** the cfDNA derived from bladder cancer cells
i) has a lower Tm value than that of cfDNA having a double helix structure derived from normal cells, or
ii) is denatured in a condition in which the cfDNA having a double helix structure derived from normal cells is not denatured.

93. The method for diagnosing bladder cancer according to claim 91, wherein the cfDNA derived from bladder cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

94. The method for diagnosing bladder cancer according to claim 91, wherein the cfDNA derived from bladder cancer cells is circulating tumor DNA derived from bladder cancer.

95. The method for diagnosing bladder cancer according to claim 94, wherein the probe having a sequence complementary to cfDNA complementarily binds to at least any one gene selected from the group consisting *of OGT, FGFR3, TP53, NUMA1, COCH, CELSR3, HMOX1, KIF1A, MGC17624, MTAP, PFKFB4, S100A8, RSPH9, FOXM1, FANCB, FANCC, FANCD2, RUSC1-AS1, CACNA1B, IMP-1, PDE3A, POU3F4, SOX3, DMC1, PLXDC2, ZNF312, SYCP2L, HOXA9, ISL1, ALDH1A3,* and a combination thereof.

96. The method for diagnosing bladder cancer according to claim 91, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

97. The method for diagnosing bladder cancer according to claim 91, wherein the sample is any one selected from the group consisting of urine, cerebrospinal fluid, plasma, blood, pleural fluid, ascites, saliva, sputum, and body fluid.

98. The method for diagnosing bladder cancer according to claim 91, wherein the positively charged material is a positively charged nanowire.

99. The method for diagnosing bladder cancer according to claim 98, wherein the nanowire comprises a conductive polymer.

100. The method for diagnosing bladder cancer according to claim 99, wherein the nanowire further comprises biotin.

101. The method for diagnosing bladder cancer according to claim 99, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

102. The method for diagnosing bladder cancer according to claim 91, wherein the probe is composed of a 15-mer to 30-mer nucleotide.

103. The method for diagnosing bladder cancer according to claim 91, wherein at least one biotin is bound to the probe.

104. The method for diagnosing bladder cancer according to claim 91, wherein the marker is any one selected from the group consisting of a quantum dot, a HRP (horse-radish peroxidase), a fluorescent protein, a phosphor, an alkaline phosphatase, and a luciferase.

105. The method for diagnosing bladder cancer according to claim 104, wherein an avidin-based protein is bound to the marker.

106. The method for diagnosing bladder cancer according to claim 104, wherein the avidin-based protein is any one selected from the group consisting of avidin, streptavidin, and a combination thereof.

107. The method for diagnosing bladder cancer according to claim 91, wherein the marker is a nanoparticle comprising a conductive polymer; hyaluronic acid; avidin or streptavidin; and a HRP (horse-radish peroxidase) or a fluorescent protein.

108. The method for diagnosing bladder cancer according to claim 91, wherein the cfDNA further comprises one derived from at least any one gene selected from the group consisting of *KRT19, CCNB1, CPT1A, IFNG, CD279, CD274, ERBB2, EGFR, FOLR1, EPCAM,* and a combination thereof.

109. The method for diagnosing bladder cancer according to claim 91, wherein the marker is detected in the step e) by a color change, a UV absorbance change, a fluorescence change, or an electrochemical change.

110. A diagnostic kit for diagnosing bladder cancer, comprising
a probe to which biotin is bound complementarily binding to a gene specifically expressed in bladder cancer;
a positively charged material;
a marker to which an avidin-based protein is bound; and
a manual,
wherein the manual describes that the kit is configured to be capable of diagnosing bladder cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

111. The diagnostic kit according to claim 110, wherein the gene specifically expressed in bladder cancer is any one or more gene selected from the group consisting of *OGT, FGFR3, TP53, NUMA1, COCH, CELSR3, HMOX1, K1F1A, MGC17624, MTAP, PFKFB4, S100A8, RSPH9, FOXM1, FANCB, FANCC, FANCD2, RUSC1-AS1, CACNA1B, IMP-1, PDE3A, POU3F4, SOX3, DMC1, PLXDC2, ZNF312, SYCP2L, HOXA9, ISL1, ALDH1A3,* and a combination thereof.

112. The diagnostic kit according to claim 110, wherein the kit further comprises a probe to which biotin is bound complementarily binding to at least any one gene selected from the group consisting of *KRT19, CCNB1, CPT1A, IFNG, CD279, CD274, ERBB2, EGFR, FOLR1, EPCAM,* and a combination thereof.

113. The diagnostic kit according to claim 110, wherein the positively charged material is a positively charged nanowire.

114. The diagnostic kit according to claim 113, wherein the nanowire comprises a conductive polymer.

115. The diagnostic kit according to claim 114, wherein the nanowire further comprises biotin.

116. The diagnostic kit according to claim 113, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

117. The diagnostic kit according to claim 110, wherein the marker is a nanoparticle comprising a conductive polymer; hyaluronic acid; avidin or streptavidin; and a HRP (horse-radish peroxidase) or a fluorescent protein.

118. A device for diagnosing bladder cancer by detecting a gene derived from bladder cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in bladder cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from bladder cancer in the sample depending on whether the marker is detected.

119. The device for diagnosing bladder cancer according to claim 118, wherein the gene specifically expressed in bladder cancer further comprises a probe to which biotin is bound complementarily binding to at least any one gene selected from the group consisting of *OGT, FGFR3, TP53, NUMA1, COCH, CELSR3, HMOX1, KIF1A, MGC17624, MTAP, PFKFB4, S100A8, RSPH9, FOXM1, FANCB, FANCC, FANCD2, RUSC1-AS1, CACNA1B, IMP-1, PDE3A, POU3F4, SOX3, DMC1, PLXDC2, ZNF312, SYCP2L, HOXA9, ISL1, ALDH1A3,* and a combination thereof.

120. The device for diagnosing bladder cancer according to claim 119, wherein the device further comprises a probe to which biotin is bound complementarily binding to at least any one gene selected from the group consisting of *KRT19, CCNB1, CPT1A, IFNG, CD279, CD274, ERBB2, EGFR, FOLR1, EPCAM,* and a combination thereof.

121. A method for diagnosing breast cancer by detecting a gene derived from breast cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from breast cancer cells, and
wherein the probe having a sequence complementary to cfDNA complementarily binds to a gene known as a breast cancer biomarker.

122. The method for diagnosing breast cancer according to claim 121, **characterized in that** the cfDNA derived from breast cancer cells
i) has a lower Tm value than that of cfDNA having a double helix structure derived from normal cells, or
ii) is denatured in a condition in which the cfDNA having a double helix structure derived from normal cells is not denatured.

123. The method for diagnosing breast cancer according to claim 121, wherein the cfDNA derived from breast cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

124. The method for diagnosing breast cancer according to claim 121, wherein the cfDNA derived from breast cancer cells is circulating tumor DNA derived from breast cancer.

125. The method for diagnosing breast cancer according to claim 124, wherein the probe having a sequence complementary to cfDNA complementarily binds to at least any one gene selected from the group consisting *ofMEST, NR1D1, BIRC5, RACGAP1, DHCR7, STC2, AZGP1, RBBP8, IL6ST, MGP, TRBC1, MMP11, COL10A1, C10orf64, COL11A1, POTEG, FSIP1, HER2,* and a combination thereof.

126. The method for diagnosing breast cancer according to claim 121, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

127. The method for diagnosing breast cancer according to claim 121, wherein the sample is any one selected from the group consisting of urine, cerebrospinal fluid, plasma, blood, pleural fluid, ascites, saliva, sputum, and body fluid.

128. The method for diagnosing breast cancer according to claim 1, wherein the positively charged material is a positively charged nanowire.

129. The method for diagnosing breast cancer according to claim 128, wherein the nanowire comprises a conductive polymer.

130. The method for diagnosing breast cancer according to claim 129, wherein the nanowire further comprises biotin.

131. The method for diagnosing breast cancer according to claim 129, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

132. The method for diagnosing breast cancer according to claim 121, wherein the probe is composed of a 15-mer to 30-mer nucleotide.

133. The method for diagnosing breast cancer according to claim 121, wherein at least one biotin is bound to the probe.

134. The method for diagnosing breast cancer according to claim 121, wherein the marker is any one selected from the group consisting of a quantum dot, a HRP (horse-radish peroxidase), a fluorescent protein, a phosphor, an alkaline phosphatase, and a luciferase.

135. The method for diagnosing breast cancer according to claim 134, wherein an avidin-based protein is bound to the marker.

136. The method for diagnosing breast cancer according to claim 134, wherein the avidin-based protein is any one selected from the group consisting of avidin, streptavidin, and a combination thereof.

137. The method for diagnosing breast cancer according to claim 121, wherein the marker is a nanoparticle comprising a conductive polymer; hyaluronic acid; avidin or streptavidin; and a HRP (horse-radish peroxidase) or a fluorescent protein.

138. The method for diagnosing breast cancer according to claim 121, wherein the cfDNA further comprises one derived from at least any one gene selected from the group consisting of *MUC1, ACPP, TYRO3, UBE2C, CPT1A, IFNG, CD279, CD274, ERBB2, EGFR, FOLR1, EPCAM,* and a combination thereof.

139. The method for diagnosing breast cancer according to claim 121, wherein the marker is detected in the step e) by a color change, a UV absorbance change, a fluorescence change, or an electrochemical change.

140. A diagnostic kit for diagnosing breast cancer, comprising
a probe to which biotin is bound complementarily binding to a gene specifically expressed in breast cancer;
a positively charged material;
a marker to which an avidin-based protein is bound; and
a manual,
wherein the manual describes that the kit is configured to be capable of diagnosing breast cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

141. The diagnostic kit according to claim 140, wherein the gene specifically expressed in breast cancer is any one or more gene selected from the group consisting *ofMEST, NR1D1, BIRC5, RACGAP1, DHCR7, STC2, AZGP1, RBBP8, IL6ST, MGP, TRBC1, MMP11, COL10A1, C10orf64, COL11A1, POTEG, FSIP1, HER2,* and a combination thereof.

142. The diagnostic kit according to claim 140, wherein the kit further comprises a probe to which biotin is bound complementarily binding to at least any one gene selected from the group consisting of *MUC1, ACPP, TYRO3, UBE2C, CPT1A, IFNG, CD279, CD274, ERBB2, EGFR, FOLR1, EPCAM,* and a combination thereof.

143. The diagnostic kit according to claim 140, wherein the positively charged material is a positively charged nanowire.

144. The diagnostic kit according to claim 143, wherein the nanowire comprises a conductive polymer.

145. The diagnostic kit according to claim 144, wherein the nanowire further comprises biotin.

146. The diagnostic kit according to claim 143, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

147. The diagnostic kit according to claim 140, wherein the marker is a nanoparticle comprising a conductive polymer; hyaluronic acid; avidin or streptavidin; and a HRP (horse-radish peroxidase) or a fluorescent protein.

148. A device for diagnosing breast cancer by detecting a gene derived from breast cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in breast cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from breast cancer in the sample depending on whether the marker is detected.

149. The device for diagnosing breast cancer according to claim 148, wherein the gene specifically expressed in breast cancer further comprises a probe to which biotin is bound complementarily binding to at least any one gene selected from the group consisting of *MEST, NR1D1, BIRC5, RACGAP1, DHCR7, STC2, AZGP1, RBBP8, IL6ST, MGP, TREC1, MMP11, COL10A1, C10orf64, COL11A1, POTEG, FSIP1, HER2,* and a combination thereof.

150. The device for diagnosing breast cancer according to claim 149, wherein the device further comprises a probe to which biotin is bound complementarily binding to at least any one gene selected from the group consisting of *MUC1, ACPP, TYRO3, UBE2C, CPT1A, IFNG, CD279, CD274, ERBB2, EGFR, FOLR1, EPCAM,* and a combination thereof.

151. A method for diagnosing colorectal cancer by detecting a gene derived from colorectal cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from colorectal cancer cells, and
wherein the probe having a sequence complementary to cfDNA complementarily binds to a gene known as a colorectal cancer biomarker.

152. The method for diagnosing colorectal cancer according to claim 151, **characterized in that** the cfDNA derived from colorectal cancer cells
i) has a lower Tm value than that of cfDNA having a double helix structure derived from normal cells, or
ii) is denatured in a condition in which the cfDNA having a double helix structure derived from normal cells is not denatured.

153. The method for diagnosing colorectal cancer according to claim 151, wherein the cfDNA derived from colorectal cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

154. The method for diagnosing colorectal cancer according to claim 151, wherein the cfDNA derived from colorectal cancer cells is circulating tumor DNA derived from colorectal cancer.

155. The method for diagnosing colorectal cancer according to claim 154, wherein the probe having a sequence complementary to cfDNA complementarily binds to at least any one gene selected from the group consisting of *NCKAP1, AUNIP, NOTUM, KRT5, TUBB, COL6A1, JUP, CDX2, MELTF, EFEMP2, DEFA5, CHEK1, MAD2L1, ENC1, CSE1L, RAD51AP1, ERICH3, SLC7A11, KRT23, PLAU, CDCA1, KLK6, DPEP1, CDH3, ANLN, CXCL1, CTHRC1, LCN2, HS6ST2, EGFL6, CXCL3, CA9, PROX1, SPP1, CST1, CXCL2, TSTA3, RRM2, MMP3, MMP7, MMP10, CXCL5, SERPINB5, TEAD4, BUB1, CDC2, CLDN2, HSPH1, LY6G6D, PRC1, PUS1, SQLE, TTK, ECT2, RNF183, FBXO39 TEX38, TTLL2, PRR7, CANP, KIAA0101,* and a combination thereof.

156. The method for diagnosing colorectal cancer according to claim 151, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

157. The method for diagnosing colorectal cancer according to claim 151, wherein the sample is any one selected from the group consisting of urine, cerebrospinal fluid, plasma, blood, pleural fluid, ascites, saliva, sputum, and body fluid.

158. The method for diagnosing colorectal cancer according to claim 151, wherein the positively charged material is a positively charged nanowire.

159. The method for diagnosing colorectal cancer according to claim 158, wherein the nanowire comprises a conductive polymer.

160. The method for diagnosing colorectal cancer according to claim 159, wherein the nanowire further comprises biotin.

161. The method for diagnosing colorectal cancer according to claim 159, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

162. The method for diagnosing colorectal cancer according to claim 151, wherein the probe is composed of a 15-mer to 30-mer nucleotide.

163. The method for diagnosing colorectal cancer according to claim 151, wherein at least one biotin is bound to the probe.

164. The method for diagnosing colorectal cancer according to claim 151, wherein the marker is any one selected from the group consisting of a quantum dot, a HRP (horse-radish peroxidase), a fluorescent protein, a phosphor, an alkaline phosphatase, and a luciferase.

165. The method for diagnosing colorectal cancer according to claim 164, wherein an avidin-based protein is bound to the marker.

166. The method for diagnosing colorectal cancer according to claim 164, wherein the avidin-based protein is any one selected from the group consisting of avidin, streptavidin, and a combination thereof.

167. The method for diagnosing colorectal cancer according to claim 151, wherein the marker is a nanoparticle comprising a conductive polymer; hyaluronic acid; avidin or streptavidin; and a HRP (horse-radish peroxidase) or a fluorescent protein.

168. The method for diagnosing colorectal cancer according to claim 151, wherein the cfDNA further comprises one derived from at least any one gene selected from the group consisting *of ACPP, FLU3, TYRO3, COTL1, CK7, CK20, MUC2, SDC2, ASB9, CCNB1, MELK, CKS2, IFITM1, CEACAM6, ATAD2, TOP2A, CPT1A, DSCC1, IFNG, CD279, CD274, ERBB2, EGFR, FOLR1, EPCA,* and a combination thereof.

169. The method for diagnosing colorectal cancer according to claim 151, wherein the marker is detected in the step e) by a color change, a UV absorbance change, a fluorescence change, or an electrochemical change.

170. A diagnostic kit for diagnosing colorectal cancer, comprising
a probe to which biotin is bound complementarily binding to a gene specifically expressed in colorectal cancer;
a positively charged material;
a marker to which an avidin-based protein is bound; and
a manual,
wherein the manual describes that the kit is configured to be capable of diagnosing colorectal cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

171. The diagnostic kit according to claim 170, wherein the gene specifically expressed in colorectal cancer is any one or more gene selected from the group consisting of *NCKAP1, AUNIP, NOTUM, KRT5, TUBB, COL6A1, JUP, CDX2, MELTF, EFEMP2, DEFA5, CHEK1, MAD2L1, ENC1, CSE1L, RAD51AP1, ERICH3, SLC7A11, KRT23, PLAU, CDCA1, KLK6, DPEP1, CDH3, ANLN, CXCL1, CTHRC1, LCN2, HS6ST2, EGFL6, CXCL3, CA9, PROX1, SPP1, CST1, CXCL2, TSTA3, RRM2, MMP3, MMP7, MMP10, CXCL5, SERPINB5, TEAD4, BUB1, CDC2, CLDN2, HSPH1, LY6G6D, PRC1, PUS1, SQLE, TTK, ECT2, RNF183, FBXO39 TEX38, TTLL2, PRR7, CANP, KIAA0101,* and a combination thereof.

172. The diagnostic kit according to claim 170, wherein the kit further comprises a probe to which biotin is bound complementarily binding to at least any one gene selected from the group consisting of *ACPP, FLU3, TYRO3, COTL1, CK7, CK20, MUC2, SDC2, ASB9, CCNB1, MELK, CKS2, IFITM1, CEACAM6, ATAD2, TOP2A, CPT1A, DSCC1, IFNG, CD279, CD274, ERBB2, EGFR, FOLR1, EPCAM,* and a combination thereof.

173. The diagnostic kit according to claim 170, wherein the positively charged material is a positively charged nanowire.

174. The diagnostic kit according to claim 173, wherein the nanowire comprises a conductive polymer.

175. The diagnostic kit according to claim 174, wherein the nanowire further comprises biotin.

176. The diagnostic kit according to claim 173, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

177. The diagnostic kit according to claim 170, wherein the marker is a nanoparticle comprising a conductive polymer; hyaluronic acid; avidin or streptavidin; and a HRP (horse-radish peroxidase) or a fluorescent protein.

178. A device for diagnosing colorectal cancer by detecting a gene derived from colorectal cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in colorectal cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from colorectal cancer in the sample depending on whether the marker is detected.

179. The device for diagnosing colorectal cancer according to claim 178, wherein the gene specifically expressed in colorectal cancer further comprises a probe to which biotin is bound complementarily binding to at least any one gene selected from the group consisting of *NCKAP1, AUNIP, NOTUM, KRT5, TUBB, COL6A1, JUP, CDX2, MELTF, EFEMP2, DEFA5, CHEK1, MAD2L1, ENC1, CSE1L, RAD51AP1, ERICH3, SLC7A11, KRT23, PLAU, CDCA1, KLK6, DPEP1, CDH3, ANLN, CXCL1, CTHRC1, LCN2, HS6ST2, EGFL6, CXCL3, CA9, PROX1, SPP1, CST1, CXCL2, TSTA3, RRM2, MMP3, MMP7, MMP10, CXCL5, SERPINB5, TEAD4, BUB1, CDC2, CLDN2, HSPH1, LY6G6D, PRC1, PUS1, SQLE, TTK, ECT2, RNF183, FBXO39 TEX38, TTLL2, PRR7, CANP, KIAA0101,* and a combination thereof.

180. The device for diagnosing colorectal cancer according to claim 179, wherein the device further comprises a probe to which biotin is bound complementarily binding to at least any one gene selected from the group consisting of *ACPP, FLU3, TYRO3, COTL1, CK7, CK20, MUC2, SDC2, ASB9, CCNB1, MELK, CKS2, IFITM1, CEACAM6, ATAD2, TOP2A, CPT1A, DSCC1, IFNG, CD279, CD274, ERBB2, EGFR, FOLR1, EPCAM,* and a combination thereof.

181. A method for diagnosing biliary tract cancer by detecting a gene derived from biliary tract cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from biliary tract cancer cells, and
wherein the probe having a sequence complementary to cfDNA complementarily binds to a gene known as a biliary tract cancer biomarker.

182. The method for diagnosing biliary tract cancer according to claim 181, **characterized in that** the cfDNA derived from biliary tract cancer cells
i) has a lower Tm value than that of cfDNA having a double helix structure derived from normal cells, or
ii) is denatured in a condition in which the cfDNA having a double helix structure derived from normal cells is not denatured.

183. The method for diagnosing biliary tract cancer according to claim 181, wherein the cfDNA derived from biliary tract cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

184. The method for diagnosing biliary tract cancer according to claim 181, wherein the cfDNA derived from biliary tract cancer cells is circulating tumor DNA derived from biliary tract cancer.

185. The method for diagnosing biliary tract cancer according to claim 184, wherein the probe having a sequence complementary to cfDNA complementarily binds to at least any one gene selected from the group consisting of *MUC16, ASH1L, DOCK7,* and a combination thereof.

186. The method for diagnosing biliary tract cancer according to claim 181, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

187. The method for diagnosing biliary tract cancer according to claim 181, wherein the sample is any one selected from the group consisting of urine, cerebrospinal fluid, plasma, blood, pleural fluid, ascites, saliva, sputum, and body fluid.

188. The method for diagnosing biliary tract cancer according to claim 181, wherein the positively charged material is a positively charged nanowire.

189. The method for diagnosing biliary tract cancer according to claim 188, wherein the nanowire comprises a conductive polymer.

190. The method for diagnosing biliary tract cancer according to claim 189, wherein the nanowire further comprises biotin.

191. The method for diagnosing biliary tract cancer according to claim 189, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

192. The method for diagnosing biliary tract cancer according to claim 181, wherein the probe is composed of a 15-mer to 30-mer nucleotide.

193. The method for diagnosing biliary tract cancer according to claim 181, wherein at least one biotin is bound to the probe.

194. The method for diagnosing biliary tract cancer according to claim 181, wherein the marker is any one selected from the group consisting of a quantum dot, a HRP (horse-radish peroxidase), a fluorescent protein, a phosphor, an alkaline phosphatase, and a luciferase.

195. The method for diagnosing biliary tract cancer according to claim 194, wherein an avidin-based protein is bound to the marker.

196. The method for diagnosing biliary tract cancer according to claim 194, wherein the avidin-based protein is any one selected from the group consisting of avidin, streptavidin, and a combination thereof.

197. The method for diagnosing biliary tract cancer according to claim 181, wherein the marker is a nanoparticle comprising a conductive polymer; hyaluronic acid; avidin or streptavidin; and a HRP (horse-radish peroxidase) or a fluorescent protein.

198. The method for diagnosing biliary tract cancer according to claim 181, wherein the cfDNA further comprises one derived from at least any one gene selected from the group consisting *of ACPP, FLU3, CPT1A, DSCC1, IFNG, CD279, CD274, ERBB2, EGFR, FOLR1, EPCAM,* and a combination thereof.

199. The method for diagnosing biliary tract cancer according to claim 181, wherein the marker is detected in the step e) by a color change, a UV absorbance change, a fluorescence change, or an electrochemical change.

200. A diagnostic kit for diagnosing biliary tract cancer, comprising
a probe to which biotin is bound complementarily binding to a gene specifically expressed in biliary tract cancer;
a positively charged material;
a marker to which an avidin-based protein is bound; and
a manual,
wherein the manual describes that the kit is configured to be capable of diagnosing biliary tract cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

201. The diagnostic kit according to claim 200, wherein the gene specifically expressed in biliary tract cancer is any one or more gene selected from the group consisting of *MUC16, ASH1L, DOCK7,* and a combination thereof.

202. The diagnostic kit according to claim 200, wherein the kit further comprises a probe to which biotin is bound complementarily binding to at least any one gene selected from the group consisting of *ACPP, FLU3, CPT1A, DSCC1, IFNG, CD279, CD274, ERBB2, EGFR, FOLR1, EPCAM,* and a combination thereof.

203. The diagnostic kit according to claim 200, wherein the positively charged material is a positively charged nanowire.

204. The diagnostic kit according to claim 203, wherein the nanowire comprises a conductive polymer.

205. The diagnostic kit according to claim 204, wherein the nanowire further comprises biotin.

206. The diagnostic kit according to claim 203, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

207. The diagnostic kit according to claim 200, wherein the marker is a nanoparticle comprising a conductive polymer; hyaluronic acid; avidin or streptavidin; and a HRP (horse-radish peroxidase) or a fluorescent protein.

208. A device for diagnosing biliary tract cancer by detecting a gene derived from biliary tract cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in biliary tract cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from biliary tract cancer in the sample depending on whether the marker is detected.

209. The device for diagnosing biliary tract cancer according to claim 208, wherein the gene specifically expressed in biliary tract cancer further comprises a probe to which biotin is bound complementarily binding to at least any one gene selected from the group consisting of *MUC16, ASH1L, DOCK7,* and a combination thereof.

210. The device for diagnosing biliary tract cancer according to claim 209, wherein the device further comprises a probe to which biotin is bound complementarily binding to at least any one gene selected from the group consisting of *ACPP, FLU3, CPT1A, DSCC1, IFNG, CD279, CD274, ERBB2, EGFR, FOLR1, EPCAM,* and a combination thereof.

211. A method for diagnosing gastric cancer by detecting a gene derived from gastric cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from gastric cancer cells, and
wherein the probe having a sequence complementary to cfDNA complementarily binds to a gene known as a gastric cancer biomarker.

212. The method for diagnosing gastric cancer according to claim 211, **characterized in that** the cfDNA derived from gastric cancer cells
i) has a lower Tm value than that of cfDNA having a double helix structure derived from normal cells, or
ii) is denatured in a condition in which the cfDNA having a double helix structure derived from normal cells is not denatured.

213. The method for diagnosing gastric cancer according to claim 211, wherein the cfDNA derived from gastric cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

214. The method for diagnosing gastric cancer according to claim 211, wherein the cfDNA derived from gastric cancer cells is circulating tumor DNA derived from gastric cancer.

215. The method for diagnosing gastric cancer according to claim 214, wherein the probe having a sequence complementary to cfDNA complementarily binds to at least any one gene selected from the group consisting of *CGB, PARP1, FOXO3A, MED30, CCNE1, MYC, TFF1, FABP1, LAMP5, MATN3, CLIP4, NOX4, ADRA2C, CSK, FZD9, GALR1, GRM6, INSR, LPHN1, LYN, MRGPRX3, ADCY3, HDAC2, CFL1, NRP2, ANXA10, TFF2, CDCA5, NUSAP1,* and a combination thereof.

216. The method for diagnosing gastric cancer according to claim 211, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

217. The method for diagnosing gastric cancer according to claim 211, wherein the sample is any one selected from the group consisting of urine, cerebrospinal fluid, plasma, blood, pleural fluid, ascites, saliva, sputum, and body fluid.

218. The method for diagnosing gastric cancer according to claim 211, wherein the positively charged material is a positively charged nanowire.

219. The method for diagnosing gastric cancer according to claim 218, wherein the nanowire comprises a conductive polymer.

220. The method for diagnosing gastric cancer according to claim 219, wherein the nanowire further comprises biotin.

221. The method for diagnosing gastric cancer according to claim 219, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

222. The method for diagnosing gastric cancer according to claim 211, wherein the probe is composed of a 15-mer to 30-mer nucleotide.

223. The method for diagnosing gastric cancer according to claim 211, wherein at least one biotin is bound to the probe.

224. The method for diagnosing gastric cancer according to claim 211, wherein the marker is any one selected from the group consisting of a quantum dot, a HRP (horse-radish peroxidase), a fluorescent protein, a phosphor, an alkaline phosphatase, and a luciferase.

225. The method for diagnosing gastric cancer according to claim 224, wherein an avidin-based protein is bound to the marker.

226. The method for diagnosing gastric cancer according to claim 224, wherein the avidin-based protein is any one selected from the group consisting of avidin, streptavidin, and a combination thereof.

227. The method for diagnosing gastric cancer according to claim 211, wherein the marker is a nanoparticle comprising a conductive polymer; hyaluronic acid; avidin or streptavidin; and a HRP (horse-radish peroxidase) or a fluorescent protein.

228. The method for diagnosing gastric cancer according to claim 211, wherein the cfDNA further comprises one derived from at least any one gene selected from the group consisting of *ACPP, FLU3, KRT19, ERBB2, EGFR, KRAS, DSCC1, CK20, MUC2, SDC2, COTL1, ATAD2, ASB9, MMP1, CEACAM6, DSCC1, CKS2, CST1, IFITM1, MELK, LGALS3BP, CPT1A, IFNG, CD279, CD274, ERBB2, EGFR, FOLR1, EPCAM,* and a combination thereof.

229. The method for diagnosing gastric cancer according to claim 211, wherein the marker is detected in the step e) by a color change, a UV absorbance change, a fluorescence change, or an electrochemical change.

230. A diagnostic kit for diagnosing gastric cancer, comprising
a probe to which biotin is bound complementarily binding to a gene specifically expressed in gastric cancer;
a positively charged material;
a marker to which an avidin-based protein is bound; and
a manual,
wherein the manual describes that the kit is configured to be capable of diagnosing gastric cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

231. The diagnostic kit according to claim 230, wherein the gene specifically expressed in gastric cancer is any one or more gene selected from the group consisting *of CGB, PARP1, FOXO3A, MED30, CCNE1, MYC, TFF1, FABP1, LAMP5, MATN3, CLIP4, NOX4, ADRA2C, CSK, FZD9, GALR1, GRM6, INSR, LPHN1, LYN, MRGPRX3, ADCY3, HDAC2, CFL1, NRP2, ANXA10, TFF2, CDCA5, NUSAP1,* and a combination thereof.

232. The diagnostic kit according to claim 230, wherein the kit further comprises a probe to which biotin is bound complementarily binding to at least any one gene selected from the group consisting of *ACPP, FLU3, KRT19, ERBB2, EGFR, KRAS, DSCC1, CK20, MUC2, SDC2, COTL1, ATAD2, ASB9, MMP1, CEACAM6, DSCC1, CKS2, CST1, IFITM1, MELK, LGALS3BP, CPT1A, IFNG, CD279, CD274, ERBB2, EGFR, FOLR1, EPCAM,* and a combination thereof.

233. The diagnostic kit according to claim 230, wherein the positively charged material is a positively charged nanowire.

234. The diagnostic kit according to claim 233, wherein the nanowire comprises a conductive polymer.

235. The diagnostic kit according to claim 234, wherein the nanowire further comprises biotin.

236. The diagnostic kit according to claim 233, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

237. The diagnostic kit according to claim 230, wherein the marker is a nanoparticle comprising a conductive polymer; hyaluronic acid; avidin or streptavidin; and a HRP (horse-radish peroxidase) or a fluorescent protein.

238. A device for diagnosing gastric cancer by detecting a gene derived from gastric cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in gastric cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from gastric cancer in the sample depending on whether the marker is detected.

239. The device for diagnosing gastric cancer according to claim 238, wherein the gene specifically expressed in gastric cancer further comprises a probe to which biotin is bound complementarily binding to at least any one gene selected from the group consisting *of CGB, PARP1, FOXO3A, MED30, CCNE1, MYC, TFF1, FABP1, LAMP5, MATN3, CLIP4, NOX4, ADRA2C, CSK, FZD9, GALR1, GRM6, INSR, LPHN1, LYN, MRGPRX3, ADCY3, HDAC2, CFL1, NRP2, ANXA10, TFF2, CDCA5, NUSAP1,* and a combination thereof.

240. The device for diagnosing gastric cancer according to claim 239, wherein the device further comprises a probe to which biotin is bound complementarily binding to at least any one gene selected from the group consisting of *ACPP, FLU3, KRT19, ERBB2, EGFR, KRAS, DSCC1, CK20, MUC2, SDC2, COTL1, ATAD2, ASB9, MMP1, CEACAM6, DSCC1, CKS2, CST1, IFITM1, MELK, LGALS3BP, CPT1A, IFNG, CD279, CD274, ERBB2, EGFR, FOLR1, EPCAM,* and a combination thereof.

241. A method for diagnosing pancreatic cancer by detecting a gene derived from pancreatic cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from pancreatic cancer cells, and
wherein the probe having a sequence complementary to cfDNA complementarily binds to a gene known as a pancreatic cancer biomarker.

242. The method for diagnosing pancreatic cancer according to claim 241, **characterized in that** the cfDNA derived from pancreatic cancer cells
i) has a lower Tm value than that of cfDNA having a double helix structure derived from normal cells, or
ii) is denatured in a condition in which the cfDNA having a double helix structure derived from normal cells is not denatured.

243. The method for diagnosing pancreatic cancer according to claim 241, wherein the cfDNA derived from pancreatic cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

244. The method for diagnosing pancreatic cancer according to claim 241, wherein the cfDNA derived from pancreatic cancer cells is circulating tumor DNA derived from pancreatic cancer.

245. The method for diagnosing pancreatic cancer according to claim 244, wherein the probe having a sequence complementary to cfDNA complementarily binds to at last any one gene selected from the group consisting of *SMAD4, APC, GNAS,* and a combination thereof.

246. The method for diagnosing pancreatic cancer according to claim 241, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

247. The method for diagnosing pancreatic cancer according to claim 241, wherein the sample is any one selected from the group consisting of urine, cerebrospinal fluid, plasma, blood, pleural fluid, ascites, saliva, sputum, and body fluid.

248. The method for diagnosing pancreatic cancer according to claim 241, wherein the positively charged material is a positively charged nanowire.

249. The method for diagnosing pancreatic cancer according to claim 248, wherein the nanowire comprises a conductive polymer.

250. The method for diagnosing pancreatic cancer according to claim 249, wherein the nanowire further comprises biotin.

251. The method for diagnosing pancreatic cancer according to claim 249, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

252. The method for diagnosing pancreatic cancer according to claim 241, wherein the probe is composed of a 15-mer to 30-mer nucleotide.

253. The method for diagnosing pancreatic cancer according to claim 241, wherein at least one biotin is bound to the probe.

254. The method for diagnosing pancreatic cancer according to claim 241, wherein the marker is any one selected from the group consisting of a quantum dot, a HRP (horse-radish peroxidase), a fluorescent protein, a phosphor, an alkaline phosphatase, and a luciferase.

255. The method for diagnosing pancreatic cancer according to claim 254, wherein an avidin-based protein is bound to the marker.

256. The method for diagnosing pancreatic cancer according to claim 254, wherein the avidin-based protein is any one selected from the group consisting of avidin, streptavidin, and a combination thereof.

257. The method for diagnosing pancreatic cancer according to claim 241, wherein the marker is a nanoparticle comprising a conductive polymer; hyaluronic acid; avidin or streptavidin; and a HRP (horse-radish peroxidase) or a fluorescent protein.

258. The method for diagnosing pancreatic cancer according to claim 241, wherein the cfDNA further comprises one derived from at least any one gene selected from the group consisting of *KRAS, MUC1, MSLN, PROX1, CEACAM5, MUC16,* and a combination thereof.

259. The method for diagnosing pancreatic cancer according to claim 241, wherein the marker is detected in the step e) by a color change, a UV absorbance change, a fluorescence change, or an electrochemical change.

260. A diagnostic kit for diagnosing pancreatic cancer, comprising
a probe to which biotin is bound complementarily binding to a gene specifically expressed in pancreatic cancer;
a positively charged material;
a marker to which an avidin-based protein is bound; and
a manual,
wherein the manual describes that the kit is configured to be capable of diagnosing pancreatic cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

261. The diagnostic kit according to claim 260, wherein the gene specifically expressed in pancreatic cancer is any one or more gene selected from the group consisting of *SMAD4, APC, GNAS,* and a combination thereof.

262. The diagnostic kit according to claim 260, wherein the kit further comprises a probe to which biotin is bound complementarily binding to at least any one gene selected from the group consisting of *KRAS, MUC1, MSLN, PROX1, CEACAM5, MUC16,* and a combination thereof.

263. The diagnostic kit according to claim 260, wherein the positively charged material is a positively charged nanowire.

264. The diagnostic kit according to claim 263, wherein the nanowire comprises a conductive polymer.

265. The diagnostic kit according to claim 264, wherein the nanowire further comprises biotin.

266. The diagnostic kit according to claim 263, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

267. The diagnostic kit according to claim 260, wherein the marker is a nanoparticle comprising a conductive polymer; hyaluronic acid; avidin or streptavidin; and a HRP (horse-radish peroxidase) or a fluorescent protein.

268. A device for diagnosing pancreatic cancer by detecting a gene derived from pancreatic cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in pancreatic cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from pancreatic cancer in the sample depending on whether the marker is detected.

269. The device for diagnosing pancreatic cancer according to claim 268, wherein the gene specifically expressed in pancreatic cancer further comprises a probe to which biotin is bound complementarily binding to at least any one gene selected from the group consisting of *SMAD4, APC, GNAS,* and a combination thereof.

270. The device for diagnosing pancreatic cancer according to claim 269, wherein the device further comprises a probe to which biotin is bound complementarily binding to at least any one gene selected from the group consisting of *KRAS, MUC1, MSLN, CEACAM1, CEACAM5, MUC16,* and a combination thereof.

271. A method for early diagnosing cancer or predicting the prognosis of cancer by detecting a gene derived from cancer cells from a sample without amplification, wherein the method comprises
a) a step of mixing a biological sample isolated from an individual comprising cell-free DNA (hereinafter cfDNA) and a positively charged material;
b) a step of isolating the positively charged material to which cfDNA is bound;
c) a step of sequentially or simultaneously mixing a probe having a sequence complementary to the cfDNA and a marker in the mixture;
d) a step of removing the probe that does not bind to cfDNA and the marker; and
e) a step of detecting the marker,
wherein the cfDNA is derived from cancer cells, and
wherein the probe having a sequence complementary to cfDNA complementarily binds to a gene known as a cancer biomarker.

272. The method for early diagnosing cancer or predicting the prognosis of cancer according to claim 271, **characterized in that** the cfDNA derived from cancer cells
i) has a lower Tm value than that of cfDNA having a double helix structure derived from normal cells, or
ii) is denatured in a condition in which the cfDNA having a double helix structure derived from normal cells is not denatured.

273. The method for early diagnosing cancer or predicting the prognosis of cancer according to claim 271, wherein the cfDNA derived from cancer cells is capable of binding with a 15-mer to 30-mer probe that can complementarily bind to cfDNA in any one of the following conditions:
i) a condition of allowing to stand for 1 minute to 120 minutes at ambient temperature;
ii) a condition of heating at 90 °C to 95 °C for 1 second to 3 minutes;
iii) a condition of heating at 75 °C to 90 °C for 1 second to 5 minutes;
iv) a condition of heating at 60 °C to 75 °C for 30 seconds to 30 minutes;
v) a condition of heating at 25 °C to 40 °C for 10 minutes to 120 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

274. The method for early diagnosing cancer or predicting the prognosis of cancer according to claim 271, wherein the cfDNA derived from cancer cells is circulating tumor DNA derived from cancer.

275. The method for early diagnosing cancer or predicting the prognosis of cancer according to claim 274, wherein the probe having a sequence complementary to cfDNA complementarily binds to at least any one gene selected from the group consisting of *CPT1A, IFNG, IFNGR1, CD279, CD274,* and a combination thereof.

276. The method for early diagnosing cancer or predicting the prognosis of cancer according to claim 271, **characterized in that** the method further comprises a step of denaturing the sample or cfDNA bound to the positively charged material prior to step c) above in any one of the following conditions:
i) a condition of allowing to stand for 1 minute to 10 minutes at ambient temperature;
ii) a condition of heating at 90 °C to 95 °C for 1 second to 1 minute;
iii) a condition of heating at 75 °C to 90 °C for 10 seconds to 3 minutes;
iv) a condition of heating at 60 °C to 75 °C for 1 minute to 30 minutes;
v) a condition of heating at 25 °C to 40 °C for 5 minutes to 60 minutes;
vi) a condition of treating with protease for 1 minute to 10 minutes;
vii) a condition of treating with DNase for 1 minute to 10 minutes; and
viii) a condition of treating with a chemical substance (for example, sodium hydroxide, DMSO, a surfactant, and the like).

277. The method for early diagnosing cancer or predicting the prognosis of cancer according to claim 271, wherein the sample is any one selected from the group consisting of urine, cerebrospinal fluid, plasma, blood, pleural fluid, ascites, saliva, sputum, and body fluid.

278. The method for early diagnosing cancer or predicting the prognosis of cancer according to claim 271, wherein the positively charged material is a positively charged nanowire.

279. The method for early diagnosing cancer or predicting the prognosis of cancer according to claim 278, wherein the nanowire comprises a conductive polymer.

280. The method for early diagnosing cancer or predicting the prognosis of cancer according to claim 279, wherein the nanowire further comprises biotin.

281. The method for early diagnosing cancer or predicting the prognosis of cancer according to claim 279, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

282. The method for early diagnosing cancer or predicting the prognosis of cancer according to claim 271, wherein the probe is composed of a 15-mer to 30-mer nucleotide.

283. The method for early diagnosing cancer or predicting the prognosis of cancer according to claim 271, wherein at least one biotin is bound to the probe.

284. The method for early diagnosing cancer or predicting the prognosis of cancer according to claim 271, wherein the marker is any one selected from the group consisting of a quantum dot, a HRP (horse-radish peroxidase), a fluorescent protein, a phosphor, an alkaline phosphatase, and a luciferase.

285. The method for early diagnosing cancer or predicting the prognosis of cancer according to claim 284, wherein an avidin-based protein is bound to the marker.

286. The method for early diagnosing cancer or predicting the prognosis of cancer according to claim 284, wherein the avidin-based protein is any one selected from the group consisting of avidin, streptavidin, and a combination thereof.

287. The method for early diagnosing cancer or predicting the prognosis of cancer according to claim 271, wherein the marker is a nanoparticle comprising a conductive polymer; hyaluronic acid; avidin or streptavidin; and a HRP (horse-radish peroxidase) or a fluorescent protein.

288. The method for early diagnosing cancer or predicting the prognosis of cancer according to claim 271, wherein the cfDNA further comprises one derived from at least any one gene selected from the group consisting of *NSE, SCC, CEA, cyfra21-1, TPA, NMP22, OGT, Thyroglobulin(TG), Calcitonin(CALCA), BRAF V600E, TERT C228T*/*C250T, AFP, β-HCG(CGB). CA19-9, PSA,PSMA, PAP, PCA3, TMPRSS2-ERG, CA125, HIF-1a, VEGF, CA15-3, HER2, SCC(SART3), TOP2A, MCM2, p16INK4a(CDKN2A), Ki-67(MKI167), HE4(WEDC2),* and a combination thereof.

289. The method for early diagnosing cancer or predicting the prognosis of cancer according to claim 271, wherein the marker is detected in the step e) by a color change, a UV absorbance change, a fluorescence change, or an electrochemical change.

290. A diagnostic kit for early diagnosing cancer or predicting the prognosis of cancer, comprising
a probe to which biotin is bound complementarily binding to a gene specifically expressed in cancer;
a positively charged material;
a marker to which an avidin-based protein is bound; and
a manual,
wherein the manual describes that the kit is configured to be capable of diagnosing cancer without gene amplification by the following protocol:
a) isolate cfDNA from a biological sample isolated from an individual using a positively charged material contained in the kit; b) mix sequentially or simultaneously a probe to which biotin is bound contained in the kit and a marker contained in the kit in the isolated cfDNA; c) remove the probe that does not bind to cfDNA and the marker; and d) detect the signal of the marker.

291. The diagnostic kit for early diagnosing cancer or predicting the prognosis of cancer according to claim 290, wherein the gene specifically expressed in cancer is any one or more gene selected from the group consisting of *CPT1A, IFNG, IFNGR1, CD279, CD274,* and a combination thereof.

292. The diagnostic kit for early diagnosing cancer or predicting the prognosis of cancer according to claim 290, wherein the kit further comprises a probe to which biotin is bound complementarily binding to at least any one gene selected from the group consisting of *NSE, SCC, CEA, cyfra21-1, TPA, NMP22, OGT, Thyroglobulin(TG), Calcitonin(CALCA), BRAF V600E, TERT C228T*/*C250T, AFP, β-HCG(CGB). CA19-9, PSA,PSMA, PAP, PCA3, TMPRSS2-ERG, CA125, HIF-1a, VEGF, CA15-3, HER2, SCC(SART3), TOP2A, MCM2, p16INK4a(CDKN2A), Ki-67(MKI167), HE4(WEDC2),* and a combination thereof.

293. The diagnostic kit for early diagnosing cancer or predicting the prognosis of cancer according to claim 290, wherein the positively charged material is a positively charged nanowire.

294. The diagnostic kit for early diagnosing cancer or predicting the prognosis of cancer according to claim 293, wherein the nanowire comprises a conductive polymer.

295. The diagnostic kit for early diagnosing cancer or predicting the prognosis of cancer according to claim 294, wherein the nanowire further comprises biotin.

296. The diagnostic kit for early diagnosing cancer or predicting the prognosis of cancer according to claim 293, wherein the conductive polymer is any one selected from the group consisting of poly(acetylene), poly(pyrrole), poly(thiophene), poly(para-phenylene), poly(3,4-ethylenedioxythiophene), poly(phenylene sulfide), poly(para-phenylene vinylene), and polyaniline.

297. The diagnostic kit for early diagnosing cancer or predicting the prognosis of cancer according to claim 290, wherein the marker is a nanoparticle comprising a conductive polymer; hyaluronic acid; avidin or streptavidin; and a HRP (horse-radish peroxidase) or a fluorescent protein.

298. A device for early diagnosing cancer or predicting the prognosis of cancer by detecting a gene derived from cancer cells from a sample without amplification, wherein the device comprises
a) a mixing part for mixing a biological sample isolated from an individual comprising cfDNA and a positively charged material;
b) an obtaining part for removing the sample except for the positively charged material to which cfDNA is bound;
c) a reaction part for adding sequentially or simultaneously a probe to which biotin is bound capable of complementarily binding to the gene specifically expressed in cancer; and a nanoparticle comprising streptavidin and a marker to the positively charged material to which cfDNA is bound;
d) a detection part for detecting the marker; and
e) an information processing part for determining whether there is cfDNA having a sequence complementary to the probe and derived from cancer in the sample depending on whether the marker is detected.

299. The device for early diagnosing cancer or predicting the prognosis of cancer according to claim 298, wherein the gene specifically expressed in cancer further comprises a probe to which biotin is bound complementarily binding to at least any one gene selected from the group consisting of *CPT1A, IFNG, IFNGR1, CD279, CD274,* and a combination thereof.

300. The device for early diagnosing cancer or predicting the prognosis of cancer according to claim 299, wherein the device further comprises a probe to which biotin is bound complementarily binding to at least any one gene selected from the group consisting *of NSE, SCC, CEA, cyfra21-1, TPA, NMP22, OGT, Thyroglobulin(TG), Calcitonin(CALCA), BRAF V600E, TERT C228T*/*C250T, AFP, β-HCG(CGB). CA19-9, PSA,PSMA, PAP, PCA3, TMPRSS2-ERG, CA125, HIF-1a, VEGF, CA15-3, HER2, SCC(SART3), TOP2A, MCM2, p16INK4a(CDKN2A), Ki-67(MKI167), HE4(WEDC2),* and a combination thereof.


<110>     Genopsy Co., Ltd.

<120>     METHOD FOR DIAGNOSING CANCER USING CFDNA

<130>     SPO20-008

<160>     166

<170>     KoPatentIn 3.0

<210>     1
<211>     21
<212>     DNA
<213>     Artificial Sequence

<220>
<223>     nucleotide seqeunce for biotinylated PD-L1 probe 1(Exon5)


<400>     1
tgggagccat cttattatgc c                                                  21


<210>     2
<211>     20
<212>     DNA
<213>     Artificial Sequence

<220>
<223>     nucleotide seqeunce for biotinylated PD-L1 probe 2(Exon5)


<400>     2
cggaagatga atgtcagtgc                                                    20


<210>     3
<211>     21
<212>     DNA
<213>     Artificial Sequence

<220>
<223>     nucleotide seqeunce for biotinylated PD-L1 probe 3(Exon2)


<400>     3
cctactggca tttgctgaac g                                                  21


<210>     4
<211>     21
<212>     DNA
<213>     Artificial Sequence

<220>
<223>     nucleotide seqeunce for biotinylated PD-L1 probe 4(Exon3)


<400>     4
accatagctg atcatgcagc g                                                  21


<210>     5

<211>    21
<212>    DNA
<213>    Artificial Sequence

<220>
<223>    nucleotide seqeunce for biotinylated EpCAM probe 1(Exon1)


<400>    5
acagagcgct agtccttcgg c                                                21


<210>    6
<211>    21
<212>    DNA
<213>    Artificial Sequence

<220>
<223>    nucleotide seqeunce for biotinylated EpCAM probe 2(Exon4)


<400>    6
aaccttatga tagtaaaagt t                                                21


<210>    7
<211>    21
<212>    DNA
<213>    Artificial Sequence

<220>
<223>    nucleotide seqeunce for biotinylated EpCAM probe 3(Exon7)


<400>    7
ggtctaaaag ctggtgttat t                                                21


<210>    8
<211>    21
<212>    DNA
<213>    Artificial Sequence

<220>
<223>    nucleotide seqeunce for biotinylated EpCAM probe 4(Exon9)


<400>    8
gaaactggct ttaccaatct t                                                21


<210>    9
<211>    21
<212>    DNA
<213>    Artificial Sequence

<220>
<223>    nucleotide seqeunce for biotinylated FOLR1 probe 1(Exon4)


<400>    9
tgttctacca acaccagcca g                                                21

<210>    10
<211>    21
<212>    DNA
<213>    Artificial Sequence

<220>
<223>    nucleotide seqeunce for biotinylated FOLR1 probe 2(Exon2)


<400>    10
actgaaccca aaggatcacc t                                                21


<210>    11
<211>    21
<212>    DNA
<213>    Artificial Sequence

<220>
<223>    nucleotide seqeunce for biotinylated FOLR1 probe 3(Exon1)


<400>    11
cctaggccac taaaccacag c                                                21


<210>    12
<211>    21
<212>    DNA
<213>    Artificial Sequence

<220>
<223>    nucleotide seqeunce for biotinylated FOLR1 probe 4(Exon5)


<400>    12
ggctgggccc tgggcagcct g                                                21


<210>    13
<211>    21
<212>    DNA
<213>    Artificial Sequence

<220>
<223>    nucleotide seqeunce for biotinylated EGFR probe 1(Exon1)


<400>    13
aacgccacaa ccaccgcgca c                                                21


<210>    14
<211>    21
<212>    DNA
<213>    Artificial Sequence

<220>
<223>    nucleotide seqeunce for biotinylated EGFR probe 2(Exon21)


<400>    14

ggaccgtcgc ttggtgcacc g                                                21

<210>    15
<211>    21
<212>    DNA
<213>    Artificial Sequence

<220>
<223>    nucleotide seqeunce for biotinylated EGFR probe 3(Exon19)

<400>    15
tctggaccca gaaggtgaga a                                                21

<210>    16
<211>    21
<212>    DNA
<213>    Artificial Sequence

<220>
<223>    nucleotide seqeunce for biotinylated EGFR probe 4(Exon20)

<400>    16
tgctgggcat ctgcctcacc t                                                21

<210>    17
<211>    21
<212>    DNA
<213>    Artificial Sequence

<220>
<223>    nucleotide seqeunce for biotinylated ERBB2 probe 1(Exon1)

<400>    17
tcgcagcacc ccgcgccccg c                                                21

<210>    18
<211>    21
<212>    DNA
<213>    Artificial Sequence

<220>
<223>    nucleotide seqeunce for biotinylated ERBB2 probe 2(Exon5)

<400>    18
cctgttctcc gatgtgtaag g                                                21

<210>    19
<211>    21
<212>    DNA
<213>    Artificial Sequence

<220>
<223>    nucleotide seqeunce for biotinylated ERBB2 probe 3(Exon10)

<400>    19
ccgctccagc cagagcagct c                                                21


<210>    20
<211>    21
<212>    DNA
<213>    Artificial Sequence

<220>
<223>    nucleotide seqeunce for biotinylated ERBB2 probe 4(Exon17)


<400>    20
aagatccgga agtacacgat g                                                21


<210>    21
<211>    18
<212>    DNA
<213>    Artificial Sequence

<220>
<223>    nucleotide seqeunce for Biotinylated OGT (O-GlcNAc transferase)
         probe 1


<400>    21
atggcgtctt ccgtgggc                                                    18


<210>    22
<211>    18
<212>    DNA
<213>    Artificial Sequence

<220>
<223>    nucleotide seqeunce for Biotinylated OGT (O-GlcNAc transferase)
         probe 2


<400>    22
tgccctgtgg ccatggac                                                    18


<210>    23
<211>    21
<212>    DNA
<213>    Artificial Sequence

<220>
<223>    nucleotide seqeunce for biotinylated CEA (Prostatic Acid
         Phosphatase; ACPP gene) probe 1


<400>    23
aatctgaacc tctcctgcca c                                                21


<210>    24
<211>    21
<212>    DNA

<213>    Artificial Sequence

<220>
<223>    nucleotide seqeunce for biotinylated CEA (Prostatic Acid
         Phosphatase; ACPP gene) probe 2


<400>    24
acccttcatc accagcaaca a                                                21


<210>    25
<211>    21
<212>    DNA
<213>    Artificial Sequence

<220>
<223>    nucleotide seqeunce for biotinylated CEA (Prostatic Acid
         Phosphatase; ACPP gene) probe 3


<400>    25
tattcttggc tgattgatgg g                                                21


<210>    26
<211>    21
<212>    DNA
<213>    Artificial Sequence

<220>
<223>    nucleotide seqeunce for biotinylated CEA (Prostatic Acid
         Phosphatase; ACPP gene) probe 4


<400>    26
cagcaacaac tccaaacccg t                                                21


<210>    27
<211>    21
<212>    DNA
<213>    Artificial Sequence

<220>
<223>    nucleotide seqeunce for biotinylated PSA (KLK3) probe 1


<400>    27
cagtctgcgg cggtgttctg g                                                21


<210>    28
<211>    21
<212>    DNA
<213>    Artificial Sequence

<220>
<223>    nucleotide seqeunce for biotinylated PSA (KLK3) probe 2


<400>    28
gcaagttcac cctcagaagg t                                                21

<210>    29
<211>    21
<212>    DNA
<213>    Artificial Sequence

<220>
<223>    nucleotide seqeunce for biotinylated PSA (KLK3) probe 3


<400>    29
gaggtccagg gttgctagga a                                                21


<210>    30
<211>    18
<212>    DNA
<213>    Artificial Sequence

<220>
<223>    nucleotide seqeunce for biotinylated CA19-9 probe 1


<400>    30
gatcatcacg gcacggtc                                                    18


<210>    31
<211>    21
<212>    DNA
<213>    Artificial Sequence

<220>
<223>    nucleotide seqeunce for biotinylated CA19-9 probe 2


<400>    31
gtgcagagag atcatcacgg c                                                21


<210>    32
<211>    21
<212>    DNA
<213>    Artificial Sequence

<220>
<223>    nucleotide seqeunce for biotinylated CA19-9 probe 3


<400>    32
tctctcttac ctgggacctc a                                                21


<210>    33
<211>    21
<212>    DNA
<213>    Artificial Sequence

<220>
<223>    nucleotide seqeunce for biotinylated C125 (MUC16 gene) probe 1

<400>    33
ctggaactga ttctatcaac c                                                21


<210>    34
<211>    21
<212>    DNA
<213>    Artificial Sequence

<220>
<223>    nucleotide seqeunce for biotinylated C125 (MUC16 gene) probe 2


<400>    34
tgtggcagaa accagctatc c                                                21


<210>    35
<211>    21
<212>    DNA
<213>    Artificial Sequence

<220>
<223>    nucleotide seqeunce for biotinylated C125 (MUC16 gene) probe 3


<400>    35
gaggtcctga ggatgtgtca t                                                21


<210>    36
<211>    21
<212>    DNA
<213>    Artificial Sequence

<220>
<223>    nucleotide seqeunce for biotinylated AFP probe 1


<400>    36
tgagtcagaa gtttaccaaa g                                                21


<210>    37
<211>    21
<212>    DNA
<213>    Artificial Sequence

<220>
<223>    nucleotide seqeunce for biotinylated AFP probe 2


<400>    37
tgcaaactga ccacgctgga a                                                21


<210>    38
<211>    21
<212>    DNA
<213>    Artificial Sequence

<220>
<223>    nucleotide seqeunce for biotinylated AFP probe 3

<400>    38
cttgaatgcc aagataaagg a                                                21

<210>    39
<211>    21
<212>    DNA
<213>    Artificial Sequence

<220>
<223>    nucleotide seqeunce for biotinylated PSMA (FOLH1) probe 1

<400>    39
catagtgctc ccttttgatt g                                                21

<210>    40
<211>    21
<212>    DNA
<213>    Artificial Sequence

<220>
<223>    nucleotide seqeunce for biotinylated PSMA (FOLH1) probe 2

<400>    40
agtggaaaga atttggcctg g                                                21

<210>    41
<211>    21
<212>    DNA
<213>    Artificial Sequence

<220>
<223>    nucleotide seqeunce for biotinylated PSMA (FOLH1) probe 3

<400>    41
acctagaaga acaattttgt t                                                21

<210>    42
<211>    21
<212>    DNA
<213>    Artificial Sequence

<220>
<223>    nucleotide seqeunce for biotinylated PAP (Prostatic Acid
         Phosphatase; ACPP gene) probe 1

<400>    42
gcagcattat gaacttggag a                                                21

<210>    43
<211>    21
<212>    DNA
<213>    Artificial Sequence

<220>
<223>    nucleotide seqeunce for biotinylated PAP (Prostatic Acid
         Phosphatase; ACPP gene) probe 2


<400>    43
tcggaatgag acgcagcacg a                                                21


<210>    44
<211>    21
<212>    DNA
<213>    Artificial Sequence

<220>
<223>    nucleotide seqeunce for biotinylated PCA3 (Prostate cancer
         antigen 3; PCA3 gene) probe 1


<400>    44
cttagtgttt caatgaacac c                                                21


<210>    45
<211>    21
<212>    DNA
<213>    Artificial Sequence

<220>
<223>    nucleotide seqeunce for biotinylated PCA3 (Prostate cancer
         antigen 3; PCA3 gene) probe 2


<400>    45
gcttagcctt gtactgaggc t                                                21


<210>    46
<211>    21
<212>    DNA
<213>    Artificial Sequence

<220>
<223>    nucleotide seqeunce for biotinylated NSE (Neuron-Specific
         Enolase; ENO2 gene) probe 1


<400>    46
ccatagagaa gatctgggcc c                                                21


<210>    47
<211>    21
<212>    DNA
<213>    Artificial Sequence

<220>
<223>    nucleotide seqeunce for biotinylated NSE (Neuron-Specific
         Enolase; ENO2 gene) probe 2


<400>    47

gatcctccca gtgggagctg a                                                21

<210>    48
<211>    21
<212>    DNA
<213>    Artificial Sequence

<220>
<223>    nucleotide seqeunce for biotinylated NSE (Neuron-Specific
         Enolase; ENO2 gene) probe 3

<400>    48
ggtcatggtg agtcatcgct c                                                21

<210>    49
<211>    21
<212>    DNA
<213>    Artificial Sequence

<220>
<223>    nucleotide seqeunce for biotinylated SCC (Squamous Cell Carcinoma
         Antigen; SART3 gene) probe 1

<400>    49
gctggagtgg ctgcatgacg a                                                21

<210>    50
<211>    21
<212>    DNA
<213>    Artificial Sequence

<220>
<223>    nucleotide seqeunce for biotinylated SCC (Squamous Cell Carcinoma
         Antigen; SART3 gene) probe 2

<400>    50
gcgccctggt cgagaactgc c                                                21

<210>    51
<211>    21
<212>    DNA
<213>    Artificial Sequence

<220>
<223>    nucleotide seqeunce for biotinylated SCC (Squamous Cell Carcinoma
         Antigen; SART3 gene) probe 3

<400>    51
gtggctagag tattacaacc t                                                21

<210>    52
<211>    21
<212>    DNA
<213>    Artificial Sequence

<220>
<223>    nucleotide seqeunce for biotinylated SCC (Squamous Cell Carcinoma
         Antigen; SART3 gene) probe 4

<400>    52
tcgagaacag catccctgca g                                                21

<210>    53
<211>    20
<212>    DNA
<213>    Artificial Sequence

<220>
<223>    nucleotide seqeunce for biotinylated Cyfra21-1 (cytokeratin
         19-fragments; KRT19 gene) probe 1

<400>    53
ctggcctcct acctggacaa                                                  20

<210>    54
<211>    21
<212>    DNA
<213>    Artificial Sequence

<220>
<223>    nucleotide seqeunce for biotinylated Cyfra21-1 (cytokeratin
         19-fragments; KRT19 gene) probe 2

<400>    54
gacctggaga tgcagatcga a                                                21

<210>    55
<211>    21
<212>    DNA
<213>    Artificial Sequence

<220>
<223>    nucleotide seqeunce for biotinylated Cyfra21-1 (cytokeratin
         19-fragments; KRT19 gene) probe 3

<400>    55
gtcgctggcc acacggagca g                                                21

<210>    56
<211>    21
<212>    DNA
<213>    Artificial Sequence

<220>
<223>    nucleotide seqeunce for biotinylated TPA (Tissue plasminogen
         activator; PLAT gene) probe 1

<400>    56

gctgtgaagc aatcatggat g                                                21

<210>    57
<211>    21
<212>    DNA
<213>    Artificial Sequence

<220>
<223>    nucleotide seqeunce for biotinylated TPA (Tissue plasminogen
         activator; PLAT gene) probe 2

<400>    57
gcacctgcca gcaggccctg t                                                21

<210>    58
<211>    21
<212>    DNA
<213>    Artificial Sequence

<220>
<223>    nucleotide seqeunce for biotinylated TPA (Tissue plasminogen
         activator; PLAT gene) probe 3

<400>    58
actggacgga gtgtgagctc t                                                21

<210>    59
<211>    21
<212>    DNA
<213>    Artificial Sequence

<220>
<223>    nucleotide seqeunce for biotinylated Thyroglobulin (TG gene)
         probe 1

<400>    59
caacaccaca gacatgatga t                                                21

<210>    60
<211>    21
<212>    DNA
<213>    Artificial Sequence

<220>
<223>    nucleotide seqeunce for biotinylated Thyroglobulin (TG gene)
         probe 2

<400>    60
tgcggcctgg ctcgagcagc a                                                21

<210>    61
<211>    21
<212>    DNA
<213>    Artificial Sequence

<220>
<223>    nucleotide seqeunce for biotinylated Thyroglobulin (TG gene)
         probe 3

<400>    61
aatcaatcac tatccagcca g                                                21

<210>    62
<211>    21
<212>    DNA
<213>    Artificial Sequence

<220>
<223>    nucleotide seqeunce for biotinylated Thyroglobulin (TG gene)
         probe 4

<400>    62
actctctgga gcactccacg g                                                21

<210>    63
<211>    21
<212>    DNA
<213>    Artificial Sequence

<220>
<223>    nucleotide seqeunce for biotinylated Calcitonin (Calcitonin
         Gene-Related Peptide; CALCA gene) probe 1

<400>    63
gaggtgtcat gggcttccaa a                                                21

<210>    64
<211>    21
<212>    DNA
<213>    Artificial Sequence

<220>
<223>    nucleotide seqeunce for biotinylated Calcitonin (Calcitonin
         Gene-Related Peptide; CALCA gene) probe 2

<400>    64
gtaatctgag tacttgcatg c                                                21

<210>    65
<211>    22
<212>    DNA
<213>    Artificial Sequence

<220>
<223>    nucleotide seqeunce for biotinylated FGFR3 probe 1

<400>    65
agtggcggtg gtggtgaggg ag                                               22

<210>    66
<211>    20
<212>    DNA
<213>    Artificial Sequence

<220>
<223>    nucleotide seqeunce for biotinylated FGFR3 probe 2


<400>    66
cccacagctt ctgcccccga                                                    20


<210>    67
<211>    17
<212>    DNA
<213>    Artificial Sequence

<220>
<223>    nucleotide seqeunce for biotinylated FGFR3 probe 3


<400>    67
gggcatccat gggagcc                                                       17


<210>    68
<211>    16
<212>    DNA
<213>    Artificial Sequence

<220>
<223>    nucleotide seqeunce for biotinylated FGFR3 probe 4


<400>    68
cagcgtgggc cgaggt                                                        16


<210>    69
<211>    20
<212>    DNA
<213>    Artificial Sequence

<220>
<223>    nucleotide seqeunce for biotinylated FGFR3 probe 5


<400>    69
ccctgagatg ctgggagcag                                                    20


<210>    70
<211>    19
<212>    DNA
<213>    Artificial Sequence

<220>
<223>    nucleotide seqeunce for biotinylated FGFR3 probe 6

<400>    70
gtgtgggaag gcggtgttg                                                    19


<210>    71
<211>    19
<212>    DNA
<213>    Artificial Sequence

<220>
<223>    nucleotide seqeunce for biotinylated TP53 probe 1


<400>    71
gccctgactt tcaactctg                                                    19


<210>    72
<211>    18
<212>    DNA
<213>    Artificial Sequence

<220>
<223>    nucleotide seqeunce for biotinylated TP53 probe 2


<400>    72
acgacagggc tggttgcc                                                     18


<210>    73
<211>    19
<212>    DNA
<213>    Artificial Sequence

<220>
<223>    nucleotide seqeunce for biotinylated TP53 probe 3


<400>    73
tccccaggcc tctgattcc                                                    19


<210>    74
<211>    18
<212>    DNA
<213>    Artificial Sequence

<220>
<223>    nucleotide seqeunce for biotinylated TP53 probe 4


<400>    74
cctccaggtg agcagtag                                                     18


<210>    75
<211>    19
<212>    DNA
<213>    Artificial Sequence

<220>
<223>    nucleotide seqeunce for biotinylated TP53 probe 5

<400>    75
cttgggcctg tgttatctc                                                    19


<210>    76
<211>    18
<212>    DNA
<213>    Artificial Sequence

<220>
<223>    nucleotide seqeunce for biotinylated TP53 probe 6


<400>    76
cccacctctt accgattt                                                     18


<210>    77
<211>    20
<212>    DNA
<213>    Artificial Sequence

<220>
<223>    nucleotide seqeunce for biotinylated TP53 probe 7


<400>    77
acaagggtgg ttgggagtag                                                   20


<210>    78
<211>    19
<212>    DNA
<213>    Artificial Sequence

<220>
<223>    nucleotide seqeunce for biotinylated TP53 probe 8


<400>    78
ggacaagaag cggtggagg                                                    19


<210>    79
<211>    21
<212>    DNA
<213>    Artificial Sequence

<220>
<223>    nucleotide seqeunce for biotinylated NMP22 (nuclear matrix
         protein-22; NUMA1 gene) probe 1


<400>    79
atggcactga agagggacag c                                                 21


<210>    80
<211>    21
<212>    DNA
<213>    Artificial Sequence

<220>
<223>    nucleotide seqeunce for biotinylated NMP22 (nuclear matrix
         protein-22; NUMA1 gene) probe 2

<400>    80
gccaaggaag agctggagca g                                                21

<210>    81
<211>    21
<212>    DNA
<213>    Artificial Sequence

<220>
<223>    nucleotide seqeunce for biotinylated NMP22 (nuclear matrix
         protein-22; NUMA1 gene) probe 3

<400>    81
gagcagctag aggtatttca g                                                21

<210>    82
<211>    21
<212>    DNA
<213>    Artificial Sequence

<220>
<223>    nucleotide seqeunce for biotinylated NMP22 (nuclear matrix
         protein-22; NUMA1 gene) probe 4

<400>    82
aggtagaatc cctggagagt c                                                21

<210>    83
<211>    21
<212>    DNA
<213>    Artificial Sequence

<220>
<223>    nucleotide seqeunce for biotinylated CA27.29/CA15-3 (Carcinoma
         Antigen 15-3; MUC1 gene) probe 1

<400>    83
gcgcctgcct gaatctgttc t                                                21

<210>    84
<211>    21
<212>    DNA
<213>    Artificial Sequence

<220>
<223>    nucleotide seqeunce for biotinylated CA27.29/CA15-3 (Carcinoma
         Antigen 15-3; MUC1 gene) probe 2

<400>    84

ttctgggcct ctccaatatt a                                                21

<210>    85
<211>    21
<212>    DNA
<213>    Artificial Sequence

<220>
<223>    nucleotide seqeunce for biotinylated CA27.29/CA15-3 (Carcinoma
         Antigen 15-3; MUC1 gene) probe 3

<400>    85
ggatacctac catcctatga g                                                21

<210>    86
<211>    21
<212>    DNA
<213>    Artificial Sequence

<220>
<223>    nucleotide seqeunce for biotinylated biotinylated Beta-HCG (human
         chorionic gonadotropin; CGB gene) probe 1

<400>    86
tgctgagcat gggcgggaca t                                                21

<210>    87
<211>    21
<212>    DNA
<213>    Artificial Sequence

<220>
<223>    nucleotide seqeunce for biotinylated biotinylated Beta-HCG (human
         chorionic gonadotropin; CGB gene) probe 2

<400>    87
tggctctcag ctgtcaatgt g                                                21

<210>    88
<211>    21
<212>    DNA
<213>    Artificial Sequence

<220>
<223>    nucleotide seqeunce for biotinylated CPT1A probe 1

<400>    88
gagccatgaa gctcttagac a                                                21

<210>    89
<211>    21
<212>    DNA
<213>    Artificial Sequence

<220>
<223>    nucleotide seqeunce for biotinylated CPT1A probe 2


<400>    89
cttccagaca tcgctgcctc g                                                  21


<210>    90
<211>    21
<212>    DNA
<213>    Artificial Sequence

<220>
<223>    nucleotide seqeunce for biotinylated CPT1A probe 3


<400>    90
tggacaatac ctcggagcct c                                                  21


<210>    91
<211>    21
<212>    DNA
<213>    Artificial Sequence

<220>
<223>    nucleotide seqeunce for biotinylated CPT1A probe 4


<400>    91
catgacccgg ctcttccgag a                                                  21


<210>    92
<211>    21
<212>    DNA
<213>    Artificial Sequence

<220>
<223>    nucleotide seqeunce for biotinylated IFN-gamma probe 1


<400>    92
atcgtatatt gggagtacca g                                                  21


<210>    93
<211>    21
<212>    DNA
<213>    Artificial Sequence

<220>
<223>    nucleotide seqeunce for biotinylated IFN-gamma probe 2


<400>    93
taaatggaga cgagcaggaa g                                                  21


<210>    94
<211>    21
<212>    DNA

<213>    Artificial Sequence

<220>
<223>    nucleotide seqeunce for biotinylated IFN-gamma probe 3


<400>    94
tagtgcttag cctggtattc a                                                  21


<210>    95
<211>    21
<212>    DNA
<213>    Artificial Sequence

<220>
<223>    nucleotide seqeunce for biotinylated PD1 probe 1


<400>    95
gtaccgcatg agccccagca a                                                  21


<210>    96
<211>    21
<212>    DNA
<213>    Artificial Sequence

<220>
<223>    nucleotide seqeunce for biotinylated PD1 probe 2


<400>    96
gttccaaacc ctggtggttg g                                                  21


<210>    97
<211>    20
<212>    DNA
<213>    Artificial Sequence

<220>
<223>    nucleotide seqeunce for biotinylated PD1 probe 3


<400>    97
gagcagacgg agtatgccac                                                    20


<210>    98
<211>    21
<212>    DNA
<213>    Artificial Sequence

<220>
<223>    nucleotide seqeunce for biotinylated IFN-gamma receptor probe 1


<400>    98
atcgtatatt gggagtacca g                                                  21


<210>    99

<211>    21
<212>    DNA
<213>    Artificial Sequence

<220>
<223>    nucleotide seqeunce for biotinylated IFN-gamma receptor probe 2


<400>    99
taaatggaga cgagcaggaa g                                                21


<210>    100
<211>    21
<212>    DNA
<213>    Artificial Sequence

<220>
<223>    nucleotide seqeunce for biotinylated IFN-gamma receptor probe 3


<400>    100
tagtgcttag cctggtattc a                                                21


<210>    101
<211>    24
<212>    DNA
<213>    Artificial Sequence

<220>
<223>    HPV 16-CP


<400>    101
gaggaggagg atgaaataga tggt                                             24


<210>    102
<211>    21
<212>    DNA
<213>    Artificial Sequence

<220>
<223>    HPV 16-DP


<400>    102
ttggaagacc tgttaatggg c                                                21


<210>    103
<211>    22
<212>    DNA
<213>    Artificial Sequence

<220>
<223>    HPV 18-CP


<400>    103
cacattgtgg cacaatcttt ta                                               22

<210>    104
<211>    20
<212>    DNA
<213>    Artificial Sequence

<220>
<223>    HPV 18-DP


<400>    104
gccatatcgc tttcatctgt                                                    20


<210>    105
<211>    24
<212>    DNA
<213>    Artificial Sequence

<220>
<223>    EGFR 19-CP


<400>    105
ggaattaaga gaagcaacat ctcc                                               24


<210>    106
<211>    19
<212>    DNA
<213>    Artificial Sequence

<220>
<223>    EGFR 19-DP


<400>    106
aacctcaggc ccacctttt                                                     19


<210>    107
<211>    21
<212>    DNA
<213>    Artificial Sequence

<220>
<223>    EGFR 21-CP


<400>    107
ccaggaacgt actggtgaaa a                                                  21


<210>    108
<211>    22
<212>    DNA
<213>    Artificial Sequence

<220>
<223>    EGFR 21-DP


<400>    108

ggaagagaaa gaataccatg ca                                                22

<210>    109
<211>    24
<212>    DNA
<213>    Artificial Sequence

<220>
<223>    EGFR Exon 19-probe1 CP1

<400>    109
ggaattaaga gaagcaacat ctcc                                              24

<210>    110
<211>    18
<212>    DNA
<213>    Artificial Sequence

<220>
<223>    EGFR Exon 19-probe1 DP

<400>    110
aacctcaggc ccaccttt                                                     18

<210>    111
<211>    21
<212>    DNA
<213>    Artificial Sequence

<220>
<223>    EGFR Exon 19-probe2 CP2

<400>    111
aaaattcccg tcgctatcaa g                                                 21

<210>    112
<211>    19
<212>    DNA
<213>    Artificial Sequence

<220>
<223>    EGFR Exon 19-probe2 DP

<400>    112
aacctcaggc ccacctttt                                                    19

<210>    113
<211>    24
<212>    DNA
<213>    Artificial Sequence

<220>
<223>    EGFR Exon 19-probe3 CP3

<400>    113
ggactctgga tcccagaagg tgag                                              24


<210>    114
<211>    19
<212>    DNA
<213>    Artificial Sequence

<220>
<223>    EGFR Exon 19-probe3 DP


<400>    114
   aacctcaggc ccacctttt                                                      19


<210>    115
<211>    24
<212>    DNA
<213>    Artificial Sequence

<220>
<223>    EGFR Exon 20-probe1 CP1


<400>    115
ccatgagtac gtattttgaa actc                                              24


<210>    116
<211>    24
<212>    DNA
<213>    Artificial Sequence

<220>
<223>    EGFR Exon 20-probe1 DP


<400>    116
gcaagagttt gccatgggga tatg                                              24


<210>    117
<211>    27
<212>    DNA
<213>    Artificial Sequence

<220>
<223>    EGFR Exon 20-probe2 CP2


<400>    117
ccaccgtgca gctcatcacg cagctca                                           27


<210>    118
<211>    24
<212>    DNA
<213>    Artificial Sequence

<220>

<223>    EGFR Exon 20-probe2 DP

<400>    118
gcaagagttt gccatgggga tatg                                              24

<210>    119
<211>    23
<212>    DNA
<213>    Artificial Sequence

<220>
<223>    EGFR Exon 20-probe3 CP3

<400>    119
gaagcctacg tgatggccag cgt                                               23

<210>    120
<211>    24
<212>    DNA
<213>    Artificial Sequence

<220>
<223>    EGFR Exon 20-probe3 DP

<400>    120
gcaagagttt gccatgggga tatg                                              24

<210>    121
<211>    21
<212>    DNA
<213>    Artificial Sequence

<220>
<223>    EGFR Exon 21-probe1 CP1

<400>    121
ccaggaacgt actggtgaaa a                                                 21

<210>    122
<211>    22
<212>    DNA
<213>    Artificial Sequence

<220>
<223>    EGFR Exon 21-probe1 DP

<400>    122
ggaagagaaa gaataccatg ca                                                22

<210>    123
<211>    22
<212>    DNA
<213>    Artificial Sequence

<220>
<223>    EGFR Exon 21-probe2 CP2

<400>    123
aagatcacag attttgggcg gg                                              22

<210>    124
<211>    22
<212>    DNA
<213>    Artificial Sequence

<220>
<223>    EGFR Exon 21-probe2 DP

<400>    124
ggaagagaaa gaataccatg ca                                              22

<210>    125
<211>    24
<212>    DNA
<213>    Artificial Sequence

<220>
<223>    EGFR Exon 21-probe3 CP3

<400>    125
ggcatgaact acttggagga ccgt                                            24

<210>    126
<211>    22
<212>    DNA
<213>    Artificial Sequence

<220>
<223>    EGFR Exon 21-probe3 DP

<400>    126
ggaagagaaa gaataccatg ca                                              22

<210>    127
<211>    21
<212>    DNA
<213>    Artificial Sequence

<220>
<223>    KRAS exon2-probe CP1

<400>    127
aaatgactga atataaactt g                                               21

<210>    128
<211>    21

<212>    DNA
<213>    Artificial Sequence

<220>
<223>    KRAS exon2-probe DP


<400>    128
gagtgccttg acgatacagc t                                                21


<210>    129
<211>    20
<212>    DNA
<213>    Artificial Sequence

<220>
<223>    ALK-EML4 variant 1-probe CP2


<400>    129
tagagcccac acctgggaaa                                                  20


<210>    130
<211>    20
<212>    DNA
<213>    Artificial Sequence

<220>
<223>    ALK-EML4 variant 1-probe DP


<400>    130
cggagcttgc tcagcttgta                                                  20


<210>    131
<211>    26
<212>    DNA
<213>    Artificial Sequence

<220>
<223>    ALK-EML4 variant 3-probe CP3


<400>    131
gcataaagat gtcatcatca accaag                                           26


<210>    132
<211>    20
<212>    DNA
<213>    Artificial Sequence

<220>
<223>    ALK-EML4 variant 3-probe DP


<400>    132
cggagcttgc tcagcttgta                                                  20

<210>    133
<211>    32
<212>    DNA
<213>    Artificial Sequence

<220>
<223>    BRCA1 Exon 7 of normal cell


<400>    133
caaagtatgg gctacagaaa ccgtgccaaa ag                                    32


<210>    134
<211>    32
<212>    DNA
<213>    Artificial Sequence

<220>
<223>    BRCA1 Exon 7 of cancer cell


<400>    134
caaagtatgg gcttcagaaa ccgtgccaaa ag                                    32


<210>    135
<211>    32
<212>    DNA
<213>    Artificial Sequence

<220>
<223>    BRCA1 Exon 10 of normal cell


<400>    135
tgggaaaacc tatcggaaga aggcaagcct cc                                    32


<210>    136
<211>    32
<212>    DNA
<213>    Artificial Sequence

<220>
<223>    BRCA1 Exon 10 of cancerl cell


<400>    136
tgggaaaacc tatcggtaga aggcaagcct cc                                    32


<210>    137
<211>    30
<212>    DNA
<213>    Artificial Sequence

<220>
<223>    BRCA1 Exon 11 of normal cell


<400>    137
ggggccaaga aattagagtc ctcagaagag                                       30

<210>    138
<211>    31
<212>    DNA
<213>    Artificial Sequence

<220>
<223>    BRCA1 Exon 11 of cancer cell


<400>    138
ggggccaaga aaattagagt cctcagaaga g                                    31


<210>    139
<211>    33
<212>    DNA
<213>    Artificial Sequence

<220>
<223>    BRCA1 Exon 15 of normal cell


<400>    139
atatacagga tatgcgaatt aagaagaaac aaa                                  33


<210>    140
<211>    33
<212>    DNA
<213>    Artificial Sequence

<220>
<223>    BRCA1 Exon 15 of cancer cell


<400>    140
atatacagga tatgtgaatt aagaagaaac aaa                                  33


<210>    141
<211>    32
<212>    DNA
<213>    Artificial Sequence

<220>
<223>    TP53 of normal cell


<400>    141
taggaggccg agctctgttg cttcgaactc ca                                   32


<210>    142
<211>    31
<212>    DNA
<213>    Artificial Sequence

<220>
<223>    TP53 of cancer cell

<400>    142
taggaggccg agctctttgc ttcgaactcc a                                    31


<210>    143
<211>    31
<212>    DNA
<213>    Artificial Sequence

<220>
<223>    MSH2 of normal cell


<400>    143
tgaggaggtt tcgacatggc ggtgcagccg a                                    31


<210>    144
<211>    31
<212>    DNA
<213>    Artificial Sequence

<220>
<223>    MSH2 of cancer cell


<400>    144
tgaggaggtt tcgacctggc ggtgcagccg a                                    31


<210>    145
<211>    32
<212>    DNA
<213>    Artificial Sequence

<220>
<223>    EGFR of normal cell


<400>    145
aaaaagatca aagtgctggg ctccggtgcg tt                                   32


<210>    146
<211>    32
<212>    DNA
<213>    Artificial Sequence

<220>
<223>    EGFR of cancer cell


<400>    146
aaaaagatca aagtgctgag ctccggtgcg tt                                   32


<210>    147
<211>    33
<212>    DNA
<213>    Artificial Sequence

<220>
<223>    FGFR3 of normal cell

<400>    147
atcctctctc tgaaatcact gagcaggaga aag                                    33


<210>    148
<211>    33
<212>    PRT
<213>    Artificial Sequence

<220>
<223>    EGFR Exon 19 Deletion amino acid sequence


<400>    148
Gly Leu Trp Ile Pro Glu Gly Glu Lys Val Lys Ile Pro Val Ala Ile
1               5                   10                  15

Lys Glu Leu Arg Glu Ala Thr Ser Pro Lys Ala Asn Lys Glu Ile Leu
            20                  25                  30

Asp




<210>    149
<211>    120
<212>    DNA
<213>    Artificial Sequence

<220>
<223>    Exon 19 deletion


<400>    149
gcagcatgtg gcaccatctc acaattgcca gttaacgtct tccttctctc tctgtcatag       60

gtgagtttct gctttgctgt gtgggggtcc atggctctga acctcaggcc caccttttct      120

                                                                       120


<210>    150
<211>    24
<212>    DNA
<213>    Artificial Sequence

<220>
<223>    CP_!


<400>    150
ggaattaaga gaagcaacat ctcc                                              24


<210>    151
<211>    19
<212>    DNA
<213>    Artificial Sequence

<220>
<223>    DP_1

<400>    151
aaaaggtggg cctgaggtt                                                    19


<210>    152
<211>    21
<212>    DNA
<213>    Artificial Sequence

<220>
<223>    CP_2


<400>    152
aaaattcccg tcgctatcaa g                                                 21


<210>    153
<211>    24
<212>    DNA
<213>    Artificial Sequence

<220>
<223>    CP_3


<400>    153
ggactctgga tcccagaagg tgag                                              24


<210>    154
<211>    238
<212>    DNA
<213>    Artificial Sequence

<220>
<223>    Exon 19 deletion


<400>    154
agggactctg gatcccagaa ggtgagaaag ttaaaattcc cgtcgctatc aaggaattaa      60

gagaagcaac atctccgaaa gccaacaagg aaatcctcga tgtgagtttc tgctttgctg     120

tgtgggggtc catggctctg aacctcaggc ccaccttttc tcatgtctgg cagctgctct     180

gctctagacc ctgctcatct ccacatccta aatgttcact ttctatgtct ttcccttt       238


<210>    155
<211>    658
<212>    DNA
<213>    Artificial Sequence

<220>
<223>    EGFR Exon 20 T790M


<400>    155
ccagatgcac ccaggagggg ccctctccca ctgcatctgt cacttcacag ccctgcgtaa      60

acgtccctgt gctaggtctt ttgcaggcac agcttttcct ccatgagtac gtattttgaa 120

actcaagatc gcattcatgc gtcttcacct ggaaggggtc catgtgcccc tccttctggc 180

caccatgcga agccacactg acgtgcctct ccctccctcc aggaagccta cgtgatggcc 240

agcgtggaca acccccacgt gtgccgcctg ctgggcatct gcctcacctc caccgtgcag 300

ctcatcacgc agctcatgcc cttcggctgc ctcctggact atgtccggga acacaaagac 360

aatattggct cccagtacct gctcaactgg tgtgtgcaga tcgcaaaggt aatcagggaa 420

gggagatacg gggaggggag ataaggagcc aggatcctca catgcggtct gcgctcctgg 480

gatagcaaga gtttgccatg gggatatgtg tgtgcgtgca tgcagcacac acacattcct 540

ttattttgga ttcaatcaag ttgatcttct tgtgcacaaa tcagtgcctg tcccatctgc 600

atgtggaaac tctcatcaat cagctacctt tgaagaattt tctctttatt gagtgctc 658

<210> 156
<211> 24
<212> DNA
<213> Artificial Sequence

<220>
<223> CP1

<400> 156
ccatgagtac gtattttgaa actc 24

<210> 157
<211> 24
<212> DNA
<213> Artificial Sequence

<220>
<223> DP1

<400> 157
catatcccca tggcaaactc ttgc 24

<210> 158
<211> 22
<212> DNA
<213> Artificial Sequence

<220>
<223> CP2

<400> 158
ccaccgtgca gctcatcatg ca 22

<210> 159
<211> 23
<212> DNA

<213>    Artificial Sequence

<220>
<223>    CP3


<400>    159
gaagcctacg tgatggccag cgt                                              23


<210>    160
<211>    36
<212>    DNA
<213>    Artificial Sequence

<220>
<223>    EGFR Exon 21 L858R


<400>    160
gtcaagatca cagattttgg gctggccaaa ctgctg                                36


<210>    161
<211>    156
<212>    DNA
<213>    Artificial Sequence

<220>
<223>    EGFR Exon 21 L858R


<400>    161
ggcatgaact acttggagga ccgtcgcttg gtgcaccgcg acctggcagc caggaacgta      60

ctggtgaaaa caccgcagca tgtcaagatc acagattttg ggctggccaa actgctgggt     120

gcggaagaga aagaatacca tgcagaagga ggcaaa                               156


<210>    162
<211>    21
<212>    DNA
<213>    Artificial Sequence

<220>
<223>    CP1


<400>    162
ccaggagcgt actggtgaaa a                                                21


<210>    163
<211>    22
<212>    DNA
<213>    Artificial Sequence

<220>
<223>    DP


<400>    163

ggaagagaaa gaataccatg ca                                                22

<210>    164
<211>    22
<212>    DNA
<213>    Artificial Sequence

<220>
<223>    CP2

<400>    164
aagatcacag attttgggcg gg                                                22

<210>    165
<211>    24
<212>    DNA
<213>    Artificial Sequence

<220>
<223>    CP3

<400>    165
ggcatgaact acttggagga ccgt                                              24

<210>    166
<211>    33
<212>    DNA
<213>    Artificial Sequence

<220>
<223>    FGFR3 of cancer cell

<400>    166
atcctctctc tgaaatcact gcgcaggaga aag                                    33